(19) Europäisches Patentamt
European Patent Office
Office européen des brevets

(11) EP 2 643 479 B1

(12) **EUROPEAN PATENT SPECIFICATION**

(45) Date of publication and mention of the grant of the patent:
**13.09.2017 Bulletin 2017/37**

(21) Application number: **11842532.1**

(22) Date of filing: **01.11.2011**

(51) Int Cl.:
***C12Q 1/68*** *(2006.01)*

(86) International application number:
**PCT/IL2011/000849**

(87) International publication number:
**WO 2012/070037 (31.05.2012 Gazette 2012/22)**

(54) **METHODS AND MATERIALS FOR CLASSIFICATION OF TISSUE OF ORIGIN OF TUMOR SAMPLES**

VERFAHREN UND MATERIALIEN ZUR KLASSIFIZIERUNG DES URSPRUNGSGEWEBES VON TUMORPROBEN

PROCÉDÉS ET MATÉRIAUX POUR LA CLASSIFICATION DE TISSUS ORIGINAIRES D'ÉCHANTILLONS TUMORAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.11.2010 US 415875 P**

(43) Date of publication of application:
**02.10.2013 Bulletin 2013/40**

(73) Proprietor: **Rosetta Genomics Ltd**
**76706 Rehovot (IL)**

(72) Inventors:
- **AHRONOV, Ranit**
  **69395 Tel Aviv (IL)**
- **ROSENWALD, Shai**
  **74208 Nes-Ziona (IL)**
- **DROMI, Nir**
  **7624804 Rehovot (IL)**

(74) Representative: **Pearl Cohen Zedek Latzer Baratz UK LLP**
**16 Upper Woburn Place**
**London WC1H 0BS (GB)**

(56) References cited:
**WO-A1-2009/033140 WO-A2-2010/073248**
**US-A1- 2010 178 653 US-A1- 2010 286 044**

- **JUN LU ET AL: "MicroRNA expression profiles classify human cancers", NATURE, vol. 435, no. 7043, 9 June 2005 (2005-06-09), pages 834-838, XP55027393, ISSN: 0028-0836, DOI: 10.1038/nature03702**
- **PENG S ET AL: "Multi-class cancer classification through gene expression profiles: microRNA versus mRNA", JOURNAL OF GENETICS AND GENOMICS, ELSEVIER BV, NL, vol. 36, no. 7, 1 July 2009 (2009-07-01), pages 409-416, XP026352175, ISSN: 1673-8527 [retrieved on 2009-07-01]**
- **M. RAPONI ET AL: "MicroRNA Classifiers for Predicting Prognosis of Squamous Cell Lung Cancer", CANCER RESEARCH, vol. 69, no. 14, 15 July 2009 (2009-07-15) , pages 5776-5783, XP55040886, ISSN: 0008-5472, DOI: 10.1158/0008-5472.CAN-09-0587**
- **"Human Genome U95Av2", INTERNET CITATION, 2 October 2002 (2002-10-02), XP002215481, Retrieved from the Internet: URL:http://www.affymetrix.com [retrieved on 2002-10-02]**
- **THOMSON J MICHAEL ET AL: "A custom microarray platform for analysis of microRNA gene expression", HHS PUBLIC ACCESS AUTHOR MANUSCRIPT, NATURE PUBLISHING GROUP, GB, vol. 1, no. 1, 1 October 2004 (2004-10-01) , pages 47-53, XP002532712, ISSN: 1548-7091, DOI: 10.1038/NMETH704**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 643 479 B1

SEQUENCE LISTING

<110> ROSETTA GENOMICS LTD

<120> METHODS AND MATERIALS FOR CLASSIFICATION OF TISSUE OF ORIGIN OF TUMOR SAMPLES Ranit Aharonov, Shai Rosenwald, Nir Dromi

<130> Pct_175

<160> 390

<170> PatentIn version 3.3

<210> 1
<211> 22
<212> RNA
<213> Homo sapiens

<400> 1
ugagguagua gguuguaugg uu    22

<210> 2
<211> 22
<212> RNA
<213> Homo sapiens

<400> 2
ugagguagga gguuguauag uu    22

<210> 3
<211> 22
<212> RNA
<213> Homo sapiens

<400> 3
aacccguaga uccgaacuug ug 22

<210> 4
<211> 23
<212> RNA
<213> Homo sapiens

<400> 4
uacccuguag auccgaauuu gug 23

<210> 5
<211> 23
<212> RNA
<213> Homo sapiens

<400> 5
uacccuguag aaccgaauuu gug 23

<210> 6
<211> 22
<212> RNA
<213> Homo sapiens

<400> 6
uggaguguga caaugguguu ug 22

<210> 7
<211> 24
<212> RNA
<213> Homo sapiens

<400> 7
ucccugagac ccuuuaaccu guga 24

<210> 8
<211> 22
<212> RNA
<213> Homo sapiens

<400> 8
ucccugagac ccuaacuugu ga 22

<210> 9
<211> 22
<212> RNA
<213> Homo sapiens

<400> 9
ucguaccgug aguaauaaug cg 22

<210> 10
<211> 22
<212> RNA
<213> Homo sapiens

<400> 10
cagugcaaug uuaaaagggc au 22

<210> 11
<211> 23
<212> RNA
<213> Homo sapiens

<400> 11
agcugguguu gugaaucagg ccg 23

<210> 12
<211> 21
<212> RNA
<213> Homo sapiens

<400> 12
uaccacaggg uagaaccacg g 21

<210> 13
<211> 22
<212> RNA
<213> Homo sapiens

<400> 13
uaacacuguc ugguaaagau gg 22

<210> 14
<211> 21
<212> RNA
<213> Homo sapiens

<400> 14
ugagaugaag cacuguagcu c 21

<210> 15
<211> 23
<212> RNA
<213> Homo sapiens

<400> 15
guccaguuuu cccaggaauc ccu 23

<210> 16
<211> 22
<212> RNA
<213> Homo sapiens

<400> 16
ugagaacuga auuccauggg uu 22

<210> 17
<211> 22
<212> RNA
<213> Homo sapiens

<400> 17
ugagaacuga auuccauagg cu 22

<210> 18
<211> 22

<212> > RNA
<213> Homo sapiens

<400> 18
ucagugcacu acagaacuuu gu 22

<210> 19
<211> 23
<212> > RNA
<213> Homo sapiens

<400> 19
ucuggcuccg ugucuucacu ccc 23

<210> 20
<211> 23
<212> RNA
<213> Homo sapiens

<400> 20
caaagugcuu acagugcagg uag 23

<210> 21
<211> 23
<212> RNA
<213> Homo sapiens

<400> 21
aacauucaac gcugucggug agu 23

<210> 22
<211> 22
<212> RNA
<213> Homo sapiens

<400> 22
accaucgacc guugauugua cc 22

<210> 23
<211> 22
<212> RNA
<213> Homo sapiens

<400> 23
uggagagaaa ggcaguuccu ga 22

<210> 24
<211> 23
<212> RNA
<213> Homo sapiens

<400> 24
caacggaauc ccaaaagcag cug 23

<210> 25
<211> 22
<212> RNA
<213> Homo sapiens

<400> 25
aacuggccua caaaguccca gu 22

<210> 26
<211> 22
<212> RNA
<213> Homo sapiens

<400> 26
ugggucuuug cgggcgagau ga 22

<210> 27
<211> 22
<212> RNA
<213> Homo sapiens

<400> 27
uguaacagca acuccaugug ga 22

<210> 28
<211> 22
<212> RNA
<213> Homo sapiens

<400> 28
uaacacuguc ugguaacgau gu 22

<210> 29
<211> 22
<212> RNA
<213> Homo sapiens

<400> 29
uaauacugcc ugguaaugau ga 22

<210> 30
<211> 23
<212> RNA
<213> Homo sapiens

<400> 30
uaauacugcc ggguaaugau gga 23

<210> 31
<211> 20
<212> RNA
<213> Homo sapiens

<400> 31
agagguauag ggcaugggaa 20

<210> 32
<211> 22
<212> RNA
<213> Homo sapiens

<400> 32
uccuucauuc caccggaguc ug 22

<210> 33
<211> 22
<212> RNA
<213> Homo sapiens

<400> 33
uggaauguaa ggaagugugu gg 22

<210> 34
<211> 22
<212> RNA
<213> Homo sapiens

<400> 34
uagcuuauca gacugauguu ga 22

<210> 35
<211> 21
<212> RNA
<213> Homo sapiens

<400> 35
caacaccagu cgaugggcug u 21

<210> 36
<211> 22
<212> RNA
<213> Homo sapiens

<400> 36
cugugcgugu gacagcggcu ga 22

<210> 37
<211> 22
<212> RNA
<213> Homo sapiens

<400> 37
acagcaggca cagacaggca gu 22

<210> 38
<211> 22
<212> RNA
<213> Homo sapiens

<400> 38
ugccugucua cacuugcugu gc 22

<210> 39
<211> 22
<212> RNA
<213> Homo sapiens

<400> 39
aagcugccag uugaagaacu gu 22

<210> 40
<211> 21

<212> RNA
<213> Homo sapiens

<400> 40
agcuacaucu ggcuacuggg u 21

<210> 41
<211> 22
<212> RNA
<213> Homo sapiens

<400> 41
ugucaguuug ucaaauaccc ca 22

<210> 42
<211> 21
<212> RNA
<213> Homo sapiens

<400> 42
caagucacua gugguuccgu u 21

<210> 43
<211> 22
<212> RNA
<213> Homo sapiens

<400> 43
uagcaccauc ugaaaucggu ua 22

<210> 44
<211> 22
<212> RNA
<213> Homo sapiens

<400> 44
uagcaccauu ugaaaucggu ua 22

<210> 45
<211> 22
<212> RNA
<213> Homo sapiens

<400> 45
ugaccgauuu cuccuggugu uc 22

<210> 46
<211> 22
<212> RNA
<213> Homo sapiens

<400> 46
uguaaacauc cucgacugga ag 22

<210> 47
<211> 22
<212> RNA
<213> Homo sapiens

<400> 47
uguaaacauc cccgacugga ag 22

<210> 48
<211> 22
<212> RNA
<213> Homo sapiens

<400> 48
uguaaacauc cuugacugga ag 22

<210> 49
<211> 21
<212> RNA
<213> Homo sapiens

<400> 49
aggcaagaug cuggcauagc u 21

<210> 50
<211> 23
<212> RNA
<213> Homo sapiens

<400> 50
ucucacacag aaaucgcacc cgu 23

<210> 51
<211> 22
<212> RNA
<213> Homo sapiens

<400> 51
gcugacuccu aguccagggc uc 22

<210> 52
<211> 22
<212> RNA
<213> Homo sapiens

<400> 52
uggcagugu c uuagcugguu gu 22

<210> 53
<211> 23
<212> RNA
<213> Homo sapiens

<400> 53
aggcagugua guuagcugau ugc 23

<210> 54
<211> 22
<212> RNA
<213> Homo sapiens

<400> 54
uuaucagaau cuccaggggu ac 22

<210> 55
<211> 23
<212> RNA
<213> Homo sapiens

<400> 55
aaagugcugc gacauuugag cgu 23

<210> 56
<211> 22
<212> RNA
<213> Homo sapiens

<400> 56
uuuguucguu cggcucgcgu ga 22

<210> 57
<211> 21
<212> RNA
<213> Homo sapiens

<400> 57
acuggacuug gagucagaag g 21

<210> 58
<211> 22
<212> RNA
<213> Homo sapiens

<400> 58
uaugugccuu uggacuacau cg 22

<210> 59
<211> 22
<212> RNA
<213> Homo sapiens

<400> 59
aaucguacag ggucauccac uu 22

<210> 60
<211> 21
<212> RNA
<213> Homo sapiens

<400> 60
cagcagcaca cugugguuug u 21

<210> 61
<211> 22
<212> RNA
<213> Homo sapiens

<400> 61
ugauugguac gucugugggu ag 22

<210> 62
<211> 23

<212> RNA
<213> Homo sapiens

<400> 62
uucucgagga aagaagcacu uuc 23

<210> 63
<211> 23
<212> RNA
<213> Homo sapiens

<400> 63
ugagugugug ugugugagug ugu 23

<210> 64
<211> 21
<212> RNA
<213> Homo sapiens

<400> 64
aauggcgcca cuaggguugu g 21

<210> 65
<211> 23
<212> RNA
<213> Homo sapiens

<400> 65
uggaagacua gugauuuugu ugu 23

<210> 66
<211> 22
<212> RNA
<213> Homo sapiens

<400> 66
auaaagcuag auaaccgaaa gu 22

<210> 67
<211> 22
<212> RNA
<213> Homo sapiens

<400> 67
uauugcacuu gucccggccu gu 22

<210> 68
<211> 22
<212> RNA
<213> Homo sapiens

<400> 68
uauugcacuc gucccggccu cc 22

<210> 69
<211> 22
<212> RNA
<213> Homo sapiens

<400> 69
ugucuacuac uggagacacu gg 22

<210> 70
<211> 84
<212> RNA
<213> Homo sapiens

<400> 70

gcauccgggu ugagguagua gguuguaugg uuuagaguua cacccuggga guuaacugua 60

caaccuucua gcuuuccuug gagc 84

<210> 71
<211> 79
<212> RNA
<213> Homo sapiens

<400> 71

cccgggcuga gguaggaggu uguauaguug aggaggacac ccaaggagau cacuauacgg 60

ccuccuagcu uuccccagg 79

<210> 72
<211> 80
<212> RNA
<213> Homo sapiens

<400> 72

ccuguugcca caaacccgua gauccgaacu ugugguauua guccgcacaa gcuuguaucu 60

auagguaugu gucuguuagg 80

<210> 73
<211> 110
<212> RNA
<213> Homo sapiens

<400> 73

gaucugucug ucuucuguau auacccugua gauccgaauu uguguaagga auuuuguggu 60

cacaaauucg uaucuagggg aauauguagu ugacauaaac acuccgcucu 110

<210> 74
<211> 110
<212> RNA
<213> Homo sapiens

<400> 74

ccagagguug uaacguuguc uauauauacc cuguagaacc gaauuugugu gguauccgua 60

uagucacaga uucgauucua ggggaauaua uggucgaugc aaaaacuuca 110

<210> 75
<211> 85
<212> RNA
<213> Homo sapiens

<400> 75

ccuuagcaga gcuguggagu gugacaaugg uguuugugucc uaaacuauca aacgccauua 60

ucacacuaaa uagcuacugc uaggc 85

<210> 76
<211> 86
<212> RNA
<213> Homo sapiens

<400> 76

ugccagucuc uagguccug agacccuuua accugugagg acauccaggg ucacagguga 60

gguucuuggg agccuggcgu cuggcc 86

<210> 77
<211> 88
<212> RNA
<213> Homo sapiens

<400> 77

ugcgcuccuc ucagucccug agacccuaac uugugauguu uaccguuuaa auccacgggu 60

uaggcucuug ggagcugcga gucgugcu 88

<210> 78
<211> 89
<212> RNA
<213> Homo sapiens

<400> 78

accagacuuu uccuaguccc ugagacccua acuugugagg uauuuuagua acaucacaag 60

ucaggcucuu gggaccuagg cggaggga 89

<210> 79
<211> 85
<212> RNA
<213> Homo sapiens

<400> 79

cgcuggcgac gggacauuau uacuuuuggu acgcgcugug acacuucaaa cucguaccgu 60

gaguaauaau gcgccgucca cggca 85

<210> 80
<211> 89
<212> RNA

<213> Homo sapiens

<400> 80

ugcugcuggc cagagcucuu uucacauugu gcuacugucu gcaccuguca cuagcagugc 60

aauguuaaaa gggcauuggc cguguagug 89

<210> 81
<211> 99
<212> RNA
<213> Homo sapiens

<400> 81

cccuggcaug guguggugg gcagcuggug uugugaauca ggccguugcc aaucagagaa 60

cggcuacuuc acaacaccag ggccacacca cacuacagg 99

<210> 82
<211> 84
<212> RNA
<213> Homo sapiens

<400> 82

cguugcugca gcugguguug ugaaucaggc cgacgagcag cgcauccucu uacccggcua 60

uuucacgaca ccaggguugc auca 84

<210> 83
<211> 100
<212> RNA
<213> Homo sapiens

<400> 83

ugugucucuc ucuguguccu gccagugguu uuacccuaug guagguuacg ucaugcuguu 60

cuaccacagg guagaaccac ggacaggaua ccggggcacc 100

<210> 84
<211> 95
<212> RNA
<213> Homo sapiens

<400> 84

cggccggccc ugggguccauc uuccaguaca guguuggaug gucuaauugu gaagcuccua 60

acacugucug guaaagaugg cucccgggug gguuc 95

<210> 85
<211> 106
<212> RNA
<213> Homo sapiens

<400> 85

gcgcagcgcc cugucuccca gccugaggug cagugcugca ucucugguca guugggaguc 60

ugagaugaag cacuguagcu caggaagaga gaaguuguuc ugcagc 106

<210> 86
<211> 88
<212> RNA
<213> Homo sapiens

<400> 86

caccuugucc ucacggucca guuuucccag gaaucccuua gaugcuaaga uggggauucc 60

uggaaauacu guucuugagg ucaugguu 88

<210> 87
<211> 99
<212> RNA
<213> Homo sapiens

<400> 87

ccgaugugua uccucagcuu ugagaacuga auuccauggg uugugucagu gucagaccuc 60

ugaaauucag uucuucagcu gggauaucuc ugucaucgu 99

<210> 88
<211> 73
<212> RNA
<213> Homo sapiens

<400> 88

ccuggcacug agaacugaau uccauaggcu gugagcucua gcaaugcccu guggacucag 60

uucuggugcc cgg 73

<210> 89
<211> 68
<212> RNA
<213> Homo sapiens

<400> 89

gaggcaaagu ucugagacac uccgacucug aguaugauag aagucagugc acuacagaac 60

uuugucuc 68

<210> 90
<211> 89
<212> RNA
<213> Homo sapiens

<400> 90

gccggcgccc gagcucuggc uccgugucuu cacucccgug cuuguccgag gagggaggga 60

gggacggggg cugugcuggg gcagcugga 89

<210> 91
<211> 84
<212> RNA
<213> Homo sapiens

<400> 91

gucagaauaa ugucaaagug cuuacagugc agguagugau augugcaucu acugcaguga 60

aggcacuugu agcauuaugg ugac 84

<210> 92
<211> 110
<212> RNA
<213> Homo sapiens

<400> 92

ugaguuuuga gguugcuuca gugaacauuc aacgcugucg gugaguuugg aauuaaaauc 60

aaaaccaucg accguugauu guacccuaug gcuaaccauc aucuacucca 110

<210> 93
<211> 110
<212> RNA
<213> Homo sapiens

<400> 93

agaagggcua ucaggccagc cuucagagga cuccaaggaa cauucaacgc ugucggugag 60

uuugggauuu gaaaaaacca cugaccguug acuguaccuu gggguccuua 110

<210> 94
<211> 82
<212> RNA
<213> Homo sapiens

<400> 94

agggggcgag ggauuggaga gaaaggcagu uccugauggu ccccucccca ggggcuggcu 60

uuccucuggu ccuucccucc ca 82

<210> 95
<211> 92
<212> RNA
<213> Homo sapiens

<400> 95

cggcuggaca gcgggcaacg gaaucccaaa agcagcuguu gucuccagag cauuccagcu 60

gcgcuuggau uucgucccccu gcucuccugc cu 92

<210> 96
<211> 88
<212> RNA

<213> Homo sapiens

<400> 96

cgaggauggg agcugagggc ugggucuuug cgggcgagau gagggugucg gaucaacugg 60

ccuacaaagu cccaguucuc ggcccccg 88

<210> 97
<211> 85
<212> RNA
<213> Homo sapiens

<400> 97

augguguuau caaguguaac agcaacucca uguggacugu guaccaauuu ccaguggaga 60

ugcuguuacu uuugaugguu accaa 85

<210> 98
<211> 85
<212> RNA
<213> Homo sapiens

<400> 98

ugguucccgc cccuguaac agcaacucca uguggaagug cccacugguu ccaguggggc 60

ugcuguuauc uggggcgagg gccag 85

<210> 99
<211> 90
<212> RNA
<213> Homo sapiens

<400> 99

ccgggccccu gugagcaucu uaccggacag ugcuggauuu cccagcuuga cucuaacacu 60

gucugguaac gauguucaaa ggugacccgc 90

<210> 100
<211> 95
<212> RNA
<213> Homo sapiens

<400> 100

ccagcucggg cagccguggc caucuuacug ggcagcauug gauggaguca ggucucuaau 60

acugccuggu aaugaugacg gcggagcccu gcacg 95

<210> 101
<211> 68
<212> RNA
<213> Homo sapiens

<400> 101

```
cccucgucuu acccagcagu guuugggugc gguugggagu cucuaauacu gccggguaau      60
gauggagg                                                               68
```

<210> 102
<211> 110
<212> RNA
<213> Homo sapiens

<400> 102

```
cgccucagag ccgcccgccg uuccuuuuuc cuaugcauau acuucuuuga ggaucuggcc      60
uaaagaggua uagggcaugg gaaaacgggg cggucggguc cucccccagcg               110
```

<210> 103
<211> 110
<212> RNA
<213> Homo sapiens

<400> 103

```
aaagauccuc agacaaucca ugugcuucuc uuguccuuca uuccaccgga gucugucuca      60
uacccaacca gauuucagug gagugaaguu caggaggcau ggagcugaca                110
```

<210> 104
<211> 86
<212> RNA
<213> Homo sapiens

<400> 104

```
ugcuucccga ggccacaugc uucuuuauau ccccauaugg auuacuuugc uauggaaugu      60
aaggaagugu gugguuucgg caagug                                           86
```

<210> 105
<211> 72
<212> RNA
<213> Homo sapiens

<400> 105

```
ugucggguag cuuaucagac ugauguugac uguugaaucu cauggcaaca ccagucgaug      60
ggcugucuga ca                                                          72
```

<210> 106
<211> 110
<212> RNA
<213> Homo sapiens

<400> 106

acccggcagu gccuccaggc gcagggcagc cccugcccac cgcacacugc gcugccccag 60

acccacugug cgugugacag cggcugaucu gugccugggc agcgcgaccc 110

<210> 107
<211> 110
<212> RNA
<213> Homo sapiens

<400> 107

ggccuggcug gacagaguug ucaugugucu gccugucuac acuugcugug cagaacaucc 60

gcucaccugu acagcaggca cagacaggca gucacaugac aacccagccu 110

<210> 108
<211> 85
<212> RNA
<213> Homo sapiens

<400> 108

ggcugagccg caguaguucu ucaguggcaa gcuuuauguc cugacccagc uaaagcugcc 60

aguugaagaa cuguugcccu cugcc 85

<210> 109
<211> 110
<212> RNA
<213> Homo sapiens

<400> 109

gcugcuggaa gguguaggua cccucaaugg cucaguagcc aguguagauc cugucuuucg 60

uaaucagcag cuacaucugg cuacugggucu ucugauggca ucuucuagcu 110

<210> 110
<211> 110
<212> RNA
<213> Homo sapiens

<400> 110

ccuggccucc ugcagugcca cgcuccgugu auuugacaag cugaguugga cacuccaugu 60

gguagagugu caguuuguca aauaccccaa gugcggcaca ugcuuaccag 110

<210> 111
<211> 81
<212> RNA
<213> Homo sapiens

<400> 111

gggcuuucaa gucacuagug guuccguuua guagaugauu gugcauuguu ucaaaauggu 60

gcccuaguga cuacaaagcc c 81

<210> 112
<211> 64
<212> RNA
<213> Homo sapiens

<400> 112

augacugauu ucuuuuggug uucagaguca auauaauuuu cuagcaccau cugaaaucgg 60

uuau 64

<210> 113
<211> 88
<212> RNA
<213> Homo sapiens

<400> 113

aucucuuaca caggcugacc gauuucuccu gguguucaga gucuguuuuu gucuagcacc 60

auuugaaauc gguuaugaug uaggggga 88

<210> 114
<211> 71
<212> RNA
<213> Homo sapiens

<400> 114

gcgacuguaa acauccucga cuggaagcug ugaagccaca gaugggcuuu cagucggaug 60

uuugcagcug c 71

<210> 115
<211> 70
<212> RNA
<213> Homo sapiens

<400> 115

guuguuguaa acaucccccga cuggaagcug uaagacacag cuaagcuuuc agucagaugu 60

uugcugcuac 70

<210> 116
<211> 92
<212> RNA
<213> Homo sapiens

<400> 116

gggcagucuu ugcuacugua aacauccuug acuggaagcu guaagguguu cagaggagcu 60

uucagucgga uguuuacagc ggcaggcugc ca 92

<210> 117
<211> 71
<212> RNA

<213> Homo sapiens

<400> 117

ggagaggagg caagaugcug gcauagcugu ugaacuggga accugcuaug ccaacauauu 60

gccaucuuuc c 71

<210> 118
<211> 99
<212> RNA
<213> Homo sapiens

<400> 118

gaaacugggc ucaaggugag gggugcuauc ugugauugag ggacaugguu aauggaauug 60

ucucacacag aaaucgcacc cgucaccuug gccuacuua 99

<210> 119
<211> 98
<212> RNA
<213> Homo sapiens

<400> 119

acccaaaccc uaggucugcu gacuccuagu ccagggcucg ugauggcugg ugggcccuga 60

acgagggguc uggaggccug gguuugaaua ucgacagc 98

<210> 120
<211> 110
<212> RNA
<213> Homo sapiens

<400> 120

ggccagcugu gaguguuucu uuggcagugu cuuagcuggu uguugugagc aauaguaagg 60

aagcaaucag caaguauacu gcccuagaag ugcugcacgu uguggggccc 110

<210> 121
<211> 77
<212> RNA
<213> Homo sapiens

<400> 121

agucuaguua cuaggcagug uaguuagcug auugcuaaua guaccaauca cuaaccacac 60

ggccagguaa aaagauu 77

<210> 122
<211> 72
<212> RNA
<213> Homo sapiens

<400> 122

ggagcuuauc agaaucucca gggguacuuu auaauuucaa aaaguccccc aggugugauu 60

cugauuugcu uc 72

<210> 123
<211> 67
<212> RNA
<213> Homo sapiens

<400> 123

gugggccuca aauguggagc acuauucuga uguccaagug gaaagugcug cgacauuuga 60

gcgucac 67

<210> 124
<211> 64
<212> RNA
<213> Homo sapiens

<400> 124

ccccgcgacg agccccucgc acaaaccgga ccugagcguu uuguucguuc ggcucgcgug 60

aggc 64

<210> 125
<211> 66
<212> RNA
<213> Homo sapiens

<400> 125

agggcuccug acuccagguc cugugugua ccuagaaaua gcacuggacu uggagucaga 60

aggccu 66

<210> 126
<211> 96
<212> RNA
<213> Homo sapiens

<400> 126

ucccuggcgu gaggguaugu gccuuuggac uacaucgugg aagccagcac caugcagucc 60

augggcauau acacuugccu caaggccuau gucauc 96

<210> 127
<211> 84
<212> RNA
<213> Homo sapiens

<400> 127

uugguacuug gagagugguu aucccugucc uguucguuuu gcucaugucg aaucguacag 60

ggucauccac uuuuucagua ucaa 84

<210> 128
<211> 112
<212> RNA
<213> Homo sapiens

<400> 128

ccaccccggu ccugcucccg ccccagcagc acacuguggu uuguacggca cuguggccac 60

guccaaacca cacugugguq uuagagcgag ggugggggag gcaccgccga gg 112

<210> 129
<211> 94
<212> RNA
<213> Homo sapiens

<400> 129

caugcugugu gugguacccu acugcagaca guggcaauca uguauaauua aaaaugauug 60

guacgucugu ggguagagua cugcaugaca caug 94

<210> 130
<211> 91
<212> RNA
<213> Homo sapiens

<400> 130

caugcugugu gugguacccu acugcagaca guggcaauca uguauaauua aaaaugauug 60

guacgucugu ggguagagua cugcaugaca c 91

<210> 131
<211> 75
<212> RNA
<213> Homo sapiens

<400> 131

gugguacccu acugcagacg uggcaaucau guauaauuaa aaaugauugg uacgucugug 60

gguagaguac ugcau 75

<210> 132
<211> 90
<212> RNA
<213> Homo sapiens

<400> 132

ucucaggcug ugaccuucuc gaggaaagaa gcacuuucug uugucugaaa gaaaagaaag 60

ugcuuccuuu cagaggguua cgguuugaga 90

<210> 133
<211> 90
<212> RNA

<213> Homo sapiens

<400> 133

```
ucucagguug ugaccuucuc gaggaaagaa gcacuuucug uugucugaaa gaaaagaaag      60
ugcuuccuuu cagaggguua cgguuugaga                                       90
```

<210> 134
<211> 96
<212> RNA
<213> Homo sapiens

<400> 134

```
gggaccugcg ugggugcggg cgugugagug ugugugugug agugugugucc gcuccggguc      60
cacgcucaug cacacaccca cacgcccaca cucagg                                96
```

<210> 135
<211> 98
<212> RNA
<213> Homo sapiens

<400> 135

```
acgaauggcu augcacugca caacccuagg agagggugcc auucacauag acuauaauug      60
aauggcgcca cuaggguugu gcagugcaca accuacac                              98
```

<210> 136
<211> 110
<212> RNA
<213> Homo sapiens

<400> 136

```
uuggauguug gccuaguucu guguggaaga cuagugauuu uguuguuuuu agauaacuaa      60
aucgacaaca aaucacaguc ugccauaugg cacaggccau gccucuacag                110
```

<210> 137
<211> 110
<212> RNA
<213> Homo sapiens

<400> 137

```
cuggauacag aguggaccgg cuggccccau cuggaagacu agugauuuug uuguugucuu      60
acugcgcuca acaacaaauc ccagucuacc uaauggugcc agccaucgca                110
```

<210> 138
<211> 110
<212> RNA
<213> Homo sapiens

<400> 138

agauuagagu ggcugugguc uagugcugug uggaagacua gugauuuugu uguucugaug 60

uacuacgaca acaagucaca gccggccuca uagcgcagac ucccuucgac 110

<210> 139
<211> 89
<212> RNA
<213> Homo sapiens

<400> 139

cgggguuggu uguuaucuuu gguuaucuag cuguaugagu gguguggagu cuucauaaag 60

cuagauaacc gaaaguaaaa auaacccca 89

<210> 140
<211> 87
<212> RNA
<213> Homo sapiens

<400> 140

ggaagcgagu uguuaucuuu gguuaucuag cuguaugagu guauuggucu ucauaaagcu 60

agauaaccga aaguaaaaac uccuuca 87

<210> 141
<211> 90
<212> RNA
<213> Homo sapiens

<400> 141

ggaggcccgu uucucucuuu gguuaucuag cuguaugagu gccacagagc cgucauaaag 60

cuagauaacc gaaaguagaa augauucuca 90

<210> 142
<211> 78
<212> RNA
<213> Homo sapiens

<400> 142

cuuucuacac agguugggau cgguugcaau gcuguguuuc uguaugguau ugcacuuguc 60

ccggccuguu gaguuugg 78

<210> 143
<211> 75
<212> RNA
<213> Homo sapiens

<400> 143

ucaucccugg guggggauuu guugcauuac uuguguucua uauaaaguau ugcacuuguc 60

ccggccugug gaaga 75

<210> 144
<211> 96
<212> RNA
<213> Homo sapiens

<400> 144

cgggccccgg gcgggcggga gggacgggac gcggugcagu guuguuuuuu cccccgccaa 60

uauugcacuc gucccggccu ccggcccccc cggccc 96

<210> 145
<211> 83
<212> RNA
<213> Homo sapiens

<400> 145

agaaauaagg cuucugucua cuacuggaga cacugguagu auaaaaccca gagucuccag 60

uaauggacgg gagccuuauu ucu 83

<210> 146
<211> 24
<212> RNA
<213> Homo sapiens

<400> 146
agccugauua aacacaugcu cuga 24

<210> 147
<211> 23
<212> RNA
<213> Homo sapiens

<400> 147
agcuacauug ucugcugggu uuc 23

<210> 148
<211> 23
<212> RNA
<213> Homo sapiens

<400> 148
caaagugcug uucgugcagg uag 23

<210> 149
<211> 85
<212> RNA
<213> Homo sapiens

<400> 149

uuuacaguuu gccaugauga aaugcauguu aaguccgugu uucagcugau cagccugauu 60

aaacacaugc ucugagcaga cuaaa 85

<210> 150
<211> 110
<212> RNA
<213> Homo sapiens

<400> 150

ugaacaucca ggucuggggc augaaccugg cauacaaugu agauuucugu guucguuagg 60

caacagcuac auugucugcu ggguuucagg cuaccuggaa acauguucuc 110

<210> > 151
<211> 80
<212> RNA
<213> Homo sapiens

<400> 151

cugggggcuc caaagugcug uucgugcagg uagugugauu acccaaccua cugcugagcu 60

agcacuuccc gagcccccgg 80

<210> 152
<211> 24
<212> RNA
<213> Homo sapiens

<400> 152
uuuggcaaug guagaacuca cacu 24

<210> 153
<211> 22
<212> RNA
<213> Homo sapiens

<400> 153
agagguagua gguugcauag uu 22

<210> 154
<211> 23
<212> RNA
<213> Homo sapiens

<400> 154
aacauucauu gcugucggug ggu 23

<210> 155
<211> 22
<212> RNA
<213> Homo sapiens

<400> 155
ucggauccgu cugagcuugg cu 22

<210> 156
<211> 22
<212> RNA
<213> Homo sapiens

<400> 156
ugagguagga gguuguauag uu 22

<210> 157
<211> 22
<212> RNA
<213> Homo sapiens

<400> 157
ugagguagua guuugugcug uu 22

<210> 158
<211> 23
<212> RNA
<213> Homo sapiens

<400> 158
aaaagugcuu acagugcagg uag 23

<210> 159
<211> 20
<212> RNA
<213> Homo sapiens

<400> 159
uaaggcacgc ggugaaugcc 20

<210> 160
<211> 27
<212> RNA
<213> Homo sapiens

<400> 160
accuucuugu auaagcacug ugcuaaa 27

<210> 161
<211> 21
<212> RNA
<213> Homo sapiens

<400> 161
ucacagugaa ccggucucuu u 21

<210> 162
<211> 22
<212> RNA
<213> Homo sapiens

<400> 162
aagcccuuac cccaaaaagc au 22

<210> 163
<211> 22

<212> RNA
<213> Homo sapiens

<400> 163
ucaaaacuga ggggcauuuu cu 22

<210> 164
<211> 21
<212> RNA
<213> Homo sapiens

<400> 164
cauaaaguag aaagcacuac u 21

<210> 165
<211> 22
<212> RNA
<213> Homo sapiens

<400> 165
ggugcagugc ugcaucucug gu 22

<210> 166
<211> 22
<212> RNA
<213> Homo sapiens

<400> 166
ugcccugugg acucaguucu gg 22

<210> 167
<211> 21
<212> RNA
<213> Homo sapiens

<400> 167
agggagggac gggggcugug c 21

<210> 168
<211> 22
<212> RNA
<213> Homo sapiens

<400> 168
ucucccaacc cuuguaccag ug 22

<210> 169
<211> 21
<212> RNA
<213> Homo sapiens

<400> 169
ucagugcaug acagaacuug g 21

<210> 170
<211> 23
<212> RNA
<213> Homo sapiens

<400> 170
uuaaugcuaa ucgugauagg ggu 23

<210> 171
<211> 22
<212> RNA
<213> Homo sapiens

<400> 171
uagcagcaca uaaugguuug ug 22

<210> 172
<211> 22
<212> RNA
<213> Homo sapiens

<400> 172
uagcagcaca ucaugguuua ca 22

<210> 173
<211> 22
<212> RNA
<213> Homo sapiens

<400> 173
aacauucaac cugucgguga gu 22

<210> 174
<211> 23
<212> RNA
<213> Homo sapiens

<400> 174
aacauucauu guugucggug ggu 23

<210> 175
<211> 22
<212> RNA
<213> Homo sapiens

<400> 175
uauggcacug guagaauuca cu 22

<210> 176
<211> 23
<212> RNA
<213> Homo sapiens

<400> 176
uaaggugcau cuagugcaga uag 23

<210> 177
<211> 21
<212> RNA
<213> Homo sapiens

<400> 177
cugaccuaug aauugacagc c 21

<210> 178
<211> 22
<212> RNA
<213> Homo sapiens

<400> 178
aacuggcccu caaagucccg cu 22

<210> 179
<211> 21
<212> RNA
<213> Homo sapiens

<400> 179
uagcagcaca gaaauauugg c 21

<210> 180
<211> 19
<212> RNA
<213> Homo sapiens

<400> 180
accgugcaaa gguagcaua 19

<210> 181
<211> 22
<212> RNA
<213> Homo sapiens

<400> 181
acaguagucu gcacauuggu ua 22

<210> 182
<211> 23
<212> > RNA
<213> Homo sapiens

<400> 182
cccaguguuc agacuaccug uuc 23

<210> 183
<211> 23
<212> RNA
<213> Homo sapiens

<400> 183
cccaguguuu agacuaucug uuc 23

<210> 184
<211> 22
<212> RNA
<213> Homo sapiens

<400> 184
gugaaauguu uaggaccacu ag 22

<210> 185
<211> 21

<212> RNA
<213> Homo sapiens

<400> 185
gauuucagug gagugaaguu c 21

<210> 186
<211> 23
<212> RNA
<213> Homo sapiens

<400> 186
uaaagugcuu auagugcagg uag 23

<210> 187
<211> 22
<212> RNA
<213> Homo sapiens

<400> 187
agaauugugg cuggacaucu gu 22

<210> 188
<211> 22
<212> RNA
<213> Homo sapiens

<400> 188
cauugcacuu gucucggucu ga 22

<210> 189
<211> 21
<212> RNA
<213> Homo sapiens

<400> 189
uucacagugg cuaaguucug c 21

<210> 190
<211> 23
<212> RNA
<213> Homo sapiens

<400> 190
uagcaccauu ugaaaucagu guu 23

<210> 191
<211> 22
<212> RNA
<213> Homo sapiens

<400> 191
uagcaccauu ugaaaucggu ua 22

<210> 192
<211> 23
<212> RNA
<213> Homo sapiens

<400> 192
uaagugcuuc cauguuuugg uga 23

<210> 193
<211> 23
<212> RNA
<213> Homo sapiens

<400> 193
acuuaaacgu ggauguacuu gcu 23

<210> 194
<211> 23
<212> RNA
<213> Homo sapiens

<400> 194
uaagugcuuc cauguuugag ugu 23

<210> 195
<211> 22
<212> RNA
<213> Homo sapiens

<400> 195
cuuucagucg gauguuugca gc 22

<210> 196
<211> 23
<212> RNA
<213> Homo sapiens

<400> 196
uguaaacauc cuacacucuc agc 23

<210> 197
<211> 21
<212> RNA
<213> Homo sapiens

<400> 197
gccccugggc cuauccuaga a 21

<210> 198
<211> 21
<212> RNA
<213> Homo sapiens

<400> 198
aggggugcua ucugugauug a 21

<210> 199
<211> 22
<212> RNA
<213> Homo sapiens

<400> 199
aauugcacgg uauccaucug ua 22

<210> 200
<211> 23
<212> RNA
<213> Homo sapiens

<400> 200
aagugccgcc aucuuuugag ugu 23

<210> 201
<211> 20
<212> RNA
<213> Homo sapiens

<400> 201
acucaaacug ugggggcacu 20

<210> 202
<211> 21
<212> RNA
<213> Homo sapiens

<400> 202
acuggacuug gagucagaag g 21

<210> 203
<211> 22
<212> RNA
<213> Homo sapiens

<400> 203
acuggacuua gggucagaag gc 22

<210> 204
<211> 23
<212> RNA
<213> Homo sapiens

<400> 204
aaugacacga ucacucccgu uga 23

<210> 205
<211> 22
<212> RNA
<213> Homo sapiens

<400> 205
aaaccguuac cauuacugag uu 22

<210> 206
<211> 21
<212> RNA
<213> Homo sapiens

<400> 206
gcaguccaug ggcauauaca c 21

<210> 207
<211> 22

<212> RNA
<213> Homo sapiens

<400> 207
uccuguacug agcugccccg ag 22

<210> 208
<211> 21
<212> > RNA
<213> Homo sapiens

<400> 208
cagcagcaca cugugguuug u 21

<210> 209
<211> 23
<212> RNA
<213> Homo sapiens

<400> 209
uuucaagcca gggggcguuu uuc 23

<210> 210
<211> 23
<212> RNA
<213> Homo sapiens

<400> 210
cacucagccu ugagggcacu uuc 23

<210> 211
<211> 23
<212> RNA
<213> Homo sapiens

<400> 211
uucucgagga aagaagcacu uuc 23

<210> 212
<211> 22
<212> RNA
<213> Homo sapiens

<400> 212
aucuggaggu aagaagcacu uu 22

<210> 213
<211> 22
<212> RNA
<213> Homo sapiens

<400> 213
aucgugcauc ccuuuagagu gu 22

<210> 214
<211> 22
<212> RNA
<213> Homo sapiens

<400> 214
aucgugcauc cuuuuagagu gu 22

<210> 215
<211> 22
<212> RNA
<213> Homo sapiens

<400> 215
gaaagcgcuu cccuuugcug ga 22

<210> 216
<211> 21
<212> RNA
<213> Homo sapiens

<400> 216
aaagcgcuuc ccuucagagu g 21

<210> 217
<211> 22
<212> RNA
<213> Homo sapiens

<400> 217
cucuagaggg aagcacuuuc uc 22

<210> 218
<211> 22
<212> RNA
<213> Homo sapiens

<400> 218
aaagugcauc cuuuuagagu gu 22

<210> 219
<211> 22
<212> RNA
<213> Homo sapiens

<400> 219
caaagugccu cccuuuagag ug 22

<210> 220
<211> 21
<212> RNA
<213> Homo sapiens

<400> 220
cuccagaggg aaguacuuuc u 21

<210> 221
<211> 22
<212> RNA
<213> Homo sapiens

<400> 221
aaagugcuuc cuuuuagagg gu 22

<210> 222
<211> 20
<212> RNA
<213> Homo sapiens

<400> 222
cuacaaaggg aagcccuuuc 20

<210> 223
<211> 22
<212> RNA
<213> Homo sapiens

<400> 223
cuacaaaggg aagcacuuuc uc 22

<210> 224
<211> 20
<212> RNA
<213> Homo sapiens

<400> 224
cugcaaaggg aagcccuuuc 20

<210> 225
<211> 21
<212> RNA
<213> Homo sapiens

<400> 225
gcgacccaua cuugguuuca g 21

<210> 226
<211> 21
<212> RNA
<213> Homo sapiens

<400> 226
agggggaaag uucuauaguc c 21

<210> 227
<211> 23
<212> RNA
<213> Homo sapiens

<400> 227
ugcaccaugg uugucugagc aug 23

<210> 228
<211> 21
<212> RNA
<213> Homo sapiens

<400> 228
cgcgggugcu uacugacccu u 21

<210> 229
<211> 23

<212> RNA
<213> Homo sapiens

<400> 229
ucuuugguua ucuagcugua uga 23

<210> 230
<211> 23
<212> RNA
<213> Homo sapiens

<400> 230
cgggucggag uuagcucaag cgg 23

<210> 231
<211> 22
<212> RNA
<213> Homo sapiens

<400> 231
aacccguaga uccgaucuug ug 22

<210> 232
<211> 22
<212> RNA
<213> Homo sapiens

<400> 232
caagcucgcu ucuaugggguc ug 22

<210> 233
<211> 23
<212> RNA
<213> Homo sapiens

<400> 233
gaagugcuuc gauuuugggg ugu 23

<210> 234
<211> 22
<212> RNA
<213> Homo sapiens

<400> 234
gauuagggug cuuagcuguu aa 22

<210> 235
<211> 21
<212> RNA
<213> Homo sapiens

<400> 235
gguuuggucc uagccuuucu a 21

<210> 236
<211> 22
<212> RNA
<213> Homo sapiens

<400> 236
uggacuugga gucaggaggc cu 22

<210> 237
<211> 22
<212> RNA
<213> Homo sapiens

<400> 237
aaucugcagg gggagccugg gu 22

<210> 238
<211> 21
<212> RNA
<213> Homo sapiens

<400> 238
acaugaaaag gggagagggc a 21

<210> 239
<211> 22
<212> RNA
<213> Homo sapiens

<400> 239
acccccccca gccauacaua ga 22

<210> 240
<211> 25
<212> RNA
<213> Homo sapiens

<400> 240
acuaccccag gaugccagca uaguu 25

<210> 241
<211> 22
<212> RNA
<213> Homo sapiens

<400> 241
agcugguuug auggggagcc au 22

<210> 242
<211> 22
<212> RNA
<213> Homo sapiens

<400> 242
agggugacag ggaacaguag au 22

<210> 243
<211> 22
<212> RNA
<213> Homo sapiens

<400> 243
augugggugg uggucaccgu uu 22

<210> 244
<211> 22
<212> RNA
<213> Homo sapiens

<400> 244
cacugauuau cgaggcgauu cu 22

<210> 245
<211> 20
<212> RNA
<213> Homo sapiens

<400> 245
gaacccuacu ccugguacca 20

<210> 246
<211> 22
<212> RNA
<213> Homo sapiens

<400> 246
gaauuuccug aggggagggg gc 22

<210> 247
<211> 22
<212> RNA
<213> Homo sapiens

<400> 247
ggcaggacgg cguaggucuu ga 22

<210> 248
<211> 22
<212> RNA
<213> Homo sapiens

<400> 248
gggcugggca gguuucagga au 22

<210> 249
<211> 21
<212> RNA
<213> Homo sapiens

<400> 249
uaggucaagg uguagcccau a 21

<210> 250
<211> 22
<212> RNA
<213> Homo sapiens

<400> 250
uauguacaag guggaggggg cg 22

<210> 251
<211> 22

<212> RNA
<213> Homo sapiens

<400> 251
ucccccaccc uuagcuuaga ua 22

<210> 252
<211> 22
<212> RNA
<213> Homo sapiens

<400> 252
uggagcaggc uggggcuuug ag 22

<210> 253
<211> 23
<212> RNA
<213> Homo sapiens

<400> 253
ugugcuccgg aguuaccucg uuu 23

<210> 254
<211> 18
<212> RNA
<213> Homo sapiens

<400> 254
uguggguucg aguuccau 18

<210> 255
<211> 18
<212> RNA
<213> Homo sapiens

<400> 255
uucccggcca augcauua 18

<210> 256
<211> 22
<212> RNA
<213> Homo sapiens

<400> 256
ugagguagua gguuguauag uu 22

<210> 257
<211> 22
<212> RNA
<213> Homo sapiens

<400> 257
ugagguagua gguugugugg uu 22

<210> 258
<211> 22
<212> RNA
<213> Homo sapiens

<400> 258
ugagguagua gauuguauag uu 22

<210> 259
<211> 22
<212> RNA
<213> Homo sapiens

<400> 259
ugagguagua guuuguacag uu 22

<210> 260
<211> 21
<212> RNA
<213> Homo sapiens

<400> 260
uaaagugcug acagugcaga u 21

<210> 261
<211> 22
<212> RNA
<213> Homo sapiens

<400> 261
uuuccggcuc gcgugggugu gu 22

<210> 262
<211> 22
<212> RNA
<213> Homo sapiens

<400> 262
cugaagcuca gagggcucug au 22

<210> 263
<211> 22
<212> RNA
<213> Homo sapiens

<400> 263
aagcccuuac cccaaaaagu au 22

<210> 264
<211> 21
<212> RNA
<213> Homo sapiens

<400> 264
cuuuuugcgg ucugggcuug c 21

<210> 265
<211> 22
<212> RNA
<213> Homo sapiens

<400> 265
cagugcaaug augaaagggc au 22

<210> 266
<211> 22
<212> RNA
<213> Homo sapiens

<400> 266
uaacagucua cagccauggu cg 22

<210> 267
<211> 22
<212> RNA
<213> Homo sapiens

<400> 267
uuuggucccc uucaaccagc ug 22

<210> 268
<211> 22
<212> RNA
<213> Homo sapiens

<400> 268
uuuggucccc uucaaccagc ua 22

<210> 269
<211> 22
<212> RNA
<213> Homo sapiens

<400> 269
ugugacuggu ugaccagagg gg 22

<210> 270
<211> 22
<212> RNA
<213> Homo sapiens

<400> 270
ucuacagugc acgugucucc ag 22

<210> 271
<211> 22
<212> RNA
<213> Homo sapiens

<400> 271
cagugguuuu acccuauggu ag 22

<210> 272
<211> 22
<212> RNA
<213> Homo sapiens

<400> 272
ggauuccugg aaauacuguu cu 22

<210> 273
<211> 22

<212> RNA
<213> Homo sapiens

<400> 273
ucagugcauc acagaacuuu gu 22

<210> 274
<211> 21
<212> RNA
<213> Homo sapiens

<400> 274
cuagacugaa gcuccuugag g 21

<210> 275
<211> 22
<212> RNA
<213> Homo sapiens

<400> 275
uagguuaucc guguugccuu cg 22

<210> 276
<211> 22
<212> RNA
<213> Homo sapiens

<400> 276
aaucauacac gguugaccua uu 22

<210> 277
<211> 22
<212> RNA
<213> Homo sapiens

<400> 277
acugcaguga aggcacuugu ag 22

<210> 278
<211> 22
<212> RNA
<213> Homo sapiens

<400> 278
accacugacc guugacugua cc 22

<210> 279
<211> 27
<212> RNA
<213> Homo sapiens

<400> 279
auugaucauc gacacuucga acgcaau 27

<210> 280
<211> 22
<212> RNA
<213> Homo sapiens

<400> 280
ucgugucuug uguugcagcc gg 22

<210> 281
<211> 21
<212> RNA
<213> Homo sapiens

<400> 281
caucccuugc augguggagg g 21

<210> 282
<211> 22
<212> RNA
<213> Homo sapiens

<400> 282
uagguaguuu cauguuguug gg 22

<210> 283
<211> 22
<212> RNA
<213> Homo sapiens

<400> 283
cucccacugc uucacuugac ua 22

<210> 284
<211> 23
<212> RNA
<213> Homo sapiens

<400> 284
ugugcaaauc caugcaaaac uga 23

<210> 285
<211> 23
<212> RNA
<213> Homo sapiens

<400> 285
caaagugcuc auagugcagg uag 23

<210> 286
<211> 22
<212> RNA
<213> Homo sapiens

<400> 286
uaaucucagc uggcaacugu ga 22

<210> 287
<211> 22
<212> RNA
<213> Homo sapiens

<400> 287
aaaucucugc aggcaaaugu ga 22

<210> 288
<211> 23
<212> RNA
<213> Homo sapiens

<400> 288
uacugcauca ggaacugauu gga 23

<210> 289
<211> 22
<212> RNA
<213> Homo sapiens

<400> 289
aguucuucag uggcaagcuu ua 22

<210> 290
<211> 22
<212> RNA
<213> Homo sapiens

<400> 290
accuggcaua caauguagau uu 22

<210> 291
<211> 22
<212> RNA
<213> Homo sapiens

<400> 291
cucaguagcc aguguagauc cu 22

<210> 292
<211> 21
<212> RNA
<213> Homo sapiens

<400> 292
aucacauugc cagggauuuc c 21

<210> 293
<211> 21
<212> RNA
<213> Homo sapiens

<400> 293
aucacauugc cagggauuac c 21

<210> 294
<211> 22
<212> RNA
<213> Homo sapiens

<400> 294
uggcucaguu cagcaggaac ag 22

<210> 295
<211> 22

<212> RNA
<213> Homo sapiens

<400> 295
uucaaguaau ccaggauagg cu 22

<210> 296
<211> 21
<212> RNA
<213> Homo sapiens

<400> 296
uucaaguaau ucaggauagg u 21

<210> 297
<211> 21
<212> RNA
<213> Homo sapiens

<400> 297
uucacagugg cuaaguuccg c 21

<210> 298
<211> 22
<212> RNA
<213> Homo sapiens

<400> 298
cacuagauug ugagcuccug ga 22

<210> 299
<211> 21
<212> RNA
<213> Homo sapiens

<400> 299
agggcccccc cucaauccug u 21

<210> 300
<211> 22
<212> RNA
<213> Homo sapiens

<400> 300
uaugugggau gguaaaccgc uu 22

<210> 301
<211> 22
<212> RNA
<213> Homo sapiens

<400> 301
cugguuucac augguggcuu ag 22

<210> 302
<211> 23
<212> RNA
<213> Homo sapiens

<400> 302
cagugcaaua guauugucaa agc 23

<210> 303
<211> 22
<212> RNA
<213> Homo sapiens

<400> 303
uguaaacauc cuacacucag cu 22

<210> 304
<211> 22
<212> RNA
<213> Homo sapiens

<400> 304
cuuucagucg gauguuuaca gc 22

<210> 305
<211> 22
<212> RNA
<213> Homo sapiens

<400> 305
ugcuaugcca acauauugcc au 22

<210> 306
<211> 21
<212> RNA
<213> Homo sapiens

<400> 306
cacauuacac ggucgaccuc u 21

<210> 307
<211> 23
<212> RNA
<213> Homo sapiens

<400> 307
cgcauccccu agggcauugg ugu 23

<210> 308
<211> 22
<212> RNA
<213> Homo sapiens

<400> 308
cuggcccucu cugcccuucc gu 22

<210> 309
<211> 22
<212> RNA
<213> Homo sapiens

<400> 309
aacacaccug guuaaccucu uu 22

<210> 310
<211> 23
<212> RNA
<213> Homo sapiens

<400> 310
gcaaagcaca cggccugcag aga 23

<210> 311
<211> 23
<212> RNA
<213> Homo sapiens

<400> 311
ucaagagcaa uaacgaaaaa ugu 23

<210> 312
<211> 21
<212> RNA
<213> Homo sapiens

<400> 312
gaacggcuuc auacaggagu u 21

<210> 313
<211> 22
<212> RNA
<213> Homo sapiens

<400> 313
uccagcauca gugauuuugu ug 22

<210> 314
<211> 23
<212> RNA
<213> Homo sapiens

<400> 314
ucccccaggu gugauucuga uuu 23

<210> 315
<211> 22
<212> RNA
<213> Homo sapiens

<400> 315
aacacaccua uucaaggauu ca 22

<210> 316
<211> 24
<212> RNA
<213> Homo sapiens

<400> 316
aauccuugga accuaggugu gagu 24

<210> 317
<211> 22

<212> RNA
<213> Homo sapiens

<400> 317
agaucgaccg uguuauauuc gc 22

<210> 318
<211> 22
<212> RNA
<213> Homo sapiens

<400> 318
gccugcuggg guggaaccug gu 22

<210> 319
<211> 21
<212> RNA
<213> Homo sapiens

<400> 319
aucauagagg aaaauccacg u 21

<210> 320
<211> 21
<212> RNA
<213> Homo sapiens

<400> 320
aacauagagg aaauuccacg u 21

<210> 321
<211> 22
<212> RNA
<213> Homo sapiens

<400> 321
agagguugcc cuuggugaau uc 22

<210> 322
<211> 21
<212> RNA
<213> Homo sapiens

<400> 322
ugguagacua uggaacguag g 21

<210> 323
<211> 22
<212> RNA
<213> Homo sapiens

<400> 323
uauacaaggg caagcucucu gu 22

<210> 324
<211> 22
<212> RNA
<213> Homo sapiens

<400> 324
gaaguuguuc gugguggauu cg 22

<210> 325
<211> 22
<212> RNA
<213> Homo sapiens

<400> 325
gaauguugcu cggugaaccc cu 22

<210> 326
<211> 23
<212> RNA
<213> Homo sapiens

<400> 326
agguuacccg agcaacuuug cau 23

<210> 327
<211> 21
<212> RNA
<213> Homo sapiens

<400> 327
aauauaacac agauggccug u 21

<210> 328
<211> 21
<212> RNA
<213> Homo sapiens

<400> 328
uaguagaccg uauagcguac g 21

<210> 329
<211> 22
<212> RNA
<213> Homo sapiens

<400> 329
aucgggaaug ucguguccgc cc 22

<210> 330
<211> 22
<212> RNA
<213> Homo sapiens

<400> 330
caggucgucu ugcagggcuu cu 22

<210> 331
<211> 23
<212> RNA
<213> Homo sapiens

<400> 331
ucuuggagua ggucauuggg ugg 23

<210> 332
<211> 22
<212> RNA
<213> Homo sapiens

<400> 332
aucaugaugg gcuccucggu gu 22

<210> 333
<211> 21
<212> RNA
<213> Homo sapiens

<400> 333
ucacuccucu ccucccgucu u 21

<210> 334
<211> 22
<212> RNA
<213> Homo sapiens

<400> 334
aagacgggag gaaagaaggg ag 22

<210> 335
<211> 22
<212> RNA
<213> Homo sapiens

<400> 335
gucauacacg gcucuccucu cu 22

<210> 336
<211> 22
<212> RNA
<213> Homo sapiens

<400> 336
agaggcuggc cgugaugaau uc 22

<210> 337
<211> 22
<212> RNA
<213> Homo sapiens

<400> 337
aaucauacag ggacauccag uu 22

<210> 338
<211> 22
<212> RNA
<213> Homo sapiens

<400> 338
ugaaacauac acgggaaacc uc 22

<210> 339
<211> 22

<212> RNA
<213> Homo sapiens

<400> 339
aaacaaacau ggugcacuuc uu 22

<210> 340
<211> 23
<212> RNA
<213> Homo sapiens

<400> 340
uaauccuugc uaccugggug aga 23

<210> 341
<211> 22
<212> RNA
<213> Homo sapiens

<400> 341
augcaccugg gcaaggauuc ug 22

<210> 342
<211> 22
<212> RNA
<213> Homo sapiens

<400> 342
aaugcacccg ggcaaggauu cu 22

<210> 343
<211> 22
<212> RNA
<213> Homo sapiens

<400> 343
aaugcaccug ggcaaggauu ca 22

<210> 344
<211> 23
<212> RNA
<213> Homo sapiens

<400> 344
uagcagcggg aacaguucug cag 23

<210> 345
<211> 21
<212> RNA
<213> Homo sapiens

<400> 345
uaaggcaccc uucugaguag a 21

<210> 346
<211> 22
<212> RNA
<213> Homo sapiens

<400> 346
uacugcagac guggcaauca ug 22

<210> 347
<211> 18
<212> RNA
<213> Homo sapiens

<400> 347
uucacaggga ggugucau 18

<210> 348
<211> 22
<212> RNA
<213> Homo sapiens

<400> 348
ccucccacac ccaaggcuug ca 22

<210> 349
<211> 22
<212> RNA
<213> Homo sapiens

<400> 349
caugccuuga guguaggacc gu 22

<210> 350
<211> 22
<212> RNA
<213> Homo sapiens

<400> 350
ggagaaauua uccuuggugu gu 22

<210> 351
<211> 23
<212> RNA
<213> Homo sapiens

<400> 351
ucggggauca ucaugucacg aga 23

<210> 352
<211> 22
<212> RNA
<213> Homo sapiens

<400> 352
aaacauucgc ggugcacuuc uu 22

<210> 353
<211> 22
<212> RNA
<213> Homo sapiens

<400> 353
uacgucaucg uugucaucgu ca 22

<210> 354
<211> 25
<212> RNA
<213> Homo sapiens

<400> 354
gcugggcagg gcuucugagc uccuu 25

<210> 355
<211> 22
<212> RNA
<213> Homo sapiens

<400> 355
uaugucugcu gaccaucacc uu 22

<210> 356
<211> 25
<212> RNA
<213> Homo sapiens

<400> 356
ggcggaggga aguagguccg uuggu 25

<210> 357
<211> 22
<212> RNA
<213> Homo sapiens

<400> 357
uacccauugc auaucggagu ug 22

<210> 358
<211> 20
<212> RNA
<213> Homo sapiens

<400> 358
accaggaggc ugaggccccu 20

<210> 359
<211> 23
<212> RNA
<213> Homo sapiens

<400> 359
aaggagcuua caaucuagcu ggg 23

<210> 360
<211> 21
<212> RNA
<213> Homo sapiens

<400> 360
gcaggaacuu gugagucucc u 21

<210> 361
<211> 22

<212> RNA
<213> Homo sapiens

<400> 361
cugcccuggc ccgagggacc ga 22

<210> 362
<211> 22
<212> RNA
<213> Homo sapiens

<400> 362
ugcaacgaac cugagccacu ga 22

<210> 363
<211> 22
<212> RNA
<213> Homo sapiens

<400> 363
cacccguaga accgaccuug cg 22

<210> 364
<211> 22
<212> RNA
<213> Homo sapiens

<400> 364
aacuggggcg ggaaggggga ag 22

<210> 365
<211> 22
<212> RNA
<213> Homo sapiens

<400> 365
aagugauugg aggugggugg gg 22

<210> 366
<211> 22
<212> RNA
<213> Homo sapiens

<400> 366
agaagcugaa gggagagaga ca 22

<210> 367
<211> 22
<212> RNA
<213> Homo sapiens

<400> 367
gugguuaucc cugcuguguu cg 22

<210> 368
<211> 22
<212> RNA
<213> Homo sapiens

<400> 368
ugcagcuggu ggagucuggg gg 22

<210> 369
<211> 22
<212> RNA
<213> Homo sapiens

<400> 369
aaucugcagg gggagccugg gu 22

<210> 370
<211> 24
<212> RNA
<213> Homo sapiens

<400> 370
acucccaugu cccuugggaa gguc 24

<210> 371
<211> 23
<212> RNA
<213> Homo sapiens

<400> 371
agcgagguug cccuuuguau auu 23

<210> 372
<211> 19
<212> RNA
<213> Homo sapiens

<400> 372
agggcugggg acagagaug 19

<210> 373
<211> 19
<212> RNA
<213> Homo sapiens

<400> 373
agugaagcau uggacugua 19

<210> 374
<211> 18
<212> RNA
<213> Homo sapiens

<400> 374
aucccacucc ugacacca 18

<210> 375
<211> 21
<212> RNA
<213> Homo sapiens

<400> 375
cauccuagcc cuaagucugg c 21

<210> 376
<211> 28
<212> RNA
<213> Homo sapiens

<400> 376
cccaggcugg aguguagugg cgugaucu 28

<210> 377
<211> 22
<212> RNA
<213> Homo sapiens

<400> 377
cgccugugaa uagucacugc ac 22

<210> 378
<211> 20
<212> RNA
<213> Homo sapiens

<400> 378
gaaagcugag cgugaacgug 20

<210> 379
<211> 20
<212> RNA
<213> Homo sapiens

<400> 379
gaaucccacu ucugacacca 20

<210> 380
<211> 23
<212> RNA
<213> Homo sapiens

<400> 380
guuccuguug gccgagugga gac 23

<210> 381
<211> 19
<212> RNA
<213> Homo sapiens

<400> 381
uaaaaggaac ucggcaaau 19

<210> 382
<211> 17
<212> RNA.
<213> Homo sapiens

<400> 382
ugcagaucuu gguggua 17

<210> 383
<211> 22

<212> RNA
<213> Homo sapiens

<400> 383
uggggccucc cacagcuguu uc 22

<210> 384
<211> 17
<212> RNA
<213> Homo sapiens

<400> 384
ugguggucua gugguua 17

<210> 385
<211> 25
<212> RNA
<213> Homo sapiens

<400> 385
uguccaaagu aaacgcccug acgca 25

<210> 386
<211> 19
<212> RNA
<213> Homo sapiens

<400> 386
ugucccuucg uggucgcca 19

<210> 387
<211> 20
<212> RNA
<213> Homo sapiens

<400> 387
uucaugggga agcagauuug 20

<210> 388
<211> 23
<212> RNA
<213> Homo sapiens

<400> 388
ugaggggcag agagcgagac uuu 23

<210> 389
<211> 24
<212> RNA
<213> Homo sapiens

<400> 389
cauagcccgg ucgcugguac auga 24

<210> 390
<211> 23
<212> RNA
<213> Homo sapiens

<400> 390
gccgagacua gagucacauc cug 23

## Description

## FIELD OF THE INVENTION

The present invention relates to methods and materials for classification of cancers and the identification of their tissue of origin. Specifically the invention relates to microRNA molecules associated with specific cancers, as well as various nucleic acid molecules relating thereto or derived therefrom.

## BACKGROUND OF THE INVENTION

microRNAs (miRs, miRNAs) are a novel class of non-coding, regulatory RNA genes[1-3] which are involved in oncogenesis[4] and show remarkable tissue-specificity[5-7]. They have emerged as highly tissue-specific biomarkers[2,5,6] postulated to play important roles in encoding developmental decisions of differentiation. Various studies have tied microRNAs to the development of specific malignancies[4]. MicroRNAs are also stable in tissue, stored frozen or as formalin-fixed, paraffin-embedded (FFPE) samples, and in serum.

Hundreds of thousands of patients in the U.S. are diagnosed each year with a cancer that has already metastasized, without a clearly identified primary site. Oncologists and pathologists are constantly faced with a diagnostic dilemma when trying to identify the primary origin of a patient's metastasis. As metastases need to be treated according to their primary origin, accurate identification of the metastases' primary origin can be critical for determining appropriate treatment.

Once a metastatic tumor is found, the patient may undergo a wide range of costly, time consuming, and at times inefficient tests, including physical examination of the patient, histopathology analysis of the biopsy, imaging methods such as chest X-ray, CT and PET scans, in order to identify the primary origin of the metastasis.

Metastatic cancer of unknown primary (CUP) accounts for 3-5% of all new cancer cases, and as a group is usually a very aggressive disease with a poor prognosis[10]. The concept of CUP comes from the limitation of present methods to identify cancer origin, despite an often complicated and costly process which can significantly delay proper treatment of such patients. Recent studies revealed a high degree of variation in clinical management, in the absence of evidence based treatment for CUP[11]. Many protocols were evaluated[12] but have shown relatively small benefit[13]. Determining tumor tissue of origin is thus an important clinical application of molecular diagnostics[9].

Molecular classification studies for tumor tissue origin[14-17] have generally used classification algorithms that did not utilize domain-specific knowledge: tissues were treated as a-priori equivalents, ignoring underlying similarities between tissue types with a common developmental origin in embryogenesis. An exception of note is the study by Shedden and co-workers[18], that was based on a pathology classification tree. These studies used machine-learning methods that average effects of biological features (*e.g.*, mRNA expression levels), an approach which is more amenable to automated processing but does not use or generate mechanistic insights.

Lu *et al.* describe a study in which microRNA expression profiling was used for the classification of poorly differentiated tumors [Lu et al. (2005) Nature vol.435, no. 7043, pp.834-838].

WO 2010/073248 describes a process for classification of cancers through the analysis of the expression patterns of 48 microRNAs which determine 26 different tumor origins.

Various markers have been proposed to indicate specific types of cancers and tumor tissue of origin. However, the diagnostic accuracy of tumor markers has not yet been defined. There is thus a need for a more efficient and effective method for diagnosing and classifying specific types of cancers.

## SUMMARY OF THE INVENTION

The present application discloses specific nucleic acid sequences for use in the identification, classification and diagnosis of specific cancers and tumor tissue of origin. The nucleic acid sequences can also be used as prognostic markers for prognostic evaluation and determination of appropriate treatment of a subject based on the abundance of the nucleic acid sequences in a biological sample. The present invention provides a method for accurate identification of tumor tissue origin.

The invention is based in part on the development of a microRNA-based classifier for tumor classification. microRNA expression levels were measured in 1300 primary and metastatic tumor paraffin-embedded samples. microRNAs were profiled using a custom array platform. Using the custom array platform, a set of over 300 microRNAs was identified for the normalization of the array data and 65 microRNAs were used for the accurate classification of over 40 different tumor types. The accuracy of the assay exceeds 85%.

The findings demonstrate the utility of microRNA as novel biomarkers for the tissue of origin of a metastatic tumor. The classifier has wide biological as well as diagnostic applications.

According to a first aspect, the present invention provides a method of identifying a tissue of origin of a cancer, the method comprising measuring the relative abundance of nucleic acid sequences comprising SEQ ID NOS: 1-390, or a sequence having at least about 90% identity thereto in a cancer sample previously obtained from a subject,; and comparing the measurement to a reference abundance of the nucleic acid sequence by using a classifier algorithm, wherein the relative abundance of said nucleic acid sequences allows for the identification of the tissue of origin of said sample.

In an embodiment, the measurement of the relative abundance of SEQ ID NOS: 372, 233, 55, 200, 201 or a sequence having at least about 90% identity thereto in said sample compared to the reference abundance allows for distinguishing between a cancer originating from a germ-cell tumor and a cancer originating from non-germ-cell tumors.

In an embodiment, the measurement of the relative abundance of SEQ ID NOS: 6, 30, 13 or a sequence having at least about 90% identity thereto in said sample compared to the reference abundance allows for distinguishing between a cancer originating from hepatobiliary tumors and a cancer originating from non-germ-cell non-hepatobiliary tumors.

In an embodiment, the measurement of the relative abundance of SEQ ID NOS: 28, 29, 231, 9 or a sequence having at least about 90% identity thereto in said sample compared to the reference abundance allows for distinguishing between a cancer originating from liver tumors and a cancer originating from biliary-tract carcinomas.

In an embodiment, the measurement of the relative abundance of SEQ ID NOS: 46, 5, 12, 30, 29, 28, 32, 13, 152, 49 or a sequence having at least about 90% identity thereto in said sample compared to the reference abundance allows for distinguishing between a cancer originating from tumors from an epithelial origin and a cancer originating from tumors from a non-epithelial origin.

In an embodiment, the measurement of the relative abundance of SEQ ID NOS: 164, 168, 170, 16, 198, 50, 176, 186, 11, 158, 20, 155, 231, 4, 8, 46, 3, 2, 7 or a sequence having at least about 90% identity thereto in said sample compared to the reference abundance allows for distinguishing between a cancer originating from melanoma and lymphoma and a cancer originating from all other non-epithelial tumors.

In an embodiment, the measurement of the relative abundance of SEQ ID NOS: 159, 66, 225, 187, 162, 161, 68, 232, 173, 11, 8, 174, 155, 231, 4, 182, 181, 37 or a sequence having at least about 90% identity thereto in said sample compared to the reference abundance allows for distinguishing between a cancer originating from brain tumors and a cancer originating from all non-brain, non-epithelial tumors.

In an embodiment, the measurement of the relative abundance of SEQ ID NOS: 40, 208, 60, 153, 230, 228, 147, 34, 206, 35, 52, 25, 229, 161, 187, 179 or a sequence having at least about 90% identity thereto in said sample compared to the reference abundance allows for distinguishing between a cancer originating from astrocytoma and a cancer originating from oligodendroglioma.

In an embodiment, the measurement of the relative abundance of SEQ ID NOS: 56, 65, 25, 175, 152, 155, 32, 49, 35, 181, or a sequence having at least about 90% identity thereto in said sample compared to the reference abundance allows for distinguishing between a cancer originating from neuroendocrine tumors and a cancer originating from all non-neuroendocrine, epithelial tumors.

In an embodiment, the measurement of the relative abundance of SEQ ID NOS: 27, 177, 4, 32, 35 or a sequence having at least about 90% identity thereto in said sample compared to the reference abundance allows for distinguishing between a cancer originating from gastrointestinal epithelial tumors and a cancer originating from non-gastrointestinal epithelial tumors.

In an embodiment, the measurement of the relative abundance of SEQ ID NOS: 56, 199, 14, 15, 165, 231, 36, 154, 21, 49 or a sequence having at least about 90% identity thereto in said sample compared to the reference abundance allows for distinguishing between a cancer originating from prostate tumors and a cancer originating from all other non-gastrointestinal epithelial tumors.

In an embodiment, the measurement of the relative abundance of SEQ ID NOS: 222, 62, 29, 28, 211, 214, 227, 215, 218, 152, 216, 212, 224, 13, 194, 192, 221, 217, 205, 219, 32, 193, 223, 220, 210, 209, 213, 163, 30 or a sequence having at least about 90% identity thereto in said sample compared to the reference abundance allows for distinguishing between a cancer originating from seminoma and a cancer originating from non-seminoma testis-tumors.

In an embodiment, the measurement of the relative abundance of SEQ ID NOS: 42, 32, 36, 178, 243, 242, 49, 240, 57, 11, 46, 17, 47, 51, 7, 8, 154, 190, 157, 196, 197, or a sequence having at least about 90% identity thereto in said sample compared to the reference abundance allows for distinguishing between a cancer originating from squamous cell carcinoma, transitional cell carcinoma and thymoma, and a cancer originating from non-gastrointestinal adenocarcinoma tumors.

In an embodiment, the measurement of the relative abundance of SEQ ID NOS: 56, 46, 25, 152, 50, 45, 191, 181, 179, 49, 32, 42, 184, 40, 147, 236, 57, 203, 36, or a sequence having at least about 90% identity thereto in said sample compared to the reference abundance allows for distinguishing between a cancer originating from breast adenocarcinoma, and a cancer originating from squamous cell carcinoma, transitional cell carcinoma, thymomas and ovarian carcinoma.

In an embodiment, the measurement of the relative abundance of SEQ ID NOS: 253, 32, 4, 39, 10, 46, 5, 226, 2, 195, 32, 185, 11, 168, 184, 16, 242, 12, 237, 243, 250, 49, 246, 167 or a sequence having at least about 90% identity thereto in said sample compared to the reference abundance allows for distinguishing between a cancer originating from ovarian carcinoma, and a cancer originating from squamous cell carcinoma, transitional cell carcinoma and thymomas.

In an embodiment, the measurement of the relative abundance of SEQ ID NOS: 11, 147, 17, 157, 40, 8, 49, 9, 191, 205, 207, 195, 51, 46, 45, 52, 234, 231, 21, 169, 43, 3, 196, 154, 390, 171, 255, 197, 190, 189, 39, 7, , 47, 32, 36, 4, 178, 37, 181, 25, 183, 182, 35, 240, 57, 242, 204, 236, 176, 158, 148, 206, 50, 20, 34, 186, 239, 251, 244, 24, 188, 172, 238 or a sequence having at least about 90% identity thereto in said sample compared to the reference abundance allows for distinguishing between a cancer originating from thyroid carcinoma, and a cancer originating from breast adenocarcinoma, lung large cell carcinoma, lung adenocarcinoma and ovarian carcinoma.

In an embodiment, the measurement of the relative abundance of SEQ ID NOS: 249, 180, 65, 235, 241, 248, 254, 247, 160, 243, 245, 252, 17, 49, 166, 225, 168, 34 or a sequence having at least about 90% identity thereto in said sample compared to the reference abundance allows for distinguishing between a cancer originating from follicular thyroid carcinoma and a cancer originating from papillary thyroid carcinoma.

In an embodiment, the measurement of the relative abundance of SEQ ID NOS: 32, 56, 50, 45, 25, 253, 152, 9, 46, 191, 178, 49, 40, 10, 147, 4, 36, 228, 236, 230, 189, 240, 67, 202, 17 or a sequence having at least about 90% identity thereto in said sample compared to the reference abundance allows for distinguishing between a cancer originating from breast adenocarcinoma and a cancer originating from lung adenocarcinoma and ovarian carcinoma.

In an embodiment, the measurement of the relative abundance of SEQ ID NOS: 56, 11, 168, 16, 237, 21, 52, 12, 154, 279, 9, 39, 47, 23, 50, 167, 383, 34, 35, 388, 5, 359, 245, 254, 10, 240, 236, 202, 4, 25, 203, 231, 20, 158, 186, 258, 244, 172, 2, 235, 256, 28, 277, 296, 374, 153, 181 or a sequence having at least about 90% identity thereto in said sample compared to the reference abundance allows for distinguishing between a cancer originating from lung adenocarcinoma and a cancer originating from ovarian carcinoma.

In an embodiment, the measurement of the relative abundance of SEQ ID NOS: 161, 164, 22, 53, 285, 3, 152, 191, 154, 21, 206, 174, 19, 45, 171, 179, 8, 296, 284, 18, 51, 258, 49, 184, 35, 34, 37, 42, 228, 15, 14, 242, 230, 253, 36, 182, 293, 292, 4, 294, 297, 354, 377, 189, 30, 386, 249, 5, 274 or a sequence having at least about 90% identity thereto in said sample compared to the reference abundance allows for distinguishing between a cancer originating from thymic carcinoma and a cancer originating from transitional cell carcinoma and squamous cell carcinoma.

In an embodiment, the measurement of the relative abundance of SEQ ID NOS: 69, 28, 280, 13, 191, 152, 29, 175, 30, 204, 4, 24, 5, 329, 273, 170, 184, 26, 231, 368, 37, 16, 169, 155, 35, 40, 17 or a sequence having at least about 90% identity thereto in said sample compared to the reference abundance allows for distinguishing between a cancer originating from transitional cell carcinoma and a cancer originating from squamous cell carcinoma.

In an embodiment, the measurement of the relative abundance of SEQ ID NOS: 164, 5, 231, 54, 1, 242, 372, 249, 167, 254, 354, 381, 380, 245, 358, 364, 240, 11, 378 or a sequence having at least about 90% identity thereto in said sample compared to the reference abundance allows for distinguishing between squamous cell carcinoma cancers originating from the uterine cervix, and squamous cell carcinoma cancers originating from anus and skin, lung, head & neck and esophagus.

In an embodiment, the measurement of the relative abundance of SEQ ID NOS: 305, 184, 41, 183, 49, 382, 235, 291, 181, 5, 296, 289, 206, 338, 334, 25, 11, 19, 198, 23 or a sequence having at least about 90% identity thereto in said sample compared to the reference abundance allows for distinguishing between squamous cell carcinoma cancers originating from anus and skin, and between squamous cell carcinoma cancers originating from lung, head & neck and esophagus.

In an embodiment, the measurement of the relative abundance of SEQ ID NOS: 4, 11, 46, 8, 274, 169, 36, 47, 363, 231, 303, 349, 10, 7, 3, 16, 164, 170, 168, 198, 50, 245, 365, 45, 382, 259, 296, 364, 314, 12 or a sequence having at least about 90% identity thereto in said sample compared to the reference abundance allows for distinguishing between a cancer originating from melanoma and a cancer originating from lymphoma.

In an embodiment, the measurement of the relative abundance of SEQ ID NOS: 11, 191, 48, 35, 228 or a sequence having at least about 90% identity thereto in said sample compared to the reference abundance allows for distinguishing between a cancer originating from B-cell lymphoma and a cancer originating from T-cell lymphoma.

In an embodiment, the measurement of the relative abundance of SEQ ID NOS: 158, 20, 176, 186, 148, 36, 51, 172, 260, 265, 67, 188, 277, 284, 302, 68, 168, 242, 204, 162, 177, 27, 65, 263, 155, 191, 190, 45, 59, 43, 56, 266, 14, 15, 8, 7, 39, 189, 249, 231, 293, 2 or a sequence having at least about 90% identity thereto in said sample compared to the reference abundance allows for distinguishing between a cancer originating from lung small cell carcinoma and a cancer originating from lung carcinoid, medullary thyroid carcinoma, gastrointestinal tract carcinoid and pancreatic islet cell tumor.

In an embodiment, the measurement of the relative abundance of SEQ ID NOS: 159, 40, 147, 11, 311, 4, 8, 231, 301, 297, 68, 67, 265, 36 or a sequence having at least about 90% identity thereto in said sample compared to the reference abundance allows for distinguishing between a cancer originating from medullary thyroid carcinoma and a cancer originating from other neuroendocrine tumors selected from lung carcinoid, gastrointestinal tract carcinoid and pancreatic islet cell tumor.

In an embodiment, the measurement of the relative abundance of SEQ ID NOS: 331, 162, 59, 326, 306, 350, 317, 155, 325, 318, 339, 264, 332, 262, 336, 324, 322, 330, 321, 263, 309, 53, 320, 275, 352, 312, 355, 367, 269, 64, 308, 175, 190, 54, 302, 152, 301, 266, 47, 313, 359, 65, 307, 191, 242, 4, 147, 40, 372, 168, 16, 182, 167, 356, 148, 382, 37, 364, 35 or a sequence having at least about 90% identity thereto in said sample compared to the reference abundance allows for distinguishing between a cancer originating from lung carcinoid tumors, and a cancer originating from gastrointestinal neuroendocrine tumors selected from gastrointestinal tract carcinoid and pancreatic islet cell tumor.

In an embodiment, the measurement of the relative abundance of SEQ ID NOS: 263, 288, 18, 286, 162, 225, 287, 206, 205, 296, 258, 313, 377, 373, 256, 153, 259, 265, 303, 268, 267, 165, 15, 272, 14, 202, 236, 203, 4, 168, 310, 298, 27, 29, 34, 228, 3, 349, 35, 26 or a sequence having at least about 90% identity thereto in said sample compared to the reference abundance allows for distinguishing between a cancer originating from pancreatic islet cell tumors and a Gastrointestinal neuroendocrine carcinoid cancer originating from small intestine and duodenum; appendicitis, stomach and pancreas.

In an embodiment, the measurement of the relative abundance of SEQ ID NOS: 36, 267, 268, 165, 15, 14, 356, 167, 372, 272, 370, 42, 41, 146 or a sequence having at least about 90% identity thereto in said sample compared to the reference abundance allows for distinguishing between adenocarcinoma tumors of the gastrointestinal system originating from:

a) gastric and esophageal adenocarcinoma, and
b) cholangiocarcinoma or adenocarcinoma of the extrahepatic biliary tract, pancreatic adenocarcinoma and colorectal adenocarcinoma; or

In an embodiment, the measurement of the relative abundance of SEQ ID NOS: 42, 184, 67, 158, 20, 186, 284, 389, 203, 240, 236, 146, 204, 43, 176, 202, 49, 46, 38, 363 or a sequence having at least about 90% identity thereto in said sample compared to the reference abundance allows for distinguishing between a cancer originating from colorectal adenocarcinoma and a cancer originating from adenocarcinoma of biliary tract or pancreas.

In an embodiment, the measurement of the relative abundance of SEQ ID NOS: 49, 11, 13, 373, 154, 5, 30, 45, 178, 147, 274, 16, 40, 21, 43, 253, 245, 256, 12, 374, 379, 180, 153, 51, 52, 1, 295, 257, 3 85, 293, 294 or a sequence having at least about 90% identity thereto in said compared to the reference abundance sample allows for distinguishing between a cancer originating from pancreatic adenocarcinoma, and a cancer originating from cholangiocarcinoma or adenocarcinoma of the extrahepatic biliary tract.

In an embodiment, the measurement of the relative abundance of SEQ ID NOS: 29, 28, 30, 46, 49, 195, 152, 175, 47, 4, 387, 196, 177, 375, 27, 304, 40, 191, 147, 35, 16, 34, 5, 155, 181, 312, 183, 182, 320, 59, 38, 324, 323, 37, 322, 325, 19, 42, 334, 265, 22 or a sequence having at least about 90% identity thereto in said sample compared to the reference abundance allows for distinguishing between a cancer originating from:

c) renal cell tumors selected from chromophobe renal cell carcinoma, clear cell renal cell carcinoma and papillary renal cell carcinoma, and
d) sarcomas, adrenal tumors and pleural mesothelioma.

In an embodiment, the measurement of the relative abundance of SEQ ID NOS: 65, 56, 11, 162, 59, 331, 350, 155, 335, 159, 336, 332, 263, 306, 339, 337, 275, 301, 276, 330, 317, 309, 45, 318, 324, 352, 191, 262, 269, 313, 19, 367, 326, 325, 322, 327, 190, 261, 321, 360, 353, 312, 371, 5, 328, 205, 183, 38, 181, 37, 40, 182, 147, 17, 42, 382, 34, 18, 3 or a sequence having at least about 90% identity thereto in said sample compared to the reference abundance allows for distinguishing between a cancer originating from pheochromocytoma, and a cancer originating from all sarcoma, adrenal carcinoma and mesothelioma tumors.

In an embodiment, the measurement of the relative abundance of SEQ ID NOS: 61, 333, 31, 347, 346, 344, 345, 387, 334, 351, 324, 326, 269, 155, 320, 322, 59, 318, 325, 245, 254, 331, 275, 180, 355, 370, 323, 312, 178, 249, 183, 181, 38, 182, 37, 3, 25 or a sequence having at least about 90% identity thereto in said sample compared to the reference abundance allows for distinguishing between a cancer originating from adrenal carcinoma and a cancer originating from mesothelioma and sarcoma tumors.

In an embodiment, the measurement of the relative abundance of SEQ ID NOS: 165, 14, 15, 333, 272, 270, 45, 301, 191, 46, 195, 266, 190, 19, 334, 155, 25, 147, 40, 34 or a sequence having at least about 90% identity thereto in said sample compared to the reference abundance allows for distinguishing between a cancer originating from a gastrointestinal stomal tumor and a cancer originating from mesothelioma and sarcoma tumors.

In an embodiment, the measurement of the relative abundance of SEQ ID NOS: 13, 30, 361, 280, 362, 147, 40, 291, 387, 290, 299, 152, 178, 303, 242, 49, 11, 35, 34, 36, 206, 16, 170, 177, 17 or a sequence having at least about 90% identity thereto in said sample compared to the reference abundance allows for distinguishing between a cancer originating from a chromophobe renal cell carcinoma tumor and a cancer originating from clear cell and papillary renal cell carcinoma tumors.

In an embodiment, the measurement of the relative abundance of SEQ ID NOS: 344, 382, 9, 338, 29, 49, 28, 195, 46, 4, 11, 254 or a sequence having at least about 90% identity thereto in said sample compared to the reference abundance allows for distinguishing between a renal carcinoma cancer originating from a clear cell tumor and a cancer originating from a papillary tumor.

In an embodiment, the measurement of the relative abundance of SEQ ID NOS: 49, 35, 17, 34, 25, 36, 168, 170, 26, 4, 190, 46, 10, 240, 43, 39, 385, 63, 202, 181, 37, 5, 183, 182, 38, 206, 296, 1 or a sequence having at least about 90% identity thereto in said sample compared to the reference abundance allows for distinguishing between a cancer originating from pleural mesothelioma and a cancer originating from sarcoma tumors.

In an embodiment, the measurement of the relative abundance of SEQ ID NOS: 152, 29, 159, 28, 339, 275, 352, 19, 320, 155, 262, 38, 37, 182, 331, 317, 323, 355, 3, 282, 312, 181, 269, 318, 59, 266, 322, 8, 324, 10, 40, 147, 169, 205, 34, 168, 14, 15, 12, 46, 255, 39, 23, 190, 236, 386, 379, 202 or a sequence having at least about 90% identity thereto in said sample compared to the reference abundance allows for distinguishing between a cancer originating from a synovial sarcoma and a cancer originating from other sarcoma tumors.

In an embodiment, the measurement of the relative abundance of SEQ ID NOS: 12, 271, 206, 333, 11, 58, 36, 18, 178, 293, 189, 382, 381, 240, 249, 5, 377, 235, 17, 20, 385, 384, 46, 283 or a sequence having at least about 90% identity thereto in said sample compared to the reference abundance allows for distinguishing between a cancer originating from chondrosarcoma and a cancer originating from other non-synovial sarcoma tumors.

In an embodiment, measurement of the relative abundance of SEQ ID NOS: 295, 205, 25, 26, 231, 183, 42, 254, 168, 64, 14, 178, 15, 39, 36, 154, 265, 174, 384, 67 or a sequence having at least about 90% identity thereto in said sample compared to the reference abundance allows for distinguishing between a cancer originating from liposarcoma and a cancer originating from other non chondrosarcoma and non synovial sarcoma tumors.

In an embodiment, the measurement of the relative abundance of SEQ ID NOS: 22, 154, 21, 174, 205, 158, 186, 148, 20, 59, 8, 183, 231 or a sequence having at least about 90% identity thereto in said sample compared to the reference abundance allows for distinguishing between a cancer originating from:

e) Ewing sarcoma and osteosarcoma, and
f) rhabdomyosarcoma, malignant fibrous histiocytoma and fibrosarcoma.

In an embodiment, the measurement of the relative abundance of SEQ ID NOS: 155, 179, 43, 208, 278, 17, 385, 174, 5, 52, 257, 366, 48, 49, 12, 25, 169, 34, 35, 23, 384, 189, 377, 265, 294, 293, 292 or a sequence having at least about 90% identity thereto in said sample compared to the reference abundance allows for distinguishing between a cancer originating from Ewing sarcoma and a cancer originating from osteosarcoma.

In an embodiment, the measurement of the relative abundance of SEQ ID NOS: 33, 268, 267, 333, 276, 319, 306, 320, 334, 323, 300, 281, 59, 339, 316, 176, 348, 352, 349, 67, 357, 315, 343, 342, 355, 340, 344, 10, 341, 331, 20, 277, 318, 158, 265, 284, 36, 183, 40, 63, 147, 43, 289, 52, 190, 4, 5, 39, 169, 208 or a sequence having at least about 90% identity thereto in said sample compared to the reference abundance allows for distinguishing between a cancer originating from rhabdomyosarcoma and a cancer originating from malignant fibrous histiocytoma and fibrosarcoma.

According to one aspect, the classifier algorithm is selected from the group consisting of decision tree classifier, K-nearest neighbor classifier (KNN), logistic regression classifier, nearest neighbor classifier, neural network classifier, Gaussian mixture model (GMM), Support Vector Machine (SVM) classifier, nearest centroid classifier, linear regression classifier and random forest classifier.

According to some aspects of the invention the biological sample is selected from the group consisting of bodily fluid, a tissue sample, a biopsy sample, a needle biopsy sample, a fine needle biopsy (FNA) sample, a surgically removed sample, and a sample obtained by tissue-sampling procedures such as endoscopy, bronchoscopy, or laparoscopic methods. According to some embodiments, the tissue is a fresh, frozen, fixed, wax-embedded or formalin-fixed paraffin-embedded (FFPE) tissue.

According to additional aspects of the invention the nucleic acid sequence relative abundance is determined by a method selected from the group consisting of nucleic acid hybridization and nucleic acid amplification. According to some embodiments, the nucleic acid hybridization is performed using a solid-phase nucleic acid biochip array or *in situ* hybridization. According to some embodiments, the nucleic acid amplification method is real-time PCR. According to some embodiments, the real-time PCR comprises forward and reverse primers. According to additional embodiments, the real-time PCR method further comprises a probe, said probe comprising a sequence complementary to SEQ ID NOS: 1; 2 or 156; 3-7; 9-12; 14-21; 23-27; 29-40; 42; 43; 44 or 191; 45-47; 49-51; 53-56; 57 or 202; 58; 59; 60 or 208; 61; 62 or 211; 64-69; and 146-148, fragments thereof, and sequences having at least about 90% identity thereto.

These and other embodiments of the present invention will become apparent in conjunction with the figures, description and claims that follow.

## BRIEF DESCRIPTION OF THE DRAWINGS

**Figures 1A-1F** demonstrate the structure of the binary decision-tree classifier, with 45 nodes and 46 leaves. Each node is a binary decision between two sets of samples, those to the left and right of the node. A series of binary decisions, starting at node #1 and moving downwards, lead to one of the possible tumor types, which are the "leaves" of the tree. A sample which is classified to the right branch at node #1 continues to node #2, otherwise it continues to node #11. A sample which is classified to the right branch at node #2 continues to node #4, otherwise it continues to node #3. A sample that reaches node #3, is further classified to either the left branch at node #3, and is assigned to the "hepatocellular carcinoma" class, or to the right branch at node #3, and is assigned to the "biliary tract adenocarcinoma" class.

Decisions are made at consecutive nodes using microRNA expression levels, until an end-point ("leaf" of the tree) is reached, indicating the predicted class for this sample. In specifying the tree structure, clinico-pathological considerations were combined with properties observed in the training set data.

**Figure 2** demonstrates binary decisions at node #1 of the decision-tree. When training a decision algorithm for a given node, only samples from classes which are possible outcomes of this node are used for training. The "non germ cell" classes (right branch at node #1); are easily distinguished from tumors of the "germ cell" classes (left branch at node #1) using the expression levels of hsa-miR-373 (SEQ ID NO: 233, 2A), hsa-miR-372 (SEQ ID NO: 55, 2B), hsa-miR-371-3p (SEQ ID NO: 200, 2C), and hsa-miR-371-5p (SEQ ID NO: 201, 2D). The boxplot presentations comparing distribution of the expression of the statistically significant miRs in tumor samples from the "germ cell" classes (left box) and "non germ cell" classes (right box). The line in the box indicates the median value. The box contains 50% of the data and the horizontal lines and crosses (outliers) show the full range of signals in this group.

**Figure 3** demonstrates binary decisions at node #3 of the decision-tree. Tumors of hepatocellular carcinoma (HCC) origin (left branch at node #3, marked by squares) are easily distinguished from tumors of biliary tract adenocarcinoma origin (right branch at node #3, marked by diamonds) using the expression levels of hsa-miR-200b (SEQ ID NO: 29 , y-axis) and hsa-miR-126 (SEQ ID NO: 9, x-axis).

**Figure 4** demonstrates binary decisions at node #4 of the decision-tree. Tumors originating in epithelial (diamonds) are easily distinguished from tumors of non-epithelial origin (squares) using the expression levels of hsa-miR-30a (SEQ ID NO: 46, y-axis) and hsa-miR-200c (SEQ ID NO: 30, x-axis).

**Figure 5** demonstrates binary decisions at node #5 of the decision-tree. Tumors originating in the lymphoma or melanoma (diamonds) are easily distinguished from tumors of non epithelial, non lymphoma /melanoma origin (squares) using the expression levels of hsa-miR-146a (SEQ ID NO: 16, y-axis), hsa-miR-30a (SEQ ID NO: 46, x-axis) and hsa-let-7e (SEQ ID NO: 2, z-axis).

**Figure 6** demonstrates binary decisions at node #6 of the decision-tree. Tumors originating in the brain (left branch at node #6, marked by diamonds) are easily distinguished from tumors of non epithelial, non brain (right branch at node #6, marked by squares) using the expression levels of hsa-miR-9* (SEQ ID NO: 66, y-axis) and hsa-miR-92b (SEQ ID NO: 68, x-axis).

**Figure 7** demonstrates binary decisions at node #7 of the decision-tree. Tumors originating in astrocytoma (right branch at node # 7, marked by diamonds) are easily distinguished from tumors of oligodendroglioma origins (left branch at node # 7, marked by squares) using the expression levels of hsa-miR-497 (SEQ ID NO: 60, y-axis) and hsa-miR-222 (SEQ ID NO: 40, x-axis).

**Figure 8** demonstrates binary decisions at node #8 of the decision-tree. Tumors originating in the neuroendocrine (diamonds) are easily distinguished from tumors of epithelial, origin (squares) using the expression levels of hsa-miR-193a-3p (SEQ ID NO: 181, y-axis), hsa-miR-7 (SEQ ID NO: 65, x-axis) and hsa-miR-375 (SEQ ID NO: 56, z-axis).

**Figure 9** demonstrates binary decisions at node #9 of the decision-tree. Tumors originating in gastro-intestinal (GI) (left branch at node # 9, marked by diamonds) are easily distinguished from tumors of non GI origins (right branch at node # 9, marked by squares) using the expression levels of hsa-miR-21 * (SEQ ID NO: 35, y-axis) and hsa-miR-194 (SEQ ID NO: 27, x-axis).

**Figure 10** demonstrates binary decisions at node #10 of the decision-tree. Tumors originating in prostate adenocarcinoma (left branch at node # 10, marked by diamonds) are easily distinguished from tumors of non prostate origins (right branch at node # 10, marked by squares) using the expression levels of hsa-miR-181a (SEQ ID NO: 21, y-axis) and hsa-miR-143 (SEQ ID NO: 14, x-axis).

**Figure 11** demonstrates binary decisions at node #12 of the decision-tree. Tumors originating in seminiomatous testicular germ cell (left branch at node # 12, marked by diamonds) are easily distinguished from tumors of non seminiomatous origins (right branch at node # 12, marked by squares) using the expression levels of hsa-miR-516a-5p (SEQ ID NO: 62, y-axis) and hsa-miR-200b (SEQ ID NO: 29, x-axis).

**Figure 12** demonstrates binary decisions at node #16 of the decision-tree. Tumors originating in thyroid carcinoma (diamonds) are easily distinguished from tumors of adenocarcinoma of the lung, breast and ovarian origin (squares) using the expression levels of hsa-miR-93 (SEQ ID NO: 148, y-axis), hsa-miR-138 (SEQ ID NO: 11, x-axis) and hsa-miR-10a (SEQ ID NO: 4, z-axis).

**Figure 13** demonstrates binary decisions at node #17 of the decision-tree. Tumors originating in follicular thyroid carcinoma (left branch at node # 17, marked by diamonds) are easily distinguished from tumors of papillary thyroid carcinoma origins (right branch at node # 17, marked by squares) using the expression levels of hsa-miR-21 (SEQ ID NO: 34, y-axis) and hsa-miR-146b-5p (SEQ ID NO: 17, x-axis).

**Figure 14** demonstrates binary decisions at node #18 of the decision-tree. Tumors originating in breast (diamonds) are easily distinguished from tumors of lung and ovarian origin (squares) using the expression levels of hsa-miR-92a (SEQ ID NO: 67, y-axis), hsa-miR-193a-3p (SEQ ID NO: 25, x-axis) and hsa-miR-31 (SEQ ID NO: 49, z-axis).

**Figure 15** demonstrates binary decisions at node #19 of the decision-tree. Tumors originating in lung adenocarcinoma (diamonds) are easily distinguished from tumors of ovarian carcinoma origin (squares) using the expression levels of hsa-miR-21 (SEQ ID NO: 34, y-axis), hsa-miR-378 (SEQ ID NO: 57, x-axis) and hsa-miR-138 (SEQ ID NO: 11, z-axis).

**Figure 16** demonstrates binary decisions at node #20 of the decision-tree. Tumors originating in thymic carcinoma (left branch at node # 20, marked by diamonds) are easily distinguished from tumors of urothelial carcinoma, transitional cell carcinoma (TCC) carcinoma and squamous cell carcinoma (SCC) origins (right branch at node # 20, marked by squares) using the expression levels of hsa-miR-21 (SEQ ID NO: 34, y-axis) and hsa-miR-100 (SEQ ID NO: 3, x-axis).

**Figure 17** demonstrates binary decisions at node #22 of the decision-tree. Tumors originating in SCC of the uterine cervix (diamonds) are easily distinguished from tumors of other SCC origin (squares) using the expression levels of hsa-miR-361-5p (SEQ ID NO: 54, y-axis), hsa-let-7c (SEQ ID NO: 1, x-axis) and hsa-miR-10b (SEQ ID NO: 5, z-axis).

**Figure 18** demonstrates binary decisions at node #24 of the decision-tree. Tumors originating in melanoma (diamonds) are easily distinguished from tumors of lymphoma origin (squares) using the expression levels of hsa-miR-342-3p (SEQ ID NO: 50, y-axis) and hsa-miR-30d (SEQ ID NO: 47, x-axis).

**Figure 19** demonstrates binary decisions at node #27 of the decision-tree. Tumors originating in thyroid carcinoma, medullary (diamonds) are easily distinguished from tumors of other neuroendocrine origin (squares) using the expression levels of hsa-miR-92b (SEQ ID NO: 68, y-axis), hsa-miR-222 (SEQ ID NO: 40, x-axis) and hsa-miR-92a (SEQ ID NO: 67, z-axis).

**Figure 20** demonstrates binary decisions at node #30 of the decision-tree. Tumors originating in gastric or esophageal adenocarcinoma (diamonds) are easily distinguished from tumors of other GI adenocarcinoma origin (squares) using the expression levels of hsa-miR-1201 (SEQ ID NO: 146, y-axis), hsa-miR-224 (SEQ ID NO: 42, x-axis) and hsa-miR-210 (SEQ ID NO: 36, z-axis).

**Figure 21** demonstrates binary decisions at node #31 of the decision-tree. Tumors originating in colorectal adenocarcinoma (diamonds) are easily distinguished from tumors of adenocarcinoma of biliary tract or pancreas origin (squares) using the expression levels of hsa-miR-30a (SEQ ID NO: 46, y-axis), hsa-miR-17 (SEQ ID NO: 20, x-axis) and hsa-miR-29a (SEQ ID NO: 43, z-axis).

**Figure 22** demonstrates binary decisions at node #33 of the decision-tree. Tumors originating in kidney (diamonds) are easily distinguished from tumors of adrenal, mesothelioma and sarcoma origin (squares) using the expression levels of hsa-miR-200b (SEQ ID NO: 29, y-axis), hsa-miR-30a (SEQ ID NO: 46, x-axis) and hsa-miR-149 (SEQ ID NO: 19, z-axis).

**Figure 23** demonstrates binary decisions at node #34 of the decision-tree. Tumors originating in pheochromocytoma (diamonds) are easily distinguished from tumors of adrenal, mesothelioma and sarcoma origin (squares) using the expression levels of hsa-miR-375 (SEQ ID NO: 56, y-axis) and hsa-miR-7 (SEQ ID NO: 65, x-axis).

**Figure 24** demonstrates binary decisions at node #44 of the decision-tree. Tumors originating in Ewing sarcoma (diamonds) are easily distinguished from tumors of osteosarcoma origin (squares) using the expression levels of hsa-miR-31 (SEQ ID NO: 49, y-axis) and hsa-miR-193a-3p (SEQ ID NO: 25, x-axis).

**Figure 25** demonstrates binary decisions at node #45 of the decision-tree. Tumors originating in Rhabdomyosarcoma (diamonds) are easily distinguished from tumors of malignant fibrous histiocytoma (MFH) or fibrosarcoma origin (squares) using the expression levels of hsa-miR-206 (SEQ ID NO: 33, y-axis), hsa-miR-22 (SEQ ID NO: 39, x-axis) and hsa-miR-487b (SEQ ID NO: 59, z-axis).

## DETAILED DESCRIPTION OF THE INVENTION

Identification of the tissue-of-origin of a tumor is vital to its management. The present invention is based in part on the discovery that specific nucleic acid sequences can be used for the identification of the tissue-of-origin of a tumor. The present invention provides a sensitive, specific and accurate method which can be used to distinguish between different tumor origins. A new microRNA-based classifier was developed for determining tissue origin of tumors based on 65 microRNAs markers. The classifier uses a specific algorithm and allows a clear interpretation of the specific biomarkers.

According to the present invention each node in the classification tree may be used as an independent differential diagnosis tool, for example in the identification of different types of lung cancers. The possibility to distinguish between different tumor origins facilitates providing the patient with the best and most suitable treatment.

The present application discloses diagnostic assays and methods, both quantitative and qualitative for detecting, diagnosing, monitoring, staging and prognosticating cancers by comparing the levels of the specific microRNA molecules of the invention. Such levels are preferably measured in at least one of biopsies, tumor samples, fine-needle aspiration (FNA), cells, tissues and/or bodily fluids. The methods provided in the present invention are particularly useful for discriminating between different cancers.

All the methods of the present invention may optionally further include measuring levels of additional cancer markers. The cancer markers measured in addition to said microRNA molecules depend on the cancer being tested and are known to those of skill in the art.

Assay techniques can be used to determine levels of gene expression, such as genes encoding the nucleic acids of the present invention in a sample derived from a patient. Such assay methods, which are well known to those of skill in the art, include, but are not limited to, nucleic acid microarrays and biochip analysis, reverse transcriptase PCR (RT-PCR) assays, immunohistochemistry assays, *in situ* hybridization assays, competitive-binding assays, northern blot analyses and ELISA assays.

According to one embodiment, the assay is based on expression level of 65 microRNAs in RNA extracted from FFPE metastatic tumor tissue.

The expression levels are used to infer the sample origin using analysis techniques such as, but not limited to, decision-tree classifier, K nearest neighbors classifier, logistic regression classifier, linear regression classifier, nearest neighbor classifier, neural network classifier and nearest centroid classifier.

In use of the decision tree classifier the expression levels are used to make binary decisions (at each relevant node) following the pre-defined structure of the binary decision-tree (defined using a training set).

At each node, the expressions of one or several microRNAs are combined together using a function of the form P=exp (β0 + β1*miR1 + β2*miR2 + β3*miR3...)/(1- exp (β0 + β1*miR1 + β2*miR2 + β3*miR3...)), where the values of β0, β1, β2... and the identities of the microRNAs have been pre-determined (using a training set). The resulting P is compared to a probability threshold level ($P_{TH}$, which was also determined using the training set), and the classification continues to the left or right branch according to whether P is larger or smaller than the $P_{TH}$ for that node. This continues until an end-point ("leaf") of the tree is reached. According to some embodiments, $P_{TH}$ = 0.5 for all nodes, and the value of β0 is adjusted accordingly. According to further embodiments, β0, β1, β2, ... are adjusted so that the slope of the log of the odds ratio function is limited.

Training the tree algorithm means determining the tree structure- which nodes there are and what is on each side, and, for each node: which miRs are used, the values of β0, β1, β2... and the $P_{TH}$. These are determined by a combination of machine learning, optimization algorithm, and trial and error by experts in machine learning and diagnostic algorithms.

An arbitrary threshold of the expression level of one or more nucleic acid sequences can be set for assigning a sample to one of two groups. Alternatively, in a preferred embodiment, expression levels of one or more nucleic acid sequences of the invention are combined by a method such as logistic regression to define a metric which is then compared to previously measured samples or to a threshold. The threshold is treated as a parameter that can be used to quantify the confidence with which samples are assigned to each class. The threshold can be scaled to favor sensitivity or specificity, depending on the clinical scenario. The correlation value to the reference data generates a continuous score that can be scaled and provides diagnostic information on the likelihood that a sample belongs to a certain class of cancer origin or type. In multivariate analysis the microRNA signature provides a high level of prognostic information.

In another preferred embodiment, expression level of nucleic acids is used to classify a test sample by comparison to a training set of samples. In this embodiment, the test sample is compared in turn to each one of the training set samples. The comparison is performed by comparing the expression levels of one or multiple nucleic acids between the test sample and the specific training sample. Each such pairwise comparison generates a combined metric for the multiple nucleic acids, which can be calculated by various numeric methods such as correlation, cosine, Euclidian distance, mean square distance, or other methods known to those skilled in the art. The training samples are then ranked according to this metric, and the samples with the highest values of the metric (or lowest values, according to the type of metric) are identified, indicating those samples that are most similar to the test sample. By choosing a parameter K, this generates a list that includes the K training samples that are most similar to the test sample. Various methods can then be applied to identify from this list the predicted class of the test sample. In a favored embodiment, the test sample is predicted to belong to the class that has the highest number of representative in the list of K most-similar training samples (this method is known as the K Nearest Neighbors method). Other embodiments may provide a list of predictions including all or part of the classes represented in the list, those classes that are represented more than a given minimum number of times, or other voting schemes whereby classes are grouped together.

## 1. Definitions

It is to be understood that the terminology used herein is for the purpose of describing particular embodiments only and is not intended to be limiting. It must be noted that, as used in the specification and the appended claims, the singular forms "a," "an" and "the" include plural referents unless the context clearly dictates otherwise.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9 and 7.0 are explicitly contemplated.

### about

As used herein, the term "about" refers to +/-10%.

### attached

"Attached" or "immobilized", as used herein, to refer to a probe and a solid support means that the binding between the probe and the solid support is sufficient to be stable under conditions of binding, washing, analysis, and removal. The binding may be covalent or non-covalent. Covalent bonds may be formed directly between the probe and the solid support or may be formed by a cross linker or by inclusion of a specific reactive group on either the solid support or the probe or both molecules. Non-covalent binding may be one or more of electrostatic, hydrophilic, and hydrophobic interactions. Included in non-covalent binding is the covalent attachment of a molecule, such as streptavidin, to the support and the non-covalent binding of a biotinylated probe to the streptavidin. Immobilization may also involve a combination of covalent and non-covalent interactions.

### baseline

"Baseline", as used herein, means the initial cycles of PCR, in which there is little change in fluorescence signal.

### biological sample

"Biological sample", as used herein, means a sample of biological tissue or fluid that comprises nucleic acids. Such samples include, but are not limited to, tissue or fluid isolated from subjects. Biological samples may also include sections of tissues such as biopsy and autopsy samples, FFPE samples, frozen sections taken for histological purposes, blood, blood fraction, plasma, serum, sputum, stool, tears, mucus, hair, skin, urine, effusions, ascitic fluid, amniotic fluid, saliva, cerebrospinal fluid, cervical secretions, vaginal secretions, endometrial secretions, gastrointestinal secretions, bronchial secretions, cell line, tissue sample, or secretions from the breast. A biological sample may be provided by fine-needle aspiration (FNA), pleural effusion or bronchial brushing. A biological sample may be provided by removing a sample of cells from a subject but can also be accomplished by using previously isolated cells (e.g., isolated by another person, at another time, and/or for another purpose), or by performing the methods described herein *in vivo.* Archival tissues, such as those having treatment or outcome history, may also be used. Biological samples also include explants and primary and/or transformed cell cultures derived from animal or human tissues.

### cancer

The term "cancer" is meant to include all types of cancerous growths or oncogenic processes, metastatic tissues or malignantly transformed cells, tissues, or organs, irrespective of histopathologic type or stage of invasiveness. Examples of cancers include, but are not limited, to solid tumors and leukemias, including: apudoma, choristoma, branchioma, malignant carcinoid syndrome, carcinoid heart disease, carcinoma (*e.g.*, Walker, basal cell, basosquamous, Brown-Pearce, ductal, Ehrlich tumor, non-small cell lung *(e.g.,* lung squamous cell carcinoma, lung adenocarcinoma and lung undifferentiated large cell carcinoma), oat cell, papillary, bronchiolar, bronchogenic, squamous cell, and transitional cell), histiocytic disorders, leukemia (*e.g.*, B cell, mixed cell, null cell, T cell, T-cell chronic, HTLV-II-associated, lymphocytic acute, lymphocytic chronic, mast cell, and myeloid), histiocytosis malignant, Hodgkin disease, immunoproliferative small, non-Hodgkin lymphoma, plasmacytoma, reticuloendotheliosis, melanoma, chondroblastoma, chondroma, chondrosarcoma, fibroma, fibrosarcoma, giant cell tumors, histiocytoma, lipoma, liposarcoma, mesothelioma, myxoma, myxosarcoma, osteoma, osteosarcoma, Ewing sarcoma, synovioma, adenofibroma, adenolymphoma, carcinosarcoma, chordoma, craniopharyngioma, dysgerminoma, hamartoma, mesenchymoma, mesonephroma, myosarcoma, ameloblastoma, cementoma, odontoma, teratoma, thymoma, trophoblastic tumor, adeno-carcinoma, adenoma, cholangioma, cholesteatoma, cylindroma, cystadenocarcinoma, cystadenoma, granulosa cell tumor, gynandroblastoma, hepatoma, hidradenoma, islet cell tumor, Leydig cell tumor, papilloma, Sertoli cell tumor, theca cell tumor, leiomyoma, leiomyosarcoma, myoblastoma, myosarcoma, rhabdomyoma, rhabdomyosarcoma, ependymoma, ganglioneuroma, glioma, medulloblastoma, meningioma, neurilemmoma, neuroblastoma, neuroepithelioma, neurofibroma, neuroma, paraganglioma, paraganglioma nonchromaffin, angiokeratoma, angiolymphoid hyperplasia with eosinophilia, angioma sclerosing, angiomatosis, glomangioma, hemangioendothelioma, hemangioma, hemangiopericytoma, hemangiosarcoma, lymphangioma, lymphangiomyoma, lymphangiosarcoma, pinealoma, carcinosarcoma, chondrosarcoma, cystosarcoma, phyllodes, fibrosarcoma, hemangiosarcoma, leimyosarcoma, leukosarcoma, liposarcoma, lymphangiosarcoma, myosarcoma, myxosarcoma, ovarian carcinoma, rhabdomyosarcoma, sarcoma (*e.g.,* Ewing, experimental, Kaposi, and mast cell), neurofibromatosis, and cervical dysplasia, and other conditions in which cells have become immortalized or transformed.

**classification**

The term classification refers to a procedure and/or algorithm in which individual items are placed into groups or classes based on quantitative information on one or more characteristics inherent in the items (referred to as traits, variables, characters, features, etc.) and based on a statistical model and/or a training set of previously labeled items. A "classification tree" places categorical variables into classes.

**complement**

"Complement" or "complementary" is used herein to refer to a nucleic acid may mean Watson-Crick (*e.g.,* A-T/U and C-G) or Hoogsteen base pairing between nucleotides or nucleotide analogs of nucleic acid molecules. A full complement or fully complementary means 100% complementary base pairing between nucleotides or nucleotide analogs of nucleic acid molecules. In some embodiments, the complementary sequence has a reverse orientation (5'-3').

Ct

Ct signals represent the first cycle of PCR where amplification crosses a threshold (cycle threshold) of fluorescence. Accordingly, low values of Ct represent high abundance or expression levels of the microRNA.

In some embodiments the PCR Ct signal is normalized such that the normalized Ct remains inversed from the expression level. In other embodiments the PCR Ct signal may be normalized and then inverted such that low normalized-inverted Ct represents low abundance or low expression levels of the microRNA.

**data processing routine**

As used herein, a "data processing routine" refers to a process that can be embodied in software that determines the biological significance of acquired data (*i.e.,* the ultimate results of an assay or analysis). For example, the data processing routine can determine a tissue of origin based upon the data collected. In the systems and methods herein, the data processing routine can also control the data collection routine based upon the results determined. The data processing routine and the data collection routines can be integrated and provide feedback to operate the data acquisition, and hence provide assay-based judging methods.

**data set**

As use herein, the term "data set" refers to numerical values obtained from the analysis. These numerical values associated with analysis may be values such as peak height and area under the curve.

**data structure**

As used herein, the term "data structure" refers to a combination of two or more data sets, an application of one or more mathematical manipulation to one or more data sets to obtain one or more new data sets, or a manipulation of two or more data sets into a form that provides a visual illustration of the data in a new way. An example of a data structure prepared from manipulation of two or more data sets would be a hierarchical cluster.

**detection**

"Detection" means detecting the presence of a component in a sample. Detection also means detecting the absence of a component. Detection also means determining the level of a component, either quantitatively or qualitatively.

**differential expression**

"Differential expression" means qualitative or quantitative differences in the temporal and/or spatial gene expression patterns within and among cells and tissue. Thus, a differentially expressed gene may qualitatively have its expression altered, including an activation or inactivation, in, *e.g.,* normal versus diseased tissue. Genes may be turned on or turned off in a particular state, relative to another state, thus permitting comparison of two or more states. A qualitatively regulated gene may exhibit an expression pattern within a state or cell type which may be detectable by standard techniques. Some genes may be expressed in one state or cell type, but not in both. Alternatively, the difference in expression may be quantitative, *e.g.,* in that expression is modulated: up-regulated, resulting in an increased amount of transcript, or down-regulated, resulting in a decreased amount of transcript. The degree to which expression differs needs only to be large enough to quantify via standard characterization techniques such as expression arrays, quantitative reverse transcriptase PCR, northern blot analysis, real-time PCR, *in situ* hybridization and RNase protection.

**epithelial tumors**

"Epithelial tumors" is meant to include all types of tumors from epithelial origin. Examples of epithelial tumors include, but are not limited to cholangioca or adenoca of extrahepatic biliary tract, urothelial carcinoma, adenocarcinoma of the breast, lung large cell or adenocarcinoma, lung small cell carcinoma, carcinoid, lung, ovarian carcinoma, pancreatic adenocarcinoma, prostatic adenocarcinoma, gastric or esophageal adenocarcinoma, thymoma/thymic carcinoma, follicular thyroid carcinoma, papillary thyroid carcinoma, medullary thyroid carcinoma, anus or skin squamous cell carcinoma, lung, head&neck, or esophagus squamous cell carcinoma, uterine cervix squamous cell carcinoma, gastrointestinal tract carcinoid, pancreatic islet cell tumor and colorectal adenocarcinoma.

**non epithelial tumors**

"Non epithelial tumors" is meant to include all types of tumors from non epithelial origin. Examples of non epithelial tumors include, but are not limited to adrenocortical carcinoma, chromophobe renal cell carcinoma, clear cell renal cell carcinoma, papillary renal cell carcinoma, pleural mesothelioma, astrocytic tumor, oligodendroglioma, pheochromocytoma, B-cell lymphoma, T-cell lymphoma, melanoma, gastrointestinal stromal tumor (GIST), Ewing Sarcoma, chondrosarcoma, malignant fibrous histiocytoma (MFH) or fibrosarcoma, osteosarcoma, rhabdomyosarcoma, synovial sarcoma and liposarcoma.

**expression profile**

The term "expression profile" is used broadly to include a genomic expression profile, *e.g.,* an expression profile of microRNAs. Profiles may be generated by any convenient means for determining a level of a nucleic acid sequence, *e.g.,* quantitative hybridization of microRNA, labeled microRNA, amplified microRNA, cDNA, etc., quantitative PCR, ELISA for quantitation, and the like, and allow the analysis of differential gene expression between two samples. A subject or patient tumor sample, *e.g.,* cells or collections thereof, *e.g.,* tissues, is assayed. Samples are collected by any convenient method, as known in the art. Nucleic acid sequences of interest are nucleic acid sequences that are found to be predictive, including the nucleic acid sequences provided above, where the expression profile may include expression data for 5, 10, 20, 25, 50, 100 or more of the nucleic acid sequences, including all of the listed nucleic acid sequences. According to some embodiments, the term "expression profile" means measuring the relative abundance of the nucleic acid sequences in the measured samples.

**expression ratio**

"Expression ratio", as used herein, refers to relative expression levels of two or more nucleic acids as determined by detecting the relative expression levels of the corresponding nucleic acids in a biological sample.

**FDR (False Discovery Rate)**

When performing multiple statistical tests, for example in comparing between the signal of two groups in multiple data features, there is an increasingly high probability of obtaining false positive results, by random differences between the groups that can reach levels that would otherwise be considered statistically significant. In order to limit the proportion of such false discoveries, statistical significance is defined only for data features in which the differences reached a p-value (by two-sided t-test) below a threshold, which is dependent on the number of tests performed and the distribution of p-values obtained in these tests.

**fragment**

"Fragment" is used herein to indicate a non-full-length part of a nucleic acid. Thus, a fragment is itself also a nucleic acid.

**gastrointestinal tumors**

"gastrointestinal tumors" is meant to include all types of tumors from gastrointestinal origin. Examples of gastrointestinal tumors include, but are not limited to cholangioca. or adenoca of extrahepatic biliary tract, pancreatic adenocarcinoma, gastric or esophageal adenocarcinoma, and colorectal adenocarcinoma.

**gene**

"Gene", as used herein, may be a natural (*e.g.,* genomic) or synthetic gene comprising transcriptional and/or translational regulatory sequences and/or a coding region and/or non-translated sequences (*e.g.,* introns, 5'- and 3'-untranslated sequences). The coding region of a gene may be a nucleotide sequence coding for an amino acid sequence or a functional RNA, such as tRNA, rRNA, catalytic RNA, siRNA, miRNA or antisense RNA. A gene may also be an mRNA or cDNA corresponding to the coding regions (*e.g.,* exons and miRNA) optionally comprising 5'- or 3'-untranslated sequences linked thereto. A gene may also be an amplified nucleic acid molecule produced *in vitro,* comprising all or a part of the coding region and/or 5'- or 3'-untranslated sequences linked thereto.

**germ cell tumors**

"Germ cell tumors" as used herein, include, but are not limited, to non-seminomatous testicular germ cell tumors, seminomatous testicular germ cell tumors and ovarian primitive germ cell tumors.

**groove binder/minor groove binder (MGB)**

"Groove binder" and/or "minor groove binder" may be used interchangeably and refer to small molecules that fit into the minor groove of double-stranded DNA, typically in a sequence-specific manner. Minor groove binders may be long, flat molecules that can adopt a crescent-like shape and thus fit snugly into the minor groove of a double helix, often displacing water. Minor groove binding molecules may typically comprise several aromatic rings connected by bonds with torsional freedom such as furan, benzene, or pyrrole rings. Minor groove binders may be antibiotics such as netropsin, distamycin, berenil, pentamidine and other aromatic diamidines, Hoechst 33258, SN 6999, aureolic anti-tumor drugs such as chromomycin and mithramycin, CC-1065, dihydrocyclopyrroloindole tripeptide ($DPI_3$), 1,2-dihydro-(3H)-pyrrolo[3,2-e]indole-7-carboxylate ($CDPI_3$), and related compounds and analogues, including those described in Nucleic Acids in Chemistry and Biology, 2nd ed., Blackburn and Gait, eds., Oxford University Press, 1996, and PCT Published Application No. WO 03/078450. A minor groove binder may be a component of a primer, a probe, a hybridization tag complement, or combinations thereof. Minor groove binders may increase the $T_m$ of the primer or a probe to which they are attached, allowing such primers or probes to effectively hybridize at higher temperatures.

**high expression miR-205 tumors**

"High expression miR-205 tumors" as used herein include, but are not limited, to urothelial carcinoma (TCC), thymoma/thymic carcinoma, anus or skin squamous cell carcinoma, lung, head&neck, or esophagus squamous cell carcinoma and uterine cervix squamous cell carcinoma.

**low expression 205 tumors**

"Low expression miR-205 tumors" as used herein include, but are not limited, to lung, large cell or adenocarcinoma, follicular thyroid carcinoma and papillary thyroid carcinoma.

**host cell**

"Host cell", as used herein, may be a naturally occurring cell or a transformed cell that may contain a vector and may support replication of the vector. Host cells may be cultured cells, explants, cells *in vivo,* and the like. Host cells may be prokaryotic cells, such as *E. coli,* or eukaryotic cells, such as yeast, insect, amphibian, or mammalian cells, such as CHO and HeLa cells.

**identity**

"Identical" or "identity", as used herein, in the context of two or more nucleic acids or polypeptide sequences mean that the sequences have a specified percentage of residues that are the same over a specified region. The percentage may be calculated by optimally aligning the two sequences, comparing the two sequences over the specified region, determining the number of positions at which the identical residue occurs in both sequences to yield the number of matched positions, dividing the number of matched positions by the total number of positions in the specified region, and multiplying the result by 100 to yield the percentage of sequence identity. In cases where the two sequences are of different lengths or the alignment produces one or more staggered ends and the specified region of comparison includes only a single sequence, the residues of single sequence are included in the denominator but not the numerator of the calculation. When comparing DNA and RNA sequences, thymine (T) and uracil (U) may be considered equivalent. Identity may be performed manually or by using a computer sequence algorithm such as BLAST or BLAST 2.0.

***in situ* detection**

*"In situ* detection", as used herein, means the detection of expression or expression levels in the original site, hereby meaning in a tissue sample such as biopsy.

**K-nearest neighbor**

The phrase "K-nearest neighbor" refers to a classification method that classifies a point by calculating the distances between it and points in the training data set. It then assigns the point to the class that is most common among its K-nearest neighbors (where K is an integer).

**leaf**

A leaf, as used herein, is the terminal group in a classification or decision tree.

**label**

"Label", as used herein, means a composition detectable by spectroscopic, photochemical, biochemical, immunochemical, chemical, or other physical means. For example, useful labels include $^{32}P$, fluorescent dyes, electron-dense reagents, enzymes (e.g., as commonly used in an ELISA), biotin, digoxigenin, or haptens and other entities which can be made detectable. A label may be incorporated into nucleic acids and proteins at any position.

**logistic regression**

Logistic regression is part of a category of statistical models called generalized linear models. Logistic regression can allow one to predict a discrete outcome, such as group membership, from a set of variables that may be continuous, discrete, dichotomous, or a mix of any of these. The dependent or response variable can be dichotomous, for example, one of two possible types of cancer. Logistic regression models the natural log of the odds ratio, *i.e.,* the ratio of the probability of belonging to the first group (*P*) over the probability of belonging to the second group (*1-P*), as a linear combination of the different expression levels (in log-space). The logistic regression output can be used as a classifier by prescribing that a case or sample will be classified into the first type if *P* is greater than 0.5 or 50%. Alternatively, the calculated probability *P* can be used as a variable in other contexts, such as a 1D or 2D threshold classifier.

**metastasis**

"Metastasis" means the process by which cancer spreads from the place at which it first arose as a primary tumor to other locations in the body. The metastatic progression of a primary tumor reflects multiple stages, including dissociation from neighboring primary tumor cells, survival in the circulation, and growth in a secondary location.

**neuroendocrine tumors**

"Neuroendocrine tumors" is meant to include all types of tumors from neuroendocrine origin. Examples of neuroendocrine tumors include, but are not limited to lung small cell carcinoma, lung carcinoid, gastrointestinal tract carcinoid, pancreatic islet cell tumor and medullary thyroid carcinoma.

**node**

A "node" is a decision point in a classification (*i.e.,* decision) tree. Also, a point in a neural net that combines input from other nodes and produces an output through application of an activation function.

**nucleic acid**

"Nucleic acid" or "oligonucleotide" or "polynucleotide", as used herein, mean at least two nucleotides covalently linked together. The depiction of a single strand also defines the sequence of the complementary strand. Thus, a nucleic acid also encompasses the complementary strand of a depicted single strand. Many variants of a nucleic acid may be used for the same purpose as a given nucleic acid. Thus, a nucleic acid also encompasses substantially identical nucleic acids and complements thereof. A single strand provides a probe that may hybridize to a target sequence under stringent hybridization conditions. Thus, a nucleic acid also encompasses a probe that hybridizes under stringent hybridization conditions.

Nucleic acids may be single-stranded or double-stranded, or may contain portions of both double-stranded and single-stranded sequences. The nucleic acid may be DNA, both genomic and cDNA, RNA, or a hybrid, where the nucleic acid may contain combinations of deoxyribo- and ribo-nucleotides, and combinations of bases including uracil, adenine, thymine, cytosine, guanine, inosine, xanthine hypoxanthine, isocytosine and isoguanine. Nucleic acids may be obtained by chemical synthesis methods or by recombinant methods.

A nucleic acid will generally contain phosphodiester bonds, although nucleic acid analogs may be included that may have at least one different linkage, *e.g.,* phosphoramidate, phosphorothioate, phosphorodithioate, or O-methylphosphoroamidite linkages and peptide nucleic acid backbones and linkages. Other analog nucleic acids include those with positive backbones, non-ionic backbones and non-ribose backbones, including those described in U.S. Pat. Nos. 5,235,033 and 5,034,506. Nucleic acids containing one or more non-naturally occurring or modified nucleotides are also included within one definition of nucleic acids. The modified nucleotide analog may be located for example at the 5'-end and/or the 3'-end of the nucleic acid molecule. Representative examples of nucleotide analogs may be selected from sugar- or backbone-modified ribonucleotides. It should be noted, however, that also nucleobase-modified ribonucleotides, i.e., ribonucleotides, containing a non-naturally occurring nucleobase instead of a naturally occurring nucleobase such as uridine or cytidine modified at the 5-position, *e.g.,* 5-(2-amino) propyl uridine, 5-bromo uridine; adenosine and guanosine modified at the 8-position, *e.g.,* 8-bromo guanosine; deaza nucleotides, *e.g.,* 7-deaza-adenosine; O- and N-alkylated nucleotides, e.g., N6-methyl adenosine are suitable. The 2'-OH-group may be replaced by a group selected from H, OR, R, halo, SH, SR, $NH_2$, NHR, $NR_2$ or CN, wherein R is C1-C6 alkyl, alkenyl or alkynyl and halo is F, Cl, Br or I. Modified nucleotides also include nucleotides conjugated with cholesterol through, e.g., a hydroxyprolinol linkage as described in Krutzfeldt et al., Nature 2005; 438:685-689, Soutschek et al., Nature 2004; 432:173-178, and U.S. Patent Publication No. 20050107325. Additional modified nucleotides and nucleic acids are described in U.S. Patent Publication No. 2005/0182005. Modifications of the ribose-phosphate backbone may be done for a variety of reasons, *e.g.,* to increase the stability and half-life of such molecules in physiological environments, to enhance diffusion across cell membranes, or as probes on a biochip. The backbone modification may also enhance resistance to degradation, such as in the harsh endocytic environment of cells. The backbone modification may also reduce nucleic acid clearance by hepatocytes, such as in the liver and kidney. Mixtures of naturally occurring nucleic acids and analogs may be made; alternatively, mixtures of different nucleic acid analogs, and mixtures of naturally occurring nucleic acids and analogs may be made.

**probe**

"Probe", as used herein, means an oligonucleotide capable of binding to a target nucleic acid of complementary sequence through one or more types of chemical bonds, usually through complementary base pairing, usually through hydrogen bond formation. Probes may bind target sequences lacking complete complementarity with the probe sequence depending upon the stringency of the hybridization conditions. There may be any number of base pair mismatches which will interfere with hybridization between the target sequence and the single-stranded nucleic acids described herein. However, if the number of mutations is so great that no hybridization can occur under even the least stringent of hybridization conditions, the sequence is not a complementary target sequence. A probe may be single-stranded or partially single- and partially double-stranded. The strandedness of the probe is dictated by the structure, composition, and properties of the target sequence. Probes may be directly labeled or indirectly labeled such as with biotin to which a streptavidin complex may later bind.

**reference value**

As used herein, the term "reference value" or "reference expression profile" refers to a criterion expression value to which measured values are compared in order to identify a specific cancer. The reference value may be based on the abundance of the nucleic acids, or may be based on a combined metric score thereof.

In preferred embodiments the reference value is determined from statistical analysis of studies that compare microRNA expression with known clinical outcomes.

**sarcoma**

Sarcoma is meant to include all types of tumors from sarcoma origin. Examples of sarcoma tumors include, but are not limited to gastrointestinal stromal tumor (GIST), Ewing sarcoma, chondrosarcoma, malignant fibrous histiocytoma (MFH) or fibrosarcoma, osteosarcoma, rhabdomyosarcoma, synovial sarcoma and liposarcoma.

**sensitivity**

"Sensitivity", as used herein, may mean a statistical measure of how well a binary classification test correctly identifies a condition, for example, how frequently it correctly classifies a cancer into the correct class out of two possible classes. The sensitivity for class A is the proportion of cases that are determined to belong to class "A" by the test out of the cases that are in class "A", as determined by some absolute or gold standard.

**specificity**

"Specificity", as used herein, may mean a statistical measure of how well a binary classification test correctly identifies a condition, for example, how frequently it correctly classifies a cancer into the correct class out of two possible classes. The specificity for class A is the proportion of cases that are determined to belong to class "not A" by the test out of the cases that are in class "not A", as determined by some absolute or gold standard.

**stringent hybridization conditions**

"Stringent hybridization conditions", as used herein, mean conditions under which a first nucleic acid sequence (*e.g.,* probe) will hybridize to a second nucleic acid sequence (*e.g.,* target), such as in a complex mixture of nucleic acids. Stringent conditions are sequence-dependent and will be different in different circumstances. Stringent conditions may be selected to be about 5-10°C lower than the thermal melting point ($T_m$) for the specific sequence at a defined ionic strength pH. The $T_m$ may be the temperature (under defined ionic strength, pH, and nucleic concentration) at which 50% of the probes complementary to the target hybridize to the target sequence at equilibrium (as the target sequences are present in excess, at $T_m$, 50% of the probes are occupied at equilibrium). Stringent conditions may be those in which the salt concentration is less than about 1.0 M sodium ion, such as about 0.01-1.0 M sodium ion concentration (or other salts) at pH 7.0 to 8.3 and the temperature is at least about 30°C for short probes (*e.g.,* about 10-50 nucleotides) and at least about 60°C for long probes (*e.g.,* greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C.

**substantially complementary**

"Substantially complementary", as used herein, means that a first sequence is at least 60%, 65%, 70%, 75%,

80%, 85%, 90%, 95%, 97%, 98% or 99% identical to the complement of a second sequence over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleotides, or that the two sequences hybridize under stringent hybridization conditions.

**substantially identical**

"Substantially identical", as used herein, means that a first and a second sequence are at least 60%, 65%, 70%, 75%, 80%, 85%, 90%, 95%, 97%, 98% or 99% identical over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleotides or amino acids, or with respect to nucleic acids, if the first sequence is substantially complementary to the complement of the second sequence.

**subject**

As used herein, the term "subject" refers to a mammal, including both human and other mammals. The methods of the present invention are preferably applied to human subjects.

**target nucleic acid**

"Target nucleic acid", as used herein, means a nucleic acid or variant thereof that may be bound by another nucleic acid. A target nucleic acid may be a DNA sequence. The target nucleic acid may be RNA. The target nucleic acid may comprise a mRNA, tRNA, shRNA, siRNA or Piwi-interacting RNA, or a pri-miRNA, pre-miRNA, miRNA, or anti-miRNA.

The target nucleic acid may comprise a target miRNA binding site or a variant thereof. One or more probes may bind the target nucleic acid. The target binding site may comprise 5-100 or 10-60 nucleotides. The target binding site may comprise a total of 5, 6, 7, 8, 9, 10, 11, 12, 13, 14, 15,16, 17, 18, 19, 20, 21, 22, 23, 24, 25, 26, 27, 28, 29, 30-40, 40-50, 50-60, 61, 62 or 63 nucleotides. The target site sequence may comprise at least 5 nucleotides of the sequence of a target miRNA binding site disclosed in U.S. Patent Publications Nos. US 2008/0182237 and US 2007/0259352.

**1D/2D threshold classifier**

"1D/2D threshold classifier", as used herein, may mean an algorithm for classifying a case or sample such as a cancer sample into one of two possible types such as two types of cancer. For a 1D threshold classifier, the decision is based on one variable and one predetermined threshold value; the sample is assigned to one class if the variable exceeds the threshold and to the other class if the variable is less than the threshold. A 2D threshold classifier is an algorithm for classifying into one of two types based on the values of two variables. A threshold may be calculated as a function (usually a continuous or even a monotonic function) of the first variable; the decision is then reached by comparing the second variable to the calculated threshold, similar to the 1D threshold classifier.

**tissue sample**

As used herein, a tissue sample is tissue obtained from a tissue biopsy using methods well known to those of ordinary skill in the related medical arts. The phrase "suspected of being cancerous", as used herein, means a cancer tissue sample believed by one of ordinary skill in the medical arts to contain cancerous cells. Methods for obtaining the sample from the biopsy include gross apportioning of a mass, microdissection, laser-based microdissection, or other art-known cell-separation methods.

**tumor**

"Tumor", as used herein, refers to all neoplastic cell growth and proliferation, whether malignant or benign, and all pre-cancerous and cancerous cells and tissues.

**variant**

"Variant", as used herein, referring to a nucleic acid means (i) a portion of a referenced nucleotide sequence; (ii) the complement of a referenced nucleotide sequence or portion thereof; (iii) a nucleic acid that is substantially identical to a referenced nucleic acid or the complement thereof; or (iv) a nucleic acid that hybridizes under stringent conditions to the referenced nucleic acid, complement thereof, or a sequence substantially identical thereto.

**wild type**

As used herein, the term "wild-type" sequence refers to a coding, a non-coding or an interface sequence which is an allelic form of sequence that performs the natural or normal function for that sequence. Wild-type sequences include multiple allelic forms of a cognate sequence, for example, multiple alleles of a wild type sequence may encode silent or conservative changes to the protein sequence that a coding sequence encodes.

The present invention employs miRNAs for the identification, classification and diagnosis of specific cancers and the identification of their tissues of origin.

**1. microRNA processing**

A gene coding for microRNA (miRNA) may be transcribed leading to production of a miRNA primary transcript known as the pri-miRNA. The pri-miRNA may comprise a hairpin with a stem and loop structure. The stem of the hairpin may comprise mismatched bases. The pri-miRNA may comprise several hairpins in a polycistronic structure.

The hairpin structure of the pri-miRNA may be recognized by Drosha, which is an RNase III endonuclease. Drosha may recognize terminal loops in the pri-miRNA and cleave approximately two helical turns into the stem to produce a 60-70 nt precursor known as the pre-miRNA. Drosha may cleave the pri-miRNA with a staggered cut typical of RNase III endonucleases yielding a pre-miRNA stem loop with a 5' phosphate and ~2 nucleotide 3' overhang. Approximately one helical turn of stem (~10 nucleotides) extending beyond the Drosha cleavage site may be essential for efficient processing. The pre-miRNA may then be actively transported from the nucleus to the cytoplasm by Ran-GTP and the export receptor Ex-portin-5.

The pre-miRNA may be recognized by Dicer, which is also an RNase III endonuclease. Dicer may recognize the double-stranded stem of the pre-miRNA. Dicer may also cut off the terminal loop two helical turns away from the base of the stem loop, leaving an additional 5' phosphate and a~2 nucleotide 3' overhang. The resulting siRNA-like duplex, which may comprise mismatches, comprises the mature miRNA and a similar-sized fragment known as the miRNA*. The miRNA and miRNA* maybe derived from opposing arms of the pri-miRNA and pre-miRNA. MiRNA* sequences may be found in libraries of cloned miRNAs, but typically at lower frequency than the miRNAs.

Although initially present as a double-stranded species with miRNA*, the miRNA may eventually become incorporated as a single-stranded RNA into a ribonucleoprotein complex known as the RNA-induced silencing complex (RISC). Various proteins can form the RISC, which can lead to variability in specificity for miRNA/miRNA* duplexes, binding site of the target gene, activity of miRNA (repress or activate), and which strand of the miRNA/miRNA* duplex is loaded in to the RISC.

When the miRNA strand of the miRNA:miRNA* duplex is loaded into the RISC, the miRNA* may be removed and degraded. The strand of the miRNA:miRNA* duplex that is loaded into the RISC may be the strand whose 5' end is less tightly paired. In cases where both ends of the miRNA:miRNA* have roughly equivalent 5' pairing, both miRNA and miRNA* may have gene silencing activity.

The RISC may identify target nucleic acids based on high levels of complementarity between the miRNA and the mRNA, especially by nucleotides 2-7 of the miRNA. Only one case has been reported in animals where the interaction between the miRNA and its target was along the entire length of the miRNA. This was shown for miR-196 and Hox B8 and it was further shown that miR-196 mediates the cleavage of the Hox B8 mRNA (Yekta et al. Science 2004; 304:594-596). Otherwise, such interactions are known only in plants (Bartel & Bartel 2003; 132:709-717).

A number of studies have looked at the base-pairing requirement between miRNA and its mRNA target for achieving efficient inhibition of translation (reviewed by Bartel 2004;116:281-297). In mammalian cells, the first 8 nucleotides of the miRNA may be important (Doench & Sharp GenesDev 2004; 18:504-511). However, other parts of the microRNA may also participate in mRNA binding. Moreover, sufficient base pairing at the 3' can compensate for insufficient pairing at the 5' (Brennecke et al., PloS Biol 2005; 3:e85). Computation studies, analyzing miRNA binding on whole genomes have suggested a specific role for bases 2-7 at the 5' of the miRNA in target binding but the role of the first nucleotide, found usually to be "A" was also recognized (Lewis et al. Cell 2005; 120:15-20). Similarly, nucleotides 1-7 or 2-8 were used to identify and validate targets by Krek et al. (Nat Genet 2005; 37:495-500).

The target sites in the mRNA may be in the 5' UTR, the 3' UTR or in the coding region. Interestingly, multiple miRNAs may regulate the same mRNA target by recognizing the same or multiple sites. The presence of multiple miRNA binding sites in most genetically identified targets may indicate that the cooperative action of multiple RISCs provides the most efficient translational inhibition.

miRNAs may direct the RISC to down-regulate gene expression by either of two mechanisms: mRNA cleavage or translational repression. The miRNA may specify cleavage of the mRNA if the mRNA has a certain degree of complementarity to the miRNA. When a miRNA guides cleavage, the cut may be between the nucleotides pairing to residues 10 and 11 of the miRNA. Alternatively, the miRNA may repress translation if the miRNA does not have the requisite degree of complementarity to the miRNA. Translational repression may be more prevalent in animals since animals may have a lower degree of complementarity between the miRNA and binding site.

It should be noted that there may be variability in the 5' and 3' ends of any pair of miRNA and miRNA*. This variability may be due to variability in the enzymatic processing of Drosha and Dicer with respect to the site of cleavage. Variability at the 5' and 3' ends of miRNA and miRNA* may also be due to mismatches in the stem structures of the pri-miRNA and pre-miRNA. The mismatches of the stem strands may lead to a population of different hairpin structures. Variability in the stem structures may also lead to variability in the products of cleavage by Drosha and Dicer.

## 2. Nucleic Acids

Nucleic acids are provided herein. The nucleic acids comprise the sequences of SEQ ID NOS: 1-390 or variants thereof. The variant may be a complement of the referenced nucleotide sequence. The variant may also be a nucleotide sequence that is substantially identical to the referenced nucleotide sequence or the complement thereof. The variant may also be a nucleotide sequence which hybridizes under stringent conditions to the referenced nucleotide sequence, complements thereof, or nucleotide sequences substantially identical thereto.

The nucleic acid may have a length of from about 10 to about 250 nucleotides. The nucleic acid may have a length of at least 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 125, 150, 175, 200 or 250 nucleotides. The nucleic acid may be synthesized or expressed in a cell (*in vitro* or *in vivo*) using a synthetic gene described herein. The nucleic acid may be synthesized as a single-strand molecule and hybridized to a substantially complementary nucleic acid to form a duplex. The nucleic acid may be introduced to a cell, tissue or organ in a single- or double-stranded form or capable of being expressed by a synthetic gene using methods well known to those skilled in the art, including as described in U.S. Patent No. 6,506,559.

**Table 1:** SEQ ID NOS of sequences used in the invention

| SEQ ID NOs 1-34 are in accordance with Sanger database version 10; SEQ ID NOs 35-390 are in accordance with Sanger database version 11; | | |
|---|---|---|
| **hairpin SEQ ID NO** | **miR SEQ ID NO** | **miR name** |
| 70 | 1 | hsa-let-7c |
| 71 | 2, 156 | hsa-let-7e |
| 72 | 3 | hsa-miR-100 |
| 73 | 4 | hsa-miR-10a |
| 74 | 5 | hsa-miR-10b |
| 75 | 6 | hsa-miR-122 |
| 76 | 7 | hsa-miR-125a-5p |
| 77,78 | 8 | hsa-miR-125b |
| 79 | 9 | hsa-miR-126 |
| 80 | 10 | hsa-miR-130a |
| 81, 82 | 11 | hsa-miR-138 |
| 83 | 12 | hsa-miR-140-3p |
| 84 | 13 | hsa-miR-141 |
| 85 | 14 | hsa-miR-143 |
| 86 | 15 | hsa-miR-145 |
| 87 | 16 | hsa-miR-146a |
| 88 | 17 | hsa-miR-146b-5p |
| 89 | 18 | hsa-miR-148a |
| 90 | 19 | hsa-miR-149 |
| 91 | 20 | hsa-miR-17 |

(continued)

| hairpin SEQ ID NO | miR SEQ ID NO | miR name |
|---|---|---|
| 92, 93 | 21 | hsa-miR-181a |
| 92, 93 | 22 | hsa-miR-181a* |
| 94 | 23 | hsa-miR-185 |
| 95 | 24 | hsa-miR-191 |
| 96 | 25 | hsa-miR-193a-3p |
| 96 | 26 | hsa-miR-193a-5p |
| 97, 98 | 27 | hsa-miR-194 |
| 99 | 28 | hsa-miR-200a |
| 100 | 29 | hsa-miR-200b |
| 101 | 30 | hsa-miR-200c |
| 102 | 31 | hsa-miR-202 |
| 103 | 32 | hsa-miR-205 |
| 104 | 33 | hsa-miR-206 |
| 105 | 34 | hsa-miR-21 |
| 105 | 35 | hsa-miR-21* |
| 106 | 36 | hsa-miR-210 |
| 107 | 37 | hsa-miR-214 |
| 107 | 38 | hsa-miR-214* |
| 108 | 39 | hsa-miR-22 |
| 109 | 40 | hsa-miR-222 |
| 110 | 41 | hsa-miR-223 |
| 111 | 42 | hsa-miR-224 |
| 112 | 43 | hsa-miR-29a |
| 113 | 44,191 | hsa-miR-29c |
| 113 | 45 | hsa-miR-29c* |
| 114 | 46 | hsa-miR-30a |
| 115 | 47 | hsa-miR-30d |
| 116 | 48 | hsa-miR-30e |
| 117 | 49 | hsa-miR-31 |
| 118 | 50 | hsa-miR-342-3p |
| 119 | 51 | hsa-miR-345 |
| 120 | 52 | hsa-miR-34a |
| 121 | 53 | hsa-miR-34c-5p |
| 122 | 54 | hsa-miR-361-5p |
| 123 | 55 | hsa-miR-372 |
| 124 | 56 | hsa-miR-375 |
| 125 | 57,202 | hsa-miR-378 |
| 126 | 58 | hsa-miR-455-5p |

(continued)

| hairpin SEQ ID NO | miR SEQ ID NO | miR name |
|---|---|---|
| 127 | 59 | hsa-miR-487b |
| 128 | 60,208 | hsa-miR-497 |
| 129, 130, 131 | 61 | hsa-miR-509-3p |
| 132, 133 | 62,211 | hsa-miR-516a-5p |
| 134 | 63 | hsa-miR-574-5p |
| 135 | 64 | hsa-miR-652 |
| 136, 137, 138 | 65 | hsa-miR-7 |
| 139, 140, 141 | 66 | hsa-miR-9* |
| 142, 143 | 67 | hsa-miR-92a |
| 144 | 68 | hsa-miR-92b |
| 145 | 69 | hsa-miR-934 |
| 149 | 146 | hsa-miR-1201 |
| 150 | 147 | hsa-miR-221 |
| 151 | 148 | hsa-miR-93 |
| | 152 | hsa-miR-182 |
| | 153 | hsa-let-7d |
| | 154 | hsa-miR-181b |
| | 155 | hsa-miR-127-3p |
| | 157 | hsa-let-7i |
| | 158 | hsa-miR-106a |
| | 159 | hsa-miR-124 |
| | 160 | hsa-miR-1248 |
| | 161 | hsa-miR-128 |
| | 162 | hsa-miR-129-3p |
| | 163 | hsa-miR-1323 |
| | 164 | hsa-miR-142-5p |
| | 165 | hsa-miR-143* |
| | 166 | hsa-miR-146b-3p |
| | 167 | hsa-miR-149* |
| | 168 | hsa-miR-150 |
| | 169 | hsa-miR-152 |
| | 170 | hsa-miR-155 |
| | 171 | hsa-miR-15a |
| | 172 | hsa-miR-15b |
| | 173 | hsa-miR-181c |
| | 174 | hsa-miR-181d |
| | 175 | hsa-miR-183 |
| | 176 | hsa-miR-18a |

(continued)

| hairpin SEQ ID NO | miR SEQ ID NO | miR name |
|---|---|---|
| | 177 | hsa-miR-192 |
| | 178 | hsa-miR-193b |
| | 179 | hsa-miR-195 |
| | 180 | hsa-miR-1973 |
| | 181 | hsa-miR-199a-3p |
| | 182 | hsa-miR-199a-5p |
| | 183 | hsa-miR-199b-5p |
| | 184 | hsa-miR-203 |
| | 185 | hsa-miR-205* |
| | 186 | hsa-miR-20a |
| | 187 | hsa-miR-219-2-3p |
| | 188 | hsa-miR-25 |
| | 189 | hsa-miR-27b |
| | 190 | hsa-miR-29b |
| | 192 | hsa-miR-302a |
| | 193 | hsa-miR-302a* |
| | 194 | hsa-miR-302d |
| | 195 | hs a-miR-30a* |
| | 196 | hsa-miR-30c |
| | 197 | hsa-miR-331-3p |
| | 198 | hsa-miR-342-5p |
| | 199 | hsa-miR-363 |
| | 200 | hsa-miR-371-3p |
| | 201 | hsa-miR-371-Sp |
| | 203 | hsa-miR-422a |
| | 204 | hsa-miR-425 |
| | 205 | hsa-miR-451 |
| | 206 | hsa-miR-455-3p |
| | 207 | hsa-miR-486-5p |
| | 209 | hsa-miR-498 |
| | 210 | hsa-miR-512-5p |
| | 212 | hsa-miR-516b |
| | 213 | hsa-miR-517a |
| | 214 | hsa-miR-517c |
| | 215 | hsa-miR-518a-3p |
| | 216 | hsa-miR-518e |
| | 217 | hsa-miR-518f* |
| | 218 | hsa-miR-519a |

(continued)

| hairpin SEQ ID NO | miR SEQ ID NO | miR name |
|---|---|---|
| | 219 | hsa-miR-519d |
| | 220 | hsa-miR-520a-5p |
| | 221 | hsa-miR-520c-3p |
| | 222 | hsa-miR-520d-5p |
| | 223 | hsa-miR-524-5p |
| | 224 | hsa-miR-527 |
| | 225 | hsa-miR-551b |
| | 226 | hsa-miR-625 |
| | 227 | hsa-miR-767-5p |
| | 228 | hsa-miR-886-3p |
| | 229 | hsa-miR-9 |
| | 230 | hsa-miR-886-5p |
| | 231 | hsa-miR-99a |
| | 232 | hsa-miR-99a* |
| | 233 | hsa-miR-373 |
| | 234 | hsa-miR-1977 |
| | 235 | hsa-miR-1978 |
| | 236 | MID-00689 |
| | 237,369 | MID-15684 |
| | 238 | MID-15867 |
| | 239 | MID-15907 |
| | 240 | MID-15965 |
| | 241 | MID-16318 |
| | 242 | MID-16489 |
| | 243 | MID-16869 |
| | 244 | MID-17144 |
| | 245 | MID-18336 |
| | 246 | MID-18422 |
| | 247 | MID-19340 |
| | 248 | MID-19533 |
| | 249 | MID-20524 |
| | 250 | MID-20703 |
| | 251 | MID-21271 |
| | 252 | MID-22664 |
| | 253 | MID-23256 |
| | 254 | MID-23291 |
| | 255 | MID-23794 |
| | 390 | MID-00405 |

(continued)

| hairpin SEQ ID NO | miR SEQ ID NO | miR name |
|---|---|---|
| | 256 | hsa-let-7a |
| | 257 | hsa-let-7b |
| | 258 | hsa-let-7f |
| | 259 | hsa-let-7g |
| | 260 | hsa-miR-106b |
| | 261 | hsa-miR-1180 |
| | 262 | hsa-miR-127-5p |
| | 263 | hsa-miR-129* |
| | 264 | hsa-miR-129-5p |
| | 265 | hsa-miR-130b |
| | 266 | hsa-miR-132 |
| | 267 | hsa-miR-133a |
| | 268 | hsa-miR-133b |
| | 269 | hsa-miR-134 |
| | 270 | hsa-miR-139-5p |
| | 271 | hsa-miR-140-5p |
| | 272 | hsa-miR-145* |
| | 273 | hsa-miR-148b |
| | 274 | hsa-miR-151-3p |
| | 275 | hsa-miR-154 |
| | 276 | hsa-miR-154* |
| | 277 | hsa-miR-17* |
| | 278 | hsa-miR-181a-2* |
| | 279 | hsa-miR-1826 |
| | 280 | hsa-miR-187 |
| | 281 | hsa-miR-188-5p |
| | 282 | hsa-miR-196a |
| | 283 | hsa-miR-1979 |
| | 284 | hsa-miR-19b |
| | 285 | hsa-miR-20b |
| | 286 | hsa-miR-216a |
| | 287 | hsa-miR-216b |
| | 288 | hsa-miR-217 |
| | 289 | hsa-miR-22* |
| | 290 | hsa-miR-221* |
| | 291 | hsa-miR-222* |
| | 292 | hsa-miR-23a |
| | 293 | hsa-miR-23b |

(continued)

| hairpin SEQ ID NO | miR SEQ ID NO | miR name |
|---|---|---|
| | 294 | hsa-miR-24 |
| | 295 | hsa-miR-26a |
| | 296 | hsa-miR-26b |
| | 297 | hsa-miR-27a |
| | 298 | hsa-miR-28-3p |
| | 299 | hsa-miR-296-5p |
| | 300 | hsa-miR-299-3p |
| | 301 | hsa-miR-29b-2* |
| | 302 | hsa-miR-301a |
| | 303 | hsa-miR-30b |
| | 304 | hsa-miR-30e* |
| | 305 | hsa-miR-31 * |
| | 306 | hsa-miR-323-3p |
| | 307 | hsa-miR-324-5p |
| | 308 | hsa-miR-328 |
| | 309 | hsa-miR-329 |
| | 310 | hsa-miR-330-3p |
| | 311 | hsa-miR-335 |
| | 312 | hsa-miR-337-5p |
| | 313 | hsa-miR-338-3p |
| | 314 | hsa-miR-361-3p |
| | 315 | hsa-miR-362-3p |
| | 316 | hsa-miR-362-5p |
| | 317 | hsa-miR-369-5p |
| | 318 | hsa-miR-370 |
| | 319 | hsa-miR-376a |
| | 320 | hsa-miR-376c |
| | 321 | hsa-miR-377* |
| | 322 | hsa-miR-379 |
| | 323 | hsa-miR-381 |
| | 324 | hsa-miR-382 |
| | 325 | hsa-miR-409-3p |
| | 326 | hsa-miR-409-5p |
| | 327 | hsa-miR-410 |
| | 328 | hsa-miR-411 |
| | 329 | hsa-miR-425* |
| | 330 | hsa-miR-431* |
| | 331 | hsa-miR-432 |

(continued)

| hairpin SEQ ID NO | miR SEQ ID NO | miR name |
|---|---|---|
| | 332 | hsa-miR-433 |
| | 333 | hsa-miR-483-3p |
| | 334 | hsa-miR-483-5p |
| | 335 | hsa-miR-485-3p |
| | 336 | hsa-miR-485-5p |
| | 337 | hsa-miR-487a |
| | 338 | hsa-miR-494 |
| | 339 | hsa-miR-495 |
| | 340 | hsa-miR-500 |
| | 341 | hsa-miR-500* |
| | 342 | hsa-miR-501-3p |
| | 343 | hsa-miR-502-3p |
| | 344 | hsa-miR-503 |
| | 345 | hsa-miR-506 |
| | 346 | hsa-miR-509-3-5p |
| | 347 | hsa-miR-513a-5p |
| | 348 | hsa-miR-532-3p |
| | 349 | hsa-miR-532-5p |
| | 350 | hsa-miR-539 |
| | 351 | hsa-miR-542-5p |
| | 352 | hsa-miR-543 |
| | 353 | hsa-miR-598 |
| | 354 | hsa-miR-612 |
| | 355 | hsa-miR-654-3p |
| | 356 | hsa-miR-658 |
| | 357 | hsa-miR-660 |
| | 358 | hsa-miR-665 |
| | 359 | hsa-miR-708 |
| | 360 | hsa-miR-873 |
| | 361 | hsa-miR-874 |
| | 362 | hsa-miR-891a |
| | 363 | hsa-miR-99b |
| | 364 | MID-00064 |
| | 365 | MID-00078 |
| | 366 | MID-00144 |
| | 367 | MID-00465 |
| | 368 | MID-00672 |
| | 370 | MID-15986 |

(continued)

| hairpin SEQ ID NO | miR SEQ ID NO | miR name |
|---|---|---|
| | 371 | MID-16270 |
| | 372 | MID-16469 |
| | 373 | MID-16582 |
| | 374 | MID-16748 |
| | 389 | MID-17356 (3651) |
| | 375 | MID-17375 |
| | 376 | MID-17576 |
| | 377 | MID-17866 |
| | 378 | MID-18307 |
| | 379 | MID-18395 |
| | 380 | MID-19898 |
| | 381 | MID-19962 |
| | 382 | MID-22331 |
| | 383 | MID-22912 |
| | 384 | MID-23017 |
| | 385 | MID-23168 |
| | 386 | MID-23178 |
| | 387 | MID-23751 |
| | 388 | hsa-miR-423-5p |

**3. Nucleic acid complexes**

The nucleic acid may further comprise one or more of the following: a peptide, a protein, a RNA-DNA hybrid, an antibody, an antibody fragment, a Fab fragment, and an aptamer.

**4. Pri-miRNA**

The nucleic acid may comprise a sequence of a pri-miRNA or a variant thereof. The pri-miRNA sequence may comprise from 45-30,000, 50-25,000, 100-20,000, 1,000-1,500 or 80-100 nucleotides. The sequence of the pri-miRNA may comprise a pre-miRNA, miRNA and miRNA*, as set forth herein, and variants thereof. The sequence of the pri-miRNA may comprise any of the sequences of SEQ ID NOS: 1-390 or variants thereof.

The pri-miRNA may comprise a hairpin structure. The hairpin may comprise a first and a second nucleic acid sequence that are substantially complimentary. The first and second nucleic acid sequence may be from 37-50 nucleotides. The first and second nucleic acid sequence may be separated by a third sequence of from 8-12 nucleotides. The hairpin structure may have a free energy of less than -25 Kcal/mole, as calculated by the Vienna algorithm with default parameters, as described in Hofacker et al. (Monatshefte f. Chemie 1994; 125:167-188). The hairpin may comprise a terminal loop of 4-20, 8-12 or 10 nucleotides. The pri-miRNA may comprise at least 19% adenosine nucleotides, at least 16% cytosine nucleotides, at least 23% thymine nucleotides and at least 19% guanine nucleotides.

**5. Pre-miRNA**

The nucleic acid may also comprise a sequence of a pre-miRNA or a variant thereof. The pre-miRNA sequence may comprise from 45-90, 60-80 or 60-70 nucleotides. The sequence of the pre-miRNA may comprise a miRNA and a miRNA* as set forth herein. The sequence of the pre-miRNA may also be that of a pri-miRNA excluding from 0-160 nucleotides from the 5' and 3' ends of the pri-miRNA. The sequence of the pre-miRNA may comprise the sequence of SEQ ID NOS: 1-390 or variants thereof.

### 6. miRNA

The nucleic acid may also comprise a sequence of a miRNA (including miRNA*) or a variant thereof. The miRNA sequence may comprise from 13-33, 18-24 or 21-23 nucleotides. The miRNA may also comprise a total of at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides. The sequence of the miRNA may be the first 13-33 nucleotides of the pre-miRNA. The sequence of the miRNA may also be the last 13-33 nucleotides of the pre-miRNA. The sequence of the miRNA may comprise the sequence of SEQ ID NOS: 1-69, 146-148, 152-390 or variants thereof.

### 7. Probes

A probe comprising a nucleic acid described herein is also provided. Probes may be used for screening and diagnostic methods, as outlined below. The probe may be attached or immobilized to a solid substrate, such as a biochip.

The probe may have a length of from 8 to 500, 10 to 100 or 20 to 60 nucleotides. The probe may also have a length of at least 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100, 120, 140, 160, 180, 200, 220, 240, 260, 280 or 300 nucleotides. The probe may further comprise a linker sequence of from 10-60 nucleotides. The probe may comprise a nucleic acid that is complementary to a sequence selected from the group consisting of SEQ ID NOS: 1-390 or variants thereof.

### 8. Biochip

A biochip is also disclosed. The biochip may comprise a solid substrate comprising an attached probe or plurality of probes described herein. The probes may be capable of hybridizing to a target sequence under stringent hybridization conditions. The probes may be attached at spatially defined addresses on the substrate. More than one probe per target sequence may be used, with either overlapping probes or probes to different sections of a particular target sequence. The probes may be capable of hybridizing to target sequences associated with a single disorder appreciated by those in the art. The probes may either be synthesized first, with subsequent attachment to the biochip, or may be directly synthesized on the biochip.

The solid substrate may be a material that may be modified to contain discrete individual sites appropriate for the attachment or association of the probes and is amenable to at least one detection method. Representative examples of substrates include glass and modified or functionalized glass, plastics (including acrylics, polystyrene and copolymers of styrene and other materials, polypropylene, polyethylene, polybutylene, polyurethanes, TeflonJ, etc.), polysaccharides, nylon or nitrocellulose, resins, silica or silica-based materials including silicon and modified silicon, carbon, metals, inorganic glasses and plastics. The substrates may allow optical detection without appreciably fluorescing.

The substrate may be planar, although other configurations of substrates may be used as well. For example, probes may be placed on the inside surface of a tube, for flow-through sample analysis to minimize sample volume. Similarly, the substrate may be flexible, such as flexible foam, including closed cell foams made of particular plastics.

The biochip and the probe may be derivatized with chemical functional groups for subsequent attachment of the two. For example, the biochip may be derivatized with a chemical functional group including, but not limited to, amino groups, carboxyl groups, oxo groups or thiol groups. Using these functional groups, the probes may be attached using functional groups on the probes either directly or indirectly using a linker. The probes may be attached to the solid support by either the 5' terminus, 3' terminus, or via an internal nucleotide.

The probe may also be attached to the solid support non-covalently. For example, biotinylated oligonucleotides can be made, which may bind to surfaces covalently coated with streptavidin, resulting in attachment. Alternatively, probes may be synthesized on the surface using techniques such as photopolymerization and photolithography.

### 9. Diagnostics

As used herein, the term "diagnosing" refers to classifying pathology, or a symptom, determining a severity of the pathology (e.g., grade or stage), monitoring pathology progression, forecasting an outcome of pathology and/or prospects of recovery.

As used herein, the phrase "subject in need thereof" refers to an animal or human subject who is known to have cancer, at risk of having cancer (*e.g.,* a genetically predisposed subject, a subject with medical and/or family history of cancer, a subject who has been exposed to carcinogens, occupational hazard, environmental hazard) and/or a subject who exhibits suspicious clinical signs of cancer (*e.g.*, blood in the stool or melena, unexplained pain, sweating, unexplained fever, unexplained loss of weight up to anorexia, changes in bowel habits (constipation and/or diarrhea), tenesmus (sense of incomplete defecation, for rectal cancer specifically), anemia and/or general weakness). Additionally or alternatively, the subject in need thereof can be a healthy human subject undergoing a routine well-being check up.

Analyzing presence of malignant or pre-malignant cells can be effected *in vivo* or *ex vivo,* whereby a biological sample (*e.g.,* biopsy, blood) is retrieved. Such biopsy samples comprise cells and may be an incisional or excisional biopsy. Alternatively, the cells may be retrieved from a complete resection.

While employing the present teachings, additional information may be gleaned pertaining to the determination of treatment regimen, treatment course and/or to the measurement of the severity of the disease.

As used herein the phrase "treatment regimen" refers to a treatment plan that specifies the type of treatment, dosage, follow-up plans, schedule and/or duration of a treatment provided to a subject in need thereof (*e.g.,* a subject diagnosed with a pathology). The selected treatment regimen can be an aggressive one which is expected to result in the best clinical outcome (*e.g.*, complete cure of the pathology) or a more moderate one which may relieve symptoms of the pathology yet results in incomplete cure of the pathology. It will be appreciated that in certain cases the treatment regimen may be associated with some discomfort to the subject or adverse side effects (*e.g.,* damage to healthy cells or tissue). The type of treatment can include a surgical intervention (*e.g.,* removal of lesion, diseased cells, tissue, or organ), a cell replacement therapy, an administration of a therapeutic drug (*e.g.,* receptor agonists, antagonists, hormones, chemotherapy agents) in a local or a systemic mode, an exposure to radiation therapy using an external source (*e.g.,* external beam) and/or an internal source (*e.g.,* brachytherapy) and/or any combination thereof. The dosage, schedule and duration of treatment can vary, depending on the severity of pathology and the selected type of treatment, and those of skill in the art are capable of adjusting the type of treatment with the dosage, schedule and duration of treatment.

A method of diagnosis is also disclosed. The method comprises detecting an expression level of a specific cancer-associated nucleic acid in a biological sample. The sample may be derived from a patient. Diagnosis of a specific cancer state in a patient may allow for prognosis and selection of therapeutic strategy. Further, the developmental stage of cells may be classified by determining temporarily expressed specific cancer-associated nucleic acids.

*In situ* hybridization of labeled probes to tissue arrays may be performed. When comparing the fingerprints between individual samples the skilled artisan can make a diagnosis, a prognosis, or a prediction based on the findings. It is further understood that the nucleic acid sequences which indicate the diagnosis may differ from those which indicate the prognosis and molecular profiling of the condition of the cells or exosomes may lead to distinctions between responsive or refractory conditions or maybe predictive of outcomes.

### 10. Kits

A kit is also disclosed and may comprise a nucleic acid described herein together with any or all of the following: assay reagents, buffers, probes and/or primers, and sterile saline or another pharmaceutically acceptable emulsion and suspension base. In addition, the kits may include instructional materials containing directions (*e.g.,* protocols) for the practice of the methods described herein. The kit may further comprise a software package for data analysis of expression profiles.

For example, the kit may be a kit for the amplification, detection, identification or quantification of a target nucleic acid sequence. The kit may comprise a poly (T) primer, a forward primer, a reverse primer, and a probe.

Any of the compositions described herein may be comprised in a kit. In a nonlimiting example, reagents for isolating miRNA, labeling miRNA, and/or evaluating a miRNA population using an array are included in a kit. The kit may further include reagents for creating or synthesizing miRNA probes. The kits will thus comprise, in suitable container means, an enzyme for labeling the miRNA by incorporating labeled nucleotide or unlabeled nucleotides that are subsequently labeled. It may also include one or more buffers, such as reaction buffer, labeling buffer, washing buffer, or a hybridization buffer, compounds for preparing the miRNA probes, components for *in situ* hybridization and components for isolating miRNA. Other kits of the invention may include components for making a nucleic acid array comprising miRNA, and thus may include, for example, a solid support.

The following examples are presented in order to more fully illustrate some embodiments of the invention. They should, in no way be construed, however, as limiting the broad scope of the invention.

## EXAMPLES

### Methods

### 1. Tumor samples

1300 primary and metastatic tumor FFPE were used in the study. Tumor samples were obtained from several sources. Institutional review approvals were obtained for all samples in accordance with each institute's institutional review board or IRB equivalent guidelines. Samples included primary tumors and metastases of defined origins, according to clinical records. Tumor content was at least 50% for >95% of samples, as determined by a pathologist based on hematoxylin-eosin (H&E) stained slides.

**2. RNA extraction**

For FFPE samples, total RNA was isolated from seven to ten 10-μm-thick tissue sections using the miR extraction protocol developed at Rosetta Genomics. Briefly, the sample was incubated a few times in xylene at 57°C to remove paraffin excess, followed by ethanol washes. Proteins were degraded by proteinase K solution at 45°C for a few hours. The RNA was extracted with acid phenol:chloroform followed by ethanol precipitation and DNAse digestion. Total RNA quantity and quality was checked by spectrophotometer (Nanodrop ND-1000).

**3. miR array platform**

Custom microarrays (Agilent Technologies, Santa Clara, CA) were produced by printing DNA oligonucleotide probes to: 982 miRs sequences, 17 negative controls, 23 spikes, and 10 positive controls (total of 1032 probes). Each probe, printed in triplicate, carried up to 28-nucleotide (nt) linker at the 3' end of the microRNA's complement sequence. 17 negative control probes were designed using as sequences which do not match the genome. Two groups of positive control probes were designed to hybridize to miR array: (i) synthetic small RNAs were spiked to the RNA before labeling to verify the labeling efficiency; and (ii) probes for abundant small RNA (*e.g.*, small nuclear RNAs (U43, U24, Z30, U6, U48, U44), 5.8s and 5s ribosomal RNA are spotted on the array to verify RNA quality.

**4. Cy-dye labeling of miRNA for miR array**

One μg of total RNA were labeled by ligation (Thomson et al. Nature Methods 2004; 1:47-53) of an RNA-linker, p-rCrU-Cy/dye (Eurogentec or eqvivalent), to the 3' end with Cy3 or Cy5. The labeling reaction contained total RNA, spikes (0.1-100 fmoles), 400 ng RNA-linker-dye, 15% DMSO, 1x ligase buffer and 20 units of T4 RNA ligase (NEB), and proceeded at 4°C for 1 h, followed by 1 h at 37°C, followed by 4 °C up to 40 min.

The labeled RNA was mixed with 30μl hybridization mixture (mixture of 45 μL of the 10X GE Agilent Blocking Agent and 246 μL of 2X Hi-RPM Hybridization). The labeling mixture was incubated at 100°C for 5 minutes followed by ice incubation in water bath for 5 minutes. Slides were Hybridize at 54°C for 16-20 hours, followed by two washes. The first wash was conducted at room temperature with Agilent GE Wash Buffer 1 for 5 min followed by a second wash with Agilent GE Wash Buffer 2 at 37°C for 5 min.

Arrays were scanned using an Agilent Microarray Scanner Bundle G2565BA (resolution of 5 μm at XDR Hi 100%, XDR Lo 5%). Array images were analyzed using Feature Extraction 10.7 software (Agilent).

**5. Array signal calculation and normalization**

Triplicate spots were combined to produce one signal for each probe by taking the logarithmic mean of reliable spots. All data were log2-transformed and the analysis was performed in log2-space. A reference data vector for normalization R was calculated by taking the median expression level for each probe across all samples. For each sample data vector S, a 2nd degree polynomial F was found so as to provide the best fit between the sample data and the reference data, such that R≈F(S). Remote data points ("outliers") were not used for fitting the polynomial F. For each probe in the sample (element Si in the vector S), the normalized value (in log-space) Mi was calculated from the initial value Si by transforming it with the polynomial function F, so that Mi=F(Si).

**6. Logistic regression**

The aim of a logistic regression model is to use several features, such as expression levels of several microRNAs, to assign a probability of belonging to one of two possible groups, such as two branches of a node in a binary decision-tree. Logistic regression models the natural log of the odds ratio, *i.e.,* the ratio of the probability of belonging to the first group, for example, the left branch in a node of a binary decision-tree (P) over the probability of belonging to the second group, for example, the right branch in such a node (1-P), as a linear combination of the different expression levels (in log-space). The logistic regression assumes that:

$$\ln(\frac{P}{1-P}) = \beta_0 + \sum_{i=1}^{N} \beta_i \cdot M_i = \beta_0 + \beta_1 \cdot M_1 + \beta_2 \cdot M_2 + \dots ,$$

where $\beta_0$ is the bias, $M_i$ is the expression level (normalized, in log2-space) of the *i*-th microRNA used in the decision

node, and $\beta_i$ is its corresponding coefficient. βi>0 indicates that the probability to take the left branch (P) increases when the expression level of this microRNA (Mi) increases, and the opposite for βi<0. If a node uses only a single microRNA (*M*), then solving for *P* results in:

$$P = \frac{e^{\beta_0+\beta_1\cdot M}}{1+e^{\beta_0+\beta_1\cdot M}}.$$

The regression error on each sample is the difference between the assigned probability P and the true "probability" of this sample, *i.e.,* 1 if this sample is in the left branch group and 0 otherwise. The training and optimization of the logistic regression model calculates the parameters $\beta$ and the p-values [for each microRNA by the Wald statistic and for the overall model by the $\chi 2$ (chi-square) difference], maximizing the likelihood of the data given the model and minimizing the total regression error

$$\sum_{\substack{Samples\\in\\first\\group}}(1-P_j)+\sum_{\substack{Samples\\in\\second\\group}}P_j.$$

The probability output of the logistic model is here converted to a binary decision by comparing *P* to a threshold, denoted by $P_{TH}$, *i.e.,* if $P > P_{TH}$ then the sample belongs to the left branch ("first group") and vice versa. Choosing at each node the branch which has a probability >0.5, *i.e.,* using a probability threshold of 0.5, leads to a minimization of the sum of the regression errors. However, as the goal was the minimization of the overall number of misclassifications (and not of their probability), a modification which adjusts the probability threshold ($P_{TH}$) was used in order to minimize the overall number of mistakes at each node (Table 2). For each node the threshold to a new probability threshold $P_{TH}$ was optimized such that the number of classification errors is minimized. This change of probability threshold is equivalent (in terms of classifications) to a modification of the bias $\beta_0$, which may reflect a change in the prior frequencies of the classes. Once the threshold was chosen $\beta_0$ was modified such that the threshold will be shifted back to 0.5. In addition, β0, β1, β2, ... were adjusted so that the slope of the log of the odds ratio function is limited.

**7. Stepwise logistic regression and feature selection**

The original data contain the expression levels of multiple microRNAs for each sample, *i.e.,* multiple of data features. In training the classifier for each node, only a small subset of these features was selected and used for optimizing a logistic regression model. In the initial training this was done using a forward stepwise scheme. The features were sorted in order of decreasing log-likelihoods, and the logistic model was started off and optimized with the first feature. The second feature was then added, and the model re-optimized. The regression error of the two models was compared: if the addition of the feature did not provide a significant advantage (a $\chi 2$ difference less than 7.88, p-value of 0.005), the new feature was discarded. Otherwise, the added feature was kept. Adding a new feature may make a previous feature redundant (*e.g.,* if they are very highly correlated). To check for this, the process iteratively checks if the feature with lowest likelihood can be discarded (without losing $\chi 2$ difference as above). After ensuring that the current set of features is compact in this sense, the process continues to test the next feature in the sorted list, until features are exhausted. No limitation on the number of features was inserted into the algorithm.

The stepwise logistic regression method was used on subsets of the training set samples by re-sampling the training set with repetition ("bootstrap"), so that each of the 20 runs contained somewhat different training set. All the features that took part in one of the 20 models were collected. A robust set of 1-3 features per each node was selected by comparing features that were repeatedly chosen in the bootstrap sets to previous evidence, and considering their signal strengths and reliability. When using these selected features to construct the classifier, the stepwise process was not used and the training optimized the logistic regression model parameters only.

**8. K-nearest-neighbors (KNN) classification algorithm**

The KNN algorithm (see *e.g.,* Ma et al., Arch Pathol Lab Med 2006; 130:465-73) calculates the distance (Pearson correlation) of any sample to all samples in the training set, and classifies the sample by the majority vote of the k samples which are most similar (k being a parameter of the classifier). The correlation is calculated on the pre-defined set of microRNAs (the microRNAs that were used by the decision-tree). KNN algorithms with k=1;10 were compared, and the optimal performer was selected, using k=5. The KNN was based on comparing the expression of all 65 microRNAs in each sample to all other samples in the training database.

**9. Reporting a final answer (prediction):**

The decision-tree and KNN each return a predicted tissue of origin and histological type where applicable. The tissue of origin and histological type may be one of the exact origins and types in the training or a variant thereof. For example, whereas the training includes brain oligondendrioglioma and brain astrocytoma, the answer may simply be brain carcinoma. In addition to the tissue of origin and histological type, the KNN and decision-tree each return a confidence measure. The KNN returns the number of samples within the K nearest neighbors that agreed with the answer reported by the KNN (denoted by V), and the decision-tree returns the probability of the result (P), which is the multiplication of the probabilities at each branch point made on the way to that answer. The classifier returns the two different predictions or a single prediction in case the predictions concur, can be unified into a single answer (for example into the prediction brain if the KNN returned brain oligondendrioglioma and the decision-tree brain astrocytoma), or if based on V and P, one answer is chosen to override the other.

**Example 1**

**Decision-tree classification algorithm**

A tumor classifier was built using the microRNA expression levels by applying a binary tree classification scheme (Figures 1A-F). This framework is set up to utilize the specificity of microRNAs in tissue differentiation and embryogenesis: different microRNAs are involved in various stages of tissue specification, and are used by the algorithm at different decision points or "nodes". The tree breaks up the complex multi-tissue classification problem into a set of simpler binary decisions. At each node, classes which branch out earlier in the tree are not considered, reducing interference from irrelevant samples and further simplifying the decision. The decision at each node can then be accomplished using only a small number of microRNA biomarkers, which have well-defined roles in the classification (Table 2). The structure of the binary tree was based on a hierarchy of tissue development and morphological similarity[18], which was modified by prominent features of the microRNA expression patterns. For example, the expression patterns of microRNAs indicated a significant difference between germ cell tumors and tumors of non-germ cell origin, and these are therefore distinguished at node #1 (Fig. 2) into separate branches (Fig. 1A).

For each of the individual nodes logistic regression models were used, a robust family of classifiers which are frequently used in epidemiological and clinical studies to combine continuous data features into a binary decision (Figs. 2-25 and Methods). Since gene expression classifiers have an inherent redundancy in selecting the gene features, bootstrapping was used on the training sample set as a method to select a stable microRNA set for each node (Methods). This resulted in a small number (usually 2-3) of microRNA features per node, totaling 65 microRNAs for the full classifier (Table 2). This approach provides a systematic process for identifying new biomarkers for differential expression.

**<u>Table 2:</u>** microRNAs used per class in the tree classifier

| miR List: | Class |
|---|---|
| hsa-miR-372 (SEQ ID NO:55) | Germ cell cancer |
| hsa-miR-372, hsa-miR-122 (SEQ ID NO:6), hsa-miR-126 (SEQ ID NO:9), hsa-miR-200b (SEQ ID NO:29) | Biliary tract adenocarcinoma |
| hsa-miR-372, hsa-miR-122, hsa-miR-126, hsa-miR-200b | Hepatocellular carcinoma (HCC) |
| hsa-miR-372, hsa-miR-122, hsa-miR-200c (SEQ ID NO:30), hsa-miR-30a (SEQ ID NO:46), hsa-miR-146a (SEQ ID NO:16), hsa-let-7e (SEQ ID NO:156), hsa-miR-9* (SEQ ID NO:66), hsa-miR-92b (SEQ ID NO:68) | Brain tumor |
| hsa-miR-372, hsa-miR-122, hsa-miR-200c, hsa-miR-30a, hsa-miR-146a, hsa-let-7e, hsa-miR-9*, hsa-miR-92b, hsa-miR-222 (SEQ ID NO:40), hsa-miR-497 (SEQ ID NO:60) | Brain - oligodendroglioma |
| hsa-miR-372, hsa-miR-122, hsa-miR-200c, hsa-miR-30a, hsa-miR-146a, hsa-let-7e, hsa-miR-9*, hsa-miR-92b, hsa-miR-222, hsa-miR-497 | Brain - astrocytoma |
| hsa-miR-372, hsa-miR-122, hsa-miR-200c, hsa-miR-30a, hsa-miR-375 (SEQ ID NO:56), hsa-miR-7 (SEQ ID NO:65), hsa-miR-193a-3p (SEQ ID NO:25), hsa-miR-194 (SEQ ID NO:27), hsa-miR-21*(SEQ ID NO:35), hsa-miR-143 (SEQ ID NO:14), hsa-miR-181a (SEQ ID NO:21) | Prostate Adenocarcinoma |

(continued)

| miR List: | Class |
|---|---|
| hsa-miR-372 | Ovarian primitive germ cell tumor |
| hsa-miR-372 | Testis |
| hsa-miR-372, hsa-miR-200b, hsa-miR-516a-5p (SEQ ID NO:62) | Seminomatous testicular germ cell tumor |
| hsa-miR-372, hsa-miR-200b, hsa-miR-516a-5p | Non seminomatous testicular germ cell tumor |
| hsa-miR-372, hsa-miR-122, hsa-miR-200c, hsa-miR-30a, hsa-miR-7, hsa-miR-194, hsa-miR-21*, hsa-miR-143, hsa-miR-181a, hsa-miR-205 (SEQ ID NO:32), hsa-miR-345 (SEQ ID NO:51), hsa-miR-125a-5p (SEQ ID NO:7), hsa-miR-193a-3p (SEQ ID NO:25), hsa-miR-375, hsa-miR-342-3p (SEQ ID NO:50) | Breast adenocarcinoma |
| hsa-miR-372, hsa-miR-122, hsa-miR-200c, hsa-miR-30a, hsa-miR-7, hsa-miR-193a-3p, hsa-miR-194, hsa-miR-21*, hsa-miR-143, hsa-miR-181a, hsa-miR-345, hsa-miR-125a-5p, hsa-miR-193a-3p, hsa-miR-375, hsa-miR-342-3p, hsa-miR-205 (SEQ ID NO:32), hsa-miR-10a (SEQ ID NO:4), hsa-miR-22 (SEQ ID NO:39) | Ovarian carcinoma |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-138 (SEQ ID NO:11), hsa-miR-93 (SEQ ID NO:148), hsa-miR-10a (SEQ ID NO:4) | Thyroid carcinoma |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-138, hsa-miR-93, hsa-miR-10a, hsa-miR-146b-5p (SEQ ID NO:17), hsa-miR-21 (SEQ ID NO:34) | Thyroid carcinoma follicular |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-138, hsa-miR-93, hsa-miR-10a, hsa-miR-146b-5p, hsa-miR-21 | Thyroid carcinoma papillary |
| hsa-miR-372, hsa-miR-122, hsa-miR-200c, hsa-miR-30a, hsa-miR-375, hsa-miR-7, hsa-miR-194, hsa-miR-21*, hsa-miR-143, hsa-miR-181a, hsa-miR-205, hsa-miR-345, hsa-miR-125a-5p, hsa-miR-138, hsa-miR-93, hsa-miR-10a, hsa-miR-193a-3p (SEQ ID NO:25), hsa-miR-31 (SEQ ID NO:49), hsa-miR-92a (SEQ ID NO:67) | Breast adenocarcinoma |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-93, hsa-miR-10a, hsa-miR-193a-3p, hsa-miR-31, hsa-miR-92a, hsa-miR-138 (SEQ ID NO:11), hsa-miR-378 (SEQ ID NO:57), hsa-miR-21 (SEQ ID NO:34) | Lung large cell or adenocarcinoma |
| hsa-miR-372, hsa-miR-122, hsa-miR-200c, hsa-miR-30a, hsa-miR-375, hsa-miR-7, hsa-miR-194, hsa-miR-21*, hsa-miR-143, hsa-miR-181a, hsa-miR-205, hsa-miR-345, hsa-miR-125a-5p, hsa-miR-93, hsa-miR-10a, hsa-miR-193a-3p, hsa-miR-31, hsa-miR-92a, hsa-miR-138, hsa-miR-378, hsa-miR-21 | Ovarian carcinoma |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-342-3p, hsa-miR-10a, hsa-miR-22, hsa-miR-100, hsa-miR-21 | Thymoma |

(continued)

| miR List: | Class |
|---|---|
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-342-3p, hsa-miR-205, hsa-miR-10a, hsa-miR-22, hsa-miR-100, hsa-miR-21, hsa-miR-934 (SEQ ID NO:69), hsa-miR-191 (SEQ ID NO:24), hsa-miR-29c (SEQ ID NO:44) | Urothelial carcinoma (TCC) |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-342-3p, hsa-miR-10a, hsa-miR-22, hsa-miR-100, hsa-miR-21, hsa-miR-934, hsa-miR-191, hsa-miR-29c | Squamous cell carcinoma (SCC) |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-342-3p, hsa-miR-10a, hsa-miR-22, hsa-miR-100, hsa-miR-21, hsa-miR-934, hsa-miR-191, hsa-miR-29c, hsa-miR-10b (SEQ ID NO:5), hsa-let-7c (SEQ ID NO:1), hsa-miR-361-5p (SEQ ID NO:54) | Uterine cervix SCC |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-193a-3p, hsa-miR-375, hsa-miR-342-3p, hsa-miR-205, | Anus or Skin SCC |
| hsa-miR-10a, hsa-miR-22, hsa-miR-100, hsa-miR-21, hsa-miR-934, hsa-miR-191, hsa-miR-29c, hsa-miR-10b hsa-let-7c, hsa-miR-361-5p, hsa-miR-138, hsa-miR-185 (SEQ ID NO:23) | |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-143, hsa-miR-181a, hsa-miR-205, hsa-miR-345, hsa-miR-125a-5p, hsa-miR-342-3p, hsa-miR-10a, hsa-miR-22, hsa-miR-100, hsa-miR-21, hsa-miR-934, hsa-miR-191, hsa-miR-29c, hsa-let-7c, hsa-miR-361-5p, hsa-miR-10b, hsa-miR-138, hsa-miR-185 | Lung, Head& Neck or Esophagus SCC |
| hsa-miR-372, hsa-miR-122, hsa-miR-200c, hsa-miR-30a, hsa-miR-146a (SEQ ID NO:16), hsa-let-7e (SEQ ID NO:2), hsa-miR-30d (SEQ ID NO:47), hsa-miR-342-3p | Melanoma |
| hsa-miR-372, hsa-miR-122, hsa-miR-200c, hsa-miR-30a, hsa-miR-146a, hsa-let-7e, hsa-miR-30d, hsa-miR-342-3p | Lymphoma |
| hsa-miR-372, hsa-miR-122, hsa-miR-200c, hsa-miR-30a, hsa-miR-146a, hsa-let-7e, hsa-miR-30d, hsa-miR-342-3p, hsa-miR-21*, hsa-miR-30e (SEQ ID NO:48) | B cell lymphoma |
| hsa-miR-372, hsa-miR-122, hsa-miR-200c, hsa-miR-30a, hsa-miR-146a, hsa-let-7e, hsa-miR-30a, hsa-miR-30d, hsa-miR-342-3p, hsa-miR-21*,hsa-miR-30e | T cell lymphoma |
| hsa-miR-372, hsa-miR-122, hsa-miR-200c, hsa-miR-30a, hsa-miR-375, hsa-miR-7, hsa-miR-193a-3p, hsa-miR-17 (SEQ ID NO:20), hsa-miR-29c* (SEQ ID NO:45) | Lung small cell carcinoma |
| hsa-miR-372, hsa-miR-122, hsa-miR-200c, hsa-miR-30a, hsa-miR-375, hsa-miR-7, hsa-miR-193a-3p, hsa-miR-17, hsa-miR-29c*, hsa-miR-222 (SEQ ID NO:40), hsa-miR-92a (SEQ ID NO:67), hsa-miR-92b (SEQ ID NO:68) | Medullary thyroid carcinoma |
| hsa-miR-372, hsa-miR-122, hsa-miR-200c, hsa-miR-30a, hsa-miR-375, hsa-miR-7, hsa-miR-193a-3p, hsa-miR-17, hsa-miR-29c*, hsa-miR-222, hsa-miR-92a, hsa-miR-92b, hsa-miR-652 (SEQ ID NO:64), hsa-miR-34c-5p (SEQ ID NO:53), hsa-miR-214 (SEQ ID NO:37) | Lung carcinoid |
| hsa-miR-372, hsa-miR-122, hsa-miR-200c, hsa-miR-30a, hsa-miR-375, hsa-miR-7, hsa-miR-193a-3p, hsa-miR-17, hsa-miR-29c*, hsa-miR-222, hsa-miR-92a, hsa-miR-92b, hsa-miR-652, hsa-miR-34c-5p, hsa-miR-214, hsa-miR-21 (SEQ ID NO:34), hsa-miR-148a (SEQ ID NO:18) | Gastrointestinal (GI) tract carcinoid |

(continued)

| miR List: | Class |
|---|---|
| hsa-miR-372, hsa-miR-122, hsa-miR-200c, hsa-miR-30a, hsa-miR-375, hsa-miR-7, hsa-miR-193a-3p, hsa-miR-17, hsa-miR-29c*, hsa-miR-222, hsa-miR-92a, hsa-miR-92b, hsa-miR-652, hsa-miR-34c-5p, hsa-miR-214, hsa-miR-21, hsa-miR-148a | Pancreas islet cell tumor |
| hsa-miR-372, hsa-miR-122, hsa-miR-200c, hsa-miR-30a, hsa-miR-375, hsa-miR-7, hsa-miR-193a-3p, hsa-miR-194 (SEQ ID NO:27), hsa-miR-21*(SEQ ID NO:35), hsa-miR-224 (SEQ ID NO:42), hsa-miR-210 (SEQ ID NO:36), hsa-miR-1201 (SEQ ID NO:146) | Gastric or Esophageal Adenocarcinoma |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-224, hsa-miR-210, hsa-miR-1201, hsa-miR-17 (SEQ ID NO:20), hsa-miR-29a (SEQ ID NO:43) | Colorectal Adenocarcinoma |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-224, hsa-miR-210, hsa-miR-1201, hsa-miR-17, hsa-miR-29a | Pancreas or bile |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-224, hsa-miR-210, hsa-miR-1201, hsa-miR-17, hsa-miR-29a, hsa-miR-345 (SEQ ID NO:51), hsa-miR-31 (SEQ ID NO:49), hsa-miR-146a (SEQ ID NO:16) | Pancreatic adenocarcinoma |
| hsa-miR-372, hsa-miR-122, hsa-miR-200c, hsa-miR-30a, hsa-miR-375, hsa-miR-7, hsa-miR-193a-3p, hsa-miR-194, hsa-miR-21*, hsa-miR-224, hsa-miR-210, hsa-miR-1201, hsa-miR-17, hsa-miR-29a, hsa-miR-345, hsa-miR-31, hsa-miR-146a | Biliary tract adenocarcinoma |
| hsa-miR-372, hsa-miR-122, hsa-miR-200c, hsa-miR-30a, hsa-miR-146a (SEQ ID NO:16), hsa-let-7e, hsa-miR-9* (SEQ ID NO:66), hsa-miR-92b (SEQ ID NO:68), hsa-miR-149 (SEQ ID NO:19), hsa-miR-200b (SEQ ID NO:29) | Renal cell carcinoma chromophobe |
| hsa-miR-372, hsa-miR-122, hsa-miR-200c, hsa-miR-30a, hsa-miR-146a, hsa-let-7e, hsa-miR-9*, hsa-miR-92b, hsa-miR-30a, hsa-miR-149, hsa-miR-200b, hsa-miR-7 (SEQ ID NO:65), hsa-miR-375 | Pheochromocytoma |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-7, hsa-miR-375, hsa-miR-202 (SEQ ID NO:31), hsa-miR-214* (SEQ ID NO:38), hsa-miR-509-3p (SEQ ID NO:61) | Adrenocortical |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-7, hsa-miR-375, hsa-miR-202, hsa-miR-214*, hsa-miR-509-3p, hsa-miR-143 (SEQ ID NO:14), hsa-miR-29c* | Gastrointestinal stomal tumor (GIST) |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-210 (SEQ ID NO:36), hsa-miR-221 (SEQ ID NO:147) | Renal cell carcinoma chromophobe |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-210, hsa-miR-221, hsa-miR-31 (SEQ ID NO:49), hsa-miR-126 (SEQ ID NO:9) | Renal cell carcinoma clear cell |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-210, hsa-miR-221, hsa-miR-31, hsa-miR-126 | Renal cell carcinoma papilary |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-7 (SEQ ID NO:65), hsa-miR-375, hsa-miR-202 (SEQ ID NO:31), hsa-miR-214* (SEQ ID NO:38), hsa-miR-509-3p (SEQ ID NO:61), hsa-miR-143 (SEQ ID NO:14), hsa-miR-29c*, hsa-miR-21* (SEQ ID NO:35), hsa-miR-130a (SEQ ID NO:10), hsa-miR-10b (SEQ ID NO:5) | Pleural mesothelioma |

(continued)

| miR List: | Class |
|---|---|
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-7, hsa-miR-375, hsa-miR-202, hsa-miR-214*, hsa-miR-509-3p, hsa-miR-143, hsa-miR-29c*, hsa-miR-21*, hsa-miR-130a, hsa-miR-10b | Sarcoma |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-7, hsa-miR-375, hsa-miR-202, hsa-miR-214*, hsa-miR-509-3p, hsa-miR-143, hsa-miR-29c*, hsa-miR-21*, hsa-miR-130a, hsa-miR-10b, hsa-miR-100 (SEQ ID NO:3), hsa-miR-222 (SEQ ID NO:40), hsa-miR-145 (SEQ ID NO:15) | Synovial sarcoma |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-7, hsa-miR-375, hsa-miR-202, hsa-miR-214*, hsa-miR-509-3p, hsa-miR-143, hsa-miR-29c*, hsa-miR-21*, hsa-miR-130a, hsa-miR-10b, hsa-miR-100, hsa-miR-222, hsa-miR-145, hsa-miR-140-3p (SEQ ID NO:12), hsa-miR-455-5p (SEQ ID NO:58) | Chondrosarcoma |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-7, hsa-miR-375, hsa-miR-202, hsa-miR-214*, hsa-miR-509-3p, hsa-miR-143, hsa-miR-29c*, hsa-miR-21*, hsa-miR-130a, hsa-miR-10b, hsa-miR-100, hsa-miR-222, hsa-miR-145, hsa-miR-140-3p, hsa-miR-455-5p, hsa-miR-210 (SEQ ID NO:36), hsa-miR-193a-5p (SEQ ID NO:26) | Liposarcoma |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-7, hsa-miR-375, hsa-miR-202, hsa-miR-214*, hsa-miR-509-3p, hsa-miR-143, hsa-miR-29c*, hsa-miR-21*, hsa-miR-130a, hsa-miR-10b, hsa-miR-100, hsa-miR-222, hsa-miR-145, hsa-miR-140-3p, hsa-miR-455-5p, hsa-miR-210, hsa-miR-193a-5p, hsa-miR-181a, hsa-miR-193a-3p (SEQ ID NO:25), hsa-miR-31 (SEQ ID NO:49) | Ewing sarcoma |
| hsa-miR-372, hsa-miR-122, hsa-miR-200c, hsa-miR-30a, hsa-miR-146a, hsa-let-7e, hsa-miR-9*, hsa-miR-92b, hsa-miR-149, hsa-miR-200b, hsa-miR-7, hsa-miR-375, hsa-miR-202, hsa-miR-214*, hsa-miR-509-3p, hsa-miR-143, hsa-miR-29c*, hsa-miR-21*, hsa-miR-130a, hsa-miR-10b, hsa-miR-100, hsa-miR-222, hsa-miR-145, hsa-miR-140-3p, hsa-miR-455-5p, hsa-miR-210, hsa-miR-193a-5p, hsa-miR-181a, hsa-miR-193a-3p, hsa-miR-31 | Osteosarcoma |
| hsa-miR-372, hsa-miR-122, hsa-miR-200c, hsa-miR-30a, hsa-miR-146a, hsa-let-7e, hsa-miR-30a, hsa-miR-9*, hsa-miR-92b, hsa-miR-30a, hsa-miR-149, hsa-miR-200b, hsa-miR-7, hsa-miR-375, hsa-miR-202, hsa-miR-214*, hsa-miR-509-3p, hsa-miR-143, hsa-miR-29c*, hsa-miR-21*, hsa-miR-130a, hsa-miR-10b, hsa-miR-100, hsa-miR-222, hsa-miR-145, hsa-miR-140-3p, hsa-miR-455-5p, hsa-miR-210, hsa-miR-193a-5p, hsa-miR-181a, hsa-miR-487b (SEQ ID NO:59), hsa-miR-22 (SEQ ID NO:39), hsa-miR-206 (SEQ ID NO:33) | Rhabdomyo sarcoma |
| hsa-miR-372, hsa-miR-122, hsa-miR-200c, hsa-miR-30a, hsa-miR-146a, hsa-let-7e, hsa-miR-30a, hsa-miR-9*, hsa-miR-92b, hsa-miR-30a, hsa-miR-149, hsa-miR-200b, hsa-miR-7, hsa-miR-375, hsa-miR-202, hsa-miR-214*, hsa-miR-509-3p, hsa-miR-143, hsa-miR-29c*, hsa-miR-21*, hsa-miR-130a, hsa-miR-10b, hsa-miR-100, hsa-miR-222, hsa-miR-145, hsa-miR-140-3p, hsa-miR-455-5p, hsa-miR-210, hsa-miR-193a-5p, hsa-miR-181a, hsa-miR-487b, hsa-miR-22, hsa-miR-206 | Malignant fibrous histiocytoma (MFH) or fibrosarcoma |

**Example 2**

**Expression of miRs provides for distinguishing between tumors**

**Table 3:** miR expression (in fluorescence units) distinguishing between the group consisting of germ-cell tumors and the group consisting of all other tumors

| median values | fold-change | p-value | SEQ ID NO. | miR name |
|---|---|---|---|---|
| 2.7e+004 - 5.0e+001 | 545.73 (+) | < e-240 | 233 | hsa-miR-373 |
| 1.8e+004 - 5.0e+001 | 365.93 (+) | < e-240 | 55 | hsa-miR-372 |
| 8.6e+003 - 5.0e+001 | 171.72 (+) | < e-240 | 200 | hsa-miR-371-3p |
| 5.9e+003 - 5.1e+001 | 115.94 (+) | 7.3e-249 | 201 | hsa-miR-371-5p |
| + for all the listed miRs, the higher expression is in tumors from a germ-cell origin. | | | | |

hsa-miR-372 (SEQ ID NO: 55) is used at node 1 of the binary-tree-classifier detailed in the invention to distinguish between germ-cell tumors and all other tumors.
Figures 2A-D are boxplot presentations comparing distribution of the expression of the statistically significant miRs in tumor samples from the "germ cell" class (left box) and "non germ cell" class (right box).

**Table 4:** miR expression (in fluorescence units) distinguishing between the group consisting of hepatobiliary tumors and the group consisting of non germ-cell non-hepatobiliary tumors

| median values | fold-change | p-value | SEQ ID NO. | miR name |
|---|---|---|---|---|
| 1.0e+005 - 5.0e+001 | 2024.31 (+) | 1.1e-123 | 6 | hsa-miR-122 |
| 7.4e+001 - 8.1e+003 | 109.63 (-) | 3.6e-010 | 30 | hsa-miR-200c |
| 5.0e+001 - 1.4e+003 | 27.92 (-) | 4.8e-010 | 13 | hsa-miR-141 |
| + the higher expression of this miR is in tumors from a hepatobiliary origin<br>- the higher expression of this miR is in tumors from a non germ-cell, non-hepatobiliary origin | | | | |

hsa-miR-122 (SEQ ID NO: 6) is used at node 2 of the binary-tree-classifier detailed in the invention to distinguish between hepatobiliary tumors and non germ-cell non-hepatobiliary tumors.

**Table 5:** miR expression (in fluorescence units) distinguishing between the group consisting of liver tumors and the group consisting of biliary-tract carcinomas (cholangiocarcinoma or gallbladder adenocarcinoma)

| median values | fold-change | p-value | SEQ ID NO. | miR name |
|---|---|---|---|---|
| 6.1e+003-4.1e+002 | 14.74 (+) | 5.5e-005 | 28 | hsa-miR-200a |
| 9.7e+003 - 9.0e+002 | 10.74 (+) | 2.4e-004 | 29 | hsa-miR-200b |
| 1.9e+003 - 7.0e+003 | 3.67 (-) | 8.5e-004 | 231 | hsa-miR-99a |
| 3.3e+003 - 7.5e+003 | 2.28 (-) | 6.2e-004 | 9 | hsa-miR-126 |
| + the higher expression of this miR is in biliary tract carcinomas<br>- the higher expression of this miR is in liver tumors | | | | |

hsa-miR-126 (SEQ ID NO: 9) and hsa-miR-200b (SEQ ID NO: 29) are used at node 3 of the binary-tree-classifier detailed in the invention to distinguish between liver tumors and biliary-tract carcinoma.
Figure 3 demonstrates that tumors of hepatocellular carcinoma (HCC) origin (marked by squares) are easily distinguished from tumors of biliary tract adenocarcinoma origin (marked by diamonds) using the expression levels of hsa-miR-200b (SEQ ID NO: 29, y-axis) and hsa-miR-126 (SEQ ID NO: 9, x-axis).

**Table 6:** miR expression (in fluorescence units) distinguishing between the group consisting of tumors from an epithelial origin and the group consisting of tumors from a non-epithelial origin

| median values | fold-change | p-value | SEQ ID NO. | miR name |
|---|---|---|---|---|
| 1.5e+004 - 7.7e+001 | 196.43 (+) | 1.5e-300 | 30 | hsa-miR-200c |

(continued)

| median values | fold-change | p-value | SEQ ID NO. | miR name |
|---|---|---|---|---|
| 9.0e+003 - 5.0e+001 | 180.07 (+) | 1.3e-208 | 29 | hsa-miR-200b |
| 3.9e+003 - 5.0e+001 | 78.09 (+) | 2.2e-187 | 28 | hsa-miR-200a |
| 2.7e+003 - 5.0e+001 | 54.64 (+) | 7.0e-078 | 32 | hsa-miR-205 |
| 2.6e+003 - 5.0e+001 | 51.98 (+) | 1.2e-265 | 13 | hsa-miR-141 |
| 5.4e+002 - 9.2e+001 | 5.90 (+) | 6.3e-048 | 152 | hsa-miR-182 |
| 1.1e+003 - 2.5e+002 | 4.35 (+) | 4.8e-022 | 49 | hsa-miR-31 |
| + for all the listed miRs, the higher expression is in tumors from epithelial origins | | | | |

A combination of the expression level of any of the miRs detailed in table 6 with the expression level of any of hsa-miR-30a (SEQ ID NO: 46), hsa-miR-10b (SEQ ID NO: 5) and hsa-miR-140-3p (SEQ ID NO: 12) also provides for distinguishing between tumors from epithelial origins and tumors from non-epithelial origins. This is demonstrated at node 4 of the binary-tree-classifier detailed in the invention with hsa-miR-200c (SEQ ID NO: 30) and hsa-miR-30a (SEQ ID NO: 46) (Figure 4). Tumors originating in epithelial (diamonds) are easily distinguished from tumors of non-epithelial origin (squares) using the expression levels of hsa-miR-30a (SEQ ID NO: 46, y-axis) and hsa-miR-200c (SEQ ID NO: 30, x-axis).

**Table 7:** miR expression (in fluorescence units) distinguishing between the group consisting of melanoma and lymphoma (B-cell, T-cell), and the group consisting of all other non-epithelial tumors

| median values | fold-change | p-value | SEQ ID NO. | miR name |
|---|---|---|---|---|
| 2.0e+003 - 7.0e+001 | 28.25 (-) | 1.9e-074 | 164 | hsa-miR-142-5p |
| 1.2e+004 - 6.3e+002 | 18.86 (-) | 6.0e-061 | 168 | hsa-miR-150 |
| 5.4e+003 - 3.1e+002 | 17.29 (-) | 5.6e-060 | 170 | hsa-miR-155 |
| 4.2e+003 - 3.5e+002 | 12.03 (-) | 8.4e-068 | 16 | hsa-miR-146a |
| 5.9e+002 - 1.4e+002 | 4.25 (-) | 8.2e-048 | 198 | hsa-miR-342-5p |
| 7.5e+003 - 1.9e+003 | 4.02 (-) | 4.8e-056 | 50 | hsa-miR-342-3p |
| 8.9e+002 - 2.5e+002 | 3.53 (-) | 6.0e-035 | 176 | hsa-miR-18a |
| 4.4e+003 - 1.4e+003 | 3.28 (-) | 8.0e-038 | 186 | hsa-miR-20a |
| 7.9e+002 - 2.6e+002 | 3.03 (-) | 7.3e-005 | 11 | hsa-miR-138 |
| 6.6e+003 - 2.3e+003 | 2.82 (-) | 4.0e-039 | 158 | hsa-miR-106a |
| 4.1e+003 - 1.4e+003 | 2.82 (-) | 2.4e-037 | 20 | hsa-miR-17 |
| 6.2e+001 - 5.9e+002 | 9.53 (+) | 3.7e-027 | 155 | hsa-miR-127-3p |
| 1.2e+003 - 7.0e+003 | 5.71 (+) | 1.5e-047 | 231 | hsa-miR-99a |
| 3.9e+002 - 1.7e+003 | 4.25 (+) | 6.6e-022 | 4 | hsa-miR-10a |
| 1.0e+004 - 4.1e+004 | 3.91 (+) | 3.2e-037 | 8 | hsa-miR-125b |
| 6.5e+002 - 2.2e+003 | 3.37 (+) | 2.4e-023 | 46 | hsa-miR-30a |
| 1.9e+003 - 5.6e+003 | 2.98 (+) | 1.0e-025 | 3 | hsa-miR-100 |
| 2.5e+003 - 7.1e+003 | 2.89 (+) | 1.8e-051 | 2 | hsa-let-7e |
| 2.9e+003 - 8.4e+003 | 2.86 (+) | 8.1e-047 | 7 | hsa-miR-125a-5p |
| + the higher expression of this miR is in the group of non-epithelial tumors excluding melanoma and lymphoma<br>- the higher expression of this miR is in the group consisting of melanoma and lymphoma | | | | |

hsa-miR-146a (SEQ ID NO: 16), hsa-let-7e (SEQ ID NO: 2) and hsa-miR-30a (SEQ ID NO: 46) are used at node 5 of the binary-tree-classifier detailed in the invention to distinguish between the group consisting of melanoma and lymphoma, and the group consisting of all other non-epithelial tumors. Figure 5 demonstrates that tumors originating in the lymphoma or melanoma (diamonds) are easily distinguished from tumors of non epithelial, non lymphoma /melanoma origin (squares) using the expression levels of hsa-miR-146a (SEQ ID NO: 16, y-axis), hsa-miR-30a (SEQ ID NO: 46, x-axis) and hsa-let-7e (SEQ ID NO: 2, z-axis).

**Table 8:** miR expression (in fluorescence units) distinguishing between the group consisting of brain tumors (astrocytic tumor and oligodendroglioma) and the group consisting of all non-brain, non-epithelial tumors

| median values | fold-change | p-value | SEQ ID NO. | miR name |
|---|---|---|---|---|
| 9.1e+003 - 5.0e+001 | 182.94 (+) | 3.8e-059 | 159 | hsa-miR-124 |
| 4.4e+003 - 5.0e+001 | 88.33 (+) | 1.1e-125 | 66 | hsa-miR-9* |
| 2.1e+003 - 6.0e+001 | 34.97 (+) | 6.0e-035 | 225 | hsa-miR-551b |
| 9.9e+002 - 5.0e+001 | 19.73 (+) | 3.0e-116 | 187 | hsa-miR-219-2-3p |
| 6.5e+002 - 5.0e+001 | 12.95 (+) | 1.8e-021 | 162 | hsa-miR-129-3p |
| 1.1e+003 - 1.0e+002 | 10.52 (+) | 2.0e-034 | 161 | hsa-miR-128 |
| 2.3e+003 - 2.5e+002 | 9.45 (+) | 2.2e-052 | 68 | hsa-miR-92b |
| 5.2e+002 - 6.8e+001 | 7.61 (+) | 6.7e-019 | 232 | hsa-miR-99a* |
| 6.9e+002 - 9.2e+001 | 7.45 (+) | 5.5e-023 | 173 | hsa-miR-181c |
| 2.2e+003 - 3.5e+002 | 6.34 (+) | 7.4e-007 | 11 | hsa-miR-138 |
| 1.2e+005 - 2.4e+004 | 4.78 (+) | 1.7e-014 | 8 | hsa-miR-125b |
| 1.8e+003 - 3.9e+002 | 4.70 (+) | 7.2e-014 | 174 | hsa-miR-181d |
| 8.5e+002 - 1.8e+002 | 4.64 (+) | 2.2e-002 | 155 | hsa-miR-127-3p |
| 1.6e+004 - 3.5e+003 | 4.60 (+) | 2.4e-010 | 231 | hsa-miR-99a |
| 8.5e+001 - 1.1e+003 | 13.55 (-) | 2.4e-014 | 4 | hsa-miR-10a |
| 7.7e+002 - 6.6e+003 | 8.58 (-) | 8.4e-017 | 182 | hsa-miR-199a-5p |
| 5.7e+002 - 4.7e+003 | 8.12 (-) | 1.8e-013 | 181 | hsa-miR-199a-3p |
| 2.8e+002 - 1.9e+003 | 6.81 (-) | 1.4e-012 | 37 | hsa-miR-214 |
| $^+$ the higher expression of this miR is in the group consisting of brain tumors<br>$^-$ the higher expression of this miR is in the group consisting of all non-brain, non-epithelial tumors | | | | |

hsa-miR-9* (SEQ ID NO: 66) and hsa-miR-92b (SEQ ID NO: 68) are used at node 6 of the binary-tree-classifier detailed in the invention to distinguish between brain tumors and the group consisting of all non-brain, non-epithelial tumors. Figure 6 demonstrates that tumors originating in the brain (marked by diamonds) are easily distinguished from tumors of non epithelial, non brain origin (marked by squares) using the expression levels of hsa-miR-9* (SEQ ID NO: 66, y-axis) and hsa-miR-92b (SEQ ID NO: 68, x-axis).

**Table 9:** miR expression (in fluorescence units) distinguishing between astrocytic tumors and oligodendrogliomas

| median values | fold-change | p-value | SEQ ID NO. | miR name |
|---|---|---|---|---|
| 2.5e+003 - 2.3e+002 | 11.10 (+) | 5.1e-011 | 230 | hsa-miR-886-5p |
| 4.4e+003 - 4.9e+002 | 9.06 (+) | 1.1e-009 | 228 | hsa-miR-886-3p |
| 1.0e+004 - 1.7e+003 | 5.99 (+) | 7.7e-008 | 147 | hsa-miR-221 |
| 1.3e+004 - 2.6e+003 | 5.03 (+) | 2.6e-006 | 40 | hsa-miR-222 |
| 3.3e+004 - 7.3e+003 | 4.54 (+) | 3.9e-004 | 34 | hsa-miR-21 |
| 8.4e+002 - 2.2e+002 | 3.78 (+) | 3.7e-006 | 206 | hsa-miR-455-3p |
| 6.0e+002 - 1.8e+002 | 3.30 (+) | 1.3e-002 | 35 | hsa-miR-21* |

(continued)

| median values | fold-change | p-value | SEQ ID NO. | miR name |
|---|---|---|---|---|
| 5.8e+003 - 1.8e+003 | 3.15 (+) | 2.4e-005 | 52 | hsa-miR-34a |
| 1.1e+003 - 3.5e+002 | 3.04 (+) | 1.0e-003 | 25 | hsa-miR-193a-3p |
| 1.6e+002 - 8.2e+002 | 5.17 (-) | 1.2e-004 | 229 | hsa-miR-9 |
| 4.6e+002 - 2.3e+003 | 5.09 (-) | 7.1e-003 | 161 | hsa-miR-128 |
| 4.1e+002 - 1.8e+003 | 4.43 (-) | 1.3e-002 | 187 | hsa-miR-219-2-3p |
| 3.8e+003 - 1.3e+004 | 3.31 (-) | 1.9e-002 | 179 | hsa-miR-195 |
| + the higher expression of this miR is in astrocytic tumors<br>- the higher expression of this miR is in oligodendrogliomas | | | | |

A combination of the expression level of any of the miRs detailed in table 9 with the expression level of hsa-miR-497 (SEQ ID NO: 208) or hsa-let-7d (SEQ ID NO: 153) also provides for classification of brain tumors as astrocytic tumors or oligodendrogliomas. This is demonstrated at node 7 of the binary-tree-classifier detailed in the invention with hsa-miR-222 (SEQ ID NO: 40) and hsa-miR-497 (SEQ ID NO: 208). In another embodiment of the invention, the expression levels of hsa-miR-222 (SEQ ID NO: 40) and hsa-let-7d (SEQ ID NO: 153) are combined to distinguish between astrocytic tumors and oligodendrogliomas.

Figure 7 demonstrates that tumors originating in astrocytoma (marked by diamonds) are easily distinguished from tumors of oligodendroglioma origins (marked by squares) using the expression levels of hsa-miR-497 (SEQ ID NO: 208, y-axis) and hsa-miR-222 (SEQ ID NO: 40, x-axis).

**Table 10:** miR expression (in fluorescence units) distinguishing between the group consisting of neuroendocrine tumors and the group consisting of all non-neuroendocrine, epithelial tumors

| median values | fold-change | p-value | SEQ ID NO. | miR name |
|---|---|---|---|---|
| 3.8e+004 - 1.5e+002 | 259.47 (+) | 5.3e-086 | 56 | hsa-miR-375 |
| 3.6e+003 - 5.2e+001 | 70.47 (+) | 4.4e-145 | 65 | hsa-miR-7 |
| 1.3e+003 - 1.8e+002 | 6.89 (+) | 4.7e-044 | 175 | hsa-miR-183 |
| 1.9e+003 - 4.4e+002 | 4.42 (+) | 3.5e-025 | 152 | hsa-miR-182 |
| 1.2e+003 - 3.0e+002 | 4.16 (+) | 5.5e-028 | 155 | hsa-miR-127-3p |
| 5.6e+001 - 7.0e+003 | 124.66 (-) | 1.4e-023 | 32 | hsa-miR-205 |
| 1.5e+002 - 1.4e+003 | 9.25 (-) | 1.8e-019 | 49 | hsa-miR-31 |
| 3.4e+002 - 1.4e+003 | 4.12 (-) | 9.5e-032 | 35 | hsa-miR-21* |
| + the higher expression of this miR is in the group consisting of neuroendocrine tumors<br>- the higher expression of this miR is in the group consisting of all non-neuroendocrine, epithelial tumors | | | | |

hsa-miR-375 (SEQ ID NO: 56), hsa-miR-7 (SEQ ID NO: 65) and hsa-miR-193a-3p (SEQ ID NO: 25) are used at node 8 of the binary-tree-classifier detailed in the invention to distinguish between the group consisting of neuroendocrine tumors and the group consisting of all non-neuroendocrine, epithelial tumors. Figure 8 demonstrates that tumors originating in the neuroendocrine (diamonds) are easily distinguished from tumors of epithelial, origin (squares) using the expression levels of hsa-miR-193a-3p (SEQ ID NO: 25, y-axis), hsa-miR-7 (SEQ ID NO: 65, x-axis) and hsa-miR-375 (SEQ ID NO: 56, z-axis).

**Table 11:** miR expression (in fluorescence units) distinguishing between the group consisting of gastrointestinal (GI) epithelial tumors and the group consisting of non-GI epithelial tumors

| median values | fold-change | p-value | SEQ ID NO. | miR name |
|---|---|---|---|---|
| 2.6e+003 - 7.1e+001 | 36.09 (+) | 2.5e-127 | 27 | hsa-miR-194 |
| 3.9e+003 - 1.2e+002 | 33.26 (+) | 1.6e-117 | 177 | hsa-miR-192 |

(continued)

| median values | fold-change | p-value | SEQ ID NO. | miR name |
|---|---|---|---|---|
| 2.6e+003 - 6.7e+002 | 3.88 (+) | 3.3e-021 | 4 | hsa-miR-10a |
| 5.0e+001 - 2.1e+004 | 411.76 (-) | 6.5e-045 | 32 | hsa-miR-205 |
| + the higher expression of this miR is in the group consisting of GI epithelial tumors<br>- the higher expression of this miR is in the group consisting of non-GI epithelial tumors | | | | |

hsa-miR-194 (SEQ ID NO: 27) and hsa-miR-21* (SEQ ID NO: 35) are used at node 9 of the binary-tree-classifier detailed in the invention to distinguish between GI epithelial tumors and non-GI epithelial tumors.

Figure 9 demonstrates that tumors originating in gastro-intestinal (GI) (marked by diamonds) are easily distinguished from tumors of non GI origins (marked by squares) using the expression levels of hsa-miR-21* (SEQ ID NO: 35, y-axis) and hsa-miR-194 (SEQ ID NO: 27, x-axis).

**Table 12:** miR expression (in fluorescence units) distinguishing between prostate tumors and all other non-GI epithelial tumors

| median values | fold-change | p-value | SEQ ID NO. | miR name |
|---|---|---|---|---|
| 5.1e+003 - 5.2e+001 | 96.76 (+) | 3.7e-016 | 56 | hsa-miR-375 |
| 1.0e+003 - 5.5e+001 | 18.27 (+) | 4.0e-025 | 199 | hsa-miR-363 |
| 6.8 e+004 - 7.2e+003 | 9.41 (+) | 1.0e-025 | 14 | hsa-miR-143 |
| 1.2e+005 - 1.4e+004 | 8.14 (+) | 7.8e-022 | 15 | hsa-miR-145 |
| 2.8e+003 - 3.5e+002 | 7.89 (+) | 1.5e-012 | 165 | hsa-miR-143* |
| 2.1e+004 - 4.4e+003 | 4.76 (+) | 2.2e-011 | 231 | hsa-miR-99a |
| 4.6e+002-2.1e+003 | 4.58 (-) | 8.0e-007 | 36 | hsa-miR-210 |
| 2.7e+002 - 1.1e+003 | 3.84 (-) | 7.8e-017 | 154 | hsa-miR-181b |
| 1.2e+003 - 4.3e+003 | 3.76 (-) | 1.2e-014 | 21 | hsa-miR-181a |
| 5.5e+002 - 2.0e+003 | 3.63 (-) | 2.3e-002 | 49 | hsa-miR-31 |
| + the higher expression of this miR is in prostate tumors<br>- the higher expression of this miR is in the group consisting of all other non-GI epithelial tumors | | | | |

hsa-miR-143 (SEQ ID NO: 14) and hsa-miR-181a (SEQ ID NO: 21) are used at node 10 of the binary-tree-classifier detailed in the invention to distinguish between prostate tumors and all other non-GI epithelial tumors.

Figure 10 demonstrates that tumors originating in prostate adenocarcinoma (marked by diamonds) are easily distinguished from tumors of non prostate origins (marked by squares) using the expression levels of hsa-miR-181a (SEQ ID NO: 21, y-axis) and hsa-miR-143 (SEQ ID NO: 14, x-axis).

**Table 13:** miR expression (in fluorescence units) distinguishing between seminiomatous and non- seminiomatous testicular tumors

| median values | fold-change | p-value | SEQ ID NO. | miR name |
|---|---|---|---|---|
| 4.3e+003 - 7.6e+002 | 5.63 (+) | 6.6e-004 | 152 | hsa-miR-182 |
| 1.0e+002 - 2.1e+003 | 20.46 (-) | 6.2e-005 | 216 | hsa-miR-518e |
| 7.8e+001 - 1.2e+003 | 15.29 (-) | 4.5e-005 | 212 | hsa-miR-516b |
| 6.8e+001 - 8.2e+002 | 11.94 (-) | 2.2e-005 | 224 | hsa-miR-527 |
| 2.1e+002 - 2.2e+003 | 10.40 (-) | 1.9e-006 | 13 | hsa-miR-141 |
| 5.3e+002 - 5.0e+003 | 9.48 (-) | 5.0e-004 | 194 | hsa-miR-302d |
| 1.4e+002 - 1.3e+003 | 8.97 (-) | 4.1e-006 | 192 | hsa-miR-302a |

(continued)

| median values | fold-change | p-value | SEQ ID NO. | miR name |
|---|---|---|---|---|
| 2.7e+002 - 2.3e+003 | 8.78 (-) | 2.9e-003 | 221 | hsa-miR-520c-3p |
| 1.3e+002 - 1.2e+003 | 8.65 (-) | 8.3e-004 | 217 | hsa-miR-518f* |
| 3.4e+003 - 2.9e+004 | 5.98 (-) | 2.6e-007 | 205 | hsa-miR-451 |
| 2.8e+002 - 1.7e+003 | 5.98 (-) | 1.1e-002 | 219 | hsa-miR-519d |
| 2.0e+002 - 1.2e+003 | 5.90 (-) | 6.8e-005 | 32 | hsa-miR-205 |
| 2.0e+002 - 1.1e+003 | 5.59 (-) | 5.8e-006 | 193 | hsa-miR-302a* |
| 1.9e+002 - 1.0e+003 | 5.27 (-) | 6.7e-003 | 223 | hsa-miR-524-5p |
| 1.5e+002 - 8.0e+002 | 5.22 (-) | 5.4e-003 | 220 | hsa-miR-520a-5p |
| 2.2e+002 - 1.1e+003 | 5.21 (-) | 4.1e-003 | 210 | hsa-miR-512-5p |
| 3.2e+002 - 1.4e+003 | 4.57 (-) | 9.2e-003 | 209 | hsa-miR-498 |
| 7.2e+002 - 3.2e+003 | 4.51 (-) | 3.1e-002 | 213 | hsa-miR-517a |
| 6.4e+002 - 2.9e+003 | 4.47 (-) | 2.9e-002 | 163 | hsa-miR-1323 |
| 9.5e+002 - 4.1e+003 | 4.29 (-) | 1.3e-004 | 30 | hsa-miR-200c |
| $^+$ the higher expression of this miR is in seminioma tumors<br>$^-$ the higher expression of this miR is in non-seminioma tumors | | | | |

A combination of the expression level of any of the miRs detailed in table 13 with the expression level of hsa-miR-200b (SEQ ID NO: 29), hsa-miR-200a (SEQ ID NO: 28), hsa-miR-516a-5p (SEQ ID NO: 211), hsa-miR-767-5p (SEQ ID NO: 227), hsa-miR-518a-3p (SEQ ID NO: 215), hsa-miR-520d-5p (SEQ ID NO: 222), hsa-miR-519a (SEQ ID NO: 218) and hsa-miR-517c (SEQ ID NO: 214) also provides for classification of seminoma and non-seminoma testis-tumors.

hsa-miR-516a-5p (SEQ ID NO: 211) and hsa-miR-200b (SEQ ID NO: 29) are used at node 12 of the binary-tree-classifier detailed in the invention to distinguish between seminoma and non-seminoma testis-tumors.

Figure 11 demonstrates that tumors originating in seminiomatous testicular germ cell (marked by diamonds) are easily distinguished from tumors of non seminiomatous origins (marked by squares) using the expression levels of hsa-miR-516a-5p (SEQ ID NO: 211, y-axis) and hsa-miR-200b (SEQ ID NO: 29, x-axis).

**Table 14:** miR expression (in fluorescence units) distinguishing between the group consisting of squamous cell carcinoma (SCC), transitional cell carcinoma (TCC), thymoma and the group consisting of non gastrointestinal (GI) adenocarcinoma tumors

| median values | fold-change | p-value | SEQ ID NO. | miR name |
|---|---|---|---|---|
| 4.6e+004 - 1.4e+002 | 321.76 (+) | 1.6e-059 | 32 | hsa-miR-205 |
| 2.9e+003 - 4.9e+002 | 5.96 (+) | 8.6e-015 | 36 | hsa-miR-210 |
| 2.5e+003 - 6.6e+002 | 3.82 (+) | 2.6e-016 | 178 | hsa-miR-193b |
| 2.5e+003 - 6.8e+002 | 3.67 (+) | 1.8e-008 | 243 | MID-16869 |
| 3.4e+003 - 9.7e+002 | 3.53 (+) | 2.2e-011 | 242 | MID-16489 |
| 3.7e+003 - 1.3e+003 | 2.82 (+) | 8.2e-004 | 49 | hsa-miR-31 |
| 6.4e+003 - 2.3e+003 | 2.78 (+) | 1.7e-010 | 240 | MID-15965 |
| 1.4e+003 - 5.2e+002 | 2.71 (+) | 2.7e-017 | 57 | hsa-miR-378 |
| 2.8e+002 - 2.2e+003 | 8.05 (-) | 2.9e-023 | 11 | hsa-miR-138 |
| 7.3e+002 - 2.7e+003 | 3.70 (-) | 1.5e-018 | 46 | hsa-miR-30a |
| 8.6e+002 - 2.2e+003 | 2.60 (-) | 4.0e-013 | 17 | hsa-miR-146b-5p |

(continued)

| median values | fold-change | p-value | SEQ ID NO. | miR name |
|---|---|---|---|---|
| 1.8e+003 - 4.3e+003 | 2.44 (-) | 1.5e-021 | 47 | hsa-miR-30d |
| 4.5e+002 - 1.1e+003 | 2.38 (-) | 2.6e-019 | 51 | hsa-miR-345 |
| 4.2e+003 - 9.6e+003 | 2.30 (-) | 3.8e-014 | 7 | hsa-miR-125a-5p |
| 1.9e+004 - 4.3e+004 | 2.26 (-) | 3.0e-009 | 8 | hsa-miR-125b |
| 9.4e+002 - 2.1e+003 | 2.24 (-) | 3.2e-008 | 154 | hsa-miR-181b |
| 7.4e+002 - 1.6e+003 | 2.13 (-) | 3.3e-010 | 190 | hsa-miR-29b |
| 6.6e+003 - 1.3e+004 | 2.04 (-) | 4.6e-014 | 157 | hsa-let-7i |
| 2.4e+003 - 4.8e+003 | 2.04 (-) | 7.9e-010 | 196 | hsa-miR-30c |
| $^{+}$ the higher expression of this miR is in SCC, TCC and thymoma<br>$^{-}$ the higher expression of this miR is in non GI adenocarcinoma | | | | |

Node 13 of the binary-tree-classifier separates tissues with high expression of miR-205 (SCC marker) such as SCC, TCC and thymomas from adenocarcinomas.
Breast adenocarcinoma and ovarian carcinoma are excluded from this separation due to a wide range of expression of miR-205.

A combination of the expression level of any of the miRs detailed in table 14 with the expression level of hsa-miR-331-3p (SEQ ID NO: 197) also provides for this classification.

hsa-miR-205 (SEQ ID NO: 32), hsa-miR-345 (SEQ ID NO: 51) and hsa-miR-125a-5p (SEQ ID NO: 7) are used at node 13 of the binary-tree-classifier detailed in the invention.

**Table 15:** miR expression (in fluorescence units) distinguishing between the group consisting of breast adenocarcinoma and the group consisting of SCC, TCC, thymomas and ovarian carcinoma

| median values | fold-change | p-value | SEQ ID NO. | miR name |
|---|---|---|---|---|
| 1.3e+003 - 5.0e+001 | 25.95 (+) | 4.1e-029 | 56 | hsa-miR-375 |
| 2.7e+003 - 8.3e+002 | 3.25 (+) | 1.6e-014 | 46 | hsa-miR-30a |
| 4.4e+003 - 1.4e+003 | 3.09 (+) | 9.5e-022 | 25 | hsa-miR-193a-3p |
| 1.2e+003 - 4.1e+002 | 2.94 (+) | 2.1e-009 | 152 | hsa-miR-182 |
| 5.5e+003 - 2.2e+003 | 2.48 (+) | 7.3e-014 | 50 | hsa-miR-342-3p |
| 6.6e+002 - 2.7e+002 | 2.48 (+) | 6.3e-008 | 45 | hsa-miR-29c* |
| 5.0e+002 - 2.2e+002 | 2.26 (+) | 5.8e-007 | 191 | hsa-miR-29c |
| 7.3e+003 - 3.3e+003 | 2.19 (+) | 1.3e-004 | 181 | hsa-miR-199a-3p |
| 2.2e+003 - 1.1e+003 | 2.05 (+) | 2.0e-006 | 179 | hsa-miR-195 |
| 2.2e+002 - 3.1e+003 | 13.81 (-) | 9.6e-014 | 49 | hsa-miR-31 |
| 6.3e+003 - 4.0e+004 | 6.32 (-) | 9.3e-008 | 32 | hsa-miR-205 |
| 8.9e+001 - 5.1e+002 | 5.72 (-) | 5.3e-010 | 42 | hsa-miR-224 |
| 1.5e+002 - 6.3e+002 | 4.05 (-) | 6.7e-007 | 184 | hsa-miR-203 |
| 5.7e+003 - 1.5e+004 | 2.64 (-) | 5.8e-018 | 40 | hsa-miR-222 |
| 3.8e+003 - 9.2e+003 | 2.41 (-) | 1.0e-018 | 147 | hsa-miR-221 |
| 4.8e+002 - 1.1e+003 | 2.39 (-) | 4.9e-010 | 236 | MID-00689 |
| 6.0e+002 - 1.4e+003 | 2.37 (-) | 1.2e-010 | 57 | hsa-miR-378 |
| 2.3e+002 - 5.2e+002 | 2.24 (-) | 2.2e-008 | 203 | hsa-miR-422a |

(continued)

| median values | fold-change | p-value | SEQ ID NO. | miR name |
|---|---|---|---|---|
| 1.2e+003 - 2.6e+003 | 2.22 (-) | 1.5e-007 | 36 | hsa-miR-210 |
| + the higher expression of this miR is in breast adenocarcinoma<br>- the higher expression of this miR is in SCC, TCC, thymomas and ovarian carcinoma | | | | |

hsa-miR-193a-3p (SEQ ID NO: 25), hsa-miR-375 (SEQ ID NO: 56) and hsa-miR-342-3p (SEQ ID NO: 50) are used at node 14 of the binary-tree-classifier detailed in the invention. According to another embodiment, hsa-miR-193a-3p (SEQ ID NO: 25), hsa-miR-375 (SEQ ID NO: 56) and hsa-miR-224 (SEQ ID NO: 42) may be used at node 14 of the binary-tree-classifier detailed in the invention.

**Table 16:** miR expression (in fluorescence units) distinguishing between the group consisting of ovarian carcinoma and the group consisting of SCC, TCC and thymomas

| median values | fold-change | p-value | SEQ ID NO. | miR name |
|---|---|---|---|---|
| 3.1e+003 - 5.5e+002 | 5.57 (+) | 1.2e-012 | 4 | hsa-miR-10a |
| 5.1e+003 - 1.5e+003 | 3.41 (+) | 1.7e-014 | 10 | hsa-miR-130a |
| 2.5e+002 - 7.5e+001 | 3.39 (+) | 1.3e-014 | 195 | hsa-miR-30a* |
| 2.4e+003 - 8.8e+002 | 2.68 (+) | 5.5e-009 | 5 | hsa-miR-10b |
| 2.9e+002 - 1.2e+002 | 2.48 (+) | 2.5e-012 | 226 | hsa-miR-625 |
| 8.8e+003 - 3.9e+003 | 2.28 (+) | 7.7e-012 | 2 | hsa-let-7e |
| 1.6e+003 - 7.3e+002 | 2.20 (+) | 3.6e-007 | 46 | hsa-miR-30a |
| 1.2e+003 - 4.6e+004 | 37.52 (-) | 1.0e-033 | 32 | hsa-miR-205 |
| 5.0e+001 - 2.7e+002 | 5.42 (-) | 4.2e-018 | 185 | hsa-miR-205* |
| 6.0e+001 2.8e+002 | 4.63 (-) | 1.1e-009 | 11 | hsa-miR-138 |
| 5.7e+002 - 2.4e+003 | 4.18 (-) | 5.2e-010 | 168 | hsa-miR-150 |
| 2.9e+002 - 8.0e+002 | 2.74 (-) | 2.5e-003 | 184 | hsa-miR-203 |
| 2.9e+002 - 7.6e+002 | 2.62 (-) | 2.1e-006 | 16 | hsa-miR-146a |
| 1.4e+003 - 3.4e+003 | 2.49 (-) | 1.9e-007 | 242 | MID-16489 |
| 9.5e+002 - 2.3e+003 | 2.42 (-) | 2.3e-015 | 12 | hsa-miR-140-3p |
| 7.6e+002 - 1.8e+003 | 2.37 (-) | 5.2e-006 | 237 | MID-15684 |
| 1.1e+003 - 2.5e+003 | 2.23 (-) | 9.8e-005 | 243 | MID-16869 |
| 2.1e+003 - 4.6e+003 | 2.18 (-) | 5.9e-004 | 250 | MID-20703 |
| 2.7e+003 - 5.8e+003 | 2.13 (-) | 6.5e-012 | 39 | hsa-miR-22 |
| 4.3e+002 - 9.2e+002 | 2.13 (-) | 1.0e-003 | 253 | MID-23256 |
| 1.7e+003 - 3.7e+003 | 2.12 (-) | 1.4e-002 | 49 | hsa-miR-31 |
| 1.7e+003 - 3.6e+003 | 2.06 (-) | 1.3e-003 | 246 | MID-18422 |
| 2.1e+003 - 4.4e+003 | 2.04 (-) | 1.4e-007 | 167 | hsa-miR-149* |
| + the higher expression of this miR is in ovarian carcinoma<br>- the higher expression of this miR is in SCC, TCC and thymomas | | | | |

hsa-miR-205 (SEQ ID NO: 32), hsa-miR-10a (SEQ ID NO: 4) and hsa-miR-22 (SEQ ID NO: 39) are used at node 15 of the binary-tree-classifier detailed in the invention.

**Table 17:** miR expression (in fluorescence units) distinguishing between the group consisting of thyroid carcinoma (follicular and papillary) and the group consisting of breast adenocarcinoma, lung large cell carcinoma, lung adenocarcinoma and ovarian carcinoma

| median values | fold-change | p-value | SEQ ID NO. | miR name |
|---|---|---|---|---|
| 4.1e+003 - 1.2e+002 | 33.86 (+) | 7.4e-033 | 11 | hsa-miR-138 |
| 3.4e+004 - 6.7e+003 | 5.03 (+) | 1.4e-009 | 147 | hsa-miR-221 |
| 4.9e+003 - 1.0e+003 | 4.74 (+) | 1.0e-006 | 17 | hsa-miR-146b-5p |
| 2.2e+004 - 5.8e+003 | 3.71 (+) | 2.8e-027 | 157 | hsa-let-7i |
| 3.9e+004 - 1.1e+004 | 3.63 (+) | 7.9e-009 | 40 | hsa-miR-222 |
| 5.4e+004 - 1.9e+004 | 2.78 (+) | 1.5e-014 | 8 | hsa-miR-125b |
| 1.3e+003 - 4.9e+002 | 2.78 (+) | 5.0e-003 | 49 | hsa-miR-31 |
| 6.9e+003 - 2.8e+003 | 2.48 (+) | 1.3e-008 | 9 | hsa-miR-126 |
| 8.1e+002 - 3.4e+002 | 2.36 (+) | 4.8e-007 | 191 | hsa-miR-29c |
| 1.3e+004 - 5.7e+003 | 2.33 (+) | 3.8e-003 | 205 | hsa-miR-451 |
| 4.8e+002 - 2.2e+002 | 2.16 (+) | 1.3e-003 | 207 | hsa-miR-486-5p |
| 4.9e+002 - 2.3e+002 | 2.12 (+) | 8.0e-006 | 195 | hsa-miR-30a* |
| 1.4e+003 - 6.6e+002 | 2.11 (+) | 2.7e-011 | 51 | hsa-miR-345 |
| .5e+003 - 1.7e+003 | 2.10 (+) | 1.5e-006 | 46 | hsa-miR-30a |
| 6.4e+002 - 3.2e+002 | 1.97 (+) | 1.5e-006 | 45 | hsa-miR-29c* |
| 7.4e+003 - 4.0e+003 | 1.88 (+) | 3.4e-007 | 52 | hsa-miR-34a |
| 6.1e+003 - 3.3e+003 | 1.88 (+) | 5.9e-008 | 234 | hsa-miR-1977 |
| 7.5e+003 - 4.1e+003 | 1.85 (+) | 1.5e-005 | 231 | hsa-miR-99a |
| 7.6e+003 -4.1e+003 | 1.85 (+) | 2.7e-004 | 21 | hsa-miR-181a |
| 1.0e+003 - 5.5e+002 | 1.82 (+) | 5.1e-008 | 169 | hsa-miR-152 |
| 1.1e+004 - 6.0e+003 | 1.79 (+) | 3.7e-008 | 43 | hsa-miR-29a |
| 5.4e+003 - 3.0e+003 | 1.77 (+) | 2.9e-005 | 3 | hsa-miR-100 |
| 5.9e+003 - 3.4e+003 | 1.73 (+) | 7.3e-007 | 196 | hsa-miR-30c |
| 2.1e+003 - 1.2e+003 | 1.69 (+) | 4.6e-004 | 154 | hsa-miR-181b |
| 4.4e+002 - 2.6e+002 | 1.66 (+) | 6.4e-003 | 390 | MID-00405 |
| 5.5e+002 - 3.3e+002 | 1.65 (+) | 5.3e-006 | 171 | hsa-miR-15a |
| 1.3e+003 - 8.1e+002 | 1.60 (+) | 6.0e-003 | 255 | MID-23794 |
| 2.3e+003 - 1.4e+003 | 1.57 (+) | 8.3e-007 | 197 | hsa-miR-331-3p |
| 1.8e+003 - 1.1e+003 | 1.57 (+) | 1.4e-005 | 190 | hsa-miR-29b |
| 3.6e+003 - 2.3e+003 | 1.56 (+) | 1.4e-003 | 189 | hsa-miR-27b |
| 6.6e+003 - 4.3e+003 | 1.52 (+) | 3.6e-005 | 39 | hsa-miR-22 |
| 9.9e+003 - 6.5e+003 | 1.52 (+) | 2.2e-006 | 7 | hsa-miR-125a-5p |
| 7.8e+002 - 5.2e+002 | 1.51 (+) | 6.2e-006 | 48 | hsa-miR-30e |
| 4.3e+003 - 2.8e+003 | 1.51 (+) | 1.2e-002 | 47 | hsa-miR-30d |
| 1.0e+002 - 2.2e+003 | 22.05 (-) | 2.2e-005 | 32 | hsa-miR-205 |
| 2.1e+002 - 1.8e+003 | 8.83 (-) | 4.8e-020 | 36 | hsa-miR-210 |

(continued)

| median values | fold-change | p-value | SEQ ID NO. | miR name |
|---|---|---|---|---|
| 3.2e+002 - 1.4e+003 | 4.34 (-) | 8.7e-011 | 4 | hsa-miR-10a |
| 4.9e+002 - 1.8e+003 | 3.59 (-) | 4.6e-014 | 178 | hsa-miR-193b |
| 1.0e+003 - 2.8e+003 | 2.74 (-) | 8.3e-006 | 37 | hsa-miR-214 |
| 2.0e+003 - 5.5e+003 | 2.67 (-) | 4.4e-005 | 181 | hsa-miR-199a-3p |
| 1.1e+003 - 2.8e+003 | 2.65 (-) | 3.1e-011 | 25 | hsa-miR-193a-3p |
| 2.7e+002 - 7.2e+002 | 2.63 (-) | 1.3e-004 | 183 | hsa-miR-199b-5p |
| 3.1e+003 - 8.1e+003 | 2.57 (-) | 1.6e-005 | 182 | hsa-miR-199a-5p |
| 5.1e+002 - 1.2e+003 | 2.41 (-) | 4.7e-006 | 35 | hsa-miR-21* |
| 2.0e+003 - 4.8e+003 | 2.39 (-) | 9.3e-003 | 240 | MID-15965 |
| 3.4e+002 - 8.0e+002 | 2.35 (-) | 3.0e-006 | 57 | hsa-miR-378 |
| 8.2e+002 - 1.9e+003 | 2.25 (-) | 1.2e-003 | 242 | MID-16489 |
| 6.0e+002 - 1.3e+003 | 2.18 (-) | 1.4e-011 | 204 | hsa-miR-425 |
| 3.0e+002 - 6.1e+002 | 2.06 (-) | 5.3e-006 | 236 | MID-00689 |
| 2.3e+002 - 4.5e+002 | 1.96 (-) | 2.0e-006 | 176 | hsa-miR-18a |
| 2.3e+003 - 4.3e+003 | 1.85 (-) | 3.5e-006 | 158 | hsa-miR-106a |
| 2.4e+003 - 4.4e+003 | 1.79 (-) | 1.9e-010 | 148 | hsa-miR-93 |
| 2.9e+002 - 5.2e+002 | 1.79 (-) | 1.9e-007 | 206 | hsa-miR-455-3p |
| 1.3e+003 - 2.3e+003 | 1.78 (-) | 7.8e-005 | 50 | hsa-miR-342-3p |
| 1.4e+003 - 2.5e+003 | 1.75 (-) | 6.0e-006 | 20 | hsa-miR-17 |
| 2.8e+004 - 4.8e+004 | 1.75 (-) | 5.1e-006 | 34 | hsa-miR-21 |
| 1.4e+003 - 2.4e+003 | 1.74 (-) | 1.1e-004 | 186 | hsa-miR-20a |
| 2.4e+002 - 4.1e+002 | 1.72 (-) | 8.4e-004 | 239 | MID-15907 |
| 3.0e+002 - 4.8e+002 | 1.62 (-) | 9.9e-003 | 251 | MID-21271 |
| 2.2e+003 - 3.6e+003 | 1.60 (-) | 4.3e-002 | 244 | MID-17144 |
| 3.8e+003 - 6.1e+003 | 1.59 (-) | 4.7e-006 | 24 | hsa-miR-191 |
| 1.0e+003 - 1.6e+003 | 1.59 (-) | 2.0e-005 | 188 | hsa-miR-25 |
| 2.1e+003 - 3.2e+003 | 1.57 (-) | 1.9e-002 | 172 | hsa-miR-15b |
| 2.6e+003 - 4.0e+003 | 1.56(-) | 9.5e-003 | 238 | MID-15867 |
| + the higher expression of this miR is in thyroid carcinoma | | | | |

the higher expression of this miR is in breast adenocarcinoma, lung large cell carcinoma, lung adenocarcinoma and ovarian carcinoma

Figure 12 demonstrates binary decisions at node #16 of the decision-tree. Tumors originating in thyroid carcinoma (diamonds) are easily distinguished from tumors of adenocarcinoma of the lung, breast and ovarian origin (squares) using the expression levels of hsa-miR-93 (SEQ ID NO: 148, y-axis), hsa-miR-138 (SEQ ID NO: 11, x-axis) and hsa-miR-10a (SEQ ID NO: 4, z-axis).

**Table 18:** miR expression (in fluorescence units) distinguishing between follicular thyroid carcinoma and papillary thyroid carcinoma

| median values | fold-change | p-value | SEQ ID NO. | miR name |
|---|---|---|---|---|
| 6.6e+003 - 7.1e+002 | 9.34 (+) | 4.5e-011 | 249 | MID-20524 |

(continued)

| median values | fold-change | p-value | SEQ ID NO. | miR name |
|---|---|---|---|---|
| 1.7e+003 - 2.2e+002 | 7.80 (+) | 1.9e-008 | 180 | hsa-miR-1973 |
| 4.5e+002 - 5.9e+001 | 7.58 (+) | 8.3e-005 | 65 | hsa-miR-7 |
| 2.5e+003 - 3.8e+002 | 6.52 (+) | 4.8e-007 | 235 | hsa-miR-1978 |
| 2.2e+003 - 3.6e+002 | 6.14 (+) | 1.5e-008 | 241 | MID-16318 |
| 4.2e+002 - 7.1e+001 | 6.00 (+) | 3.0e-004 | 248 | MID-19533 |
| 9.6e+002 - 1.7e+002 | 5.76 (+) | 1.6e-008 | 254 | MID-23291 |
| 9.9e+002 - 1.9e+002 | 5.33 (+) | 3.2e-005 | 247 | MID-19340 |
| 6.4e+002 - 1.2e+002 | 5.17 (+) | 6.8e-009 | 160 | hsa-miR-1248 |
| 1.5e+003 - 3.0e+002 | 4.97 (+) | 1.1e-006 | 243 | MID-16869 |
| 2.7e+003 - 6.1e+002 | 4.48 (+) | 1.4e-010 | 245 | MID-18336 |
| 5.0e+002 - 1.2e+002 | 4.00 (+) | 7.0e-004 | 252 | MID-22664 |
| 4.0e+002 - 2.5e+004 | 62.88 (-) | 6.7e-011 | 17 | hsa-miR-146b-5p |
| 4.4e+002 - 8.2e+003 | 18.72 (-) | 2.5e-008 | 49 | hsa-miR-31 |
| 5.0e+001 - 9.3e+002 | 18.69 (-) | 5.0e-012 | 166 | hsa-miR-146b-3p |
| 7.6e+001 - 8.3e+002 | 10.86 (-) | 4.8e-006 | 225 | hsa-miR-551b |
| 3.1e+002 - 3.3e+003 | 10.71 (-) | 3.2e-007 | 168 | hsa-miR-150 |
| 1.1e+004 - 4.7e+004 | 4.40 (-) | 3.4e-007 | 34 | hsa-miR-21 |
| $^{+}$ the higher expression of this miR is in follicular thyroid carcinoma<br>$^{-}$ the higher expression of this miR is in papillary thyroid carcinoma | | | | |

Figure 13 demonstrates binary decisions at node #17 of the decision-tree. Tumors originating in follicular thyroid carcinoma (marked by diamonds) are easily distinguished from tumors of papillary thyroid carcinoma origins (marked by squares) using the expression levels of hsa-miR-21 (SEQ ID NO: 34, y-axis) and hsa-miR-146b-5p (SEQ ID NO: 17, x-axis).

**Table 19:** miR expression (in fluorescence units) distinguishing between the group consisting of breast adenocarcinoma and the group consisting of lung adenocarcinoma and ovarian carcinoma

| median values | fold-change | p-value | SEQ ID NO. | miR name |
|---|---|---|---|---|
| 6.3e+003 - 6.0e+002 | 10.55 (+) | 8.8e-005 | 32 | hsa-miR-205 |
| 1.3 e+003 - 1.5e+002 | 8.43 (+) | 7.9e-006 | 56 | hsa-miR-375 |
| 5.5e+003 - 1.7e+003 | 3.17 (+) | 7.7e-012 | 50 | hsa-miR-342-3p |
| 6.6e+002 - 2.6e+002 | 2.52 (+) | 2.2e-008 | 45 | hsa-miR-29c* |
| 4.4e+003 - 2.0e+003 | 2.23 (+) | 2.2e-012 | 25 | hsa-miR-193a-3p |
| 9.5e+002 - 4.3e+002 | 2.20 (+) | 7.9e-005 | 253 | MID-23256 |
| 1.2e+003 -5.6e+002 | 2.15 (+) | 4.9e-005 | 152 | hsa-miR-182 |
| 3.7e+003 - 1.9e+003 | 1.94 (+) | 5.3e-005 | 9 | hsa-miR-126 |
| 2.7e+003 - 1.4e+003 | 1.90 (+) | 2.9e-004 | 46 | hsa-miR-30a |
| 5.0e+002 - 2.8e+002 | 1.78 (+) | 1.1e-004 | 191 | hsa-miR-29c |
| 2.4e+003 - 1.4e+003 | 1.72 (+) | 1.5e-006 | 178 | hsa-miR-193b |
| 2.2 e+002 - 1.3e+003 | 5.79 (-) | 7.7e-006 | 49 | hsa-miR-31 |

(continued)

| median values | fold-change | p-value | SEQ ID NO. | miR name |
|---|---|---|---|---|
| 5.7 e+003 - 1.5e+004 | 2.69 (-) | 3.0e-010 | 40 | hsa-miR-222 |
| 1.4e+003 - 3.4e+003 | 2.50 (-) | 1.6e-006 | 10 | hsa-miR-130a |
| 3.8e+003 - 9.3e+003 | 2.41 (-) | 1.2e-009 | 147 | hsa-miR-221 |
| 9.3e+002 - 2.2e+003 | 2.33 (-) | 2.2e-002 | 4 | hsa-miR-10a |
| 1.2e+003 - 2.5e+003 | 2.09 (-) | 1.5e-005 | 36 | hsa-miR-210 |
| 1.3e+003 - 2.6e+003 | 2.01 (-) | 9.9e-005 | 228 | hsa-miR-886-3p |
| 4.8e+002 - 9.4e+002 | 1.95 (-) | 4.6e-004 | 236 | MID-00689 |
| 5.3e+002 - 1.0e+003 | 1.92 (-) | 6.3e-004 | 230 | hsa-miR-886-5p |
| 1.8e+003 - 3.3e+003 | 1.86 (-) | 7.1e-005 | 189 | hsa-miR-27b |
| 3.6e+003 - 6.4e+003 | 1.79 (-) | 6.1e-003 | 240 | MID-15965 |
| 2.7e+003 - 4.7e+003 | 1.75 (-) | 3.5e-005 | 67 | hsa-miR-92a |
| 6.0e+002 - 1.0e+003 | 1.73 (-) | 3.0e-004 | 202 | hsa-miR-378 |
| 8.0e+002 - 1.4e+003 | 1.71 (-) | 2.9e-004 | 17 | hsa-miR-146b-5p |
| + the higher expression of this miR is in breast adenocarcinoma<br>- the higher expression of this miR is in lung adenocarcinoma and ovarian carcinoma | | | | |

Figure 14 demonstrates binary decisions at node #18 of the decision-tree. Tumors originating in breast (diamonds) are easily distinguished from tumors of lung and ovarian origin (squares) using the expression levels of hsa-miR-92a (SEQ ID NO: 67, y-axis), hsa-miR-193a-3p (SEQ ID NO: 25, x-axis) and hsa-miR-31 (SEQ ID NO: 49, z-axis).

**Table 20:** miR expression (in fluorescence units) distinguishing between lung adenocarcinoma and ovarian carcinoma

| median values | fold-change | p-value | SEQ ID NO. | miR name |
|---|---|---|---|---|
| 1.4e+003 - 5.2e+001 | 27.96 (+) | 3.5e-008 | 56 | hsa-miR-375 |
| 5.5e+002 - 6.0e+001 | 9.19 (+) | 8.1e-009 | 11 | hsa-miR-138 |
| 3.2e+003 - 5.7e+002 | 5.65 (+) | 5.6e-004 | 168 | hsa-miR-150 |
| 9.7e+002 - 2.9e+002 | 3.35 (+) | 2.2e-004 | 16 | hsa-miR-146a |
| 2.3e+003 - 7.6e+002 | 2.96 (+) | 3.2e-003 | 237 | MID-15684 |
| 8.4e+003 - 2.9e+003 | 2.88 (+) | 1.7e-010 | 21 | hsa-miR-181a |
| 7.0e+003 - 2.6e+003 | 2.69 (+) | 9.2e-008 | 52 | hsa-miR-34a |
| 2.4e+003 - 9.5e+002 | 2.58 (+) | 3.2e-007 | 12 | hsa-miR-140-3p |
| 2.1e+003 - 8.8e+002 | 2.39 (+) | 2.1e-007 | 154 | hsa-miR-181b |
| 1.4e+005 - 6.3e+004 | 2.28 (+) | 1.9e-003 | 279 | hsa-miR-1826 |
| 3.2e+003 - 1.4e+003 | 2.25 (+) | 6.1e-003 | 9 | hsa-miR-126 |
| 6.1e+003 - 2.7e+003 | 2.24 (+) | 2.3e-006 | 39 | hsa-miR-22 |
| 4.2e+003 - 2.2e+003 | 1.93 (+) | 8.9e-005 | 47 | hsa-miR-30d |
| 1.9e+003 - 1.0e+003 | 1.90 (+) | 3.5e-006 | 23 | hsa-miR-185 |
| 2.8e+003 - 1.5e+003 | 1.88 (+) | 1.4e-003 | 50 | hsa-miR-342-3p |
| 3.6e+003 - 2.1e+003 | 1.69 (+) | 1.8e-002 | 167 | hsa-miR-149* |
| 9.7e+002 - 5.9e+002 | 1.66 (+) | 8.7e-005 | 383 | MID-22912 |
| 6.8e+004 - 4.2e+004 | 1.64 (+) | 5.4e-004 | 34 | hsa-miR-21 |

(continued)

| median values | fold-change | p-value | SEQ ID NO. | miR name |
|---|---|---|---|---|
| 1.8e+003 - 1.2e+003 | 1.55 (+) | 5.1e-004 | 35 | hsa-miR-21* |
| 1.4e+003 - 8.9e+002 | 1.54 (+) | 1.7e-004 | 388 | hsa-miR-423-5p |
| 4.4e+002 - 2.4e+003 | 5.38 (-) | 1.0e-007 | 5 | hsa-miR-10b |
| 1.9e+002 - 7.2e+002 | 3.77 (-) | 3.3e-006 | 359 | hsa-miR-708 |
| 6.4e+002-2.1e+003 | 3.27 (-) | 5.8e-003 | 245 | MID-18336 |
| 1.8e+002 - 5.4e+002 | 2.96 (-) | 6.6e-003 | 254 | MID-23291 |
| 1.7e+003 - 5.1e+003 | 2.95 (-) | 3.4e-005 | 10 | hsa-miR-130a |
| 2.8e+003 - 8.1e+003 | 2.86 (-) | 3.7e-003 | 240 | MID-15965 |
| 5.1e+002 - 1.3e+003 | 2.65 (-) | 3.7e-004 | 236 | MID-00689 |
| 6.1e+002 - 1.6e+003 | 2.63 (-) | 1.8e-004 | 202 | hsa-miR-378 |
| 1.3e+003 - 3.1e+003 | 2.39 (-) | 1.2e-002 | 4 | hsa-miR-10a |
| 1.0e+003 - 2.3e+003 | 2.30 (-) | 1.8e-006 | 25 | hsa-miR-193a-3p |
| 2.6e+002 - 5.6e+002 | 2.15 (-) | 4.1e-004 | 203 | hsa-miR-422a |
| 3.0e+003 - 6.1e+003 | 2.04 (-) | 1.8e-002 | 231 | hsa-miR-99a |
| 2.0e+003 - 3.9e+003 | 2.01 (-) | 3.5e-005 | 20 | hsa-miR-17 |
| 3.3e+003 - 6.4e+003 | 1.96 (-) | 3.5e-005 | 158 | hsa-miR-106a |
| 1.8e+003 - 3.5e+003 | 1.88 (-) | 3.1e-005 | 186 | hsa-miR-20a |
| 3.2e+003 - 5.9e+003 | 1.85 (-) | 1.2e-004 | 258 | hsa-let-7f |
| 2.4e+003 - 4.4e+003 | 1.85 (-) | 2.0e-003 | 244 | MID-17144 |
| 2.0e+003 - 3.5e+003 | 1.72 (-) | 8.7e-004 | 172 | hsa-miR-15b |
| 5.2e+003 - 8.8e+003 | 1.69 (-) | 5.3e-004 | 2 | hsa-let-7e |
| 4.1e+002 - 6.8e+002 | 1.66 (-) | 1.2e-002 | 235 | hsa-miR-1978 |
| 3.3e+004 - 5.4e+004 | 1.66 (-) | 3.3e-005 | 256 | hsa-let-7a |
| 2.8e+003 - 4.7e+003 | 1.65 (-) | 3.5e-003 | 28 | hsa-miR-200a |
| 5.2e+002 - 8.5e+002 | 1.62 (-) | 1.6e-004 | 277 | hsa-miR-17* |
| 3.7e+002 - 5.8e+002 | 1.57 (-) | 1.9e-003 | 296 | hsa-miR-26b |
| 1.0e+005 - 1.6e+005 | 1.56 (-) | 3.7e-002 | 374 | MID-16748 |
| 6.0e+003 - 9.1e+003 | 1.53 (-) | 2.1e-005 | 153 | hsa-let-7d |
| 3.8e+003 - 5.7e+003 | 1.50 (-) | 4.1e-003 | 181 | hsa-miR-199a-3p |
| $^{+}$ the higher expression of this miR is in lung adenocarcinoma<br>$^{-}$ the higher expression of this miR is in ovarian carcinoma | | | | |

Figure 15 demonstrates binary decisions at node #19 of the decision-tree. Tumors originating in lung adenocarcinoma (diamonds) are easily distinguished from tumors of ovarian carcinoma origin (squares) using the expression levels of hsa-miR-21 (SEQ ID NO: 34, y-axis), hsa-miR-378 (SEQ ID NO: 202, x-axis) and hsa-miR-138 (SEQ ID NO: 11, z-axis).

**Table 21:** miR expression (in fluorescence units) distinguishing between the group consisting of thymic carcinoma and the group consisting of TCC and SCC

| median values | fold-change | p-value | SEQ ID NO. | miR name |
|---|---|---|---|---|
| 5.3 e+002 - 5.9e+001 | 9.00 (+) | 5.7e-026 | 161 | hsa-miR-128 |
| 7.4e+002 - 9.2e+001 | 8.04 (+) | 2.2e-007 | 164 | hsa-miR-142-5p |
| 6.8e+002 - 8.8e+001 | 7.82 (+) | 2.6e-021 | 22 | hsa-miR-181a* |
| 7.1e+002 - 1.2e+002 | 6.09 (+) | 1.2e-006 | 53 | hsa-miR-34c-5p |
| 9.1e+002 - 1.8e+002 | 5.06 (+) | 2.2e-008 | 285 | hsa-miR-20b |
| 1.3e+004 - 2.8e+003 | 4.59 (+) | 7.5e-014 | 3 | hsa-miR-100 |
| 1.6e+003 - 3.6e+002 | 4.39 (+) | 7. 1e-007 | 152 | hsa-miR-182 |
| 8.7e+002 - 2.0e+002 | 4.37 (+) | 6.4e-010 | 191 | hsa-miR-29c |
| 3.7e+003 - 9.1e+002 | 4.09 (+) | 2.6e-014 | 154 | hsa-miR-181b |
| 1.5e+004 - 3.8e+003 | 3.82 (+) | 4.8e-009 | 21 | hsa-miR-181a |
| 2.1e+003 - 6.4e+002 | 3.25 (+) | 6.4e-006 | 206 | hsa-miR-455-3p |
| 8.7e+002 - 2.7e+002 | 3.23 (+) | 1.5e-010 | 174 | hsa-miR-181d |
| 9.4e+002 - 2.9e+002 | 3.23 (+) | 1.9e-004 | 19 | hsa-miR-149 |
| 7.3e+002 - 2.6e+002 | 2.80 (+) | 2.5e-008 | 45 | hsa-miR-29c* |
| 8.7e+002 - 3.2e+002 | 2.69 (+) | 2.2e-007 | 171 | hsa-miR-15a |
| 2.7e+003 - 1.0e+003 | 2.66 (+) | 5.1e-005 | 179 | hsa-miR-195 |
| 4.4e+004 - 1.8e+004 | 2.46 (+) | 8.2e-008 | 8 | hsa-miR-125b |
| 7.3e+002 - 3.2e+002 | 2.26 (+) | 2.8e-004 | 296 | hsa-miR-26b |
| 2.7e+003 - 1.2e+003 | 2.22 (+) | 2.4e-003 | 284 | hsa-miR-19b |
| 5.7e+002 - 2.6e+002 | 2.17 (+) | 7.8e-005 | 18 | hsa-miR-148a |
| 9.1e+002 - 4.4e+002 | 2.06 (+) | 1.4e-006 | 51 | hsa-miR-345 |
| 7.5e+003 - 3.8e+003 | 2.00 (+) | 1.6e-002 | 258 | hsa-let-7f |
| 1.8e+002 - 4.4e+003 | 24.66 (-) | 3.9e-008 | 49 | hsa-miR-31 |
| 5.8e+001 - 1.0e+003 | 17.65 (-) | 1.0e-007 | 184 | hsa-miR-203 |
| 2.2e+002 - 1.6e+003 | 7.43 (-) | 4.5e-018 | 35 | hsa-miR-21* |
| 1.1e+004 - 5.5e+004 | 4.97 (-) | 1.5e-032 | 34 | hsa-miR-21 |
| 6.2e+002 - 2.5e+003 | 4.06 (-) | 2.0e-008 | 37 | hsa-miR-214 |
| 1.6e+002 - 5.8e+002 | 3.69 (-) | 6.3e-005 | 42 | hsa-miR-224 |
| 6.9e+002 - 2.5e+003 | 3.58 (-) | 2.3e-009 | 228 | hsa-miR-886-3p |
| 4.8e+003 - 1.7e+004 | 3.47 (-) | 3.9e-009 | 15 | hsa-miR-145 |
| 2.7e+003 - 8.2e+003 | 3.08 (-) | 2.7e-008 | 14 | hsa-miR-143 |
| 1.3e+003 - 3.7e+003 | 2.93 (-) | 6.7e-005 | 242 | MID-16489 |
| 2.5e+002 - 7.4e+002 | 2.91 (-) | 4.3e-006 | 230 | hsa-miR-886-5p |
| 3.5e+002 - 1.0e+003 | 2.90 (-) | 5.6e-004 | 253 | MID-23256 |
| 1.1e+003 - 3.0e+003 | 2.82 (-) | 7.9e-006 | 36 | hsa-miR-210 |
| 2.2e+003 - 5.8e+003 | 2.63 (-) | 1.2e-007 | 182 | hsa-miR-199a-5p |

(continued)

| median values | fold-change | p-value | SEQ ID NO. | miR name |
|---|---|---|---|---|
| 5.0e+003 - 1.2e+004 | 2.48 (-) | 3.8e-006 | 293 | hsa-miR-23b |
| 7.5e+003 - 1.8e+004 | 2.44 (-) | 1.1e-008 | 292 | hsa-miR-23a |
| 2.7e+002 - 6.6e+002 | 2.43 (-) | 5.4e-003 | 4 | hsa-miR-10a |
| 9.0e+003 - 2.2e+004 | 2.43 (-) | 5.5e-015 | 294 | hsa-miR-24 |
| 3.8e+003 - 8.8e+003 | 2.35 (-) | 3.0e-004 | 297 | hsa-miR-27a |
| 2.3e+002 - 5.2e+002 | 2.28 (-) | 1.6e-002 | 354 | hsa-miR-612 |
| 1.8e+003 - 3.9e+003 | 2.22 (-) | 1.1e-006 | 377 | MID-17866 |
| 1.6e+003 - 3.3e+003 | 2.11 (-) | 2.4e-004 | 189 | hsa-miR-27b |
| 6.9e+003 - 1.4e+004 | 2.08 (-) | 1.5e-004 | 30 | hsa-miR-200c |
| 3.8e+004 - 7.9e+004 | 2.05 (-) | 1.8e-008 | 386 | MID-23178 |
| 1.5e+003 - 3.1e+003 | 2.04 (-) | 2.6e-002 | 249 | MID-20524 |
| 4.6e+002 - 9.3e+002 | 2.03 (-) | 5.6e-002 | 5 | hsa-miR-10b |
| 2.5e+002 - 5.0e+002 | 2.03 (-) | 3.2e-005 | 274 | hsa-miR-151-3p |
| + the higher expression of this miR is in thymic carcinoma<br>- the higher expression of this miR is in TCC and SCC | | | | |

Figure 16 demonstrates binary decisions at node #20 of the decision-tree. Tumors originating in thymic carcinoma (marked by diamonds) are easily distinguished from tumors of urothelial carcinoma, transitional cell carcinoma (TCC) carcinoma and squamous cell carcinoma (SCC) origins (marked by squares) using the expression levels of hsa-miR-21 (SEQ ID NO: 34, y-axis) and hsa-miR-100 (SEQ ID NO: 3, x-axis).

**Table 22:** miR expression (in fluorescence units) distinguishing between TCC and SCC (of anus, skin, lung, head&neck, esophagus or uterine cervix)

| median values | fold-change | p-value | SEQ ID NO. | miR name |
|---|---|---|---|---|
| 2.5e+002 - 5.0e+001 | 5.05 (+) | 7.4e-036 | 69 | hsa-miR-934 |
| 9.1e+003 - 2.2e+003 | 4.14 (+) | 1.2e-012 | 28 | hsa-miR-200a |
| 2.1e+002 - 5.3e+001 | 3.87 (+) | 8.4e-007 | 280 | hsa-miR-187 |
| 6.0e+003 - 1.9e+003 | 3.19 (+) | 9.8e-008 | 13 | hsa-miR-141 |
| 5.6e+002 - 1.8e+002 | 3.15 (+) | 4.7e-013 | 191 | hsa-miR-29c |
| 9.4e+002 - 3.0e+002 | 3.13 (+) | 8.1e-008 | 152 | hsa-miR-182 |
| 1.8e+004 - 6.2e+003 | 2.99 (+) | 3.9e-010 | 29 | hsa-miR-200b |
| 3.2e+002 - 1.1e+002 | 2.81 (+) | 8.8e-009 | 175 | hsa-miR-183 |
| 3.1e+004 - 1.2e+004 | 2.65 (+) | 8.1e-005 | 30 | hsa-miR-200c |
| 2.1e+003 - 8.1e+002 | 2.63 (+) | 6.2e-020 | 204 | hsa-miR-425 |
| 1.2e+003 - 4.8e+002 | 2.41 (+) | 6.3e-007 | 4 | hsa-miR-10a |
| 8.5e+003 - 3.7e+003 | 2.30 (+) | 2.4e-024 | 24 | hsa-miR-191 |
| 1.8e+003 - 8.4e+002 | 2.14 (+) | 2.7e-006 | 5 | hsa-miR-10b |
| 3.5e+002 - 1.7e+002 | 2.09 (+) | 2.0e-006 | 329 | hsa-miR-425* |
| 3.4e+002 - 1.6e+002 | 2.08 (+) | 8.0e-005 | 273 | hsa-miR-148b |
| 3.1e+002 - 8.1e+002 | 2.60 (-) | 1.6e-005 | 170 | hsa-miR-155 |

(continued)

| median values | fold-change | p-value | SEQ ID NO. | miR name |
|---|---|---|---|---|
| 5.0e+002 - 1.2e+003 | 2.49 (-) | 1.0e-002 | 184 | hsa-miR-203 |
| 1.5e+002 - 3.5e+002 | 2.39 (-) | 3.0e-011 | 26 | hsa-miR-193a-5p |
| 1.9e+003 - 4.5e+003 | 2.35 (-) | 1.3e-008 | 231 | hsa-miR-99a |
| 1.2e+002 - 2.7e+002 | 2.28 (-) | 1.6e-003 | 368 | MID-00672 |
| 1.4e+003 - 3.2e+003 | 2.25 (-) | 1.3e-005 | 37 | hsa-miR-214 |
| 3.9e+002 - 8.7e+002 | 2.23 (-) | 1.5e-004 | 16 | hsa-miR-146a |
| 3.5e+002 - 7.6e+002 | 2.15 (-) | 4.2e-005 | 169 | hsa-miR-152 |
| 1.5e+002 - 3.3e+002 | 2.15 (-) | 4.4e-004 | 155 | hsa-miR-127-3p |
| 8.4e+002 - 1.7e+003 | 2.08 (-) | 1.6e-005 | 35 | hsa-miR-21* |
| 7.7e+003 - 1.6e+004 | 2.07 (-) | 5.3e-012 | 40 | hsa-miR-222 |
| 4.4e+002 - 9.0e+002 | 2.06 (-) | 1.5e-005 | 17 | hsa-miR-146b-5p |
| $^{+}$ the higher expression of this miR is in TCC<br>$^{-}$ the higher expression of this miR is in SCC | | | | |

hsa-miR-934 (SEQ ID NO: 69), hsa-miR-191 (SEQ ID NO: 24) and hsa-miR-29c (SEQ ID NO: 191) are used at node #21 of the binary-tree-classifier detailed in the invention to distinguish between TCC and SCC.

**Table 23:** miR expression (in fluorescence units) distinguishing between SCC of the uterine cervix and other SCC tumors (anus, skin, lung, head& neck or esophagus)

| median values | auROC | fold-change | p-value | SEQ ID NO. | miR name |
|---|---|---|---|---|---|
| 2.4e+002 - 9.2e+001 | 0.65 | 2.57 (+) | 2.0e-002 | 164 | hsa-miR-142-5p |
| 1.6e+003 - 7.6e+002 | 0.85 | 2.13 (+) | 1.7e-005 | 5 | hsa-miR-10b |
| 8.9e+003 - 4.4e+003 | 0.74 | 2.01 (+) | 2.1e-004 | 231 | hsa-miR-99a |
| 1.2e+003 - 9.8e+002 | 0.71 | 1.24 (+) | 1.2e-002 | 54 | hsa-miR-361-5p |
| 3.4e+004 - 2.7e+004 | 0.71 | 1.24 (+) | 3.9e-004 | 1 | hsa-let-7c |
| 1.3e+003 - 4.3e+003 | 0.81 | 3.39 (-) | 9.9e-006 | 242 | MID-16489 |
| 3.9e+002 - 1.2e+003 | 0.74 | 3.10 (-) | 2.1e-003 | 372 | MID-16469 |
| 1.1e+003 - 3.3e+003 | 0.84 | 3.09 (-) | 1.3e-008 | 249 | MID-20524 |
| 1.7e+003 - 5.2e+003 | 0.78 | 3.01 (-) | 2.4e-005 | 167 | hsa-miR-149* |
| 2.7e+002 - 8.0e+002 | 0.79 | 2.97 (-) | 1.4e-004 | 254 | MID-23291 |
| 2.2e+002 - 6.2e+002 | 0.76 | 2.77 (-) | 1.7e-004 | 354 | hsa-miR-612 |

(continued)

| median values | auROC | fold-change | p-value | SEQ ID NO. | miR name |
|---|---|---|---|---|---|
| 5.7e+002 - 1.5e+003 | 0.76 | 2.65 (-) | 7.8e-006 | 381 | MID-19962 |
| 2.3e+002 - 6.0e+002 | 0.79 | 2.63 (-) | 2.1e-005 | 380 | MID-19898 |
| 9.8e+002 - 2.4e+003 | 0.78 | 2.44 (-) | 2.8e-005 | 245 | MID-18336 |
| 1.2e+002 - 2.8e+002 | 0.73 | 2.34 (-) | 5.3e-003 | 358 | hsa-miR-665 |
| 6.1e+002 - 1.4e+003 | 0.70 | 2.31 (-) | 6.1e-003 | 364 | MID-00064 |
| 2.9e+003 - 6.7e+003 | 0.81 | 2.30 (-) | 8.8e-008 | 240 | MID-15965 |
| 1.2e+002 - 2.8e+002 | 0.66 | 2.26 (-) | 1.5e-002 | 11 | hsa-miR-138 |
| 1.0e+002 - 2.3e+002 | 0.77 | 2.24 (-) | 8.9e-005 | 378 | MID-18307 |
| $^+$ the higher expression of this miR is in SCC of the uterine cervix<br>$^-$ the higher expression of this miR is in other SCC tumors | | | | | |

Figure 17 demonstrates binary decisions at node #22 of the decision-tree. Tumors originating in SCC of the uterine cervix (diamonds) are easily distinguished from tumors of other SCC origin (squares) using the expression levels of hsa-miR-361-5p (SEQ ID NO: 54, y-axis), hsa-let-7c (SEQ ID NO: 1, x-axis) and hsa-miR-10b (SEQ ID NO: 5, z-axis).

**Table 24:** miR expression (in fluorescence units) distinguishing between anus or skin SCC and upper SCC tumors (lung, head& neck or esophagus)

| median values | auROC | fold-change | p-value | SEQ ID NO. | miR name |
|---|---|---|---|---|---|
| 3.2e+002 - 5.0e+001 | 0.78 | 6.38 (+) | 3.0e-006 | 305 | hsa-miR-31* |
| 4.3e+003 - 8.0e+002 | 0.80 | 5.39 (+) | 1.8e-006 | 184 | hsa-miR-203 |
| 8.6e+002 - 2.5e+002 | 0.78 | 3.49 (+) | 1.8e-006 | 41 | hsa-miR-223 |
| 1.7e+003 - 5.4e+002 | 0.80 | 3.12 (+) | 3.5e-006 | 183 | hsa-miR-199b-5p |
| 9.4e+003 - 3.5e+003 | 0.70 | 2.73 (+) | 2.4e-003 | 49 | hsa-miR-31 |
| 8.7e+003 - 3.2e+003 | 0.86 | 2.71 (+) | 3.6e-007 | 382 | MID-22331 |
| 1.9e+003 - 7.1e+002 | 0.87 | 2.68 (+) | 1.7e-008 | 235 | hsa-miR-1978 |
| 2.4e+002 - 9.2e+001 | 0.83 | 2.55 (+) | 9.6e-009 | 291 | hsa-miR-222* |
| 6.8e+003 - 2.9e+003 | 0.74 | 2.31 (+) | 7.4e-004 | 181 | hsa-miR-199a-3p |

(continued)

| median values | auROC | fold-change | p-value | SEQ ID NO. | miR name |
|---|---|---|---|---|---|
| 1.5e+003 - 6.7e+002 | 0.88 | 2.28 (+) | 7.1e-007 | 5 | hsa-miR-10b |
| 5.3e+002 - 2.4e+002 | 0.75 | 2.21 (+) | 1.4e-004 | 296 | hsa-miR-26b |
| 3.4e+002 - 1.6e+002 | 0.74 | 2.19 (+) | 7.7e-005 | 289 | hsa-miR-22* |
| 1.3e+003 - 6.0e+002 | 0.71 | 2.13 (+) | 1.2e-003 | 206 | hsa-miR-455-3p |
| 7.9e+003 - 3.8e+003 | 0.84 | 2.11 (+) | 4.2e-006 | 338 | hsa-miR-494 |
| 2.9e+002 - 1.4e+002 | 0.73 | 2.08 (+) | 1.1e-004 | 334 | hsa-miR-483-5p |
| 2.8e+003 - 1.3e+003 | 0.82 | 2.07 (+) | 4.5e-006 | 25 | hsa-miR-193a-3p |
| 1.1e+002 - 3.3e+002 | 0.77 | 3.03 (-) | 2.3e-005 | 11 | hsa-miR-138 |
| 1.3e+002 - 3.1e+002 | 0.65 | 2.29 (-) | 1.5e-002 | 19 | hsa-miR-149 |
| 9.7e+001 - 2.1e+002 | 0.75 | 2.16 (-) | 4.0e-005 | 198 | hsa-miR-342-5p |
| 1.1e+003 1.8e+003 | 0.83 | 1.63 (-) | 1.1e-006 | 23 | hsa-miR-185 |
| $^+$ the higher expression of this miR is in anus or skin SCC<br>$^-$ the higher expression of this miR is in upper SCC tumors | | | | | |

hsa-miR-10b (SEQ ID NO: 5), hsa-miR-138 (SEQ ID NO: 11) and hsa-miR-185 (SEQ ID NO: 23) are used at node 23 of the binary-tree-classifier detailed in the invention to distinguish between anus or skin SCC and upper SCC tumors.

**Table 25:** miR expression (in fluorescence units) distinguishing between melanoma and lymphoma (B-cell or T-cell) tumors

| median values | auROC | fold-change | p-value | SEQ ID NO. | miR name |
|---|---|---|---|---|---|
| 1.7e+003 - 3.0e+002 | 0.89 | 5.81 (+) | 2.8e-010 | 4 | hsa-miR-10a |
| 1.9e+003 - 6.0e+002 | 0.80 | 3.13 (+) | 7.9e-005 | 11 | hsa-miR-138 |
| 1.7e+003 - 5.7e+002 | 0.94 | 2.98 (+) | 2.3e-011 | 46 | hsa-miR-30a |
| 2.5e+004 - 8.8e+003 | 0.87 | 2.83 (+) | 1.1e-009 | 8 | hsa-miR-125b |
| 6.2e+002 - 2.3e+002 | 0.94 | 2.74 (+) | 9.2e-011 | 274 | hsa-miR-151-3p |
| 9.2e+002 - 3.4e+002 | 0.87 | 2.70 (+) | 1.9e-007 | 169 | hsa-miR-152 |

(continued)

| median values | auROC | fold-change | p-value | SEQ ID NO. | miR name |
|---|---|---|---|---|---|
| 1.6e+003 - 6.0e+002 | 0.77 | 2.60 (+) | 2.0e-004 | 36 | hsa-miR-210 |
| 4.8e+003 - 1.9e+003 | 0.90 | 2.56 (+) | 2.1e-011 | 47 | hsa-miR-30d |
| 1.2e+003 - 5.5e+002 | 0.88 | 2.26 (+) | 2.5e-008 | 363 | hsa-miR-99b |
| 2.4e+003 - 1.1e+003 | 0.85 | 2.24 (+) | 1.4e-006 | 231 | hsa-miR-99a |
| 6.5e+003 - 3.0e+003 | 0.80 | 2.17 (+) | 2.2e-005 | 303 | hsa-miR-30b |
| 6.4e+002 - 3.0e+002 | 0.86 | 2.14 (+) | 2.9e-008 | 349 | hsa-miR-532-5p |
| 2.1e+003 - 1.0e+003 | 0.86 | 2.08 (+) | 1.6e-006 | 10 | hsa-miR-130a |
| 5.4e+003 - 2.6e+003 | 0.81 | 2.06 (+) | 8.3e-006 | 7 | hsa-miR-125a-5p |
| 3.6e+003 - 1.8e+003 | 0.82 | 2.05 (+) | 2.5e-006 | 3 | hsa-miR-100 |
| 7.9e+003 - 3.9e+003 | 0.69 | 2.04 (+) | 1.5e-002 | 16 | hsa-miR-146a |
| 1.6e+002 - 2.2e+003 | 0.93 | 13.84 (-) | 1.1e-014 | 164 | hsa-miR-142-5p |
| 7.2e+002 - 7.5e+003 | 0.93 | 10.40 (-) | 1.1e-013 | 170 | hsa-miR-155 |
| 2.0e+003 - 1.4e+004 | 0.90 | 7.18 (-) | 2.2e-010 | 168 | hsa-miR-150 |
| 1.7e+002 - 7.0e+002 | 0.91 | 4.14 (-) | 5.2e-011 | 198 | hsa-miR-342-5p |
| 2.2e+003 - 8.3e+003 | 0.97 | 3.83 (-) | 6.2e-019 | 50 | hsa-miR-342-3p |
| 9.1e+002 - 2.6e+003 | 0.86 | 2.87 (-) | 1.3e-008 | 245 | MID-18336 |
| 2.3e+002 - 6.4e+002 | 0.77 | 2.74 (-) | 2.4e-004 | 365 | MID-00078 |
| 1.9e+002 - 5.2e+002 | 0.78 | 2.68 (-) | 4.7e-004 | 45 | hsa-miR-29c* |
| 2.8e+003 - 6.6e+003 | 0.74 | 2.34 (-) | 1.6e-003 | 382 | MID-22331 |
| 3.4e+003 - 7.9e+003 | 0.85 | 2.30 (-) | 1.2e-004 | 259 | hsa-let-7g |
| 3.8 e+002 - 8.5e+002 | 0.75 | 2.25 (-) | 4.3e-003 | 296 | hsa-miR-26b |
| 7.5e+002 - 1.6e+003 | 0.78 | 2.16 (-) | 2.3e-004 | 364 | MID-00064 |

(continued)

| median values | auROC | fold-change | p-value | SEQ ID NO. | miR name |
|---|---|---|---|---|---|
| 6.3 e+002 - 1.3e+003 | 0.79 | 2.08 (-) | 7.9e-005 | 314 | hsa-miR-361-3p |
| 2.7e+003 - 5.4e+003 | 0.80 | 2.05 (-) | 7.5e-007 | 12 | hsa-miR-140-3p |
| + the higher expression of this miR is in melanoma<br>- the higher expression of this miR is in lymphoma | | | | | |

Figure 18 demonstrates binary decisions at node #24 of the decision-tree. Tumors originating in melanoma (diamonds) are easily distinguished from tumors of lymphoma origin (squares) using the expression levels of hsa-miR-342-3p (SEQ ID NO: 50, y-axis) and hsa-miR-30d (SEQ ID NO: 47, x-axis).

**Table 26:** miR expression (in fluorescence units) distinguishing between B-cell lymphoma and T-cell lymphoma

| median values | auROC | fold-change | p-value | SEQ ID NO. | miR name |
|---|---|---|---|---|---|
| 8.3e+002 - 2.8e+002 | 0.74 | 2.96 (+) | 3.7e-005 | 11 | hsa-miR-138 |
| 6.7e+002 - 2.8e+002 | 0.72 | 2.37 (+) | 2.2e-003 | 191 | hsa-miR-29c |
| 1.2e+003 - 5.9e+002 | 0.76 | 2.02 (+) | 1.4e-003 | 48 | hsa-miR-30e |
| 6.7e+002 - 1.8e+003 | 0.79 | 2.77 (-) | 1.1e-006 | 35 | hsa-miR-21* |
| 1.5e+003 - 3.9e+003 | 0.68 | 2.68 (-) | 2.6e-003 | 228 | hsa-miR-886-3p |
| + the higher expression of this miR is in B-cell lymphoma<br>- the higher expression of this miR is in T-cell lymphoma | | | | | |

hsa-miR-30e (SEQ ID NO: 48) and hsa-miR-21* (SEQ ID NO: 35) are used at node 25 of the binary-tree-classifier detailed in the invention to distinguish between B-cell lymphoma and T-cell lymphoma.

**Table 27:** miR expression (in fluorescence units) distinguishing between lung small cell carcinoma and other neuroendocrine tumors selected from the group consisting of lung carcinoid, medullary thyroid carcinoma, gastrointestinal tract carcinoid and pancreatic islet cell tumor

| median values | auROC | fold-change | p-value | SEQ ID NO. | miR name |
|---|---|---|---|---|---|
| 1.2e+004 - 1.2e+003 | 0.99 | 9.68 (+) | 3.3e-021 | 158 | hsa-miR-106a |
| 7.3e+003 - 7.9e+002 | 1.00 | 9.17 (+) | 3.4e-022 | 20 | hsa-miR-17 |
| 1.4e+003 - 1.6e+002 | 0.99 | 8.53 (+) | 8.2e-022 | 176 | hsa-miR-18a |
| 5.8e+003 - 7.0e+002 | 1.00 | 8.38 (+) | 7.4e-021 | 186 | hsa-miR-20a |
| 1.1e+004 - 1.5e+003 | 0.98 | 7.71 (+) | 1.7e-022 | 148 | hsa-miR-93 |
| 4.7e+003 - 6.7e+002 | 0.89 | 6.99 (+) | 1.0e-008 | 36 | hsa-miR-210 |
| 2.2e+003 - 3.7e+002 | 0.95 | 5.87 (+) | 2.8e-016 | 51 | hsa-miR-345 |
| 8.9e+003 - 1.8e+003 | 0.95 | 4.96 (+) | 1.6e-010 | 172 | hsa-miR-15b |

(continued)

| median values | auROC | fold-change | p-value | SEQ ID NO. | miR name |
|---|---|---|---|---|---|
| 8.2e+003 - 1.8e+003 | 0.98 | 4.68 (+) | 6.3e-020 | 260 | hsa-miR-106b |
| 1.1e+003 - 2.4e+002 | 0.91 | 4.62 (+) | 7.7e-010 | 265 | hsa-miR-130b |
| 8.0e+003 - 1.8e+003 | 0.94 | 4.33 (+) | 2.7e-013 | 67 | hsa-miR-92a |
| 4.1e+003 - 9.8e+002 | 0.98 | 4.15 (+) | 2.6e-019 | 188 | hsa-miR-25 |
| 1.1e+003 - 3.4e+002 | 0.98 | 3.40 (+) | 7.9e-016 | 277 | hsa-miR-17* |
| 2.5e+003 - 8.3e+002 | 0.99 | 2.96 (+) | 1.8e-011 | 284 | hsa-miR-19b |
| 5.1e+002 - 1.8e+002 | 0.74 | 2.84 (+) | 6.1e-004 | 302 | hsa-miR-301a |
| 7.9e+002 - 2.9e+002 | 0.91 | 2.78 (+) | 8.7e-010 | 68 | hsa-miR-92b |
| 9.9e+002 - 4.3e+002 | 0.69 | 2.28 (+) | 5.1e-002 | 168 | hsa-miR-150 |
| 2.5e+003 - 1.1e+003 | 0.70 | 2.24 (+) | 4.5e-003 | 242 | MID-16489 |
| 1.4e+003 - 6.6e+002 | 0.91 | 2.12 (+) | 1.1e-009 | 204 | hsa-miR-425 |
| 5.0e+001 - 1.6e+003 | 0.91 | 31.23 (-) | 4.5e-009 | 162 | hsa-miR-129-3p |
| 1.1e+002 - 1.6e+003 | 0.91 | 14.13 (-) | 8.6e-009 | 177 | hsa-miR-192 |
| 7.6e+001 - 7.9e+002 | 0.91 | 10.42 (-) | 1.5e-008 | 27 | hsa-miR-194 |
| 5.5e+002 - 5.0e+003 | 0.92 | 9.14 (-) | 1.7e-009 | 65 | hsa-miR-7 |
| 7.1e+001 - 5.7e+002 | 0.78 | 8.02 (-) | 5.8e-005 | 263 | hsa-miR-129* |
| 2.5e+002 - 1.6e+003 | 0.80 | 6.30 (-) | 3.5e-005 | 155 | hsa-miR-127-3p |
| 1.5e+002 - 9.1e+002 | 0.96 | 6.05 (-) | 3.5e-015 | 191 | hsa-miR-29c |
| 3.3e+002 - 2.0e+003 | 0.93 | 5.99 (-) | 3.3e-013 | 190 | hsa-miR-29b |
| 1.7e+002 - 9.9e+002 | 0.99 | 5.76 (-) | 1.3e-020 | 45 | hsa-miR-29c* |
| 1.2e+002 - 6.6e+002 | 0.75 | 5.60 (-) | 8.0e-004 | 59 | hsa-miR-487b |
| 1.8e+003 - 8.0e+003 | 0.90 | 4.44 (-) | 1.6e-012 | 43 | hsa-miR-29a |

(continued)

| median values | auROC | fold-change | p-value | SEQ ID NO. | miR name |
|---|---|---|---|---|---|
| 1.3e+004 - 4.9e+004 | 0.88 | 3.87 (-) | 3.3e-006 | 56 | hsa-miR-375 |
| 1.6e+002 - 5.5e+002 | 0.95 | 3.37 (-) | 9.6e-011 | 266 | hsa-miR-132 |
| 4.0e+003 - 1.2e+004 | 0.82 | 2.98 (-) | 9.4e-006 | 14 | hsa-miR-143 |
| 7.8e+003 - 2.3e+004 | 0.85 | 2.89 (-) | 6.1e-006 | 15 | hsa-miR-145 |
| 1.2e+004 - 3.4e+004 | 0.79 | 2.83 (-) | 4.3e-005 | 8 | hsa-miR-125b |
| 4.5e+003 - 1.2e+004 | 0.97 | 2.70 (-) | 1.7e-014 | 7 | hsa-miR-125a-5p |
| 1.9e+003 - 5.0e+003 | 0.89 | 2.67 (-) | 3.6e-010 | 39 | hsa-miR-22 |
| 2.5e+003 - 5.7e+003 | 0.79 | 2.25 (-) | 8.7e-004 | 189 | hsa-miR-27b |
| 1.1e+003 - 2.4e+003 | 0.64 | 2.18 (-) | 4.1e-002 | 249 | MID-20524 |
| 2.2e+003 - 4.8e+003 | 0.72 | 2.14 (-) | 8.5e-003 | 231 | hsa-miR-99a |
| 9.6e+003 - 2.0e+004 | 0.82 | 2.12 (-) | 1.3e-003 | 293 | hsa-miR-23b |
| 15.e+003 - 1.0e+004 | 0.80 | 2.01 (-) | 6.3e-005 | 2 | hsa-let-7e |
| + the higher expression of this miR is in lung small cell carcinoma<br>- the higher expression of this miR is in other neuroendocrine tumors | | | | | |

hsa-miR-17 (SEQ ID NO: 20) and hsa-miR-29c* (SEQ ID NO: 45) are used at node #26 of the binary-tree-classifier detailed in the invention to distinguish between lung small cell carcinoma and other neuroendocrine tumors.

**Table 28:** miR expression (in fluorescence units) distinguishing between medullary thyroid carcinoma and other neuroendocrine tumors selected from the group consisting of lung carcinoid, gastrointestinal tract carcinoid and pancreatic islet cell tumor

| median values | auROC | fold-change | p-value | SEQ ID NO. | miR name |
|---|---|---|---|---|---|
| 4.4e+003 - 5.5e+001 | 0.84 | 79.70 (+) | 1.5e-007 | 159 | hsa-miR-124 |
| 4.0e+004 - 4.9e+003 | 0.98 | 8.07 (+) | 1.6e-015 | 40 | hsa-miR-222 |
| 1.9e+004 - 2.8e+003 | 0.98 | 6.85 (+) | 4.8e-016 | 147 | hsa-miR-221 |
| 1.1e+003 - 2.0e+002 | 0.70 | 5.55 (+) | 1.1e-003 | 11 | hsa-miR-138 |
| 3.2e+002 - 7.8e+001 | 0.83 | 4.12 (+) | 7.6e-007 | 311 | hsa-miR-335 |

(continued)

| median values | auROC | fold-change | p-value | SEQ ID NO. | miR name |
|---|---|---|---|---|---|
| 5.8e+003 - 1.5e+003 | 0.86 | 3.91 (+) | 1.3e-006 | 4 | hsa-miR-10a |
| 6.3e+004 - 1.7e+004 | 0.83 | 3.61 (+) | 3.9e-006 | 8 | hsa-miR-125b |
| 1.1e+004 - 3.2e+003 | 0.79 | 3.43 (+) | 5.5e-005 | 231 | hsa-miR-99a |
| 4.3e+002 - 2.0e+002 | 0.78 | 2.10 (+) | 2.8e-004 | 301 | hsa-miR-29b-2* |
| 7.9e+003 - 3.8e+003 | 0.82 | 2.06 (+) | 4.4e-005 | 297 | hsa-miR-27a |
| 1.4e+002 - 4.0e+002 | 0.95 | 2.95 (-) | 7.5e-011 | 68 | hsa-miR-92b |
| 1.1e+003 - 2.8e+003 | 0.87 | 2.50 (-) | 3.2e-006 | 67 | hsa-miR-92a |
| 1.8e+002 - 3.7e+002 | 0.76 | 2.07 (-) | 2.0e-003 | 265 | hsa-miR-130b |
| 4.4e+002 - 9.0e+002 | 0.75 | 2.04 (-) | 2.1e-003 | 36 | hsa-miR-210 |
| + the higher expression of this miR is in medullary thyroid carcinoma<br>- the higher expression of this miR is in other neuroendocrine tumors | | | | | |

Figure 19 demonstrates binary decisions at node #27 of the decision-tree. Tumors originating in medullary thyroid carcinoma (diamonds) are easily distinguished from tumors of other neuroendocrine origin (squares) using the expression levels of hsa-miR-92b (SEQ ID NO: 68, y-axis), hsa-miR-222 (SEQ ID NO: 40, x-axis) and hsa-miR-92a (SEQ ID NO: 67, z-axis).

**Table 29:** miR expression (in fluorescence units) distinguishing between lung carcinoid tumors and GI neuroendocrine tumors selected from the group consisting of gastrointestinal tract carcinoid and pancreatic islet cell tumor

| median values | auROC | fold-change | p-value | SEQ ID NO. | miR name |
|---|---|---|---|---|---|
| 4.0e+003 - 9.9e+001 | 0.90 | 40.08 (+) | 1.9e-010 | 331 | hsa-miR-432 |
| 6.0e+003 - 1.5e+002 | 0.86 | 39.24 (+) | 4.6e-008 | 162 | hsa-miR-129-3p |
| 6.3e+003 - 1.9e+002 | 0.87 | 34.16 (+) | 7.8e-009 | 59 | hsa-miR-487b |
| 1.3e+003 - 5.5e+001 | 0.88 | 23.36 (+) | 2.9e-010 | 326 | hsa-miR-409-5p |
| 1.1e+003 - 5.0e+001 | 0.88 | 21.14 (+) | 5.2e-010 | 306 | hsa-miR-323-3p |
| 1.0e+003 - 5.5e+001 | 0.87 | 18.59 (+) | 1.5e-009 | 350 | hsa-miR-539 |
| 7.9e+002 - 5.6e+001 | 0.84 | 14.25 (+) | 1.4e-008 | 317 | hsa-miR-369-5p |

(continued)

| median values | auROC | fold-change | p-value | SEQ ID NO. | miR name |
|---|---|---|---|---|---|
| 1.0e+004 - 7.2e+002 | 0.86 | 13.95 (+) | 3.2e-007 | 155 | hsa-miR-127-3p |
| 1.7e+003 - 1.2e+002 | 0.86 | 13.60 (+) | 2.1e-008 | 325 | hsa-miR-409-3p |
| 1.6e+003 - 1.2e+002 | 0.88 | 13.10 (+) | 4.2e-009 | 318 | hsa-miR-370 |
| 9.5e+002 - 7.3e+001 | 0.81 | 13.03 (+) | 3.1e-006 | 339 | hsa-miR-495 |
| 9.5e+002 - 7.4e+001 | 0.84 | 12.92 (+) | 5.7e-007 | 264 | hsa-miR-129-5p |
| 6.4e+002 - 5.0e+001 | 0.91 | 12.84 (+) | 1.6e-013 | 332 | hsa-miR-433 |
| 6.5e+002 - 5.7e+001 | 0.88 | 11.52 (+) | 5.1e-011 | 262 | hsa-miR-127-5p |
| 5.6e+002 - 5.2e+001 | 0.90 | 10.76 (+) | 2.7e-012 | 336 | hsa-miR-485-5p |
| 2.0e+003 - 1.9e+002 | 0.86 | 10.44 (+) | 4.2e-008 | 324 | hsa-miR-382 |
| 7.9e+002 - 7.8e+001 | 0.83 | 10.20 (+) | 1.3e-007 | 322 | hsa-miR-379 |
| 6.0e+002 - 5.9e+001 | 0.89 | 10.15 (+) | 9.6e-012 | 330 | hsa-miR-431* |
| 4.7e+002 - 5.0e+001 | 0.90 | 9.41 (+) | 6.1e-012 | 321 | hsa-miR-377* |
| 1.3e+003 - 1.4e+002 | 0.80 | 9.40 (+) | 1.5e-005 | 263 | hsa-miR-129* |
| 4.7e+002 - 5.0e+001 | 0.86 | 9.35 (+) | 1.8e-008 | 309 | hsa-miR-329 |
| 4.9e+002 - 5.3e+001 | 0.79 | 9.24 (+) | 3.1e-005 | 53 | hsa-miR-34c-5p |
| 1.1e+003 - 1.2e+002 | 0.83 | 9.05 (+) | 6.4e-007 | 320 | hsa-miR-376c |
| 1.1e+003 - 1.2e+002 | 0.86 | 8.81 (+) | 2.3e-008 | 275 | hsa-miR-154 |
| 6.5e+002 - 8.4e+001 | 0.83 | 7.73 (+) | 8.1e-007 | 352 | hsa-miR-543 |
| 9.9e+002 - 1.3e+002 | 0.82 | 7.49 (+) | 3.2e-007 | 312 | hsa-miR-337-5p |
| 6.2e+002 - 8.8e+001 | 0.86 | 7.10 (+) | 3.0e-008 | 355 | hsa-miR-654-3p |
| 3.5e+002 - 5.0e+001 | 0.91 | 7.05 (+) | 3.2e-013 | 367 | MID-00465 |
| 6.0e+002 - 1.0e+002 | 0.84 | 5.76 (+) | 5.9e-007 | 269 | hsa-miR-134 |

(continued)

| median values | auROC | fold-change | p-value | SEQ ID NO. | miR name |
|---|---|---|---|---|---|
| 3.2e+003 - 8.5e+002 | 0.91 | 3.84 (+) | 1.2e-011 | 64 | hsa-miR-652 |
| 3.2e+002 - 1.1e+002 | 0.83 | 2.84 (+) | 2.3e-005 | 308 | hsa-miR-328 |
| 2.6e+003 - 9.4e+002 | 0.74 | 2.78 (+) | 1.1e-003 | 175 | hsa-miR-183 |
| 2.8e+003 - 1.0e+003 | 0.87 | 2.73 (+) | 3.0e-006 | 190 | hsa-miR-29b |
| 3.9e+003 - 1.6e+003 | 0.88 | 2.49 (+) | 6.9e-010 | 54 | hsa-miR-361-5p |
| 4.1e+002 - 1.7e+002 | 0.67 | 2.44 (+) | 2.1e-002 | 302 | hsa-miR-301a |
| 4.0e+003 - 1.7e+003 | 0.79 | 2.41 (+) | 5.9e-004 | 152 | hsa-miR-182 |
| 4.0e+002 - 1.7e+002 | 0.88 | 2.39 (+) | 4.7e-007 | 301 | hsa-miR-29b-2* |
| 8.7e+002 - 3.7e+002 | 0.77 | 2.36 (+) | 6.8e-005 | 266 | hsa-miR-132 |
| 7.7e+003 - 3.3e+003 | 0.82 | 2.34 (+) | 5.4e-006 | 47 | hsa-miR-30d |
| 3.7e+002 - 1.6e+002 | 0.70 | 2.32 (+) | 5.8e-003 | 313 | hsa-miR-338-3p |
| 3.3e+002 - 1.5e+002 | 0.66 | 2.16 (+) | 1.3e-002 | 359 | hsa-miR-708 |
| 5.5e+003 - 2.5e+003 | 0.68 | 2.16 (+) | 4.2e-002 | 65 | hsa-miR-7 |
| 2.1e+003 - 9.9e+002 | 0.78 | 2.13 (+) | 7.0e-005 | 307 | hsa-miR-324-5p |
| 1.2e+003 - 5.9e+002 | 0.81 | 2.02 (+) | 1.6e-004 | 191 | hsa-miR-29c |
| 3.5e+002 - 1.9e+003 | 0.88 | 5.36 (-) | 1.0e-007 | 242 | MID-16489 |
| 6.5e+002 - 1.9e+003 | 0.76 | 2.96 (-) | 4.9e-004 | 4 | hsa-miR-10a |
| 1.3e+003 - 3.6e+003 | 0.84 | 2.79 (-) | 1.9e-006 | 147 | hsa-miR-221 |
| 2.2e+003 - 5.9e+003 | 0.81 | 2.75 (-) | 8.5e-006 | 40 | hsa-miR-222 |
| 2.6e+002 - 6.8e+002 | 0.76 | 2.56 (-) | 7.4e-004 | 372 | MID-16469 |
| 3.5e+002 - 8.9e+002 | 0.71 | 2.56 (-) | 4.7e-003 | 168 | hsa-miR-150 |
| 1.5e+002 - 3.7e+002 | 0.83 | 2.55 (-) | 4.8e-005 | 16 | hsa-miR-146a |

(continued)

| median values | auROC | fold-change | p-value | SEQ ID NO. | miR name |
|---|---|---|---|---|---|
| 1.9e+003 - 4.7e+003 | 0.82 | 2.40 (-) | 1.7e-005 | 182 | hsa-miR-199a-5p |
| 1.3e+003 - 3.0e+003 | 0.79 | 2.35 (-) | 1.2e-004 | 167 | hsa-miR-149* |
| 2.1e+002 - 4.8e+002 | 0.84 | 2.26 (-) | 3.1e-005 | 356 | hsa-miR-658 |
| 1.2e+003 - 2.8e+003 | 0.74 | 2.25 (-) | 1.4e-003 | 148 | hsa-miR-93 |
| 1.4e+003 - 3.1e+003 | 0.70 | 2.23 (-) | 1.9e-002 | 382 | MID-22331 |
| 8.0e+002 - 1.8e+003 | 0.83 | 2.21 (-) | 2.9e-005 | 37 | hsa-miR-214 |
| 4.4e+002 - 8.9e+002 | 0.79 | 2.01 (-) | 2.1e-004 | 364 | MID-00064 |
| 2.1e+002 - 4.2e+002 | 0.78 | 2.01 (-) | 1.1e-003 | 35 | hsa-miR-21 * |
| + the higher expression of this miR is in lung carcinoid tumors<br>- the higher expression of this miR is in GI neuroendocrine tumors | | | | | |

hsa-miR-652 (SEQ ID NO: 64), hsa-miR-34c-5p (SEQ ID NO: 53) and hsa-miR-214 (SEQ ID NO: 37) are used at node 28 of the binary-tree-classifier detailed in the invention to distinguish between lung carcinoid tumors and GI neuroendocrine tumors.

**Table 30:** miR expression (in fluorescence units) distinguishing between pancreatic islet cell tumors and GI neuroendocrine carcinoid tumors selected from the group consisting of small intestine and duodenum; appendicitis, stomach and pancreas

| median values | | auROC | fold-change | p-value | SEQ ID NO. | miR name |
|---|---|---|---|---|---|---|
| 1.1e+002 | 2.3e+003 | 0.80 | 20.91 (+) | 2.8e-004 | 263 | hsa-miR-129* |
| 5.0e+001 | 4.8e+002 | 0.72 | 9.61 (+) | 6.6e-003 | 288 | hsa-miR-217 |
| 1.9e+002 | 1.6e+003 | 0.90 | 8.54 (+) | 6.8e-006 | 18 | hsa-miR-148a |
| 5.2e+001 | 4.3e+002 | 0.68 | 8.34 (+) | 2.7e-002 | 286 | hsa-miR-216a |
| 2.5e+002 | 1.8e+003 | 0.74 | 7.22 (+) | 4.4e-003 | 162 | hsa-miR-129-3p |
| 9.9e+001 | 6.6e+002 | 0.74 | 6.65 (+) | 2.3e-003 | 225 | hsa-miR-551b |
| 5.0e+001 | 3.0e+002 | 0.75 | 6.04 (+) | 5.4e-003 | 287 | hsa-miR-216b |
| 1.9e+002 | 7.1e+002 | 0.92 | 3.75 (+) | 7.3e-007 | 206 | hsa-miR-455-3p |
| 3.4e+003 | 1.3e+004 | 0.79 | 3.65 (+) | 2.5e-003 | 205 | hsa-miR-451 |
| 2.6e+002 | 8.9e+002 | 0.83 | 3.43 (+) | 2.8e-004 | 296 | hsa-miR-26b |
| 2.6e+003 | 8.7e+003 | 0.91 | 3.29 (+) | 3.6e-004 | 258 | hsa-let-7f |
| 1.6e+002 | 5.2e+002 | 0.78 | 3.25 (+) | 3.2e-003 | 313 | hsa-miR-338-3p |
| 2.4e+003 | 6.6e+003 | 0.85 | 2.71 (+) | 5.0e-005 | 377 | MID-17866 |
| 7.6e+003 | 1.9e+004 | 0.88 | 2.45 (+) | 1.2e-005 | 373 | MID-16582 |

(continued)

| median values | | auROC | fold-change | p-value | SEQ ID NO. | miR name |
|---|---|---|---|---|---|---|
| 2.7e+004 | 6.4e+004 | 0.80 | 2.42 (+) | 1.0e-003 | 256 | hsa-let-7a |
| 4.7e+003 | 1.1e+004 | 0.89 | 2.36 (+) | 1.8e-004 | 153 | hsa-let-7d |
| 2.8e+003 | 6.4e+003 | 0.86 | 2.28 (+) | 2.2e-003 | 259 | hsa-let-7g |
| 2.3e+002 | 4.8e+002 | 0.69 | 2.11 (+) | 1.0e-002 | 265 | hsa-miR-130b |
| 3.1e+003 | 6.4e+003 | 0.75 | 2.09 (+) | 7.5e-003 | 303 | hsa-miR-30b |
| 9.7e+002 | 1.0e+002 | 0.81 | 9.40 (-) | 5.4e-004 | 268 | hsa-miR-133b |
| 1.0e+003 | 1.1e+002 | 0.80 | 9.22 (-) | 5.4e-004 | 267 | hsa-miR-133a |
| 1.9e+003 | 2.3e+002 | 0.93 | 8.37 (-) | 2.1e-006 | 165 | hsa-miR-143* |
| 9.2e+004 | 1.1e+004 | 0.94 | 8.18 (-) | 3.7e-008 | 15 | hsa-miR-145 |
| 5.3e+002 | 6.6e+001 | 0.91 | 8.05 (-) | 7.0e-006 | 272 | hsa-miR-145* |
| 3.8e+004 | 5.2e+003 | 0.96 | 7.30 (-) | 3.7e-009 | 14 | hsa-miR-143 |
| 2.0e+003 | 3.1e+002 | 0.88 | 6.35 (-) | 6.2e-006 | 202 | hsa-miR-378 |
| 1.4e+003 | 2.9e+002 | 0.88 | 4.99 (-) | 8.1e-006 | 236 | MID-00689 |
| 6.4e+002 | 1.4e+002 | 0.88 | 4.74 (-) | 7.9e-006 | 203 | hsa-miR-422a |
| 3.6e+003 | 9.2e+002 | 0.82 | 3.91 (-) | 2.4e-004 | 4 | hsa-miR-10a |
| 1.1e+003 | 3.0e+002 | 0.78 | 3.78 (-) | 4.4e-003 | 168 | hsa-miR-150 |
| 3.6e+002 | 1.1e+002 | 0.81 | 3.23 (-) | 3.4e-004 | 310 | hsa-miR-330-3p |
| 6.7e+002 | 2.1e+002 | 0.95 | 3.16 (-) | 4.6e-007 | 298 | hsa-miR-28-3p |
| 2.2e+003 | 7.2e+002 | 0.74 | 3.09 (-) | 1.4e-002 | 27 | hsa-miR-194 |
| 2.1e+004 | 7.5e+003 | 0.91 | 2.72 (-) | 7.6e-005 | 29 | hsa-miR-200b |
| 2.2e+004 | 8.5e+003 | 0.87 | 2.57 (-) | 2.8e-006 | 34 | hsa-miR-21 |
| 1.7e+003 | 6.7e+002 | 0.74 | 2.56 (-) | 8.0e-003 | 228 | hsa-miR-886-3p |
| 3.8e+003 | 1.5e+003 | 0.77 | 2.50 (-) | 3.6e-003 | 3 | hsa-miR-100 |
| 5.3e+002 | 2.5e+002 | 0.94 | 2.14 (-) | 4.3e-007 | 349 | hsa-miR-532-5p |
| 5.0e+002 | 2.4e+002 | 0.82 | 2.06 (-) | 8.5e-004 | 35 | hsa-miR-21* |
| 3.3e+002 | 1.6e+002 | 0.77 | 2.01 (-) | 5.7e-003 | 26 | hsa-miR-193a-5p |
| + the higher expression of this miR is in pancreatic islet cell tumors<br>- the higher expression of this miR is in GI neuroendocrine carcinoid tumors | | | | | | |

hsa-miR-21 (SEQ ID NO: 34), and hsa-miR-148a (SEQ ID NO: 18) are used at node 29 of the binary-tree-classifier detailed in the invention to distinguish between pancreatic islet cell tumors and GI neuroendocrine carcinoid tumors.

**Table 31:** miR expression (in fluorescence units) distinguishing between gastric or esophageal adenocarcinoma and other adenocarcinoma tumors of the gastrointestinal system selected from the group consisting of cholangiocarcinoma or adenocarcinoma of extrahepatic biliary tract, pancreatic adenocarcinoma and colorectal adenocarcinoma

| median values | | auROC | fold-change | p-value | SEQ ID NO. | miR name |
|---|---|---|---|---|---|---|
| 6.7e+001 | 6.2e+002 | 0.74 | 9.14 (+) | 4.6e-008 | 267 | hsa-miR-133a |
| 6.3e+001 | 5.5e+002 | 0.74 | 8.73 (+) | 3.9e-008 | 268 | hsa-miR-133b |

(continued)

| median values | | auROC | fold-change | p-value | SEQ ID NO. | miR name |
|---|---|---|---|---|---|---|
| 5.9e+002 | 2.5e+003 | 0.75 | 4.26 (+) | 3.9e-007 | 165 | hsa-miR-143* |
| 2.8e+004 | 7.9e+004 | 0.71 | 2.82 (+) | 4.5e-004 | 15 | hsa-miR-145 |
| 1.3e+004 | 3.2e+004 | 0.68 | 2.55 (+) | 1.3e-003 | 14 | hsa-miR-143 |
| 5.1e+002 | 1.3e+003 | 0.71 | 2.53 (+) | 8.2e-004 | 356 | hsa-miR-658 |
| 3.1e+003 | 7.2e+003 | 0.72 | 2.33 (+) | 2.2e-004 | 167 | hsa-miR-149* |
| 1.4e+003 | 3.1e+003 | 0.69 | 2.22 (+) | 7.2e-004 | 376 | MID-17576 |
| 6.5e+002 | 1.4e+003 | 0.71 | 2.20 (+) | 3.0e-004 | 372 | MID-16469 |
| 1.5e+002 | 3.2e+002 | 0.69 | 2.14 (+) | 3.0e-004 | 272 | hsa-miR-145* |
| 1.4e+003 | 2.9e+003 | 0.74 | 2.11 (+) | 3.8e-004 | 370 | MID-15986 |
| 3.6e+002 | 5.5e+001 | 0.83 | 6.57 (-) | 5.4e-008 | 42 | hsa-miR-224 |
| 4.0e+002 | 1.5e+002 | 0.73 | 2.61 (-) | 1.1e-004 | 41 | hsa-miR-223 |
| 1.2e+003 | 9.0e+002 | 0.67 | 1.28 (-) | 1.2e-002 | 146 | hsa-miR-1201 |
| + the higher expression of this miR is in gastric or esophageal adenocarcinoma<br>- the higher expression of this miR is in other adenocarcinoma tumors of the gastrointestinal system | | | | | | |

Figure 20 demonstrates binary decisions at node #30 of the decision-tree. Tumors originating in gastric or esophageal adenocarcinoma (diamonds) are easily distinguished from tumors of other GI adenocarcinoma origin (squares) using the expression levels of hsa-miR-1201 (SEQ ID NO: 146, y-axis), hsa-miR-224 (SEQ ID NO: 42, x-axis) and hsa-miR-210 (SEQ ID NO: 36, z-axis).

**Table 32:** miR expression (in fluorescence units) distinguishing between colorectal adenocarcinoma and cholangiocarcinoma or adenocarcinoma of biliary tract or pancreas

| median values | | auROC | fold-change | p-value | SEQ ID NO. | miR name |
|---|---|---|---|---|---|---|
| 2.1e+002 | 5.4e+002 | 0.69 | 2.55 (+) | 4.0e-003 | 42 | hsa-miR-224 |
| 1.8e+002 | 4.2e+002 | 0.70 | 2.28 (+) | 1.2e-003 | 184 | hsa-miR-203 |
| 3.2e+003 | 6.2e+003 | 0.77 | 1.91 (+) | 5.1e-007 | 67 | hsa-miR-92a |
| 3.1e+003 | 5.6e+003 | 0.81 | 1.81 (+) | 4.6e-007 | 158 | hsa-miR-106a |
| 1.8e+003 | 3.2e+003 | 0.81 | 1.81 (+) | 1.3e-007 | 20 | hsa-miR-17 |
| 1.8e+003 | 3.2e+003 | 0.76 | 1.80 (+) | 7.9e-005 | 186 | hsa-miR-20a |
| 1.1e+003 | 1.9e+003 | 0.76 | 1.75 (+) | 1.4e-005 | 284 | hsa-miR-19b |
| 1.6e+003 | 2.6e+003 | 0.70 | 1.67 (+) | 3.0e-003 | 389 | MID-17356 |
| 3.1e+002 | 5.1e+002 | 0.75 | 1.63 (+) | 2.1e-005 | 203 | hsa-miR-422a |
| 4.5e+003 | 7.2e+003 | 0.67 | 1.60 (+) | 5.6e-003 | 240 | MID-15965 |
| 6.9e+002 | 1.1e+003 | 0.76 | 1.59 (+) | 1.7e-005 | 236 | MID-00689 |
| 1.1e+003 | 1.6e+003 | 0.68 | 1.53 (+) | 2.5e-003 | 146 | hsa-miR-1201 |
| 9.1e+002 | 1.4e+003 | 0.69 | 1.49 (+) | 5.2e-004 | 204 | hsa-miR-425 |
| 6.5e+003 | 9.3e+003 | 0.77 | 1.44 (+) | 1.2e-005 | 43 | hsa-miR-29a |

(continued)

| median values | | auROC | fold-change | p-value | SEQ ID NO. | miR name |
|---|---|---|---|---|---|---|
| 4.5e+002 | 6.4e+002 | 0.75 | 1.44 (+) | 7.3e-006 | 176 | hsa-miR-18a |
| 9.1e+002 | 1.3e+003 | 0.72 | 1.41 (+) | 1.4e-004 | 202 | hsa-miR-378 |
| 1.8e+003 | 5.3e+002 | 0.69 | 3.39 (-) | 2.0e-003 | 49 | hsa-miR-31 |
| 2.0e+003 | 8.2e+002 | 0.82 | 2.39 (-) | 2.2e-008 | 46 | hsa-miR-30a |
| 3.7e+002 | 2.5e+002 | 0.66 | 1.47 (-) | 1.3e-002 | 38 | hsa-miR-214* |
| 1.3e+003 | 9.0e+002 | 0.73 | 1.41 (-) | 2.2e-003 | 363 | hsa-miR-99b |
| + the higher expression of this miR is in colorectal adenocarcinoma<br>- the higher expression of this miR is in other cholangiocarcinoma or adenocarcinoma tumors of biliary tract or pancreas | | | | | | |

Figure 21 demonstrates binary decisions at node #31 of the decision-tree. Tumors originating in colorectal adenocarcinoma (diamonds) are easily distinguished from tumors of cholangiocarcinoma or adenocarcinoma of biliary tract or pancreas origin (squares) using the expression levels of hsa-miR-30a (SEQ ID NO: 46, y-axis), hsa-miR-17 (SEQ ID NO: 20, x-axis) and hsa-miR-29a (SEQ ID NO: 43, z-axis).

**Table 33:** miR expression (in fluorescence units) distinguishing between cholangiocarcinoma or adenocarcinoma of extrahepatic biliary tract and pancreatic adenocarcinoma

| median values | | auROC | fold-change | p-value | SEQ ID NO. | miR name |
|---|---|---|---|---|---|---|
| 1.1e+003 | 3.4e+003 | 0.81 | 3.06 (+) | 1.5e-003 | 49 | hsa-miR-31 |
| 1.4e+002 | 3.3e+002 | 0.71 | 2.36 (+) | 1.1e-002 | 11 | hsa-miR-138 |
| 1.7e+003 | 3.0e+003 | 0.70 | 1.77 (+) | 1.7e-002 | 13 | hsa-miR-141 |
| 1.1e+004 | 1.8e+004 | 0.70 | 1.65 (+) | 1.5e-002 | 373 | MID-16582 |
| 8.4e+002 | 1.4e+003 | 0.69 | 1.63 (+) | 9.6e-002 | 154 | hsa-miR-181b |
| 4.3e+002 | 7.0e+002 | 0.69 | 1.62 (+) | 5.1e-001 | 5 | hsa-miR-10b |
| 9.3e+003 | 1.5e+004 | 0.68 | 1.61 (+) | 7.4e-002 | 30 | hsa-miR-200c |
| 2.7e+002 | 4.2e+002 | 0.72 | 1.58 (+) | 1.3e-002 | 45 | hsa-miR-29c* |
| 1.0e+003 | 1.5e+003 | 0.66 | 1.47 (+) | 1.1e-001 | 178 | hsa-miR-193b |
| 6.6e+003 | 9.0e+003 | 0.75 | 1.36 (+) | 1.2e-002 | 147 | hsa-miR-221 |
| 4.7e+002 | 6.4e+002 | 0.70 | 1.36 (+) | 4.0e-002 | 274 | hsa-miR-151-3p |
| 5.4e+002 | 7.3e+002 | 0.66 | 1.34 (+) | 2.4e-002 | 16 | hsa-miR-146a |
| 1.1e+004 | 1.5e+004 | 0.71 | 1.32 (+) | 3.7e-002 | 40 | hsa-miR-222 |
| 3.8e+003 | 4.9e+003 | 0.71 | 1.30 (+) | 8.4e-002 | 21 | hsa-miR-181a |
| 6.0e+003 | 6.8e+003 | 0.66 | 1.14 (+) | 6.3e-002 | 43 | hsa-miR-29a |
| 5.9e+002 | 3.3e+002 | 0.74 | 1.81 (-) | 2.1e-002 | 253 | MID-23256 |
| 1.9e+003 | 1.1e+003 | 0.66 | 1.70 (-) | 8.0e-002 | 245 | MID-18336 |
| 4.5e+004 | 2.7e+004 | 0.73 | 1.68 (-) | 7.4e-003 | 256 | hsa-let-7a |
| 2.7e+003 | 1.8e+003 | 0.65 | 1.51 (-) | 9.2e-002 | 12 | hsa-miR-140-3p |
| 1.4e+005 | 9.3e+004 | 0.75 | 1.47 (-) | 5.4e-003 | 374 | MID-16748 |
| 8.9e+004 | 6.1e+004 | 0.66 | 1.45 (-) | 2.9e-002 | 379 | MID-18395 |

(continued)

| median values | | auROC | fold-change | p-value | SEQ ID NO. | miR name |
|---|---|---|---|---|---|---|
| 4.7e+002 | 3.3e+002 | 0.69 | 1.41 (-) | 6.6e-002 | 180 | hsa-miR-1973 |
| 6.0e+003 | 4.3e+003 | 0.68 | 1.40 (-) | 2.6e-002 | 153 | hsa-let-7d |
| 4.4e+002 | 3.2e+002 | 0.75 | 1.39 (-) | 7.1e-002 | 51 | hsa-miR-345 |
| 6.1e+003 | 4.4e+003 | 0.70 | 1.38 (-) | 3.9e-002 | 52 | hsa-miR-34a |
| 3.3e+004 | 2.4e+004 | 0.73 | 1.37 (-) | 1.4e-002 | 1 | hsa-let-7c |
| 2.0e+004 | 1.5e+004 | 0.68 | 1.36 (-) | 2.8e-002 | 295 | hsa-miR-26a |
| 3.9e+004 | 2.9e+004 | 0.77 | 1.35 (-) | 3.3e-003 | 257 | hsa-let-7b |
| 6.1e+003 | 4.8e+003 | 0.66 | 1.26 (-) | 6.5e-002 | 385 | MID-23168 |
| 1.3e+004 | 1.0e+004 | 0.67 | 1.21 (-) | 9.0e-002 | 293 | hsa-miR-23b |
| 2.4e+004 | 2.1e+004 | 0.68 | 1.18 (-) | 2.6e-002 | 294 | hsa-miR-24 |
| + the higher expression of this miR is in pancreatic adenocarcinoma<br>- the higher expression of this miR is in cholangiocarcinoma or adenocarcinoma of extrahepatic biliary tract | | | | | | |

hsa-miR-345 (SEQ ID NO: 51), hsa-miR-31 (SEQ ID NO: 49) and hsa-miR-146a (SEQ ID NO: 16) are used at node #32 of the binary-tree-classifier detailed in the invention to distinguish between cholangio cancer or adenocarcinoma of extrahepatic biliary tract and pancreatic adenocarcinoma.

**Table 34:** miR expression (in fluorescence units) distinguishing between kidney tumors selected from the group consisting of chromophobe renal cell carcinoma, clear cell renal cell carcinoma and papillary renal cell carcinoma and other tumors selected from the group consisting of sarcoma, adrenal (pheochromocytoma, adrenocortical carcinoma) and mesothelioma (pleural mesothelioma)

| median values | | auROC | fold-change | p-value | SEQ ID NO. | miR name |
|---|---|---|---|---|---|---|
| 5.0e+001 | 4.8e+003 | 0.94 | 96.12 (+) | 7.6e-042 | 29 | hsa-miR-200b |
| 5.0e+001 | 2.3e+003 | 0.94 | 45.03 (+) | 3.3e-044 | 28 | hsa-miR-200a |
| 5.0e+001 | 7.7e+002 | 0.82 | 15.36 (+) | 1.1e-015 | 30 | hsa-miR-200c |
| 1.2e+003 | 1.1e+004 | 0.96 | 9.73 (+) | 8.6e-041 | 46 | hsa-miR-30a |
| 1.2e+002 | 1.1e+003 | 0.74 | 9.21 (+) | 1.1e-008 | 49 | hsa-miR-31 |
| 1.9e+002 | 1.7e+003 | 0.94 | 8.87 (+) | 8.6e-039 | 195 | hsa-miR-30a* |
| 7.5e+001 | 5.0e+002 | 0.74 | 6.58 (+) | 1.1e-009 | 152 | hsa-miR-182 |
| 5.0e+001 | 2.5e+002 | 0.76 | 5.07 (+) | 9.8e-011 | 175 | hsa-miR-183 |
| 2.2e+003 | 8.3e+003 | 0.92 | 3.81 (+) | 5.0e-033 | 47 | hsa-miR-30d |
| 1.5e+003 | 5.1e+003 | 0.83 | 3.52 (+) | 2.5e-016 | 4 | hsa-miR-10a |
| 7.3e+001 | 2.3e+002 | 0.75 | 3.15 (+) | 6.4e-011 | 387 | MID-23751 |
| 3.2e+003 | 9.5e+003 | 0.89 | 2.95 (+) | 1.1e-025 | 196 | hsa-miR-30c |
| 1.4e+002 | 4.0e+002 | 0.76 | 2.80 (+) | 2.1e-012 | 177 | hsa-miR-192 |
| 9.8e+001 | 2.5e+002 | 0.79 | 2.52 (+) | 1.7e-015 | 375 | MID-17375 |
| 9.0e+001 | 2.2e+002 | 0.75 | 2.43 (+) | 2.1e-012 | 27 | hsa-miR-194 |
| 1.2e+002 | 2.8e+002 | 0.76 | 2.40 (+) | 6.5e-013 | 304 | hsa-miR-30e* |
| 6.7e+003 | 1.6e+004 | 0.75 | 2.37 (+) | 1.6e-012 | 40 | hsa-miR-222 |

(continued)

| median values | | auROC | fold-change | p-value | SEQ ID NO. | miR name |
|---|---|---|---|---|---|---|
| 2.5e+002 | 5.9e+002 | 0.69 | 2.33 (+) | 9.5e-006 | 191 | hsa-miR-29c |
| 4.5e+003 | 9.8e+003 | 0.74 | 2.20 (+) | 1.4e-011 | 147 | hsa-miR-221 |
| 4.5e+002 | 9.8e+002 | 0.61 | 2.19 (+) | 7.6e-003 | 35 | hsa-miR-21* |
| 2.8e+002 | 6.1e+002 | 0.71 | 2.15 (+) | 3.7e-007 | 16 | hsa-miR-146a |
| 2.4e+004 | 4.9e+004 | 0.64 | 2.07 (+) | 1.2e-003 | 34 | hsa-miR-21 |
| 2.3e+003 | 4.7e+003 | 0.66 | 2.06 (+) | 5.4e-004 | 5 | hsa-miR-10b |
| 1.2e+003 | 1.2e+002 | 0.85 | 9.53 (-) | 2.1e-018 | 155 | hsa-miR-127-3p |
| 1.2e+004 | 1.6e+003 | 0.89 | 7.57 (-) | 4.9e-023 | 181 | hsa-miR-199a-3p |
| 3.7e+002 | 5.0e+001 | 0.86 | 7.45 (-) | 1.2e-019 | 312 | hsa-miR-337-Sp |
| 1.0e+003 | 1.4e+002 | 0.82 | 7.21 (-) | 1.7e-015 | 183 | hsa-miR-199b-5p |
| 1.7e+004 | 2.6e+003 | 0.86 | 6.48 (-) | 1.4e-017 | 182 | hsa-miR-199a-5p |
| 2.9 e+002 | 5.0e+001 | 0.86 | 5.73 (-) | 3.5e-019 | 320 | hsa-miR-376c |
| 3.4e+002 | 6.5e+001 | 0.86 | 5.23 (-) | 2.6e-016 | 59 | hsa-miR-487b |
| 4.9e+002 | 9.4e+001 | 0.82 | 5.18 (-) | 9.7e-016 | 38 | hsa-miR-214* |
| 2.4e+002 | 5.0e+001 | 0.86 | 4.83 (-) | 2.2e-017 | 324 | hsa-miR-382 |
| 2.1e+002 | 5.0e+001 | 0.83 | 4.27 (-) | 6.6e-017 | 323 | hsa-miR-381 |
| 5.0e+003 | 1.2e+003 | 0.81 | 4.22 (-) | 2.7e-013 | 37 | hsa-miR-214 |
| 2.1e+002 | 5.0e+001 | 0.86 | 4.21 (-) | 8.5e-018 | 322 | hsa-miR-379 |
| 2.1e+002 | 5.0e+001 | 0.83 | 4.14 (-) | 1.2e-015 | 325 | hsa-miR-409-3p |
| 2.5e+002 | 6.7e+001 | 0.86 | 3.76 (-) | 2.8e-016 | 19 | hsa-miR-149 |
| 2.6e+002 | 7.5e+001 | 0.71 | 3.51 (-) | 2.8e-007 | 42 | hsa-miR-224 |
| 3.2e+002 | 9.9e+001 | 0.79 | 3.25 (-) | 1.3e-011 | 334 | hsa-miR-483-5p |
| 3.0e+002 | 1.5e+002 | 0.79 | 2.08 (-) | 9.5e-012 | 265 | hsa-miR-130b |
| 2.0e+002 | 1.0e+002 | 0.76 | 2.00 (-) | 4.8e-009 | 22 | hsa-miR-181a* |
| + the higher expression of this miR is in kidney tumors<br>- the higher expression of this miR is in sarcoma, adrenal and mesothelioma tumors | | | | | | |

Figure 22 demonstrates binary decisions at node #33 of the decision-tree. Tumors originating in kidney (diamonds) are easily distinguished from tumors of adrenal, mesothelioma and sarcoma origin (squares) using the expression levels of hsa-miR-200b (SEQ ID NO: 29, y-axis), hsa-miR-30a (SEQ ID NO: 46, x-axis) and hsa-miR-149 (SEQ ID NO: 19, z-axis).

**Table 35:** miR expression (in fluorescence units) distinguishing between pheochromocytoma (neuroendocrine tumor of the adrenal) and all sarcoma, adrenal carcinoma and mesothelioma tumors

| median values | | auROC | fold-change | p-value | SEQ ID NO. | miR name |
|---|---|---|---|---|---|---|
| 5.0e+001 | 1.5e+004 | 0.96 | 295.36 (+) | 6.7e-067 | 65 | hsa-miR-7 |
| 5.0e+001 | 9.8e+003 | 0.91 | 196.58 (+) | 5.0e-036 | 56 | hsa-miR-375 |
| 1.3e+002 | 4.0e+003 | 0.85 | 29.73 (+) | 3.2e-009 | 11 | hsa-miR-138 |
| 5.0e+001 | 1.0e+003 | 0.94 | 20.53 (+) | 1.5e-021 | 162 | hsa-miR-129-3p |

(continued)

| median values | | auROC | fold-change | p-value | SEQ ID NO. | miR name |
|---|---|---|---|---|---|---|
| 2.7e+002 | 4.0e+003 | 0.84 | 15.11 (+) | 3.0e-008 | 59 | hsa-miR-487b |
| 1.5e+002 | 2.2e+003 | 0.81 | 14.54 (+) | 7.4e-008 | 331 | hsa-miR-432 |
| 7.0e+001 | 8.7e+002 | 0.84 | 12.45 (+) | 9.4e-011 | 350 | hsa-miR-539 |
| 9.6e+002 | 1.2e+004 | 0.80 | 12.36 (+) | 8.3e-005 | 155 | hsa-miR-127-3p |
| 5.8e+001 | 6.8e+002 | 0.80 | 11.61 (+) | 1.2e-008 | 335 | hsa-miR-485-3p |
| 5.0e+001 | 5.7e+002 | 0.87 | 11.48 (+) | 2.7e-008 | 159 | hsa-miR-124 |
| 5.3e+001 | 5.6e+002 | 0.86 | 10.67 (+) | 2.3e-014 | 336 | hsa-miR-485-5p |
| 5.0e+001 | 5.2e+002 | 0.83 | 10.38 (+) | 1.2e-012 | 332 | hsa-miR-433 |
| 5.0e+001 | 5.1e+002 | 0.94 | 10.28 (+) | 1.1e-029 | 263 | hsa-miR-129* |
| 5.0e+001 | 4.8e+002 | 0.82 | 9.55 (+) | 9.6e-008 | 306 | hsa-miR-323-3p |
| 1.2e+002 | 1.2e+003 | 0.79 | 9.22 (+) | 1.0e-005 | 339 | hsa-miR-495 |
| 5.8e+001 | 5.2e+002 | 0.80 | 9.01 (+) | 4.4e-006 | 337 | hsa-miR-487a |
| 1.6e+002 | 1.4e+003 | 0.80 | 8.75 (+) | 4.8e-006 | 275 | hsa-miR-154 |
| 7.1e+001 | 6.1e+002 | 0.90 | 8.56 (+) | 6.0e-013 | 301 | hsa-miR-29b-2* |
| 5.3e+001 | 4.5e+002 | 0.78 | 8.54 (+) | 3.7e-005 | 276 | hsa-miR-154* |
| 5.3e+001 | 4.1e+002 | 0.83 | 7.77 (+) | 4.7e-009 | 330 | hsa-miR-431* |
| 9.8e+001 | 7.4e+002 | 0.81 | 7.56 (+) | 1.3e-006 | 317 | hsa-miR-369-5p |
| 6.4e+001 | 4.8e+002 | 0.80 | 7.56 (+) | 1.4e-007 | 309 | hsa-miR-329 |
| 2.4e+002 | 1.8e+003 | 0.90 | 7.28 (+) | 1.4e-009 | 45 | hsa-miR-29c* |
| 1.5e+002 | 1.1e+003 | 0.79 | 7.24 (+) | 1.5e-005 | 318 | hsa-miR-370 |
| 2.2e+002 | 1.5e+003 | 0.80 | 6.74 (+) | 1.5e-005 | 324 | hsa-miR-382 |
| 1.3e+002 | 8.6e+002 | 0.76 | 6.53 (+) | 3.1e-004 | 352 | hsa-miR-543 |
| 2.3e+002 | 1.5e+003 | 0.89 | 6.44 (+) | 2.6e-008 | 191 | hsa-miR-29c |
| 9.6e+001 | 6.2e+002 | 0.79 | 6.40 (+) | 1.1e-005 | 262 | hsa-miR-127-5p |
| 1.5e+002 | 9.6e+002 | 0.77 | 6.39 (+) | 2.3e-004 | 269 | hsa-miR-134 |
| 5.4e+001 | 3.3e+002 | 0.90 | 6.03 (+) | 2.1e-012 | 313 | hsa-miR-338-3p |
| 2.2e+002 | 1.3e+003 | 0.84 | 5.80 (+) | 4.8e-008 | 19 | hsa-miR-149 |
| 5.0e+001 | 2.7e+002 | 0.82 | 5.32 (+) | 7.5e-012 | 367 | MID-00465 |
| 1.1e+002 | 5.6e+002 | 0.78 | 5.27 (+) | 5.3e-005 | 326 | hsa-miR-409-5p |
| 1.8e+002 | 9.6e+002 | 0.76 | 5.26 (+) | 3.1e-004 | 325 | hsa-miR-409-3p |
| 1.8e+002 | 9.2e+002 | 0.76 | 5.25 (+) | 8.2e-004 | 322 | hsa-miR-379 |
| 5.0e+001 | 2.5e+002 | 0.79 | 5.05 (+) | 4.6e-008 | 327 | hsa-miR-410 |
| 8.1e+002 | 4.0e+003 | 0.97 | 4.95 (+) | 3.4e-011 | 190 | hsa-miR-29b |
| 7.6e+001 | 3.7e+002 | 0.93 | 4.85 (+) | 7.6e-019 | 261 | hsa-miR-1180 |
| 6.4e+001 | 2.8e+002 | 0.79 | 4.43 (+) | 1.0e-005 | 321 | hsa-miR-377* |
| 5.0e+001 | 2.0e+002 | 0.81 | 4.09 (+) | 2.9e-009 | 360 | hsa-miR-873 |
| 5.3e+001 | 2.1e+002 | 0.88 | 4.08 (+) | 1.2e-012 | 353 | hsa-miR-598 |

(continued)

| median values | | auROC | fold-change | p-value | SEQ ID NO. | miR name |
|---|---|---|---|---|---|---|
| 3.3e+002 | 1.3e+003 | 0.73 | 4.01 (+) | 3.0e-003 | 312 | hsa-miR-337-5p |
| 6.6e+001 | 2.6e+002 | 0.77 | 3.96 (+) | 8.8e-005 | 371 | MID-16270 |
| 2.1e+003 | 7.2e+003 | 0.85 | 3.51 (+) | 6.2e-005 | 5 | hsa-miR-10b |
| 6.7e+001 | 2.3e+002 | 0.68 | 3.40 (+) | 2.6e-002 | 328 | hsa-miR-411 |
| 6.9e+003 | 2.2e+004 | 0.77 | 3.17 (+) | 4.3e-004 | 205 | hsa-miR-451 |
| 1.3e+003 | 1.1e+002 | 0.90 | 12.33 (-) | 2.9e-008 | 183 | hsa-miR-199b-5p |
| 5.5e+002 | 1.1e+002 | 0.86 | 4.75 (-) | 2.4e-006 | 38 | hsa-miR-214* |
| 1.5e+004 | 3.2e+003 | 0.84 | 4.48 (-) | 2.9e-005 | 181 | hsa-miR-199a-3p |
| 5.9e+003 | 1.3e+003 | 0.85 | 4.47 (-) | 6.8e-005 | 37 | hsa-miR-214 |
| 7.8e+003 | 1.8e+003 | 0.80 | 4.23 (-) | 5.9e-004 | 40 | hsa-miR-222 |
| 1.8e+004 | 4.5e+003 | 0.87 | 4.13 (-) | 9.0e-006 | 182 | hsa-miR-199a-5p |
| 5.2e+003 | 1.3e+003 | 0.80 | 4.11 (-) | 6.4e-004 | 147 | hsa-miR-221 |
| 7.0e+002 | 1.9e+002 | 0.85 | 3.72 (-) | 4.7e-007 | 17 | hsa-miR-146b-5p |
| 3.1e+002 | 8.9e+001 | 0.72 | 3.48 (-) | 2.3e-003 | 42 | hsa-miR-224 |
| 2.7e+003 | 8.0e+002 | 0.81 | 3.42 (-) | 3.8e-005 | 382 | MID-22331 |
| 2.6e+004 | 7.9e+003 | 0.79 | 3.35 (-) | 1.1e-004 | 34 | hsa-miR-21 |
| 4.2e+002 | 1.4e+002 | 0.74 | 3.08 (-) | 1.3e-003 | 18 | hsa-miR-148a |
| 6.3e+003 | 2.1e+003 | 0.83 | 3.03 (-) | 2.6e-005 | 3 | hsa-miR-100 |
| + the higher expression of this miR is in pheochromocytoma<br>- the higher expression of this miR is in sarcoma, adrenal carcinoma and mesothelioma tumors | | | | | | |

Figure 23 demonstrates binary decisions at node #34 of the decision-tree. Tumors originating in pheochromocytoma (diamonds) are easily distinguished from tumors of adrenal, mesothelioma and sarcoma origin (squares) using the expression levels of hsa-miR-375 (SEQ ID NO: 56, y-axis) and hsa-miR-7 (SEQ ID NO: 65, x-axis).

**Table 36:** miR expression (in fluorescence units) distinguishing between adrenal carcinoma and mesothelioma or sarcoma tumors

| median values | | auROC | fold-change | p-value | SEQ ID NO. | miR name |
|---|---|---|---|---|---|---|
| 5.0e+001 | 2.6e+003 | 0.98 | 51.10 (+) | 1.3e-040 | 61 | hsa-miR-509-3p |
| 5.4e+001 | 1.3e+003 | 0.76 | 24.55 (+) | 4.9e-007 | 333 | hsa-miR-483-3p |
| 5.0e+001 | 1.2e+003 | 0.99 | 24.01 (+) | 8.1e-066 | 31 | hsa-miR-202 |
| 6.4e+001 | 1.4e+003 | 0.95 | 21.83 (+) | 2.6e-024 | 347 | hsa-miR-513a-5p |
| 5.0e+001 | 6.0e+002 | 0.96 | 12.08 (+) | 9.3e-030 | 346 | hsa-miR-509-3-5p |
| 1.9e+002 | 2.2e+003 | 0.92 | 11.82 (+) | 2.2e-016 | 344 | hsa-miR-503 |
| 5.0e+001 | 5.1e+002 | 0.98 | 10.25 (+) | 3.8e-033 | 345 | hsa-miR-506 |
| 6.1e+001 | 5.9e+002 | 0.96 | 9.70 (+) | 1.2e-026 | 387 | MID-23751 |
| 3.1e+002 | 2.7e+003 | 0.71 | 8.66 (+) | 6.0e-005 | 334 | hsa-miR-483-5p |
| 1.4e+002 | 1.1e+003 | 0.91 | 7.79 (+) | 1.1e-015 | 351 | hsa-miR-542-5p |

(continued)

| median values | | auROC | fold-change | p-value | SEQ ID NO. | miR name |
|---|---|---|---|---|---|---|
| 2.0e+002 | 1.2e+003 | 0.72 | 5.77 (+) | 8.5e-005 | 324 | hsa-miR-382 |
| 1.0e+002 | 5.5e+002 | 0.75 | 5.44 (+) | 3.1e-007 | 326 | hsa-miR-409-5p |
| 1.4e+002 | 7.2e+002 | 0.73 | 5.31 (+) | 2.7e-004 | 269 | hsa-miR-134 |
| 7.9e+002 | 3.9e+003 | 0.69 | 4.98 (+) | 8.9e-003 | 155 | hsa-miR-127-3p |
| 2.1e+002 | 1.0e+003 | 0.68 | 4.93 (+) | 6.4e-003 | 320 | hsa-miR-376c |
| 1.6e+002 | 7.8e+002 | 0.69 | 4.84 (+) | 2.6e-003 | 322 | hsa-miR-379 |
| 2.2e+002 | 1.0e+003 | 0.72 | 4.53 (+) | 4.9e-005 | 59 | hsa-miR-487b |
| 1.5e+002 | 6.6e+002 | 0.69 | 4.49 (+) | 1.3e-003 | 318 | hsa-miR-370 |
| 1.7e+002 | 7.8e+002 | 0.71 | 4.45 (+) | 2.9e-004 | 325 | hsa-miR-409-3p |
| 1.1e+003 | 4.7e+003 | 0.92 | 4.19 (+) | 3.9e-011 | 245 | MID-18336 |
| 2.9e+002 | 1.1e+003 | 0.84 | 3.79 (+) | 1.8e-007 | 254 | MID-23291 |
| 1.4e+002 | 5.0e+002 | 0.70 | 3.71 (+) | 1.5e-004 | 331 | hsa-miR-432 |
| 1.5e+002 | 5.3e+002 | 0.69 | 3.60 (+) | 1.6e-003 | 275 | hsa-miR-154 |
| 3.1e+002 | 1.1e+003 | 0.90 | 3.48 (+) | 7.2e-011 | 180 | hsa-miR-1973 |
| 1.7e+002 | 5.4e+002 | 0.63 | 3.22 (+) | 6.9e-002 | 355 | hsa-miR-654-3p |
| 1.1e+003 | 3.5e+003 | 0.86 | 3.14 (+) | 8.6e-009 | 370 | MID-15986 |
| 1.8e+002 | 5.4e+002 | 0.64 | 3.07 (+) | 4.5e-002 | 323 | hsa-miR-381 |
| 2.9e+002 | 8.9e+002 | 0.63 | 3.07 (+) | 5.8e-002 | 312 | hsa-miR-337-5p |
| 1.8e+003 | 5.6e+003 | 0.88 | 3.03 (+) | 1.0e-008 | 178 | hsa-miR-193b |
| 1.4e+003 | 4.2e+003 | 0.81 | 3.02 (+) | 1.6e-006 | 249 | MID-20524 |
| 1.7e+003 | 9.5e+001 | 0.96 | 18.32 (-) | 1.3e-015 | 183 | hsa-miR-199b-5p |
| 1.9e+004 | 1.7e+003 | 0.95 | 10.80 (-) | 9.7e-014 | 181 | hsa-miR-199a-3p |
| 6.5e+002 | 6.1e+001 | 0.97 | 10.75 (-) | 2.6e-016 | 38 | hsa-miR-214* |
| 2.4e+004 | 2.5e+003 | 0.97 | 9.43 (-) | 1.9e-015 | 182 | hsa-miR-199a-5p |
| 7.1e+003 | 9.0e+002 | 0.96 | 7.89 (-) | 1.2e-011 | 37 | hsa-miR-214 |
| 7.5e+003 | 1.5e+003 | 0.90 | 4.87 (-) | 1.8e-012 | 3 | hsa-miR-100 |
| 2.5e+003 | 7.6e+002 | 0.83 | 3.37 (-) | 3.6e-006 | 25 | hsa-miR-193a-3p |
| 1.3e+003 | 4.3e+002 | 0.80 | 3.05 (-) | 2.5e-006 | 169 | hsa-miR-152 |
| + the higher expression of this miR is in adrenal carcinoma<br>- the higher expression of this miR is in sarcoma and mesothelioma tumors | | | | | | |

hsa-miR-202 (SEQ ID NO: 31), hsa-miR-509-3p (SEQ ID NO: 61) and hsa-miR-214* (SEQ ID NO: 38) are used at node 35 of the binary-tree-classifier detailed in the invention to distinguish between adrenal carcinoma and sarcoma or mesothelioma tumors.

**Table 37:** miR expression (in fluorescence units) distinguishing between GIST and mesothelioma or sarcoma tumors

| median values | | auROC | fold-change | p-value | SEQ ID NO. | miR name |
|---|---|---|---|---|---|---|
| 2.4e+002 | 5.6e+003 | 0.97 | 23.39 (+) | 4.2e-033 | 165 | hsa-miR-143* |

(continued)

| median values | | auROC | fold-change | p-value | SEQ ID NO. | miR name |
|---|---|---|---|---|---|---|
| 4.9e+003 | 1.0e+005 | 0.97 | 21.41 (+) | 1.7e-025 | 14 | hsa-miR-143 |
| 8.1e+003 | 1.5e+005 | 0.99 | 18.42 (+) | 4.2e-026 | 15 | hsa-miR-145 |
| 5.0e+001 | 7.9e+002 | 0.87 | 15.77 (+) | 1.5e-010 | 333 | hsa-miR-483-3p |
| 6.2e+001 | 8.4e+002 | 0.98 | 13.54 (+) | 1.5e-037 | 272 | hsa-miR-145* |
| 1.6e+002 | 1.6e+003 | 0.99 | 9.58 (+) | 2.7e-024 | 270 | hsa-miR-139-5p |
| 1.8e+002 | 1.8e+003 | 0.96 | 9.49 (+) | 2.7e-019 | 45 | hsa-miR-29c* |
| 6.1e+001 | 5.8e+002 | 0.95 | 9.48 (+) | 7.9e-028 | 301 | hsa-miR-29b-2* |
| 1.9e+002 | 1.5e+003 | 0.94 | 7.89 (+) | 1.9e-015 | 191 | hsa-miR-29c |
| 1.2e+003 | 6.3e+003 | 0.96 | 5.12 (+) | 8.8e-014 | 46 | hsa-miR-30a |
| 1.9e+002 | 7.3e+002 | 0.93 | 3.84 (+) | 6.6e-013 | 195 | hsa-miR-30a* |
| 2.4e+002 | 8.7e+002 | 0.92 | 3.66 (+) | 1.8e-008 | 266 | hsa-miR-132 |
| 6.1e+002 | 2.2e+003 | 0.91 | 3.52 (+) | 4.2e-008 | 190 | hsa-miR-29b |
| 1.9e+002 | 6.5e+002 | 0.82 | 3.50 (+) | 1.5e-006 | 19 | hsa-miR-149 |
| 2.6e+002 | 9.0e+002 | 0.82 | 3.47 (+) | 4.8e-005 | 334 | hsa-miR-483-5p |
| 9.3e+002 | 1.9e+002 | 0.70 | 5.00 (-) | 2.1e-003 | 155 | hsa-miR-127-3p |
| 3.4e+003 | 7.1e+002 | 0.88 | 4.74 (-) | 2.3e-007 | 25 | hsa-miR-193a-3p |
| 7.0e+003 | 1.9e+003 | 0.79 | 3.64 (-) | 5.5e-004 | 147 | hsa-miR-221 |
| 9.8e+003 | 2.8e+003 | 0.78 | 3.54 (-) | 1.1e-003 | 40 | hsa-miR-222 |
| 3.2e+004 | 9.8e+003 | 0.75 | 3.26 (-) | 1.1e-003 | 34 | hsa-miR-21 |
| + the higher expression of this miR is in GIST<br>- the higher expression of this miR is in sarcoma and mesothelioma tumors | | | | | | |

hsa-miR-29C* (SEQ ID NO: 45) and hsa-miR-143 (SEQ ID NO: 14) are used at node 36 of the binary-tree-classifier detailed in the invention to distinguish between GIST and sarcoma or mesothelioma tumors.

**Table 38:** miR expression (in fluorescence units) distinguishing between chromophobe renal cell carcinoma tumors and clear cell or papillary renal cell carcinoma tumors

| median values | | auROC | fold-change | p-value | SEQ ID NO. | miR name |
|---|---|---|---|---|---|---|
| 8.8e+001 | 2.1e+003 | 0.99 | 23.68 (+) | 4.7e-017 | 13 | hsa-miR-141 |
| 3.0e+002 | 5.7e+003 | 0.99 | 18.81 (+) | 8.4e-012 | 30 | hsa-miR-200c |
| 6.6e+001 | 9.8e+002 | 0.99 | 14.85 (+) | 7.5e-019 | 361 | hsa-miR-874 |
| 5.0e+001 | 7.4e+002 | 0.97 | 14.80 (+) | 1.0e-014 | 280 | hsa-miR-187 |
| 5.0e+001 | 7.2e+002 | 0.98 | 14.47 (+) | 4.7e-018 | 362 | hsa-miR-891a |
| 5.3e+003 | 7.4e+004 | 0.98 | 13.97 (+) | 5.3e-017 | 147 | hsa-miR-221 |
| 7.6e+003 | 9.0e+004 | 0.97 | 11.89 (+) | 1.4e-015 | 40 | hsa-miR-222 |
| 5.3e+001 | 5.1e+002 | 0.98 | 9.66 (+) | 1.2e-017 | 291 | hsa-miR-222* |
| 1.4e+002 | 1.1e+003 | 0.94 | 8.01 (+) | 2.7e-010 | 387 | MID-23751 |
| 7.4e+001 | 5.4e+002 | 0.97 | 7.32 (+) | 4.4e-015 | 290 | hsa-miR-221 * |
| 1.1e+002 | 5.6e+002 | 0.93 | 4.97 (+) | 3.2e-010 | 299 | hsa-miR-296-5p |

(continued)

| median values | | auROC | fold-change | p-value | SEQ ID NO. | miR name |
|---|---|---|---|---|---|---|
| 3.2e+002 | 1.5e+003 | 0.90 | 4.90 (+) | 3.1e-007 | 152 | hsa-miR-182 |
| 8.4e+002 | 3.3e+003 | 0.73 | 3.89 (+) | 6.3e-003 | 178 | hsa-miR-193b |
| 5.4e+003 | 1.7e+004 | 0.92 | 3.26 (+) | 2.2e-007 | 303 | hsa-miR-30b |
| 1.1e+003 | 3.5e+003 | 0.74 | 3.20 (+) | 8.1e-003 | 242 | MID-16489 |
| 6.2e+003 | 3.3e+002 | 0.85 | 18.53 (-) | 4.3e-006 | 49 | hsa-miR-31 |
| 6.1e+002 | 5.0e+001 | 0.90 | 12.13 (-) | 2.1e-007 | 11 | hsa-miR-138 |
| 1.8e+003 | 2.2e+002 | 0.98 | 8.38 (-) | 6.7e-014 | 35 | hsa-miR-21* |
| 7.9e+004 | 1.0e+004 | 0.95 | 7.54 (-) | 3.1e-013 | 34 | hsa-miR-21 |
| 3.7e+003 | 5.4e+002 | 0.92 | 6.79 (-) | 3.1e-009 | 36 | hsa-miR-210 |
| 9.8e+002 | 1.7e+002 | 0.97 | 5.71 (-) | 3.1e-013 | 206 | hsa-miR-455-3p |
| 1.0e+003 | 2.5e+002 | 0.91 | 4.07 (-) | 4.7e-008 | 16 | hsa-miR-146a |
| 6.0e+002 | 1.7e+002 | 0.89 | 3.64 (-) | 7.1e-007 | 170 | hsa-miR-155 |
| 7.5e+002 | 2.1e+002 | 0.78 | 3.48 (-) | 1.6e-003 | 177 | hsa-miR-192 |
| 8.6e+002 | 2.5e+002 | 0.86 | 3.39 (-) | 6.6e-006 | 17 | hsa-miR-146b-5p |
| + the higher expression of this miR is in chromophobe renal cell carcinoma tumors<br>- the higher expression of this miR is in clear cell or papillary renal cell carcinoma tumors | | | | | | |

hsa-miR-210 (SEQ ID NO: 36) and hsa-miR-221 (SEQ ID NO: 147) are used at node #37 of the binary-tree-classifier detailed in the invention to distinguish between chromophobe renal cell carcinoma tumors and clear cell or papillary renal cell carcinoma tumors.

**Table 39:** miR expression (in fluorescence units) distinguishing between clear cell and papillary renal cell carcinoma tumors

| median values | | auROC | fold-change | p-value | SEQ ID NO. | miR name |
|---|---|---|---|---|---|---|
| 1.2e+002 | 5.7e+002 | 0.89 | 4.81 (+) | 2.3e-005 | 344 | hsa-miR-503 |
| 1.6e+003 | 5.9e+003 | 0.81 | 3.65 (+) | 5.8e-003 | 382 | MID-22331 |
| 1.8e+003 | 6.4e+003 | 0.94 | 3.54 (+) | 1.1e-005 | 9 | hsa-miR-126 |
| 1.7e+003 | 5.7e+003 | 0.82 | 3.45 (+) | 3.0e-003 | 338 | hsa-miR-494 |
| 1.1e+004 | 1.3e+003 | 0.87 | 8.35 (-) | 3. 1e-004 | 29 | hsa-miR-200b |
| 8.7e+003 | 1.3e+003 | 0.81 | 6.61 (-) | 3.0e-002 | 49 | hsa-miR-31 |
| 5.1e+003 | 9.5e+002 | 0.92 | 5.30 (-) | 5.0e-00 disclosed 5 | 28 | hsa-miR-200a |
| 2.1e+003 | 5.1e+002 | 1.00 | 4.10 (-) | 1.1e-009 | 195 | hsa-miR-30a* |
| 1.9e+004 | 5.0e+003 | 1.00 | 3.70 (-) | 4.5e-010 | 46 | hsa-miR-30a |
| 5.3e+003 | 1.6e+003 | 0.86 | 3.39 (-) | 6.9e-004 | 4 | hsa-miR-10a |
| 7.6e+002 | 2.3e+002 | 0.76 | 3.23 (-) | 2.0e-002 | 11 | hsa-miR-138 |
| 6.4e+002 | 2.0e+002 | 0.79 | 3.17 (-) | 7.4e-003 | 254 | MID-23291 |
| + the higher expression of this miR is in renal clear cell carcinoma tumors<br>- the higher expression of this miR is in papillary renal cell carcinoma tumors | | | | | | |

hsa-miR-31 (SEQ ID NO: 49) and hsa-miR-126 (SEQ ID NO: 9) are used at node 38 of the binary-tree-classifier detailed in the invention to distinguish between renal clear cell and papillary cell carcinoma tumors.

**Table 40:** miR expression (in fluorescence units) distinguishing between pleural mesothelioma and sarcoma tumors

| median values | | auROC | fold-change | p-value | SEQ ID NO. | miR name |
|---|---|---|---|---|---|---|
| 1.2e+002 | 1.7e+003 | 0.78 | 13.97 (+) | 1.7e-006 | 49 | hsa-miR-31 |
| 4.3e+002 | 2.1e+003 | 0.89 | 5.01 (+) | 2.0e-011 | 35 | hsa-miR-21* |
| 5.9e+002 | 1.6e+003 | 0.84 | 2.75 (+) | 2.1e-008 | 17 | hsa-miR-146b-5p |
| 2.5e+004 | 6.9e+004 | 0.89 | 2.71 (+) | 4.8e-010 | 34 | hsa-miR-21 |
| 2.4e+003 | 6.1e+003 | 0.77 | 2.57 (+) | 2.3e-005 | 25 | hsa-miR-193a-3p |
| 1.2e+003 | 3.1e+003 | 0.75 | 2.49 (+) | 5.9e-005 | 36 | hsa-miR-210 |
| 4.6e+002 | 1.1e+003 | 0.70 | 2.33 (+) | 9.7e-004 | 168 | hsa-miR-150 |
| 2.9e+002 | 6.8e+002 | 0.75 | 2.33 (+) | 1.4e-004 | 170 | hsa-miR-155 |
| 3.2e+002 | 7.3e+002 | 0.76 | 2.25 (+) | 1.4e-005 | 26 | hsa-miR-193a-5p |
| 1.1e+003 | 2.4e+003 | 0.76 | 2.13 (+) | 1.7e-004 | 4 | hsa-miR-10a |
| 5.1e+002 | 1.0e+003 | 0.70 | 2.03 (+) | 1.1e-003 | 190 | hsa-miR-29b |
| 9.0e+002 | 1.8e+003 | 0.77 | 1.99 (+) | 1.5e-005 | 46 | hsa-miR-30a |
| 2.6e+003 | 4.9e+003 | 0.71 | 1.90 (+) | 8.9e-003 | 10 | hsa-miR-130a |
| 2.8e+003 | 5.2e+003 | 0.69 | 1.88 (+) | 1.6e-003 | 240 | MID-15965 |
| 4.3e+003 | 7.3e+003 | 0.67 | 1.71 (+) | 1.5e-002 | 43 | hsa-miR-29a |
| 5.0e+003 | 8.2e+003 | 0.72 | 1.65 (+) | 1.1e-004 | 39 | hsa-miR-22 |
| 3.8e+003 | 5.9e+003 | 0.64 | 1.57 (+) | 2.9e-002 | 385 | MID-23168 |
| 9.9e+002 | 1.5e+003 | 0.66 | 1.55 (+) | 1.4e-002 | 63 | hsa-miR-574-5p |
| 7.5e+002 | 1.2e+003 | 0.66 | 1.53 (+) | 2.8e-002 | 202 | hsa-miR-378 |
| 2.7e+004 | 7.4e+003 | 0.84 | 3.62 (-) | 3.3e-007 | 181 | hsa-miR-199a-3p |
| 1.1e+004 | 3.1e+003 | 0.81 | 3.45 (-) | 3.2e-006 | 37 | hsa-miR-214 |
| 2.8e+003 | 8.3e+002 | 0.85 | 3.36 (-) | 6.7e-008 | 5 | hsa-miR-10b |
| 2.9e+003 | 9.1e+002 | 0.74 | 3.19 (-) | 2.0e-004 | 183 | hsa-miR-199b-5p |
| 3.0e+004 | 1.1e+004 | 0.80 | 2.84 (-) | 6.0e-005 | 182 | hsa-miR-199a-5p |
| 8.4e+002 | 3.8e+002 | 0.78 | 2.22 (-) | 5.7e-005 | 38 | hsa-miR-214* |
| 1.2e+003 | 7.0e+002 | 0.75 | 1.69 (-) | 2.8e-004 | 206 | hsa-miR-455-3p |
| 7.0e+002 | 4.4e+002 | 0.75 | 1.61 (-) | 2.9e-003 | 296 | hsa-miR-26b |
| 5.0e+004 | 3.2e+004 | 0.71 | 1.58 (-) | 4.3e-003 | 1 | hsa-let-7c |
| + the higher expression of this miR is in pleural mesothelioma tumors<br>- the higher expression of this miR is in sarcoma tumors | | | | | | |

hsa-miR-21* (SEQ ID NO: 35) hsa-miR-130a (SEQ ID NO: 10) and hsa-miR-10b (SEQ ID NO: 5) are used at node 39 of the binary-tree-classifier detailed in the invention to distinguish between pleural mesothelioma tumors and sarcoma tumors.

**Table 41:** miR expression (in fluorescence units) distinguishing between synovial sarcoma and other sarcoma tumors

| median values | | auROC | fold-change | p-value | SEQ ID NO. | miR name |
|---|---|---|---|---|---|---|
| 5.1e+001 | 1.3e+003 | 0.89 | 25.03 (+) | 2.9e-009 | 152 | hsa-miR-182 |

(continued)

| median values | | auROC | fold-change | p-value | SEQ ID NO. | miR name |
|---|---|---|---|---|---|---|
| 5.0e+001 | 1.1e+003 | 0.92 | 21.59 (+) | 9.2e-009 | 29 | hsa-miR-200b |
| 5.0e+001 | 9.7e+002 | 0.88 | 19.35 (+) | 5.9e-007 | 159 | hsa-miR-124 |
| 5.0e+001 | 6.4e+002 | 0.92 | 12.81 (+) | 1.5e-008 | 28 | hsa-miR-200a |
| 1.3e+002 | 8.9e+002 | 0.88 | 7.00 (+) | 8.2e-005 | 339 | hsa-miR-495 |
| 1.6e+002 | 1.1e+003 | 0.89 | 6.94 (+) | 1.6e-005 | 275 | hsa-miR-154 |
| 1.1e+002 | 7.3e+002 | 0.87 | 6.53 (+) | 2.8e-004 | 352 | hsa-miR-54 disclosed 3 |
| 1.4e+002 | 8.8e+002 | 0.91 | 6.51 (+) | 6.1e-006 | 19 | hsa-miR-149 |
| 3.3e+002 | 2.0e+003 | 0.86 | 6.22 (+) | 9.6e-005 | 320 | hsa-miR-376c |
| 1.0e+003 | 6.2e+003 | 0.84 | 6.05 (+) | 1.5e-003 | 155 | hsa-miR-127-3p |
| 9.7e+001 | 5.2e+002 | 0.84 | 5.40 (+) | 1.7e-004 | 262 | hsa-miR-127-5p |
| 7.8e+002 | 4.2e+003 | 0.86 | 5.33 (+) | 2.5e-004 | 38 | hsa-miR-214* |
| 9.5e+003 | 5.0e+004 | 0.84 | 5.29 (+) | 7.3e-003 | 37 | hsa-miR-214 |
| 2.9e+004 | 1.4e+005 | 0.87 | 4.90 (+) | 4.7e-003 | 182 | hsa-miR-199a-5p |
| 1.4e+002 | 6.4e+002 | 0.81 | 4.56 (+) | 1.9e-003 | 331 | hsa-miR-432 |
| 1.1e+002 | 5.0e+002 | 0.84 | 4.43 (+) | 2.7e-004 | 317 | hsa-miR-369-5p |
| 2.1e+002 | 8.9e+002 | 0.78 | 4.19 (+) | 9.6e-003 | 323 | hsa-miR-381 |
| 1.9e+002 | 7.7e+002 | 0.81 | 3.96 (+) | 2.7e-003 | 355 | hsa-miR-654-3p |
| 5.6e+003 | 2.1e+004 | 0.91 | 3.79 (+) | 9.6e-006 | 3 | hsa-miR-100 |
| 1.7e+002 | 6.5e+002 | 0.86 | 3.76 (+) | 2.8e-004 | 282 | hsa-miR-196a |
| 4.1e+002 | 1.5e+003 | 0.80 | 3.55 (+) | 6.0e-003 | 312 | hsa-miR-337-5p |
| 2.6e+004 | 9.1e+004 | 0.86 | 3.52 (+) | 7.4e-003 | 181 | hsa-miR-199a-3p |
| 1.6e+002 | 5.5e+002 | 0.80 | 3.41 (+) | 6.5e-003 | 269 | hsa-miR-134 |
| 1.9e+002 | 6.4e+002 | 0.79 | 3.32 (+) | 8.3e-003 | 318 | hsa-miR-370 |
| 3.2e+002 | 1.0e+003 | 0.78 | 3.08 (+) | 7.6e-003 | 59 | hsa-miR-487b |
| 2.1e+002 | 6.4e+002 | 0.87 | 3.02 (+) | 7.1e-007 | 266 | hsa-miR-132 |
| 2.2e+002 | 6.3e+002 | 0.78 | 2.92 (+) | 1.1e-002 | 322 | hsa-miR-379 |
| 4.4e+004 | 1.2e+005 | 0.92 | 2.83 (+) | 1.6e-004 | 8 | hsa-miR-125b |
| 2.4e+002 | 6.1e+002 | 0.78 | 2.58 (+) | 8.4e-003 | 324 | hsa-miR-382 |
| 2.4e+003 | 6.0e+003 | 0.80 | 2.45 (+) | 1.8e-003 | 10 | hsa-miR-130a |
| 9.2e+003 | 8.4e+002 | 0.96 | 10.95 (-) | 2.1e-010 | 40 | hsa-miR-222 |
| 6.8e+003 | 6.7e+002 | 0.96 | 10.19 (-) | 7.9e-010 | 147 | hsa-miR-221 |
| 1.8e+003 | 3.6e+002 | 0.88 | 4.92 (-) | 2.1e-005 | 169 | hsa-miR-152 |
| 7.9e+003 | 1.7e+003 | 0.72 | 4.72 (-) | 2.8e-002 | 205 | hsa-miR-451 |
| 2.7e+004 | 5.9e+003 | 0.84 | 4.59 (-) | 4.4e-005 | 34 | hsa-miR-21 |
| 5.9e+002 | 1.4e+002 | 0.83 | 4.32 (-) | 9.2e-004 | 168 | hsa-miR-150 |
| 5.5e+003 | 1.3e+003 | 0.92 | 4.15 (-) | 2.2e-007 | 14 | hsa-miR-143 |
| 9.5e+003 | 2.9e+003 | 0.93 | 3.30 (-) | 2.7e-007 | 15 | hsa-miR-145 |

(continued)

| median values | | auROC | fold-change | p-value | SEQ ID NO. | miR name |
|---|---|---|---|---|---|---|
| 3.5e+003 | 1.0e+003 | 0.91 | 3.30 (-) | 1.6e-003 | 12 | hsa-miR-140-3p |
| 1.0e+003 | 3.5e+002 | 0.78 | 2.92 (-) | 6.1e-003 | 46 | hsa-miR-30a |
| 1.0e+003 | 3.6e+002 | 0.82 | 2.86 (-) | 4.1e-004 | 255 | MID-23794 |
| 5.8e+003 | 2.0e+003 | 0.88 | 2.82 (-) | 1.9e-005 | 39 | hsa-miR-22 |
| 1.1e+003 | 4.0e+002 | 0.77 | 2.74 (-) | 3.9e-003 | 23 | hsa-miR-185 |
| 5.8e+002 | 2.4e+002 | 0.80 | 2.45 (-) | 6.8e-003 | 190 | hsa-miR-29b |
| 7.1e+002 | 3.1e+002 | 0.79 | 2.27 (-) | 4.9e-003 | 236 | MID-00689 |
| 6.7e+004 | 3.2e+004 | 0.85 | 2.10 (-) | 2.2e-004 | 386 | MID-23178 |
| 7.7e+004 | 3.7e+004 | 0.80 | 2.08 (-) | 2.9e-003 | 379 | MID-18395 |
| 7.8e+002 | 3.8e+002 | 0.78 | 2.07 (-) | 7.4e-003 | 202 | hsa-miR-378 |

+ the higher expression of this miR is in synovial sarcoma tumors
- the higher expression of this miR is in other sarcoma tumors

hsa-miR-100 (SEQ ID NO: 3) hsa-miR-145 (SEQ ID NO: 15) and hsa-miR-222 (SEQ ID NO: 40) are used at node 40 of the binary-tree-classifier detailed in the invention to distinguish between synovial sarcoma tumors and other sarcoma tumors.

**Table 42:** miR expression (in fluorescence units) distinguishing between chondrosarcoma and other non synovial sarcoma tumors

| median values | | auROC | fold-change | p-value | SEQ ID NO. | miR name |
|---|---|---|---|---|---|---|
| 2.9e+003 | 2.2e+005 | 1.00 | 75.69 (+) | 2.1e-022 | 12 | hsa-miR-140-3p |
| 1.5e+002 | 5.1e+003 | 0.91 | 35.23 (+) | 8.5e-015 | 271 | hsa-miR-140-Sp |
| 1.1e+003 | 1.6e+004 | 0.98 | 14.49 (+) | 6.1e-015 | 206 | hsa-miR-455-3p |
| 5.0e+001 | 5.5e+002 | 0.71 | 11.03 (+) | 3.1e-003 | 333 | hsa-miR-483-3p |
| 9.5e+001 | 1.1e+003 | 0.88 | 11.01 (+) | 1.2e-006 | 11 | hsa-miR-138 |
| 9.2e+001 | 8.2e+002 | 0.87 | 8.87 (+) | 6.3e-012 | 58 | hsa-miR-455-5p |
| 1.1e+003 | 4.7e+003 | 0.91 | 4.37 (+) | 1.5e-006 | 36 | hsa-miR-210 |
| 3.6e+002 | 1.4e+003 | 0.83 | 3.98 (+) | 3.1e-004 | 18 | hsa-miR-148a |
| 1.5e+003 | 3.6e+003 | 0.72 | 2.36 (+) | 2.3e-002 | 178 | hsa-miR-193b |
| 1.3e+004 | 2.8e+004 | 0.84 | 2.13 (+) | 1.5e-004 | 293 | hsa-miR-23b |
| 2.7e+003 | 5.5e+003 | 0.80 | 2.05 (+) | 5.8e-004 | 189 | hsa-miR-27b |
| 3.4e+003 | 6.7e+002 | 0.70 | 5.01 (-) | 1.1e-004 | 382 | MID-22331 |
| 6.6e+002 | 1.7e+002 | 0.81 | 3.91 (-) | 1.2e-004 | 381 | MID-19962 |
| 3.2e+003 | 8.5e+002 | 0.83 | 3.76 (-) | 1.9e-004 | 240 | MID-15965 |
| 1.5e+003 | 4.2e+002 | 0.79 | 3.47 (-) | 8.0e-004 | 249 | MID-20524 |
| 2.9e+003 | 9.0e+002 | 0.85 | 3.27 (-) | 1.3e-005 | 5 | hsa-miR-10b |
| 2.9e+003 | 1.0e+003 | 0.78 | 2.92 (-) | 6.9e-005 | 377 | MID-17866 |
| 7.1e+002 | 2.7e+002 | 0.75 | 2.62 (-) | 1.3e-003 | 235 | hsa-miR-1978 |
| 7.0e+002 | 2.8e+002 | 0.81 | 2.48 (-) | 4.4e-005 | 17 | hsa-miR-146b-5p |

(continued)

| median values | | auROC | fold-change | p-value | SEQ ID NO. | miR name |
|---|---|---|---|---|---|---|
| 1.3e+003 | 5.7e+002 | 0.71 | 2.36 (-) | 2.7e-002 | 20 | hsa-miR-17 |
| 4.5e+003 | 1.9e+003 | 0.73 | 2.36 (-) | 8.2e-003 | 385 | MID-23168 |
| 1.1e+004 | 5.0e+003 | 0.74 | 2.16 (-) | 4.8e-003 | 384 | MID-23017 |
| 1.1e+003 | 5.4e+002 | 0.79 | 2.04 (-) | 3.2e-004 | 46 | hsa-miR-30a |
| 1.6e+004 | 8.1e+003 | 0.83 | 2.02 (-) | 3.0e-004 | 283 | hsa-miR-1979 |
| + the higher expression of this miR is in chondrosarcoma tumors<br>- the higher expression of this miR is in other non-synovial sarcoma tumors | | | | | | |

hsa-miR-140-3p (SEQ ID NO: 12) and hsa-miR-455-5p (SEQ ID NO: 58) are used at node 41 of the binary-tree-classifier detailed in the invention to distinguish between chondrosarcoma tumors and other non-synovial sarcoma tumors.

**Table 43:** miR expression (in fluorescence units) distinguishing between liposarcoma and other non chondrosarcoma and non synovial sarcoma tumors

| median values | | auROC | fold-change | p-value | SEQID NO. | miR name |
|---|---|---|---|---|---|---|
| 1.9e+004 | 1.2e+005 | 0.93 | 6.18 (+) | 1.6e-011 | 295 | hsa-miR-26a |
| 4.2e+003 | 1.8e+004 | 0.73 | 4.20 (+) | 8.1e-003 | 205 | hsa-miR-451 |
| 1.5e+003 | 5.9e+003 | 0.84 | 3.94 (+) | 6.5e-006 | 25 | hsa-miR-193a-3p |
| 2.4e+002 | 8.8e+002 | 0.88 | 3.70 (+) | 7.5e-007 | 26 | hsa-miR-193a-5p |
| 6.1e+003 | 2.0e+004 | 0.88 | 3.24 (+) | 2.2e-005 | 231 | hsa-miR-99a |
| 2.3e+003 | 5.9e+003 | 0.75 | 2.60 (+) | 1.9e-003 | 183 | hsa-miR-199b-5p |
| 3.1e+002 | 7.9e+002 | 0.79 | 2.54 (+) | 1.7e-004 | 42 | hsa-miR-224 |
| 2.9e+002 | 7.4e+002 | 0.71 | 2.54 (+) | 9.9e-003 | 254 | MID-23291 |
| 4.2e+002 | 1.0e+003 | 0.71 | 2.38 (+) | 1.5e-002 | 168 | hsa-miR-150 |
| 3.2e+002 | 7.7e+002 | 0.77 | 2.36 (+) | 1.1e-004 | 64 | hsa-miR-652 |
| 4.8e+003 | 1.1e+004 | 0.84 | 2.27 (+) | 5.4e-006 | 14 | hsa-miR-143 |
| 1.4e+003 | 3.0e+003 | 0.76 | 2.20 (+) | 2.7e-004 | 178 | hsa-miR-193b |
| 7.9e+003 | 1.7e+004 | 0.78 | 2.13 (+) | 1.1e-004 | 15 | hsa-miR-145 |
| 4.6e+003 | 9.7e+003 | 0.79 | 2.12 (+) | 9.8e-004 | 39 | hsa-miR-22 |
| 1.4e+003 | 3.1e+002 | 0.79 | 4.49 (-) | 1.8e-004 | 36 | hsa-miR-210 |
| 2.4e+003 | 9.0e+002 | 0.71 | 2.60 (-) | 1.2e-002 | 154 | hsa-miR-181b |
| 5.9e+002 | 2.6e+002 | 0.75 | 2.29 (-) | 4.0e-003 | 265 | hsa-miR-130b |
| 6.5e+002 | 3.0e+002 | 0.75 | 2.16 (-) | 3.2e-003 | 174 | hsa-miR-181d |
| 1.2e+004 | 5.6e+003 | 0.79 | 2.14 (-) | 2.0e-004 | 384 | MID-23017 |

(continued)

| median values | | auROC | fold-change | p-value | SEQID NO. | miR name |
|---|---|---|---|---|---|---|
| 3.3e+003 | 1.6e+003 | 0.80 | 2.04 (-) | 6.6e-004 | 67 | hsa-miR-92a |
| + the higher expression of this miR is in liposarcoma tumors<br>- the higher expression of this miR is in other non-chondrosarcoma and non-synovial sarcoma tumors | | | | | | |

hsa-miR-210 (SEQ ID NO: 36) and hsa-miR-193a-5p (SEQ ID NO: 26) are used at node 42 of the binary-tree-classifier detailed in the invention to distinguish between liposarcoma tumors and other non-chondrosarcoma and non-synovial sarcoma tumors.

**Table 44:** miR expression (in fluorescence units) distinguishing between Ewing sarcoma or osteosarcoma; and rhabdomyosarcoma, malignant fibrous histiocytoma (MFH) or fibrosarcoma

| median values | | auROC | fold-change | p-value | SEQID NO. | miR name |
|---|---|---|---|---|---|---|
| 1.9e+002 | 1.2e+003 | 0.87 | 6.62 (+) | 1.1e-006 | 22 | hsa-miR-181a* |
| 1.1e+003 | 6.4e+003 | 0.91 | 5.68 (+) | 8.7e-009 | 154 | hsa-miR-181b |
| 3.7e+003 | 2.1e+004 | 0.93 | 5.67 (+) | 2.9e-010 | 21 | hsa-miR-181a |
| 4.2e+002 | 1.8e+003 | 0.85 | 4.19 (+) | 3.5e-006 | 174 | hsa-miR-181d |
| 2.9e+003 | 9.4e+003 | 0.72 | 3.27 (+) | 1.2e-002 | 205 | hsa-miR-451 |
| 1.8e+003 | 4.7e+003 | 0.78 | 2.63 (+) | 2.9e-003 | 158 | hsa-miR-106a |
| 1.1e+003 | 2.8e+003 | 0.78 | 2.52 (+) | 2.9e-003 | 186 | hsa-miR-20a |
| 2.0e+003 | 4.9e+003 | 0.81 | 2.45 (+) | 9.2e-005 | 148 | hsa-miR-93 |
| 1.1e+003 | 2.6e+003 | 0.77 | 2.32 (+) | 5.1e-003 | 20 | hsa-miR-17 |
| 6.0e+002 | 1.3e+002 | 0.71 | 4.54 (-) | 1.1e-002 | 59 | hsa-miR-487b |
| 4.9e+004 | 1.7e+004 | 0.84 | 2.86 (-) | 2.9e-005 | 8 | hsa-miR-125b |
| 3.4e+003 | 1.3e+003 | 0.72 | 2.70 (-) | 9.4e-003 | 183 | hsa-miR-199b-5p |
| 8.1e+003 | 3.5e+003 | 0.76 | 2.34 (-) | 1.1e-003 | 231 | hsa-miR-99a |
| + the higher expression of this miR is in Ewing sarcoma or osteosarcoma tumors<br>- the higher expression of this miR is in rhabdomyosarcoma, malignant fibrous histiocytoma (MFH) or fibrosarcoma tumors | | | | | | |

hsa-miR-181a (SEQ ID NO: 21) is used at node 43 of the binary-tree-classifier detailed in the invention to distinguish between Ewing sarcoma or osteosarcoma tumors and rhabdomyosarcoma, malignant fibrous histiocytoma (MFH) or fibrosarcoma tumors.

**Table 45:** miR expression (in fluorescence units) distinguishing between Ewing sarcoma and osteosarcoma

| median values | | auROC | fold-change | p-value | SEQ ID NO. | miR name |
|---|---|---|---|---|---|---|
| 1.6e+002 | 1.1e+003 | 1.00 | 6.60 (+) | 3.7e-006 | 155 | hsa-miR-127-3p |
| 1.4e+003 | 8.5e+003 | 0.97 | 5.85 (+) | 8.9e-004 | 179 | hsa-miR-195 |
| 2.8e+003 | 1.4e+004 | 0.86 | 4.90 (+) | 1.4e-002 | 43 | hsa-miR-29a |
| 1.4e+003 | 6.5e+003 | 1.00 | 4.58 (+) | 1.1e-004 | 208 | hsa-miR-497 |
| 1.7e+002 | 7.6e+002 | 0.88 | 4.42 (+) | 1.0e-003 | 278 | hsa-miR-181a-2* |
| 4.0e+002 | 1.6e+003 | 0.86 | 4.05 (+) | 6.0e-003 | 17 | hsa-miR-146b-5p |

(continued)

| median values | | auROC | fold-change | p-value | SEQ ID NO. | miR name |
|---|---|---|---|---|---|---|
| 3.4e+003 | 8.9e+003 | 0.81 | 2.64 (+) | 1.4e-002 | 385 | MID-23168 |
| 8.0e+002 | 2.1e+003 | 0.77 | 2.60 (+) | 1.5e-002 | 174 | hsa-miR-181d |
| 1.6e+003 | 4.1e+003 | 0.82 | 2.55 (+) | 1.3e-002 | 5 | hsa-miR-10b |
| 2.2e+003 | 4.9e+003 | 0.84 | 2.19 (+) | 7.1e-003 | 52 | hsa-miR-34a |
| 2.5e+004 | 5.4e+004 | 0.97 | 2.16 (+) | 2.7e-004 | 257 | hsa-let-7b |
| 2.5e+002 | 5.2e+002 | 0.88 | 2.12 (+) | 2.1e-003 | 366 | MID-00144 |
| 4.5e+002 | 9.4e+002 | 0.84 | 2.06 (+) | 6.2e-003 | 48 | hsa-miR-30e |
| 1.3e+003 | 5.0e+001 | 0.96 | 25.44 (-) | 7.9e-005 | 49 | hsa-miR-31 |
| 1.2e+004 | 2.0e+003 | 0.89 | 5.72 (-) | 1.4e-003 | 12 | hsa-miR-140-3p |
| 3.8e+003 | 7.6e+002 | 0.94 | 4.92 (-) | 5.2e-005 | 25 | hsa-miR-193a-3p |
| 1.8e+003 | 4.4e+002 | 0.89 | 4.09 (-) | 3.3e-003 | 169 | hsa-miR-152 |
| 3.7e+004 | 1.2e+004 | 0.89 | 3.00 (-) | 3.2e-003 | 34 | hsa-miR-21 |
| 8.1e+002 | 2.7e+002 | 0.83 | 2.96 (-) | 1.7e-003 | 35 | hsa-miR-21* |
| 1.7e+003 | 6.7e+002 | 0.88 | 2.55 (-) | 4.2e-003 | 23 | hsa-miR-185 |
| 2.1e+004 | 8.2e+003 | 0.82 | 2.53 (-) | 1.7e-002 | 384 | MID-23017 |
| 4.3e+003 | 1.7e+003 | 0.84 | 2.52 (-) | 3.8e-003 | 189 | hsa-miR-27b |
| 5.1e+003 | 2.3e+003 | 0.80 | 2.18 (-) | 3.0e-002 | 377 | MID-17866 |
| 9.6e+002 | 4.4e+002 | 0.78 | 2.17 (-) | 3.0e-002 | 265 | hsa-miR-130b |
| 3.7e+004 | 1.8e+004 | 0.82 | 2.07 (-) | 3.3e-003 | 294 | hsa-miR-24 |
| 1.8e+004 | 8.8e+003 | 0.86 | 2.03 (-) | 9.0e-003 | 293 | hsa-miR-23b |
| 3.0e+004 | 1.5e+004 | 0.80 | 2.02 (-) | 1.6e-002 | 292 | hsa-miR-23a |
| + the higher expression of this miR is in Ewing sarcoma tumors<br>- the higher expression of this miR is in osteosarcoma tumors | | | | | | |

Figure 24 demonstrates binary decisions at node #44 of the decision-tree. Tumors originating in Ewing sarcoma (diamonds) are easily distinguished from tumors of osteosarcoma origin (squares) using the expression levels of hsa-miR-31 (SEQ ID NO: 49, y-axis) and hsa-miR-193a-3p (SEQ ID NO: 25, x-axis).

**Table 46:** miR expression (in fluorescence units) distinguishing between rhabdomyosarcoma and malignant fibrous histiocytoma (MFH) or fibrosarcoma

| median values | | auROC | fold-change | p-value | SEQID NO. | miR name |
|---|---|---|---|---|---|---|
| 5.0e+001 | 4.1e+003 | 0.96 | 81.34 (+) | 1.9e-007 | 33 | hsa-miR-206 |
| 5.7e+001 | 4.3e+003 | 0.89 | 74.89 (+) | 1.8e-004 | 268 | hsa-miR-133b |
| 5.9e+001 | 3.9e+003 | 0.88 | 66.65 (+) | 3.2e-004 | 267 | hsa-miR-133a |
| 5.0e+001 | 1.3e+003 | 0.89 | 25.89 (+) | 3.9e-006 | 333 | hsa-miR-483-3p |
| 5.3e+001 | 5.2e+002 | 0.85 | 9.90 (+) | 1.3e-004 | 276 | hsa-miR-154* |
| 5.8e+001 | 5.6e+002 | 0.85 | 9.63 (+) | 1.2e-004 | 319 | hsa-miR-376a |
| 5.7e+001 | 5.1e+002 | 0.86 | 9.00 (+) | 4.8e-005 | 306 | hsa-miR-323-3p |
| 2.5e+002 | 1.8e+003 | 0.84 | 7.01 (+) | 2.8e-003 | 320 | hsa-miR-376c |

(continued)

| median values | | auROC | fold-change | p-value | SEQID NO. | miR name |
|---|---|---|---|---|---|---|
| 2.6e+002 | 1.7e+003 | 0.82 | 6.52 (+) | 3.9e-003 | 334 | hsa-miR-483-5p |
| 3.1e+002 | 1.9e+003 | 0.87 | 6.22 (+) | 5.1e-004 | 323 | hsa-miR-381 |
| 1.0e+002 | 6.3e+002 | 0.85 | 6.19 (+) | 5.4e-004 | 300 | hsa-miR-299-3p |
| 1.3e+002 | 7.9e+002 | 0.82 | 6.18 (+) | 1.4e-003 | 281 | hsa-miR-188-5p |
| 4.1e+002 | 2.3e+003 | 0.86 | 5.73 (+) | 1.4e-003 | 59 | hsa-miR-487b |
| 1.5e+002 | 8.4e+002 | 0.85 | 5.68 (+) | 8.1e-004 | 339 | hsa-miR-495 |
| 3.7e+002 | 1.7e+003 | 0.79 | 4.57 (+) | 3.1e-002 | 316 | hsa-miR-362-5p |
| 2.0e+002 | 9.2e+002 | 0.80 | 4.49 (+) | 2.4e-003 | 176 | hsa-miR-18a |
| 2.9e+002 | 1.3e+003 | 0.82 | 4.39 (+) | 1.4e-003 | 348 | hsa-miR-532-3p |
| 1.8e+002 | 7.8e+002 | 0.85 | 4.27 (+) | 4.0e-004 | 352 | hsa-miR-543 |
| 4.0e+002 | 1.7e+003 | 0.81 | 4.18 (+) | 2.3e-002 | 349 | hsa-miR-532-5p |
| 1.9e+003 | 7.8e+003 | 0.87 | 4.14 (+) | 4.9e-004 | 67 | hsa-miR-92a |
| 5.7e+002 | 2.4e+003 | 0.86 | 4.13 (+) | 9.2e-004 | 357 | hsa-miR-660 |
| 1.3e+002 | 5.6e+002 | 0.78 | 4.13 (+) | 4.2e-003 | 315 | hsa-miR-362-3p |
| 2.3e+002 | 8.6e+002 | 0.81 | 3.73 (+) | 2.8e-003 | 343 | hsa-miR-502-3p |
| 2.0e+002 | 7.2e+002 | 0.84 | 3.64 (+) | 1.5e-003 | 342 | hsa-miR-501-3p |
| 2.3e+002 | 8.5e+002 | 0.82 | 3.62 (+) | 6.7e-003 | 355 | hsa-miR-654-3p |
| 1.9e+002 | 6.7e+002 | 0.79 | 3.56 (+) | 1.3e-002 | 340 | hsa-miR-500 |
| 2.4e+002 | 8.4e+002 | 0.80 | 3.56 (+) | 7.9e-003 | 344 | hsa-miR-503 |
| 2.2e+003 | 7.6e+003 | 0.78 | 3.53 (+) | 7.2e-003 | 10 | hsa-miR-130a |
| 2.6e+002 | 8.8e+002 | 0.80 | 3.35 (+) | 3.7e-003 | 341 | hsa-miR-500* |
| 2.6e+002 | 7.9e+002 | 0.79 | 3.06 (+) | 7.3e-003 | 331 | hsa-miR-432 |
| 9.3e+002 | 2.7e+003 | 0.77 | 2.90 (+) | 1.4e-002 | 20 | hsa-miR-17 |
| 4.3e+002 | 1.2e+003 | 0.86 | 2.90 (+) | 1.0e-003 | 277 | hsa-miR-17* |
| 2.4e+002 | 6.7e+002 | 0.83 | 2.77 (+) | 7.0e-003 | 318 | hsa-miR-370 |
| 1.6e+003 | 4.5e+003 | 0.78 | 2.75 (+) | 1.4e-002 | 158 | hsa-miR-106a |
| 4.3e+002 | 1.1e+003 | 0.83 | 2.67 (+) | 3.0e-003 | 265 | hsa-miR-130b |
| 1.0e+003 | 2.7e+003 | 0.86 | 2.63 (+) | 7.1e-004 | 284 | hsa-miR-19b |
| 8.6e+002 | 2.1e+003 | 0.82 | 2.43 (+) | 8.6e-003 | 36 | hsa-miR-210 |
| 6.1e+003 | 6.8e+002 | 0.90 | 8.92 (-) | 1.8e-004 | 183 | hsa-miR-199b-5p |
| 1.9e+004 | 4.5e+003 | 0.83 | 4.15 (-) | 8.0e-004 | 40 | hsa-miR-222 |
| 1.1e+003 | 3.1e+002 | 0.90 | 3.55 (-) | 5.6e-005 | 63 | hsa-miR-574-5p |
| 1.1e+004 | 3.2e+003 | 0.82 | 3.52 (-) | 2.2e-003 | 147 | hsa-miR-221 |
| 5.9e+003 | 1.8e+003 | 0.80 | 3.25 (-) | 2.0e-003 | 43 | hsa-miR-29a |
| 5.2e+002 | 1.6e+002 | 0.82 | 3.19 (-) | 5.4e-003 | 289 | hsa-miR-22* |
| 5.1e+003 | 1.7e+003 | 0.82 | 3.04 (-) | 7.0e-003 | 52 | hsa-miR-34a |
| 8.1e+002 | 2.9e+002 | 0.76 | 2.81 (-) | 1.4e-002 | 190 | hsa-miR-29b |

(continued)

| median values | | auROC | fold-change | p-value | SEQID NO. | miR name |
|---|---|---|---|---|---|---|
| 1.2e+003 | 4.5e+002 | 0.86 | 2.67 (-) | 4.7e-003 | 4 | hsa-miR-10a |
| 3.7e+003 | 1.5e+003 | 0.86 | 2.43 (-) | 1.3e-003 | 5 | hsa-miR-10b |
| 7.0e+003 | 2.9e+003 | 0.85 | 2.39 (-) | 1.5e-003 | 39 | hsa-miR-22 |
| 1.6e+003 | 6.9e+002 | 0.78 | 2.25 (-) | 1.5e-002 | 169 | hsa-miR-152 |
| 2.9e+003 | 1.3e+003 | 0.76 | 2.19 (-) | 2.8e-002 | 208 | hsa-miR-497 |
| + the higher expression of this miR is in rhabdomyosarcoma tumors<br>- the higher expression of this miR is in MFH or fibrosarcoma tumors | | | | | | |

Figure 25 demonstrates binary decisions at node #45 of the decision-tree. Tumors originating in Rhabdomyosarcoma (diamonds) are easily distinguished from tumors of malignant fibrous histiocytoma (MFH) or fibrosarcoma origin (squares) using the expression levels of hsa-miR-206 (SEQ ID NO: 33, y-axis), hsa-miR-22 (SEQ ID NO: 39, x-axis) and hsa-miR-487b (SEQ ID NO: 59, z-axis).

**Table 47:** β values of the decision tree classifier

The classification at node 11 is based on the gender of subject rather than on beta values; accordingly, no data is provided for this node. $P_{TH}$ = 0.5 for all nodes

| miR 3 | | | miR 2 | | | miR 1 | | | | |
|---|---|---|---|---|---|---|---|---|---|---|
| β3 | SEQ ID NO | **miR** hsa- | β2 | SEQ ID NO | **miR** hsa- | β1 | SEQ ID NO | **miR** hsa- | β0 intercept | Node |
| | | | | | | 2.3127 | 55 | miR-372 | -23.3111 | 1 |
| | | | | | | 2.3127 | 6 | miR-122 | -26.9408 | 2 |
| | | | -1.379 | 9 | miR-126 | 1.8567 | 29 | miR-200b | -3.8519 | 3 |
| | | | -1.2306 | 46 | miR-30a | 1.9582 | 30 | miR-200c | -8.2646 | 4 |
| -0.88435 | 46 | miR-30a | -1.7697 | 2 | let-7e | 1.1979 | 16 | miR-146a | 17.4706 | 5 |
| | | | 1.7188 | 68 | miR-92b | 1.5475 | 66 | miR-9* | -32.5621 | 6 |
| | | | 2.0005 | 208 | miR-497 | -1.1606 | 40 | miR-222 | -9.5521 | 7 |
| 1.6602 | 56 | miR-375 | 1.2404 | 65 | miR-7 | -1.0267 | 25 | miR-193a-3p | -23.053 | 8 |
| | | | 1.1414 | 35 | miR-21* | 2.0115 | 27 | miR-194 | -29.3207 | 9 |
| | | | 0.9879 | 14 | miR-143 | -1.5458 | 21 | miR-181a | 1.244 | 10 |
| | | | -1.256 | 211 | miR-516a-5p | -1.942 | 29 | miR-200b | 21.3416 | 12 |
| -1.0128 | 51 | miR-345 | 1.1064 | 32 | miR-205 | -1.1455 | 7 | miR-125a-5p | 10.3775 | 13 |
| 0.82076 | 56 | miR-375 | 0.93196 | 50 | miR-342-3p | 1.9505 | 25 | miR-193a-3p | -40.666 | 14 |
| -0.91632 | 32 | miR-205 | 0.61098 | 4 | miR-10a | -1.8153 | 39 | miR-22 | 26.2937 | 15 |
| -1.119 | 4 | miR-10a | 1.5494 | 11 | miR-138 | -1.3023 | 148 | miR-93 | 9.4008 | 16 |
| | | | -1.4509 | 17 | miR-146b-5p | -1.801 | 34 | miR-21 | 42.5529 | 17 |
| -1.3119 | 67 | miR-92a | -0.63021 | 49 | miR-31 | 1.7974 | 25 | miR-193a-3p | 0.52521 | 18 |
| 1.6447 | 34 | miR-21 | -1.3077 | 202 | miR-378 | 0.9662 | 11 | miR-138 | -20.7179 | 19 |
| | | | -2.0444 | 34 | miR-21 | 1.0814 | 3 | miR-100 | 15.0039 | 20 |
| 1.734 | 69 | miR-934 | 0.22547 | 191 | miR-29c | 1.5137 | 24 | miR-191 | -31.6015 | 21 |
| 1.6178 | 54 | miR-361-5p | 0.86212 | 1 | let-7c | 1.41 | 5 | miR-10b | -44.3141 | 22 |
| -1.8652 | 23 | miR-185 | 1.3275 | 5 | miR-10b | -0.32773 | 11 | miR-138 | 7.6168 | 23 |
| | | | 1.5521 | 47 | miR-30d | -1.7146 | 50 | miR-342-3p | 2.4904 | 24 |

(continued)

The classification at node 11 is based on the gender of subject rather than on beta values; accordingly, no data is provided for this node. $P_{TH}$ = 0.5 for all nodes

| miR 3 | | | miR 2 | | | miR 1 | | | | |
|---|---|---|---|---|---|---|---|---|---|---|
| β3 | SEQ ID NO | miR hsa- | β2 | SEQ ID NO | miR hsa- | β1 | SEQ ID NO | miR hsa- | β0 intercept | Node |
| | | | -1.3096 | 45 | miR-29c* | 1.9063 | 20 | miR-17 | -10.0563 | 26 |
| -0.63749 | 67 | miR-92a | -1.5907 | 68 | miR-92b | 1.5531 | 40 | miR-222 | -2.3904 | 27 |
| 1.0197 | 53 | miR-34c-5p | -0.65807 | 37 | miR-214 | 1.9688 | 64 | miR-652 | -22.027 | 28 |
| | | | -1.3936 | 18 | miR-148a | 1.8457 | 34 | miR-21 | -11.4697 | 29 |
| -0.50909 | 146 | 1201 | -0.79749 | 36 | miR-210 | -1.3059 | 42 | miR-224 | 21.7628 | 30 |
| -1.3361 | 46 | miR-30a | 1.6268 | 43 | miR-29a | 0.95763 | 20 | miR-17 | -17.747 | 31 |
| -1.8214 | 51 | miR-345 | 0.62041 | 16 | miR-146a | 1.0661 | 49 | miR-31 | -2.3716 | 32 |
| 1.0224 | 46 | miR-30a | -2.0172 | 19 | miR-149 | 0.48415 | 29 | miR-200b | -4.226 | 33 |
| | | | 0.87847 | 56 | miR-375 | 2.1394 | 65 | miR-7 | -29.6828 | 34 |
| -0.057027 | 38 | miR-214* | 0.76095 | 61 | miR-509-3p | 2.1832 | 31 | miR-202 | -23.6445 | 35 |
| | | | 1.9413 | 14 | miR-143 | 1.2571 | 45 | miR-29c* | -41.4047 | 36 |
| | | | -0.63202 | 36 | miR-210 | 2.2247 | 147 | miR-221 | -25.1227 | 37 |
| | | | 2.3043 | 9 | miR-126 | -0.19797 | 49 | miR-31 | -24.5409 | 38 |
| 1.7948 | 35 | miR-21* | -1.0484 | 5 | miR-10b | 1.014 | 10 | miR-130a | -20.7495 | 39 |
| -0.77759 | 15 | miR-145 | -1.0289 | 40 | miR-222 | 1.9198 | 3 | miR-100 | -6.0971 | 40 |
| | | | 1.6244 | 58 | miR-455-5p | 1.6462 | 12 | miR-140-3p | -38.5059 | 41 |
| | | | 1.9298 | 26 | miR-193a-5p | -0.84091 | 36 | miR-210 | -10.7873 | 42 |
| | | | | | | 2.3127 | 21 | miR-181a | -30.4778 | 43 |
| | | | -1.0974 | 49 | miR-31 | -2.0358 | 25 | miR-193a-3p | 31.0975 | 44 |
| 1.8651 | 487 | miR-206 | 1.0201 | 59 | miR-487b | -0.91078 | 39 | miR-22 | -17.5516 | 45 |

**Table 48:** Using fine-needle aspiration (FNA), pleural effusion or bronchial brushing for the identification of cancer tissue of origin

| Class identified | Biopsy Site | Histological Type | Sampling Method |
|---|---|---|---|
| lung-small | Lymph Node | Neuroendocrine; Small | percutaneous FNA |
| UpperSCC | Lung | Non-small; squamous | percutaneous FNA |
| UpperSCC | Lung | Non-small; adenocarcinoma | percutaneous FNA |
| lung-small | Lung | Neuroendocrine; Small | percutaneous FNA |
| lung-adeno | Lung | Non-small; adenocarcinoma | percutaneous FNA |
| UpperSCC | Lung | Non-small; squamous | percutaneous FNA |
| lung-small | Lymph Node | Neuroendocrine; Small | transbronchial FNA |
| lung-small | Lung | Neuroendocrine; Small | transbronchial FNA |
| lung-adeno | Lung pleura | Non-small; adenocarcinoma | Pleural effusion |
| lung-adeno | Lung pleura | Non-small;adenocarcinoma | Pleural effusion |
| Lung, small | Lung | Neuroendocrine; Small | bronchial brushing |
| Lung, small | Lung | Neuroendocrine; Small | bronchial brushing |
| Lung, small | Lung | Neuroendocrine; Small | bronchial brushing |
| Lung, small | Lung | Neuroendocrine; Small | bronchial brushing |
| Lung, small | Lung | Neuroendocrine; Small | bronchial brushing |

The foregoing description of the specific embodiments so fully reveals the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of the disclosed embodiments. Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

It should be understood that the detailed description and specific examples, while indicating preferred embodiments of the invention, are given by way of illustration only, since various changes and modifications within the scope of the invention will become apparent to those skilled in the art from this detailed description.

**References:**

1. Bentwich, I. et al. Identification of hundreds of conserved and nonconserved human microRNAs. Nat Genet (2005).
2. Farh, K.K. et al. The Widespread Impact of Mammalian MicroRNAs on mRNA Repression and Evolution. Science (2005).
3. Griffiths-Jones, S., Grocock, R.J., van Dongen, S., Bateman, A. & Enright, A.J. miRBase: microRNA sequences, targets and gene nomenclature. Nucleic Acids Res 34, D140-4 (2006).
4. He, L. et al. A microRNA polycistron as a potential human oncogene. Nature 435, 828-33 (2005).
5. Baskerville, S. & Bartel, D.P. Microarray profiling of microRNAs reveals frequent coexpression with neighboring miRNAs and host genes. Rna 11, 241-7 (2005).
6. Landgraf, P. et al. A Mammalian microRNA Expression Atlas Based on Small RNA Library Sequencing. Cell 129, 1401-14 (2007).
7. Volinia, S. et al. A microRNA expression signature of human solid tumors defines cancer gene targets. Proc Natl Acad Sci USA (2006).
8. Lu, J. et al. MicroRNA expression profiles classify human cancers. Nature 435, 834-8 (2005).
9. Varadhachary, G.R., Abbruzzese, J.L. & Lenzi, R. Diagnostic strategies for unknown primary cancer. Cancer 100, 1776-85 (2004).

10. Pimiento, J.M., Teso, D., Malkan, A., Dudrick, S.J. & Palesty, J.A. Cancer of unknown primary origin: a decade of experience in a community-based hospital. Am J Surg 194, 833-7; discussion 837-8 (2007).

11. Shaw, P.H., Adams, R., Jordan, C. & Crosby, T.D. A clinical review of the investigation and management of carcinoma of unknown primary in a single cancer network. Clin Oncol (R Coll Radiol) 19, 87-95 (2007).

12. Hainsworth, J.D. & Greco, F.A. Treatment of patients with cancer of an unknown primary site. N Engl JMed 329, 257-63 (1993).

13. Blaszyk, H., Hartmann, A. & Bjornsson, J. Cancer of unknown primary: clinicopathologic correlations. Apmis 111, 1089-94 (2003).

14. Bloom, G. et al. Multi-platform, multi-site, microarray-based human tumor classification. Am J Pathol 164, 9-16 (2004).

15. Ma, X.J. et al. Molecular classification of human cancers using a 92-gene real-time quantitative polymerase chain reaction assay. Arch Pathol Lab Med 130, 465-73 (2006).

16. Talantov, D. et al. A quantitative reverse transcriptase-polymerase chain reaction assay to identify metastatic carcinoma tissue of origin. J Mol Diagn 8, 320-9 (2006).

17. Tothill, R.W. et al. An expression-based site of origin diagnostic method designed for clinical application to cancer of unknown origin. Cancer Res 65, 4031-40 (2005).

18. Shedden, K.A. et al. Accurate molecular classification of human cancers based on gene expression using a simple classifier with a pathological tree-based framework. Am J Pathol 163, 1985-95 (2003).

19. Raver-Shapira, N. et al. Transcriptional Activation of miR-34a Contributes to p53-Mediated Apoptosis. Mol Cell (2007).

20. Xiao, C. et al. MiR-150 Controls B Cell Differentiation by Targeting the Transcription Factor c-Myb. Cell 131, 146-59 (2007).

## Claims

**1.** A method of distinguishing between cancers of different origins, said method comprising:

(a) measuring the relative abundance of nucleic acid sequences comprising SEQ ID NOS: 1-390, or a sequence having at least about 90% identity thereto, in a cancer sample previously obtained from a subject; and
(b) comparing said measurement to a reference abundance of said nucleic acid sequence by using a classifier algorithm;

wherein the relative abundance of said nucleic acid sequence in said sample compared to the reference abundance allows for distinguishing between cancers of different origins; and
wherein the measurement of the relative abundance of SEQ ID NOS: 372, 233, 55, 200, 201 or a sequence having at least about 90% identity thereto in said sample compared to the reference abundance allows for distinguishing between a cancer originating from a germ-cell tumor and a cancer originating from non-germ-cell tumors; or
wherein the measurement of the relative abundance of SEQ ID NOS: 6, 30, 13 or a sequence having at least about 90% identity thereto in said sample compared to the reference abundance allows for distinguishing between a cancer originating from hepatobiliary tumors and a cancer originating from non-germ-cell non-hepatobiliary tumors; or
wherein the measurement of the relative abundance of SEQ ID NOS: 28, 29, 231, 9 or a sequence having at least about 90% identity thereto in said sample compared to the reference abundance allows for distinguishing between a cancer originating from liver tumors and a cancer originating from biliary-tract carcinomas; or
wherein the measurement of the relative abundance of SEQ ID NOS: 46, 5, 12, 30, 29, 28, 32, 13, 152, 49 or a sequence having at least about 90% identity thereto in said sample compared to the reference abundance allows for distinguishing between a cancer originating from tumors from an epithelial origin and a cancer originating from tumors from a non-epithelial origin; or
wherein the measurement of the relative abundance of SEQ ID NOS: 164, 168, 170, 16, 198, 50, 176, 186, 11, 158, 20, 155, 231, 4, 8, 46, 3, 2, 7 or a sequence having at least about 90% identity thereto in said sample compared to the reference abundance allows for distinguishing between a cancer originating from melanoma and lymphoma and a cancer originating from all other non-epithelial tumors; or
wherein the measurement of the relative abundance of SEQ ID NOS: 159, 66, 225, 187, 162, 161, 68, 232, 173, 11, 8, 174, 155, 231, 4, 182, 181, 37 or a sequence having at least about 90% identity thereto in said sample compared to the reference abundance allows for distinguishing between a cancer originating from brain tumors and a cancer originating from all non-brain, non-epithelial tumors; or
wherein the measurement of the relative abundance of SEQ ID NOS: 40, 208, 60, 153, 230, 228, 147, 34, 206, 35, 52, 25, 229, 161, 187, 179 or a sequence having at least about 90% identity thereto in said sample compared to the reference abundance allows for distinguishing between a cancer originating from astrocytoma and a cancer originating from oligodendroglioma; or
wherein the measurement of the relative abundance of SEQ ID NOS: 56, 65, 25, 175, 152, 155, 32, 49, 35, 181, or a sequence having at least about 90% identity thereto in said sample compared to the reference abundance allows for distinguishing between a cancer originating from neuroendocrine tumors and a cancer originating from all non-neuroendocrine, epithelial tumors; or
wherein the measurement of the relative abundance of SEQ ID NOS: 27, 177, 4, 32, 35 or a sequence having at least about 90% identity thereto in said sample compared to the reference abundance allows for distinguishing between a cancer originating from gastrointestinal epithelial tumors and a cancer originating from non-gastrointestinal epithelial tumors; or
wherein the measurement of the relative abundance of SEQ ID NOS: 56, 199, 14, 15, 165, 231, 36, 154, 21, 49 or a sequence having at least about 90% identity thereto in said sample compared to the reference abundance allows for distinguishing between a cancer originating from prostate tumors and a cancer originating from all other non-gastrointestinal epithelial tumors; or
wherein the measurement of the relative abundance of SEQ ID NOS: 222, 62, 29, 28, 211, 214, 227, 215, 218, 152, 216, 212, 224, 13, 194, 192, 221, 217, 205, 219, 32, 193, 223, 220, 210, 209, 213, 163, 30 or a sequence having at least about 90% identity thereto in said sample compared to the reference abundance allows for distinguishing between a cancer originating from seminoma and a cancer originating from non-seminoma testis-tumors; or
wherein the measurement of the relative abundance of SEQ ID NOS: 42, 32, 36, 178, 243, 242, 49, 240, 57, 11, 46, 17, 47, 51, 7, 8, 154, 190, 157, 196, 197, or a sequence having at least about 90% identity thereto in said sample compared to the reference abundance allows for distinguishing between a cancer originating from squamous cell carcinoma, transitional cell carcinoma and thymoma, and a cancer originating from non-gastrointestinal adenocarcinoma tumors; or

wherein the measurement of the relative abundance of SEQ ID NOS: 56, 46, 25, 152, 50, 45, 191, 181, 179, 49, 32, 42, 184, 40, 147, 236, 57, 203, 36, or a sequence having at least about 90% identity thereto in said sample compared to the reference abundance allows for distinguishing between a cancer originating from breast adenocarcinoma, and a cancer originating from squamous cell carcinoma, transitional cell carcinoma, thymomas and ovarian carcinoma; or

wherein the measurement of the relative abundance of SEQ ID NOS: 253, 32, 4, 39, 10, 46, 5, 226, 2, 195, 32, 185, 11, 168, 184, 16, 242, 12, 237, 243, 250, 49, 246, 167 or a sequence having at least about 90% identity thereto in said sample compared to the reference abundance allows for distinguishing between a cancer originating from ovarian carcinoma, and a cancer originating from squamous cell carcinoma, transitional cell carcinoma and thymomas; or

wherein the measurement of the relative abundance of SEQ ID NOS: 11, 147, 17, 157, 40, 8, 49, 9, 191, 205, 207, 195, 51, 46, 45, 52, 234, 231, 21, 169, 43, 3, 196, 154, 390, 171, 255, 197, 190, 189, 39, 7, , 47, 32, 36, 4, 178, 37, 181, 25, 183, 182, 35, 240, 57, 242, 204, 236, 176, 158, 148, 206, 50, 20, 34, 186, 239, 251, 244, 24, 188, 172, 238 or a sequence having at least about 90% identity thereto in said sample compared to the reference abundance allows for distinguishing between a cancer originating from thyroid carcinoma, and a cancer originating from breast adenocarcinoma, lung large cell carcinoma, lung adenocarcinoma and ovarian carcinoma; or

wherein the measurement of the relative abundance of SEQ ID NOS: 249, 180, 65, 235, 241, 248, 254, 247, 160, 243, 245, 252, 17, 49, 166, 225, 168, 34 or a sequence having at least about 90% identity thereto in said sample compared to the reference abundance allows for distinguishing between a cancer originating from follicular thyroid carcinoma and a cancer originating from papillary thyroid carcinoma; or

wherein the measurement of the relative abundance of SEQ ID NOS: 32, 56, 50, 45, 25, 253, 152, 9, 46, 191, 178, 49, 40, 10, 147, 4, 36, 228, 236, 230, 189, 240, 67, 202, 17 or a sequence having at least about 90% identity thereto in said sample compared to the reference abundance allows for distinguishing between a cancer originating from breast adenocarcinoma and a cancer originating from lung adenocarcinoma and ovarian carcinoma; or

wherein the measurement of the relative abundance of SEQ ID NOS: 56, 11, 168, 16, 237, 21, 52, 12, 154, 279, 9, 39, 47, 23, 50, 167, 383, 34, 35, 388, 5, 359, 245, 254, 10, 240, 236, 202, 4, 25, 203, 231, 20, 158, 186, 258, 244, 172, 2, 235, 256, 28, 277, 296, 374, 153, 181 or a sequence having at least about 90% identity thereto in said sample compared to the reference abundance allows for distinguishing between a cancer originating from lung adenocarcinoma and a cancer originating from ovarian carcinoma; or

wherein the measurement of the relative abundance of SEQ ID NOS: 161, 164, 22, 53, 285, 3, 152, 191, 154, 21, 206, 174, 19, 45, 171, 179, 8, 296, 284, 18, 51, 258, 49, 184, 35, 34, 37, 42, 228, 15, 14, 242, 230, 253, 36, 182, 293, 292, 4, 294, 297, 354, 377, 189, 30, 386, 249, 5, 274 or a sequence having at least about 90% identity thereto in said sample compared to the reference abundance allows for distinguishing between a cancer originating from thymic carcinoma and a cancer originating from transitional cell carcinoma and squamous cell carcinoma; or

wherein the measurement of the relative abundance of SEQ ID NOS: 69, 28, 280, 13, 191, 152, 29, 175, 30, 204, 4, 24, 5, 329, 273, 170, 184, 26, 231, 368, 37, 16, 169, 155, 35, 40, 17 or a sequence having at least about 90% identity thereto in said sample compared to the reference abundance allows for distinguishing between a cancer originating from transitional cell carcinoma and a cancer originating from squamous cell carcinoma; or

wherein the measurement of the relative abundance of SEQ ID NOS: 164, 5, 231, 54, 1, 242, 372, 249, 167, 254, 354, 381, 380, 245, 358, 364, 240, 11, 378 or a sequence having at least about 90% identity thereto in said sample compared to the reference abundance allows for distinguishing between squamous cell carcinoma cancers originating from the uterine cervix, and squamous cell carcinoma cancers originating from anus and skin, lung, head & neck and esophagus; or

wherein the measurement of the relative abundance of SEQ ID NOS: 305, 184, 41, 183, 49, 382, 235, 291, 181, 5, 296, 289, 206, 338, 334, 25, 11, 19, 198, 23 or a sequence having at least about 90% identity thereto in said sample compared to the reference abundance allows for distinguishing between squamous cell carcinoma cancers originating from anus and skin, and between squamous cell carcinoma cancers originating from lung, head & neck and esophagus; or

wherein the measurement of the relative abundance of SEQ ID NOS: 4, 11, 46, 8, 274, 169, 36, 47, 363, 231, 303, 349, 10, 7, 3, 16, 164, 170, 168, 198, 50, 245, 365, 45, 382, 259, 296, 364, 314, 12 or a sequence having at least about 90% identity thereto in said sample compared to the reference abundance allows for distinguishing between a cancer originating from melanoma and a cancer originating from lymphoma; or

wherein the measurement of the relative abundance of SEQ ID NOS: 11, 191, 48, 35, 228 or a sequence having at least about 90% identity thereto in said sample compared to the reference abundance allows for distinguishing between a cancer originating from B-cell lymphoma and a cancer originating from T-cell lymphoma; or

wherein the measurement of the relative abundance of SEQ ID NOS: 158, 20, 176, 186, 148, 36, 51, 172, 260, 265, 67, 188, 277, 284, 302, 68, 168, 242, 204, 162, 177, 27, 65, 263, 155, 191, 190, 45, 59, 43, 56, 266, 14, 15, 8, 7, 39, 189, 249, 231, 293, 2 or a sequence having at least about 90% identity thereto in said sample compared to the reference abundance allows for distinguishing between a cancer originating from lung small cell carcinoma and a cancer originating from lung carcinoid, medullary thyroid carcinoma, gastrointestinal tract carcinoid and pancreatic islet cell tumor; or
wherein the measurement of the relative abundance of SEQ ID NOS: 159, 40, 147, 11, 311, 4, 8, 231, 301, 297, 68, 67, 265, 36 or a sequence having at least about 90% identity thereto in said sample compared to the reference abundance allows for distinguishing between a cancer originating from medullary thyroid carcinoma and a cancer originating from other neuroendocrine tumors selected from lung carcinoid, gastrointestinal tract carcinoid and pancreatic islet cell tumor; or
wherein the measurement of the relative abundance of SEQ ID NOS: 331, 162, 59, 326, 306, 350, 317, 155, 325, 318, 339, 264, 332, 262, 336, 324, 322, 330, 321, 263, 309, 53, 320, 275, 352, 312, 355, 367, 269, 64, 308, 175, 190, 54, 302, 152, 301, 266, 47, 313, 359, 65, 307, 191, 242, 4, 147, 40, 372, 168, 16, 182, 167, 356, 148, 382, 37, 364, 35 or a sequence having at least about 90% identity thereto in said sample compared to the reference abundance allows for distinguishing between a cancer originating from lung carcinoid tumors, and a cancer originating from gastrointestinal neuroendocrine tumors selected from gastrointestinal tract carcinoid and pancreatic islet cell tumor; or
wherein the measurement of the relative abundance of SEQ ID NOS: 263, 288, 18, 286, 162, 225, 287, 206, 205, 296, 258, 313, 377, 373, 256, 153, 259, 265, 303, 268, 267, 165, 15, 272, 14, 202, 236, 203, 4, 168, 310, 298, 27, 29, 34, 228, 3, 349, 35, 26 or a sequence having at least about 90% identity thereto in said sample compared to the reference abundance allows for distinguishing between a cancer originating from pancreatic islet cell tumors and a Gastrointestinal neuroendocrine carcinoid cancer originating from small intestine and duodenum; appendicitis, stomach and pancreas; or
wherein the measurement of the relative abundance of SEQ ID NOS: 36, 267, 268, 165, 15, 14, 356, 167, 372, 272, 370, 42, 41, 146 or a sequence having at least about 90% identity thereto in said sample compared to the reference abundance allows for distinguishing between adenocarcinoma tumors of the gastrointestinal system originating from:

a) gastric and esophageal adenocarcinoma, and
b) cholangiocarcinoma or adenocarcinoma of the extrahepatic biliary tract, pancreatic adenocarcinoma and colorectal adenocarcinoma; or

wherein the measurement of the relative abundance of SEQ ID NOS: 42, 184, 67, 158, 20, 186, 284, 389, 203, 240, 236, 146, 204, 43, 176, 202, 49, 46, 38, 363 or a sequence having at least about 90% identity thereto in said sample compared to the reference abundance allows for distinguishing between a cancer originating from colorectal adenocarcinoma and a cancer originating from adenocarcinoma of biliary tract or pancreas; or
wherein the measurement of the relative abundance of SEQ ID NOS: 49, 11, 13, 373, 154, 5, 30, 45, 178, 147, 274, 16, 40, 21, 43, 253, 245, 256, 12, 374, 379, 180, 153, 51, 52, 1, 295, 257, 385, 293, 294 or a sequence having at least about 90% identity thereto in said compared to the reference abundance sample allows for distinguishing between a cancer originating from pancreatic adenocarcinoma, and a cancer originating from cholangiocarcinoma or adenocarcinoma of the extrahepatic biliary tract; or
wherein the measurement of the relative abundance of SEQ ID NOS: 29, 28, 30, 46, 49, 195, 152, 175, 47, 4, 387, 196, 177, 375, 27, 304, 40, 191, 147, 35, 16, 34, 5, 155, 181, 312, 183, 182, 320, 59, 38, 324, 323, 37, 322, 325, 19, 42, 334, 265, 22 or a sequence having at least about 90% identity thereto in said sample compared to the reference abundance allows for distinguishing between a cancer originating from:

c) renal cell tumors selected from chromophobe renal cell carcinoma, clear cell renal cell carcinoma and papillary renal cell carcinoma, and
d) sarcomas, adrenal tumors and pleural mesothelioma; or

wherein the measurement of the relative abundance of SEQ ID NOS: 65, 56, 11, 162, 59, 331, 350, 155, 335, 159, 336, 332, 263, 306, 339, 337, 275, 301, 276, 330, 317, 309, 45, 318, 324, 352, 191, 262, 269, 313, 19, 367, 326, 325, 322, 327, 190, 261, 321, 360, 353, 312, 371, 5, 328, 205, 183, 38, 181, 37, 40, 182, 147, 17, 42, 382, 34, 18, 3 or a sequence having at least about 90% identity thereto in said sample compared to the reference abundance allows for distinguishing between a cancer originating from pheochromocytoma, and a cancer originating from all sarcoma, adrenal carcinoma and mesothelioma tumors; or
wherein the measurement of the relative abundance of SEQ ID NOS: 61, 333, 31, 347, 346, 344, 345, 387, 334, 351, 324, 326, 269, 155, 320, 322, 59, 318, 325, 245, 254, 331, 275, 180, 355, 370, 323, 312, 178, 249, 183, 181, 38, 182, 37, 3, 25 or a sequence having at least about 90% identity thereto in said sample compared to the reference abundance allows for distinguishing between a cancer originating from adrenal carcinoma and a cancer originating from mesothelioma and sarcoma tumors; or
wherein the measurement of the relative abundance of SEQ ID NOS: 165, 14, 15, 333, 272, 270, 45, 301, 191, 46, 195, 266, 190, 19, 334, 155, 25, 147, 40, 34 or a sequence having at least about 90% identity thereto in said sample compared to the reference abundance allows for distinguishing between a cancer originating from a gastrointestinal stomal tumor and a cancer originating from mesothelioma and sarcoma tumors; or
wherein the measurement of the relative abundance of SEQ ID NOS: 13, 30, 361, 280, 362, 147, 40, 291, 387, 290, 299, 152, 178, 303, 242, 49, 11, 35, 34, 36, 206, 16, 170, 177, 17 or a sequence having at least about 90% identity thereto in said sample compared to the reference abundance allows for distinguishing between a cancer originating from a chromophobe renal cell carcinoma tumor and a cancer originating from clear cell and papillary renal cell carcinoma tumors; or
wherein the measurement of the relative abundance of SEQ ID NOS: 344, 382, 9, 338, 29, 49, 28, 195, 46, 4, 11, 254 or a sequence having at least about 90% identity thereto in said sample compared to the reference abundance allows for distinguishing between a renal carcinoma cancer originating from a clear cell tumor and a cancer originating from a papillary tumor; or
wherein the measurement of the relative abundance of SEQ ID NOS: 49, 35, 17, 34, 25, 36, 168, 170, 26, 4, 190, 46, 10, 240, 43, 39, 385, 63, 202, 181, 37, 5, 183, 182, 38, 206, 296, 1 or a sequence having at least about 90% identity thereto in said sample compared to the reference abundance allows for distinguishing between a cancer originating from pleural mesothelioma and a cancer originating from sarcoma tumors; or
wherein the measurement of the relative abundance of SEQ ID NOS: 152, 29, 159, 28, 339, 275, 352, 19, 320, 155, 262, 38, 37, 182, 331, 317, 323, 355, 3, 282, 312, 181, 269, 318, 59, 266, 322, 8, 324, 10, 40, 147, 169, 205, 34, 168, 14, 15, 12, 46, 255, 39, 23, 190, 236, 386, 379, 202 or a sequence having at least about 90% identity thereto in said sample compared to the reference abundance allows for distinguishing between a cancer originating from a synovial sarcoma and a cancer originating from other sarcoma tumors; or
wherein the measurement of the relative abundance of SEQ ID NOS: 12, 271, 206, 333, 11, 58, 36, 18, 178, 293, 189, 382, 381, 240, 249, 5, 377, 235, 17, 20, 385, 384, 46, 283 or a sequence having at least about 90% identity thereto in said sample compared to the reference abundance allows for distinguishing between a cancer originating from chondrosarcoma and a cancer originating from other non-synovial sarcoma tumors; or
wherein measurement of the relative abundance of SEQ ID NOS: 295, 205, 25, 26, 231, 183, 42, 254, 168, 64, 14, 178, 15, 39, 36, 154, 265, 174, 384, 67 or a sequence having at least about 90% identity thereto in said sample compared to the reference abundance allows for distinguishing between a cancer originating from liposarcoma and a cancer originating from other non chondrosarcoma and non synovial sarcoma tumors; or
wherein the measurement of the relative abundance of SEQ ID NOS: 22, 154, 21, 174, 205, 158, 186, 148, 20, 59, 8, 183, 231 or a sequence having at least about 90% identity thereto in said sample compared to the reference abundance allows for distinguishing between a cancer originating from:

e) Ewing sarcoma and osteosarcoma, and
f) rhabdomyosarcoma, malignant fibrous histiocytoma and fibrosarcoma; or

wherein the measurement of the relative abundance of SEQ ID NOS: 155, 179, 43, 208, 278, 17, 385, 174, 5, 52, 257, 366, 48, 49, 12, 25, 169, 34, 35, 23, 384, 189, 377, 265, 294, 293, 292 or a sequence having at least about 90% identity thereto in said sample compared to the reference abundance allows for distinguishing between a cancer originating from Ewing sarcoma and a cancer originating from osteosarcoma; or
wherein the measurement of the relative abundance of SEQ ID NOS: 33, 268, 267, 333, 276, 319, 306, 320, 334, 323, 300, 281, 59, 339, 316, 176, 348, 352, 349, 67, 357, 315, 343, 342, 355, 340, 344, 10, 341, 331, 20, 277, 318, 158, 265, 284, 36, 183, 40, 63, 147, 43, 289, 52, 190, 4, 5, 39, 169, 208 or a sequence having at least about 90% identity thereto in said sample compared to the reference abundance allows for distinguishing between a cancer originating from rhabdomyosarcoma and a cancer originating from malignant fibrous histiocytoma and fibrosarcoma.

**2.** The method of claim 1, wherein said cancer sample is selected from a bodily fluid, a tissue sample, a biopsy sample, a needle biopsy sample, a fine needle biopsy (FNA) sample, a surgically removed sample, and a sample obtained by tissue-sampling procedures such as endoscopy, bronchoscopy, or laparoscopic methods; preferably wherein said tissue is a fresh, frozen, fixed, wax-embedded or formalin-fixed paraffin-embedded (FFPE) tissue.

**3.** The method of any of claims 1 or 2, wherein the nucleic acid sequence abundance is measured by a method selected from nucleic acid hybridization and nucleic acid amplification.

**4.** The method of claim 3, wherein the nucleic acid hybridization is performed using a solid-phase nucleic acid biochip array or *in situ* hybridization.

**5.** The method of claim 3, wherein said nucleic acid amplification method is real-time PCR; preferably wherein said real-time PCR comprises forward and reverse primers.

**6.** The method of claim 5, wherein said real-time PCR method further comprises a probe, said probe comprising a sequence complementary to SEQ ID NOS: 1; 2 or 156; 3-7; 9-12; 14-21; 23-27; 29-40; 42; 43; 44 or 191; 45-47; 49-51; 53-56; 57 or 202; 58; 59; 60 or 208; 61; 62 or 211; 64-69; and 146-148, fragments thereof, and sequences having at least about 90% identity thereto.

**7.** The method of claim 1, wherein said classifier algorithm is a decision tree classifier, a K-nearest neighbor classifier (KNN), a logistic regression classifier, a nearest neighbor classifier, a neural network classifier, a Gaussian mixture model (GMM), a Support Vector Machine (SVM) classifier, a nearest centroid classifier, a linear regression classifier or a random forest classifier.

**Patentansprüche**

**1.** Eine Methode zur Unterscheidung von verschiedenen Krebsarten, wobei die Methode folgendes umfasst:

(a) Messung der relativen Häufigkeit von Nukleotidsequenzen, die aus Sequenz ID Nummern 1-390 bestehen, bzw. zu mindestens 90% mit diesen identisch sind, in einer zuvor von einem Patienten entnommene Karzinom-Gewebeprobe; und
(b) die genannte Messung mit einer Referenzhäufigkeit der genannten Nukleinsäuresequenz verglichen wird, indem ein Algorithmus-Klassifikationsverfahren angewendet wird;

wobei die relative Häufigkeit der genannten Nukleinsäuresequenz in der genannten Probe im Vergleich zur Referenzhäufigkeit es ermöglicht, verschiedene Krebsarten voneinander zu unterscheiden; und
wobei die Messung der relativen Häufigkeit der Sequenz ID Nummern 372, 233, 55, 200 und 201 bzw. einer Sequenz, die mindestens zu etwa 90% die entsprechende Identität aufweist, im Vergleich zur Referenzhäufigkeit es ermöglicht, zwischen einer aus Keimzelltumoren entstandenen Krebserkrankung und einer aus andersartigen Tumoren entstandenen Krebserkrankung zu unterscheiden; oder
wobei die Messung der relativen Häufigkeit der Sequenz ID Nummern 6, 30, 13 bzw. einer Sequenz, die mindestens zu etwa 90% die entsprechende Identität aufweist, im Vergleich zur Referenzhäufigkeit es ermöglicht, zwischen einer aus hepatobiliären Tumoren entstandenen Krebserkrankung und einer nicht aus Keimzellen hervorgegangenen, und nicht hepatobiliären Tumoren entstandenen Krebserkrankung zu unterscheiden; oder
wobei die Messung der relativen Häufigkeit der Sequenz ID Nummern 28, 29, 231, 9 bzw. einer Sequenz, die mindestens zu etwa 90% die entsprechende Identität aufweist, im Vergleich zur Referenzhäufigkeit es ermöglicht, zwischen einer aus Lebertumoren entstandenen Krebserkrankung und einer aus Gallengangskarzinomen entstandenen Krebserkrankung zu unterscheiden; oder
wobei die Messung der relativen Häufigkeit der Sequenz ID Nummern 46, 5, 12, 30, 29, 28, 32, 13, 152, 49 bzw. einer Sequenz, die mindestens zu etwa 90% die entsprechende Identität aufweist, im Vergleich zur Referenzhäufigkeit es ermöglicht, zwischen einer aus Tumoren epithelialen Ursprungs entstandenen Krebserkrankung und einer aus Tumoren anderen (nicht epithelialen) Ursprungs entstandenen Krebserkrankung zu unterscheiden; oder
wobei die Messung der relativen Häufigkeit der Sequenz ID Nummern 164, 168, 170, 16, 198, 50, 176, 186, 11, 158, 20, 155, 231, 4, 8, 46, 3, 2, 7 bzw. einer Sequenz, die mindestens zu etwa 90% die entsprechende Identität aufweist, im Vergleich zur Referenzhäufigkeit es ermöglicht, zwischen einer aus einem Melanom oder einem Lymphom entstandenen Krebserkrankung und einer aus allen anderen Tumoren, die nicht epithelialen Ursprungs sind, entstandenen Krebserkrankung zu unterscheiden; oder
wobei die Messung der relativen Häufigkeit der Sequenz ID Nummern 159, 66, 225, 187, 162, 161, 68, 232, 173, 11, 8, 174, 155, 231, 4, 182, 181, 37 bzw. einer Sequenz, die mindestens zu etwa 90% die entsprechende Identität aufweist, im Vergleich zur Referenzhäufigkeit es ermöglicht, zwischen einer aus einem Gehirntumor entstandenen Krebserkrankung und einer aus allen anderen Tumoren, die keine Gehirntumore sind und die nicht epithelialen Ursprungs sind, entstandenen Krebserkrankung zu unterscheiden; oder
wobei die Messung der relativen Häufigkeit der Sequenz ID Nummern 40, 208, 60, 153, 230, 228, 147, 34, 206, 35, 52, 25, 229, 161, 187, 179 bzw. einer Sequenz, die mindestens zu etwa 90% die entsprechende Identität aufweist, im Vergleich zur Referenzhäufigkeit es ermöglicht, zwischen einer aus einem Astrozytom entstandenen Krebserkrankung und einer aus einem Oligodendrogliom entstandenen Krebserkrankung zu unterscheiden; oder
wobei die Messung der relativen Häufigkeit der Sequenz ID Nummern 56, 65, 25, 175, 152, 155, 32, 49, 35, 181 bzw. einer Sequenz, die mindestens zu etwa 90% die entsprechende Identität aufweist, im Vergleich zur Referenzhäufigkeit es ermöglicht, zwischen einer aus neuroendokrinen Tumoren entstandenen Krebserkrankung und einer aus nicht neuroendokrinen, epithelialen Tumoren entstandenen Krebserkrankung zu unterscheiden; oder
wobei die Messung der relativen Häufigkeit der Sequenz ID Nummern 27, 177, 4, 32, 35 bzw. einer Sequenz, die mindestens zu etwa 90% die entsprechende Identität aufweist, im Vergleich zur Referenzhäufigkeit es ermöglicht, zwischen einer aus gastrointestinalen epithelialen Tumoren entstandenen Krebserkrankung und einer aus nicht gastronintestinalen epithelialen Tumoren entstandenen Krebserkrankung zu unterscheiden; oder
wobei die Messung der relativen Häufigkeit der Sequenz ID Nummern 56, 199, 14, 15, 165, 231, 36, 154, 21, 49 bzw. einer Sequenz, die mindestens zu etwa 90% die entsprechende Identität aufweist, im Vergleich zur Referenzhäufigkeit es ermöglicht, zwischen einer aus Prostata-Tumoren entstandenen Krebserkrankung und einer aus Tumoren nicht gastrointestinalen, epithelialen Ursprungs entstandenen Krebserkrankung zu unterscheiden; oder
wobei die Messung der relativen Häufigkeit der Sequenz ID Nummern 222, 62, 29, 28, 211, 214, 227, 215, 218, 152, 216, 212, 224, 13, 194, 192, 221, 217, 205, 219, 32, 193, 223, 220, 210, 209, 213, 163, 30 bzw. einer Sequenz, die mindestens zu etwa 90% die entsprechende Identität aufweist, im Vergleich zur Referenzhäufigkeit es ermöglicht, zwischen einer aus einem Seminom entstandenen Krebserkrankung und einer aus andersartigen Hoden-Tumoren entstandenen Krebserkrankung zu unterscheiden; oder
wobei die Messung der relativen Häufigkeit der Sequenz ID Nummern 42, 32, 36, 178, 243, 242, 49, 240, 57, 11, 46, 17, 47, 51, 7, 8, 154, 190, 157, 196, 197 bzw. einer Sequenz, die mindestens zu etwa 90% die entsprechende Identität aufweist, im Vergleich zur Referenzhäufigkeit es ermöglicht, zwischen einer aus Plattenepithel-Tumoren, Urothelkarzinomen oder Thymomen entstandenen Krebserkrankung und einer aus nicht gastrointestinalen Adenokarzinom-Tumoren entstandenen Krebserkrankung zu unterscheiden; oder
wobei die Messung der relativen Häufigkeit der Sequenz ID Nummern 56, 46, 25, 152, 50, 45, 191, 181, 179, 49, 32, 42, 184, 40, 147, 236, 57, 203, 36 bzw. einer Sequenz, die mindestens zu etwa 90% die entsprechende Identität aufweist, im Vergleich zur Referenzhäufigkeit es ermöglicht, zwischen einer aus einem Brustadenokarzinom entstandenen Krebserkrankung und einer aus einem Plattenepithelkarzinom, einem Urothelkarzinom, einem Thymom oder einem Ovarialkarzinom entstandenen Krebserkrankung zu unterscheiden; oder
wobei die Messung der relativen Häufigkeit der Sequenz ID Nummern 253, 32, 4, 39, 10, 46, 5, 226, 2, 195, 32, 185, 11, 168, 184, 16, 242, 12, 237, 243, 250, 49, 246, 167 bzw. einer Sequenz, die mindestens zu etwa 90% die entsprechende Identität aufweist, im Vergleich zur Referenzhäufigkeit es ermöglicht, zwischen einer aus einem Ovarialkarzinom entstandenen Krebserkrankung und einer aus einem Plattenepithelkarzinom, einem Urothelkarzinom oder einem Thymom entstandenen Krebserkrankung zu unterscheiden; oder
wobei die Messung der relativen Häufigkeit der Sequenz ID Nummern 11, 147, 17, 157, 40, 8, 49, 9, 191, 205, 207, 195, 51, 46, 45, 52, 234, 231, 21, 169, 43, 3, 196, 154, 390, 171, 255, 197, 190, 189, 39, 7, 47, 32, 36, 4, 178, 37, 181, 25, 183, 182, 35, 240, 57, 242, 204, 236, 176, 158, 148, 206, 50, 20, 34, 186, 239, 251, 244, 24, 188, 172, 238 bzw. einer Sequenz, die mindestens zu etwa 90% die entsprechende Identität aufweist, im Vergleich zur Referenzhäufigkeit es ermöglicht, zwischen einer aus einem Schilddrüsenkarzinom entstandenen Krebserkrankung und einer aus einem Brustadenokarzinom, einem großzelligen Lungenkarzinom, einem Adenokarzinom der Lunge oder einem Ovarialkarzinom entstandenen Krebserkrankung zu unterscheiden; oder
wobei die Messung der relativen Häufigkeit der Sequenz ID Nummern 249, 180, 65, 235, 241, 248, 254, 247, 160, 243, 245, 252, 17, 49, 166, 225, 168, 34 bzw. einer Sequenz, die mindestens zu etwa 90% die entsprechende Identität aufweist, im Vergleich zur Referenzhäufigkeit es ermöglicht, zwischen einer aus einem follikulären Schilddrüsenkarzinom entstandenen Krebserkrankung und einer aus einem papillären Schilddrüsenkarzinom entstandenen Krebserkrankung zu unterscheiden; oder
wobei die Messung der relativen Häufigkeit der Sequenz ID Nummern 32, 56, 50, 45, 25, 253, 152, 9, 46, 191, 178, 49, 40, 10, 147, 4, 36, 228, 236, 230, 189, 240, 67, 202, 17 bzw. einer Sequenz, die mindestens zu etwa 90% die entsprechende Identität aufweist, im Vergleich zur Referenzhäufigkeit es ermöglicht, zwischen einer aus einem Brustadenokarzinom entstandenen Krebserkrankung und einer aus einem Adenokarzinom der Lunge und aus einem Ovarialkarzinom entstandenen Krebserkrankung zu unterscheiden; oder
wobei die Messung der relativen Häufigkeit der Sequenz ID Nummern 56, 11, 168, 16, 237, 21, 52, 12, 154, 279, 9, 39, 47, 23, 50, 167, 383, 34, 35, 388, 5, 359, 245, 254, 10, 240, 236, 202, 4, 25, 203, 231, 20, 158, 186, 258, 244, 172, 2, 235, 256, 28, 277, 296, 374, 153, 181 bzw. einer Sequenz, die mindestens zu etwa 90% die entsprechende Identität aufweist, im Vergleich zur Referenzhäufigkeit es ermöglicht, zwischen einer aus einem Adenokarzinom der Lunge entstandenen Krebserkrankung und einem aus einem Ovarialkarzinom entstandenen Krebserkrankung zu unterscheiden; oder
wobei die Messung der relativen Häufigkeit der Sequenz ID Nummern 161, 164, 22, 53, 285, 3, 152, 191, 154, 21, 206, 174, 19, 45, 171, 179, 8, 296, 284, 18, 51, 258, 49, 184, 35, 34, 37, 42, 228, 15, 14, 242, 230, 253, 36, 182, 293, 292, 4, 294, 297, 354, 377, 189, 30, 386, 249, 5, 274 bzw. einer Sequenz, die mindestens zu etwa 90% die entsprechende Identität aufweist, im Vergleich zur Referenzhäufigkeit es ermöglicht, zwischen einer aus einem Thymuskarzinom entstandenen Krebserkrankung und einer aus einem Urothelkarzinom oder einem Plattenepithelkarzinom entstandenen Krebserkrankung zu unterscheiden; oder

wobei die Messung der relativen Häufigkeit der Sequenz ID Nummern 69, 28, 280, 13, 191, 152, 29, 175, 30, 204, 4, 24, 5, 329, 273, 170, 184, 26, 231, 368, 37, 16, 169, 155, 35, 40, 17 bzw. einer Sequenz, die mindestens zu etwa 90% die entsprechende Identität aufweist, im Vergleich zur Referenzhäufigkeit es ermöglicht, zwischen einer aus einem Urothelkarzinom entstandenen Krebserkrankung und einer aus einem Plattenepithelkarzinom entstandenen Krebserkrankung zu unterscheiden; oder

wobei die Messung der relativen Häufigkeitt der Sequenz ID Nummern 164, 5, 231, 54, 1, 242, 372, 249, 167, 254, 354, 381, 380, 245, 358, 364, 240, 11, 378 bzw. einer Sequenz, die mindestens zu etwa 90% die entsprechende Identität aufweist, im Vergleich zur Referenzhäufigkeit es ermöglicht, zwischen einer aus einem im Gebärmutterhals entstandenen Plattenepithelkarzinom und einem vom After, der Haut, der Lunge, dem Kopf und Hals oder vom Ösophagus ausgehenden Plattenepithelkarzinom zu unterscheiden; oder

wobei die Messung der relativen Häufigkeit der Sequenz ID Nummern 305, 184, 41, 183, 49, 382, 235, 291, 181, 5, 296, 289, 206, 338, 334, 25, 11, 19, 198, 23 bzw. einer Sequenz, die mindestens zu etwa 90% die entsprechende Identität aufweist, im Vergleich zur Referenzhäufigkeit es ermöglicht, zwischen vom After und der Haut ausgehenden Plattenepithelkarzinomen und von der Lunge, von Hals und Kopf oder vom Ösophagus ausgehenden Plattenepithelkarzinomen zu unterscheiden; oder

wobei die Messung der relativen Häufigkeit der Sequenz ID Nummern 4, 11, 46, 8, 274, 169, 36, 47, 363, 231, 303, 349, 10, 7, 3, 16, 164, 170, 168, 198, 50, 245, 365, 45, 382, 259, 296, 364, 314, 12 bzw. einer Sequenz, die mindestens zu etwa 90% die entsprechende Identität aufweist, im Vergleich zur Referenzhäufigkeit es ermöglicht, zwischen einer aus einem Melanom entstandenen Krebserkrankung und einer aus einem Lymphom entstandenen Krebserkrankung zu unterscheiden; oder

wobei die Messung der relativen Häufigkeit der Sequenz ID Nummern 11, 191, 48, 35, 228 bzw. einer Sequenz, die mindestens zu etwa 90% die entsprechende Identität aufweist, im Vergleich zur Referenzhäufigkeit es ermöglicht, zwischen einer aus einem B-Zellen-Lymphom entstandenen Krebserkrankung und einer aus einem T-Zellen-Lymphom entstandenen Krebserkrankung zu unterscheiden; oder

wobei die Messung der relativen Häufigkeit der Sequenz ID Nummern 158, 20, 176, 186, 148, 36, 51, 172, 260, 265, 67, 188, 277, 284, 302, 68, 168, 242, 204, 162, 177, 27, 65, 263, 155, 191, 190, 45, 59, 43, 56, 266, 14, 15, 8, 7, 39, 189, 249, 231, 293, 2 bzw. einer Sequenz, die mindestens zu etwa 90% die entsprechende Identität aufweist, im Vergleich zur Referenzhäufigkeit es ermöglicht, zwischen einer aus einem kleinzelligen Lungenkarzinom entstandenen Krebserkrankung und einer aus einem karzinoiden Lungentumor, einem medullären Schilddrüsenkarzinom, einem karzinoiden Tumor des Gastrointestinaltrakts oder einem Inselzellen-Tumor der Pankreas zu unterscheiden; oder

wobei die Messung der relativen Häufigkeit der Sequenz ID Nummern 159, 40, 147, 11, 311, 4, 8, 231, 301, 297, 68, 67, 265, 36 bzw. einer Sequenz, die mindestens zu etwa 90% die entsprechende Identität aufweist, im Vergleich zur Referenzhäufigkeit es ermöglicht, zwischen einer aus einem medullären Schilddrüsenkarzinom entstandenen Krebserkrankung und einer aus andersartigen neuroendokrinen Tumoren, selektiert von karzinoiden Lungentumoren, Karzinoiden des Gastrointestinaltrakts und Pankreas-Inselzelltumoren entstandenen Krebserkrankung zu unterscheiden; oder

wobei die Messung der relativen Häufigkeit der Sequenz ID Nummern 331, 162, 59, 326, 306, 350, 317, 155, 325, 318, 339, 264, 332, 262, 336, 324, 322, 330, 321, 263, 309, 53, 320, 275, 352, 312, 355, 367, 269, 64, 308, 175, 190, 54, 302, 152, 301, 266, 47, 313, 359, 65, 307, 191, 242, 4, 147, 40, 372, 168, 16, 182, 167, 356, 148, 382, 37, 364, 35 bzw. einer Sequenz, die mindestens zu etwa 90% die entsprechende Identität aufweist, im Vergleich zur Referenzhäufigkeit es ermöglicht, zwischen einer aus Lungenkarzinoiden entstandenen Krebserkrankung und einer von gastrointestinalen neuroendokrinen Tumoren, die von einem Karzinoid des gastrointestinalen Trakts und einem Pankreas-Inselzellentumor selektiert wurden, entstandenen Krebserkrankung zu unterscheiden; oder

wobei die Messung der relativen Häufigkeit der Sequenz ID Nummern 263, 288, 18, 286, 162, 225, 287, 206, 205, 296, 258, 313, 377, 373, 256, 153, 259, 265, 303, 268, 267, 165, 15, 272, 14, 202, 236, 203, 4, 168, 310, 298, 27, 29, 34, 228, 3, 349, 35, 26 bzw. einer Sequenz, die mindestens zu etwa 90% die entsprechende Identität aufweist, im Vergleich zur Referenzhäufigkeit es ermöglicht, zwischen einer aus Pankreas-Inselzell-Tumoren entstandenen Krebserkrankung und einem von Dünndarm und Zwölffingerdarm, einer Appendizitis, und vom Magen und von der Pankreas ausgegangenen gastrointestinalen, neuroendokrinen Karzinoid zu unterscheiden; oder

wobei die Messung der relativen Häufigkeit der Sequenz ID Nummern 36, 267, 268, 165, 15, 14, 356, 167, 372, 272, 370, 42, 41, 146 bzw. einer Sequenz, die mindestens zu etwa 90% die entsprechende Identität aufweist, im Vergleich zur Referenzhäufigkeit es ermöglicht, zwischen Adenokarzinom-Tumoren des Magen-Darm-Systems mit den folgenden Ursprüngen zu unterscheiden:

(a) Magen-und Ösophagus-Adenokarzinome und

(b) Gallengangskarzinom oder Adenokarzinom des extrahepatischen Gallengangs, Pankreaskarzinom und kolorektales Adenokarzinom; oder

wobei die Messung der relativen Häufigkeit der Sequenz ID Nummern 42, 184, 67, 158, 20, 186, 284, 389, 203, 240, 236, 146, 204, 43, 176, 202, 49, 46, 38, 363 bzw. einer Sequenz, die mindestens zu etwa 90% die entsprechende Identität aufweist, im Vergleich zur Referenzhäufigkeit es ermöglicht, zwischen einer aus einem kolorektalen Adenokarzinom entstandenen Krebserkrankung und einer aus einem Adenokarzinom der Gallenwege entstandenen Krebserkrankung zu unterscheiden; oder
wobei die Messung der relativen Häufigkeit der Sequenz ID Nummern 49, 11, 13, 373, 154, 5, 30, 45, 178, 147, 274, 16, 40, 21, 43, 253, 245, 256, 12, 374, 379, 180, 153, 51, 52, 1, 295, 257, 385, 293, 294 bzw. einer Sequenz, die mindestens zu etwa 90% die entsprechende Identität aufweist, im Vergleich zur Referenzhäufigkeit es ermöglicht, zwischen einer aus einem Pankreaskarzinom entstandenen Krebserkrankung und einer aus einem Gallengangskarzinom oder aus einem Adenokarzinom der extrahepatischen Gallenwege entstandenen Krebserkrankung zu unterscheiden; oder
wobei die Messung der relativen Häufigkeit der Sequenz ID Nummern 29, 28, 30, 46, 49, 195, 152, 175, 47, 4, 387, 196, 177, 375, 27, 304, 40, 191, 147, 35, 16, 34, 5, 155, 181, 312, 183, 182, 320, 59, 38, 324, 323, 37, 322, 325, 19, 42, 334, 265, 22 bzw. einer Sequenz, die mindestens zu etwa 90% die entsprechende Identität aufweist, im Vergleich zur Referenzhäufigkeit es ermöglicht, zwischen Krebserkrankungen mit den folgenden Ursprüngen zu unterscheiden:

(c) von chromophoben Nierenzellkarzinom, klarzelligem Nierenzellkarzinom und papillärem Nierenzellkarzinom selektierten Nierenzelltumoren, und

(d) Sarkomen, Nebennieren-Tumore und Pleuramesotheliome; oder

wobei die Messung der relativen Häufigkeit der Sequenz ID Nummern 65, 56, 11, 162, 59, 331, 350, 155, 335, 159, 336, 332, 263, 306, 339, 337, 275, 301, 276, 330, 317, 309, 45, 318, 324, 352, 191, 262, 269, 313, 19, 367, 326, 325, 322, 327, 190, 261, 321, 360, 353, 312, 371, 5, 328, 205, 183, 38, 181, 37, 40, 182, 147, 17, 42, 382, 34, 18, 3 bzw. einer Sequenz, die mindestens zu etwa 90% die entsprechende Identität aufweist, im Vergleich zur Referenzhäufigkeit es ermöglicht, zwischen einer aus einem Phäochromozytom entstandenen Krebserkrankung und einer aus sämtlichen Sarkomen, Nebennieren-Tumoren und Mesotheliomtumoren entstandenen Krebserkrankung zu unterscheiden; oder
wobei die Messung der relativen Häufigkeit der Sequenz ID Nummern 61, 333, 31, 347, 346, 344, 345, 387, 334, 351, 324, 326, 269, 155, 320, 322, 59, 318, 325, 245, 254, 331, 275, 180, 355, 370, 323, 312, 178, 249, 183, 181, 38, 182, 37, 3, 25 bzw. einer Sequenz, die mindestens zu etwa 90% die entsprechende Identität aufweist, im Vergleich zur Referenzhäufigkeit es ermöglicht, zwischen einer aus einem Nebennieren-Tumor entstandenen Krebserkrankung und einer aus Mesotheliomtumoren oder Sarkomen entstandenen Krebserkrankung zu unterscheiden; oder
wobei die Messung der relativen Häufigkeit der Sequenz ID Nummern 165, 14, 15, 333, 272, 270, 45, 301, 191, 46, 195, 266, 190, 19, 334, 155, 25, 147, 40, 34 bzw. einer Sequenz, die mindestens zu etwa 90% die entsprechende Identität aufweist, im Vergleich zur Referenzhäufigkeit es ermöglicht, zwischen einer aus einem gastrointestinalen Stromatumor entstandenen Krebserkrankung und einer aus Mesotheliomtumoren oder Sarkomen entstandenen Krebserkrankung zu unterscheiden; oder
wobei die Messung der relativen Häufigkeit der Sequenz ID Nummern 13, 30, 361, 280, 362, 147, 40, 291, 387, 290, 299, 152, 178, 303, 242, 49, 11, 35, 34, 36, 206, 16, 170, 177, 17 bzw. einer Sequenz, die mindestens zu etwa 90% die entsprechende Identität aufweist, im Vergleich zur Referenzhäufigkeit es ermöglicht, zwischen einer aus einem chromophoben Nierenzellkarzinom entstandenen Krebserkrankung und einer aus klarzelligen und papillären Nierenzellkarzinom-Tumoren entstandenen Krebserkrankung zu unterscheiden; oder
wobei die Messung der relativen Häufigkeit der Sequenz ID Nummern 344, 382, 9, 338, 29, 49, 28, 195, 46, 4, 11, 254 bzw. einer Sequenz, die mindestens zu etwa 90% die entsprechende Identität aufweist, im Vergleich zur Referenzhäufigkeit es ermöglicht, zwischen einem aus einem klarzelligem Nierenzellkarzinom entstandenen Nierenkarzinom und einem aus einem papillären Tumor entstandenen Krebs zu unterscheiden; oder
wobei die Messung der relativen Häufigkeit der Sequenz ID Nummern 49, 35, 17, 34, 25, 36, 168, 170, 26, 4, 190, 46, 10, 240, 43, 39, 385, 63, 202, 181, 37, 5, 183, 182, 38, 206, 296, 1 bzw. einer Sequenz, die mindestens zu etwa 90% die entsprechende Identität aufweist, im Vergleich zur Referenzhäufigkeit es ermöglicht, zwischen einem aus einem Pleuramesotheliom entstandenen Krebs und einem aus Sarkomen entstandenen Krebs zu unterscheiden; oder
wobei die Messung der relativen Häufigkeit der Sequenz ID Nummern 152, 29, 159, 28, 339, 275, 352, 19, 320, 155, 262, 38, 37, 182, 331, 317, 323, 355, 3, 282, 312, 181, 269, 318, 59, 266, 322, 8, 324, 10, 40, 147, 169, 205, 34, 168, 14, 15, 12, 46, 255, 39, 23, 190, 236, 386, 379, 202 bzw. einer Sequenz, die mindestens zu etwa 90% die entsprechende Identität aufweist, im Vergleich zur Referenzhäufigkeit es ermöglicht, zwischen einem aus einem synovialen Sarkom entstandenen Krebs und einem aus anderen Sarkomen entstandenen Krebs zu unterscheiden; oder

wobei die Messung der relativen Häufigkeit der Sequenz ID Nummern 12, 271, 206, 333, 11, 58, 36, 18, 178, 293, 189, 382, 381, 240, 249, 5, 377, 235, 17, 20, 385, 384, 46, 283 bzw. einer Sequenz, die mindestens zu etwa 90% die entsprechende Identität aufweist, im Vergleich zur Referenzhäufigkeit es ermöglicht, zwischen einem aus einem Chondrosarkom entstandenen Krebs und einem aus anderen Sarkomen nicht synovialen Ursprungs entstandenen Krebs zu unterscheiden; oder

wobei die Messung der relativen Häufigkeit der Sequenz ID Nummern 295, 205, 25, 26, 231, 183, 42, 254, 168, 64, 14, 178, 15, 39, 36, 154, 265, 174, 384, 67 bzw. einer Sequenz, die mindestens zu etwa 90% die entsprechende Identität aufweist, im Vergleich zur Referenzhäufigkeit es ermöglicht, zwischen einem aus einem Liposarkom entstandenen Krebs und einem aus anderen Sarkomen, die weder Chondrosarkome noch synoviale Sarkome darstellen, entstandenen Krebs zu unterscheiden; oder

wobei die Messung der relativen Häufigkeit der Sequenz ID Nummern 22, 154, 21, 174, 205, 158, 186, 148, 20, 59, 8, 183, 231 bzw. einer Sequenz, die in der genannten Probe mindestens zu etwa 90% die entsprechende Identität aufweist, im Vergleich zur Referenzhäufigkeit es ermöglicht, bei einem Karzinom zwischen den folgenden Ursprüngen zu unterscheiden:

(e) Ewing-Sarkom und Osteosarkom, und
(f) Rhabdomyosarkom, malignes fibröses Histiozytom und Fibrosarkom; oder

wobei die Messung der relativen Häufigkeit der Sequenz ID Nummern 155, 179, 43, 208, 278, 17, 385, 174, 5, 52, 257, 366, 48, 49, 12, 25, 169, 34, 35, 23, 384, 189, 377, 265, 294, 293, 292 bzw. einer Sequenz, die mindestens zu etwa 90% die entsprechende Identität aufweist, im Vergleich zur Referenzhäufigkeit es ermöglicht, zwischen einem aus einem Ewing-Sarkom entstandenen Krebs und einem aus einem Osteosarkom entstandenen Krebs zu unterscheiden; oder

wobei die Messung der relativen Häufigkeit der Sequenz ID Nummern 33, 268, 267, 333, 276, 319, 306, 320, 334, 323, 300, 281, 59, 339, 316, 176, 348, 352, 349, 67, 357, 315, 343, 342, 355, 340, 344, 10, 341, 331, 20, 277, 318, 158, 265, 284, 36, 183, 40, 63, 147, 43, 289, 52, 190, 4, 5, 39, 169, 208 bzw. einer Sequenz, die in der genannten Probe mindestens zu etwa 90% die entsprechende Identität aufweist, im Vergleich zur Referenzhäufigkeit es ermöglicht, zwischen einem aus einem Rhabdomyosarkom entstandenen Krebs und einem aus einem malignen fibrösen Histiozytom und einem Fibrosarkom entstandenen Krebs zu unterscheiden; oder

**2.** Die Methode von Anspruch 1, wobei die genannte Tumorprobe von einer Körperflüssigkeit, einer Gewebeprobe, einer Biopsieprobe, einer Nadelbiopsieprobe, einer Feinnadelbiopsie (FNA)-Probe, einer bei einer Operation entfernten Gewebeprobe oder durch Verfahren für Gewebeprobenahmen wie Endoskopie, Bronchoskopie oder laparoskopische Verfahren entnommen wird; worin es sich bei dem genannten Gewebe vorzugsweise um ein frisches, tiefgefrorenes, fixiertes, in Wachs eingebettetes oder formalinfixiertes, Paraffin-eingebettetes (FFPE) Gewebe handelt.

**3.** Das Verfahren beider Ansprüche 1 oder 2, worin die Häufigkeit der Nukleinsäuresequenz durch eine ausgewählte Methode von Nukleinsäure-Hybridisierung und Nukleinsäure-Amplifikation gemessen wird.

**4.** Das Verfahren von Anspruch 3, worin die Methode der Nukleinsäure-Hybridisierung mittels Biochip-Arrays oder insitu-Hybridisierung ausgeführt wird.

**5.** Das Verfahren von Anspruch 3, worin die genannte Nukleinsäure-Amplifikation eine Real-Time-PCR darstellt; wobei die genannte Real-Time-PCR vorzugsweise sowohl Vorwärtsprimer als auch Rückwärtsprimer umfasst.

**6.** Das Verfahren von Anspruch 5, worin die genannte Real-Time-PCR-Methode eine Probe umfasst und die genannte Probe eine die SEQ ID Nummern 1; 2 oder 156; 3-7; 9-12; 14-21; 23-27; 29-40; 42; 43; 44 oder 191; 45-47; 49-51; 53-56; 57 oder 202; 58; 59; 60 oder 208; 61; 62 oder 211; 64-69; und 146-148, Fragmente hiervon bzw. Sequenzen, die zu mindestens etwa 90% damit identisch sind, ergänzende Sequenz enthält.

**7.** Das Verfahren von Anspruch 1, worin das genannte Algorithmus-Klassifikationsverfahren ein Entscheidungsbaum-Klassifikationsverfahren, ein Nächstgelegener-Nachbar-Klassifikationsverfahren oder "K-nearest neighbor classifier" (KNN), ein Logistische Regression-Klassifikationsverfahren, ein Gaußsches Mischverteilungsmodell (GMM), ein "Support-Vector-Machine" (SVM)-Klassifikationsverfahren, ein Nächstgelegener- Schwerpunkt-Klassifikationsverfahren oder "nearest centroid classifier", ein Lineare- Regression-Klassifikationsverfahren oder ein Random Forest-Klassifikationsverfahren darstellt.

## Revendications

**1.** La présente invention concerne un procédé pour distinguer entre les cancers de différentes origines, ledit procédé comprenant :

(a) la mesure de l'abondance relative des séquences d'acides nucléiques comportant les séquences SEQ ID N ° : 1-390, ou présentant une identité de séquence d'au moins 90 % avec celles-ci, dans un échantillon cancéreux obtenu précédemment à partir d'un sujet ; et
(b) la comparaison de ladite mesure à une abondance de référence de ladite séquence d'acide nucléique en utilisant un algorithme classificateur ;

dans lequel l'abondance relative de ladite séquence d'acide nucléique dans ledit échantillon par rapport à l'abondance de référence permet de distinguer les cancers d'origines différentes ; et
dans lequel la mesure de l'abondance relative des SEQ ID NO : 372, 233, 55, 200, 201 ou une séquence présentant une identité de séquence d'au moins 90 % avec celles-ci dans ledit échantillon par rapport à l'abondance de référence, permet de distinguer un cancer provenant d'une tumeur germinale et un cancer provenant de tumeurs non germinales ; ou
dans lequel la mesure de l'abondance relative des SEQ ID NO : 6, 30, 13 ou une séquence présentant une identité de séquence d'au moins 90 % avec celles-ci dans ledit échantillon par rapport à l'abondance de référence, permet de distinguer un cancer provenant de tumeurs hépatobiliaires et un cancer provenant de tumeurs non germinales non hépatobiliaires ; ou
dans lequel la mesure de l'abondance relative des SEQ ID NO : 28, 29, 231, 9 ou une séquence présentant une identité de séquence d'au moins 90 % avec celles-ci dans ledit échantillon par rapport à l'abondance de référence, permet de distinguer un cancer provenant de tumeurs hépatiques et un cancer provenant de carcinomes des voies biliaires ; ou
dans lequel la mesure de l'abondance relative des SEQ ID NO : 46, 5, 12, 30, 29, 28, 32, 13, 152, 49 ou une séquence présentant une identité de séquence d'au moins 90 % avec celles-ci dans ledit échantillon par rapport à l'abondance de référence, permet de distinguer un cancer provenant de tumeurs d'origine épithéliale et un cancer provenant de tumeurs d'origine non épithéliale ; ou
dans lequel la mesure de l'abondance relative des SEQ ID NO : 164, 168, 170, 16, 198, 50, 176, 186, 11, 158, 20, 155, 231, 4, 8, 46, 3, 2, 7 ou une séquence présentant une identité de séquence d'au moins 90 % avec celles-ci dans ledit échantillon par rapport à l'abondance de référence, permet de distinguer un cancer provenant d'un mélanome et d'un lymphome et un cancer provenant de toutes les autres tumeurs non épithéliales ; ou
dans lequel la mesure de l'abondance relative des SEQ ID NO : 159, 66, 225, 187, 162, 161, 68, 232, 173, 11, 8, 174, 155, 231, 4, 182, 181, 37 ou une séquence présentant une identité de séquence d'au moins 90 % avec celles-ci dans ledit échantillon par rapport à l'abondance de référence, permet de distinguer un cancer provenant de tumeurs cérébrales et un cancer provenant de toutes les tumeurs non-cérébrales, non épithéliales ; ou
dans lequel la mesure de l'abondance relative des SEQ ID NO : 40, 208, 60, 153, 230, 228, 147, 34, 206, 35, 52, 25, 229, 161, 187, 179 ou une séquence présentant une identité de séquence d'au moins 90 % avec celles-ci dans ledit échantillon par rapport à l'abondance de référence, permet de distinguer un cancer provenant d'un astrocytome et un cancer provenant d'un oligodendrogliome ; ou
dans lequel la mesure de l'abondance relative des SEQ ID NO : 56, 65, 25, 175, 152, 155, 32, 49, 35, 181, ou une séquence présentant une identité de séquence d'au moins 90 % avec celles-ci dans ledit échantillon par rapport à l'abondance de référence, permet de distinguer un cancer provenant de tumeurs neuroendocrines et un cancer provenant de toutes les tumeurs épithéliales non neuroendocrines ; ou
dans lequel la mesure de l'abondance relative des SEQ ID NO : 27, 177, 4, 32, 35 ou une séquence présentant une identité de séquence d'au moins 90 % avec celles-ci dans ledit échantillon par rapport à l'abondance de référence, permet de distinguer un cancer provenant de tumeurs épithéliales gastro-intestinales et un cancer provenant de tumeurs épithéliales non gastro-intestinales ; ou
dans lequel la mesure de l'abondance relative des SEQ ID NO : 56, 199, 14, 15, 165, 231, 36, 154, 21, 49, ou une séquence présentant une identité de séquence d'au moins 90 % avec celles-ci dans ledit échantillon par rapport à l'abondance de référence, permet de distinguer un cancer provenant de tumeurs de la prostate et un cancer provenant de toutes les autres tumeurs épithéliales non gastro-intestinales ; ou
dans lequel la mesure de l'abondance relative des SEQ ID NO : 222, 62, 29, 28, 211, 214, 227, 215, 218, 152, 216, 212, 224, 13, 194, 192, 221, 217, 205, 219, 32, 193, 223, 220, 210, 209, 213, 163, 30 ou une séquence présentant une identité de séquence d'au moins 90 % avec celles-ci dans ledit échantillon par rapport à l'abondance de référence, permet de distinguer un cancer provenant d'un séminome et un cancer provenant de tumeurs testiculaires non séminomateuses ; ou
dans lequel la mesure de l'abondance relative des SEQ ID NO : 42, 32, 36, 178, 243, 242, 49, 240, 57, 11, 46, 17, 47, 51, 7, 8, 154, 190, 157, 196, 197, ou une séquence présentant une identité de séquence d'au moins 90 % avec celles-ci dans ledit échantillon par rapport à l'abondance de référence, permet de distinguer un cancer provenant d'un carcinome épidermoïde, d'un carcinome transitionnel et d'un thymome, et un cancer provenant de tumeurs d'adénocarcinome non gastro-intestinales ; ou
dans lequel la mesure de l'abondance relative des SEQ ID NO : 56, 46, 25, 152, 50, 45, 191, 181, 179, 49, 32, 42, 184, 40, 147, 236, 57, 203, 36, ou une séquence présentant une identité de séquence d'au moins 90 % avec celles-ci dans ledit échantillon par rapport à l'abondance de référence, permet de distinguer un cancer provenant d'un adénocarcinome du sein et un cancer provenant d'un carcinome épidermoïde, d'un carcinome transitionnel, de thymomes et d'un carcinome ovarien ; ou
dans lequel la mesure de l'abondance relative des SEQ ID NO : 253, 32, 4, 39, 10, 46, 5, 226, 2, 195, 32, 185, 11, 168, 184, 16, 242, 12, 237, 243, 250, 49, 246, 167 ou une séquence présentant une identité de séquence d'au moins 90 % avec celles-ci dans ledit échantillon par rapport à l'abondance de référence, permet de distinguer un cancer provenant d'un carcinome ovarien, et un cancer provenant d'un carcinome épidermoïde, d'un carcinome transitionnel et de thymomes ; ou
dans lequel la mesure de l'abondance relative des SEQ ID NO : 11, 147, 17, 157, 40, 8, 49, 9, 191, 205, 207, 195, 51, 46, 45, 52, 234, 231, 21, 169, 43, 3, 196, 154, 390, 171, 255, 197, 190, 189, 39, 7, , 47, 32, 36, 4, 178, 37, 181, 25, 183, 182, 35, 240, 57, 242, 204, 236, 176, 158, 148, 206, 50, 20, 34, 186, 239, 251, 244, 24, 188, 172, 238 ou une séquence présentant une identité de séquence d'au moins 90 % avec celles-ci dans ledit échantillon par rapport à l'abondance de référence, permet de distinguer un cancer provenant d'un carcinome thyroïdien, et un cancer provenant d'un adénocarcinome du sein, d'un carcinome pulmonaire à grande cellule, d'un adénocarcinome pulmonaire et d'un carcinome ovarien ; ou
dans lequel la mesure de l'abondance relative des SEQ ID NO : 249, 180, 65, 235, 241, 248, 254, 247, 160, 243, 245, 252, 17, 49, 166, 225, 168, 34 ou une séquence présentant une identité de séquence d'au moins 90 % avec celles-ci dans ledit échantillon par rapport à l'abondance de référence, permet de distinguer un cancer provenant d'un carcinome folliculaire de la thyroïde et un cancer provenant d'un carcinome papillaire de la thyroïde ; ou
dans lequel la mesure de l'abondance relative des SEQ ID NO : 32, 56, 50, 45, 25, 253, 152, 9, 46, 191, 178, 49, 40, 10, 147, 4, 36, 228, 236, 230, 189, 240, 67, 202, 17 ou une séquence présentant une identité de séquence d'au moins 90 % avec celles-ci dans ledit échantillon par rapport à l'abondance de référence, permet de distinguer un cancer provenant d'un adénocarcinome du sein et un cancer provenant d'un adénocarcinome pulmonaire et d'un carcinome ovarien ; ou
dans lequel la mesure de l'abondance relative des SEQ ID NO : 56, 11, 168, 16, 237, 21, 52, 12, 154, 279, 9, 39, 47, 23, 50, 167, 383, 34, 35, 388, 5, 359, 245, 254, 10, 240, 236, 202, 4, 25, 203, 231, 20, 158, 186, 258, 244, 172, 2, 235, 256, 28, 277, 296, 374, 153, 181 ou une séquence présentant une identité de séquence d'au moins 90 % avec celles-ci dans ledit échantillon par rapport à l'abondance de référence, permet de distinguer un cancer provenant d'un adénocarcinome pulmonaire et un cancer provenant d'un carcinome ovarien ;
ou
dans lequel la mesure de l'abondance relative des SEQ ID NO : 161, 164, 22, 53, 285, 3, 152, 191, 154, 21, 206, 174, 19, 45, 171, 179, 8, 296, 284, 18, 51, 258, 49, 184, 35, 34, 37, 42, 228, 15, 14, 242, 230, 253, 36, 182, 293, 292, 4, 294, 297, 354, 377, 189, 30, 386, 249, 5, 274 ou une séquence présentant une identité de séquence d'au moins 90 % avec celles-ci dans ledit échantillon par rapport à l'abondance de référence, permet de distinguer un cancer provenant d'un carcinome thymique et un cancer provenant d'un carcinome transitionnel et d'un carcinome épidermoïde ; ou
dans lequel la mesure de l'abondance relative des SEQ ID NO : 69, 28, 280, 13, 191, 152, 29, 175, 30, 204, 4, 24, 5, 329, 273, 170, 184, 26, 231, 368, 37, 16, 169, 155, 35, 40, 17 ou une séquence présentant une identité de séquence d'au moins 90 % avec celles-ci dans ledit échantillon par rapport à l'abondance de référence, permet de distinguer un cancer provenant d'un carcinome transitionnel et un cancer provenant d'un carcinome épidermoïde ; ou
dans lequel la mesure de l'abondance relative des SEQ ID NO : 164, 5, 231, 54, 1, 242, 372, 249, 167, 254, 354, 381, 380, 245, 358, 364, 240, 11, 378 ou une séquence présentant une identité de séquence d'au moins 90 % avec celles-ci dans ledit échantillon par rapport à l'abondance de référence, permet de distinguer les cancers du carcinome épidermoïde provenant du col de l'utérus et les cancers du carcinome épidermique provenant de l'anus et de la peau, des poumons, de la tête et du cou et de l'oesophage; ou

dans lequel la mesure de l'abondance relative des SEQ ID NO : 305, 184, 41, 183, 49, 382, 235, 291, 181, 5, 296, 289, 206, 338, 334, 25, 11, 19, 198, 23 ou une séquence présentant une identité de séquence d'au moins 90 % avec celles-ci dans ledit échantillon par rapport à l'abondance de référence, permet de distinguer les cancers du carcinome épidermoïde provenant de l'anus et de la peau, et les cancers du carcinome épidermoïde provenant des poumons, de la tête et du cou et de l'oesophage ; ou
dans lequel la mesure de l'abondance relative des SEQ ID NO : 4, 11, 46, 8, 274, 169, 36, 47, 363, 231, 303, 349, 10, 7, 3, 16, 164, 170, 168, 198, 50, 245, 365, 45, 382, 259, 296, 364, 314, 12 ou une séquence présentant une identité de séquence d'au moins 90 % avec celles-ci dans ledit échantillon par rapport à l'abondance de référence, permet de distinguer un cancer provenant d'un mélanome et un cancer provenant d'un lymphome ; ou
dans lequel la mesure de l'abondance relative des SEQ ID NO : 11, 191, 48, 35, 228 ou une séquence présentant une identité de séquence d'au moins 90 % avec celles-ci dans ledit échantillon par rapport à l'abondance de référence, permet de distinguer un cancer provenant d'un lymphome à cellules B et un cancer provenant d'un lymphome à lymphocytes T; ou
dans lequel la mesure de l'abondance relative des SEQ ID NO : 158, 20, 176, 186, 148, 36, 51, 172, 260, 265, 67, 188, 277, 284, 302, 68, 168, 242, 204, 162, 177, 27, 65, 263, 155, 191, 190, 45, 59, 43, 56, 266, 14, 15, 8, 7, 39, 189, 249, 231, 293, 2 ou une séquence présentant une identité de séquence d'au moins 90 % avec celles-ci dans ledit échantillon par rapport à l'abondance de référence, permet de distinguer un cancer provenant d'un carcinome du poumon à petites cellules et un cancer provenant d'une tumeur carcinoïde pulmonaire, d'un carcinome médullaire de la thyroïde, d'une tumeur carcinoïde du tractus gastro-intestinal et d'une tumeur des cellules des îlots pancréatiques ; ou
dans lequel la mesure de l'abondance relative des SEQ ID NO : 159, 40, 147, 11, 311, 4, 8, 231, 301, 297, 68, 67, 265, 36 ou une séquence présentant une identité de séquence d'au moins 90 % avec celles-ci dans ledit échantillon par rapport à l'abondance de référence, permet de distinguer un cancer provenant du carcinome médullaire de la thyroïde et un cancer provenant d'autres tumeurs neuroendocrines sélectionnées parmi une tumeur carcinoïde pulmonaire, une tumeur carcinoïde du tractus gastro-intestinal et une tumeur des cellules des îlots pancréatiques ; ou
dans lequel la mesure de l'abondance relative des SEQ ID NO : 331, 162, 59, 326, 306, 350, 317, 155, 325, 318, 339, 264, 332, 262, 336, 324, 322, 330, 321, 263, 309, 53, 320, 275, 352, 312, 355, 367, 269, 64, 308, 175, 190, 54, 302, 152, 301, 266, 47, 313, 359, 65, 307, 191, 242, 4, 147, 40, 372, 168, 16, 182, 167, 356, 148, 382, 37, 364, 35 ou une séquence présentant une identité de séquence d'au moins 90 % avec celles-ci dans ledit échantillon par rapport à l'abondance de référence, permet de distinguer un cancer provenant de tumeurs carcinoïdes pulmonaires et un cancer provenant de tumeurs neuroendocrines gastro-intestinales sélectionnées parmi une tumeur carcinoïde du tractus gastro-intestinal et une tumeur des cellules des îlots carcinoïdes ; ou
dans lequel la mesure de l'abondance relative des SEQ ID NO : 263, 288, 18, 286, 162, 225, 287, 206, 205, 296, 258, 313, 377, 373, 256, 153, 259, 265, 303, 268, 267, 165, 15, 272, 14, 202, 236, 203, 4, 168, 310, 298, 27, 29, 34, 228, 3, 349, 35, 26 ou une séquence présentant une identité de séquence d'au moins 90 % avec celles-ci dans ledit échantillon par rapport à l'abondance de référence, permet de distinguer un cancer provenant des tumeurs des cellules des îlots pancréatiques et un cancer carcinoïde neuroendocrinien gastro-intestinal provenant de l'intestin grêle et du duodénum ; de l'appendicite, de l'estomac et du pancréas ; ou
dans lequel la mesure de l'abondance relative des SEQ ID NO : 36, 267, 268, 165, 15, 14, 356, 167, 372, 272, 370, 42, 41, 146 ou une séquence présentant une identité de séquence d'au moins 90 % avec celles-ci dans ledit échantillon par rapport à l'abondance de référence permet de distinguer les tumeurs d'adénocarcinome du système gastro-intestinal provenant :

(a) d'un adénocarcinome gastrique et oesophagien, et
(b) d'un cholangiocarcinome ou d'un adénocarcinome des voies biliaires extra-hépatiques, d'un adénocarcinome pancréatique et d'un adénocarcinome colorectal ; ou

dans lequel la mesure de l'abondance relative des SEQ ID NO : 42, 184, 67, 158, 20, 186, 284, 389, 203, 240, 236, 146, 204, 43, 176, 202, 49, 46, 38, 363 ou une séquence présentant une identité de séquence d'au moins 90 % avec celles-ci dans ledit échantillon par rapport à l'abondance de référence, permet de distinguer un cancer provenant d'un adénocarcinome colorectal et un cancer provenant d'un adénocarcinome des voies biliaires ou du pancréas ; ou
dans lequel la mesure de l'abondance relative des SEQ ID NO : 49, 11, 13, 373, 154, 5, 30, 45, 178, 147, 274, 16, 40, 21, 43, 253, 245, 256, 12, 374, 379, 180, 153, 51, 52, 1, 295, 257, 385, 293, 294 ou une séquence présentant une identité de séquence d'au moins 90 % avec celles-ci dans ledit échantillon par rapport à l'échantillon d'abondance de référence permet de distinguer un cancer provenant d'un adénocarcinome pancréatique et un cancer provenant d'un cholangiocarcinome ou d'un adénocarcinome du tractus biliaire extra-hépatique ; ou
dans lequel la mesure de l'abondance relative des SEQ ID NO : 29, 28, 30, 46, 49, 195, 152, 175, 47, 4, 387, 196, 177, 375, 27, 304, 40, 191, 147, 35, 16, 34, 5, 155, 181, 312, 183, 182, 320, 59, 38, 324, 323, 37, 322, 325, 19, 42, 334, 265, 22 ou une séquence présentant une identité de séquence d'au moins 90 % avec celles-ci dans ledit échantillon par rapport à l'abondance de référence, permet de distinguer un cancer provenant :

(c) de tumeurs des cellules rénales sélectionnées parmi un carcinome rénal à cellules chromophobes, un carcinome rénal à cellules claires et un carcinome rénal à cellules papillaires et
(d) de sarcomes, de tumeurs de la surrénale et du mésothéliome pleural ; ou

dans lequel la mesure de l'abondance relative des SEQ ID NO : 65, 56, 11, 162, 59, 331, 350, 155, 335, 159, 336, 332, 263, 306, 339, 337, 275, 301, 276, 330, 317, 309, 45, 318, 324, 352, 191, 262, 269, 313, 19, 367, 326, 325, 322, 327, 190, 261, 321, 360, 353, 312, 371, 5, 328, 205, 183, 38, 181, 37, 40, 182, 147, 17, 42, 382, 34, 18, 3 ou une séquence présentant une identité de séquence d'au moins 90 % avec celles-ci dans ledit échantillon par rapport à l'abondance de référence, permet de distinguer un cancer provenant d'un phéochromocytome et un cancer provenant de toutes les tumeurs d'un sarcome, d'un carcinome surrénal et d'un mésothéliome ; ou
dans lequel la mesure de l'abondance relative des SEQ ID NO : 61, 333, 31, 347, 346, 344, 345, 387, 334, 351, 324, 326, 269, 155, 320, 322, 59, 318, 325, 245, 254, 331, 275, 180, 355, 370, 323, 312, 178, 249, 183, 181, 38, 182, 37, 3, 25 ou une séquence présentant une identité de séquence d'au moins 90 % avec celles-ci dans ledit échantillon par rapport à l'abondance de référence, permet de distinguer un cancer provenant d'un carcinome surrénal et un cancer provenant des tumeurs d'un mésothéliome et d'un sarcome ; ou
dans lequel la mesure de l'abondance relative des SEQ ID NO : 165, 14, 15, 333, 272, 270, 45, 301, 191, 46, 195, 266, 190, 19, 334, 155, 25, 147, 40, 34 ou une séquence présentant une identité de séquence d'au moins 90 % avec celles-ci dans ledit échantillon par rapport à l'abondance de référence, permet de distinguer un cancer provenant d'une tumeur stromale gastro-intestinale et un cancer provenant de tumeurs d'un mésothéliome et d'un sarcome ; ou
dans lequel la mesure de l'abondance relative des SEQ ID NO : 13, 30, 361, 280, 362, 147, 40, 291, 387, 290, 299, 152, 178, 303, 242, 49, 11, 35, 34, 36, 206, 16, 170, 177, 17 ou une séquence présentant une identité de séquence d'au moins 90 % avec celles-ci dans ledit échantillon par rapport à l'abondance de référence, permet de distinguer un cancer provenant d'une tumeur d'un carcinome chromophobe à cellules rénales et d'un cancer provenant de tumeurs de carcinomes à cellules claires et papillaires rénales ; ou
dans lequel la mesure de l'abondance relative des SEQ ID NO : 344, 382, 9, 338, 29, 49, 28, 195, 46, 4, 11, 254 ou une séquence présentant une identité de séquence d'au moins 90 % avec celles-ci dans ledit échantillon par rapport à l'abondance de référence, permet de distinguer un cancer d'un carcinome provenant d'une tumeur à cellules claires rénales et un cancer provenant d'une tumeur papillaire ; ou
dans lequel la mesure de l'abondance relative des SEQ ID NO : 49, 35, 17, 34, 25, 36, 168, 170, 26, 4, 190, 46, 10, 240, 43, 39, 385, 63, 202, 181, 37, 5, 183, 182, 38, 206, 296, 1 ou une séquence présentant une identité de séquence d'au moins 90 % avec celles-ci dans ledit échantillon par rapport à l'abondance de référence, permet de distinguer un cancer provenant d'un mésothéliome pleural et un cancer provenant de tumeurs d'un sarcome ; ou
dans lequel la mesure de l'abondance relative des SEQ ID NO : 152, 29, 159, 28, 339, 275, 352, 19, 320, 155, 262, 38, 37, 182, 331, 317, 323, 355, 3, 282, 312, 181, 269, 318, 59, 266, 322, 8, 324, 10, 40, 147, 169, 205, 34, 168, 14, 15, 12, 46, 255, 39, 23, 190, 236, 386, 379, 202 ou une séquence présentant une identité de séquence d'au moins 90 % avec celles-ci dans ledit échantillon par rapport à l'abondance de référence, permet de distinguer un cancer provenant d'un sarcome synovial et un cancer provenant d'autres tumeurs d'un sarcome ; ou
dans lequel la mesure de l'abondance relative des SEQ ID NO : 12, 271, 206, 333, 11, 58, 36, 18, 178, 293, 189, 382, 381, 240, 249, 5, 377, 235, 17, 20, 385, 384, 46, 283 ou une séquence présentant une identité de séquence d'au moins 90 % avec celles-ci dans ledit échantillon par rapport à l'abondance de référence, permet de distinguer un cancer provenant d'un chondrosarcome et un cancer provenant d'autres tumeurs non-synoviales d'un sarcome ; ou
dans lequel la mesure de l'abondance relative des SEQ ID NO : 295, 205, 25, 26, 231, 183, 42, 254, 168, 64, 14, 178, 15, 39, 36, 154, 265, 174, 384, 67 ou une séquence présentant une identité de séquence d'au moins 90 % avec celles-ci dans ledit échantillon par rapport à l'abondance de référence, permet de distinguer un cancer provenant d'un iposarcome et un cancer provenant d'autres tumeurs non chondrosarcomateuses et ne provenant pas de tumeurs d'un sarcome synovial ; ou
dans lequel la mesure de l'abondance relative des SEQ ID NO : 22, 154, 21, 174, 205, 158, 186, 148, 20, 59, 8, 183, 231 ou une séquence présentant une identité de séquence d'au moins 90 % avec celles-ci dans ledit échantillon par rapport à l'abondance de référence permet de distinguer un cancer provenant :

(e) d'un sarcome d'Ewing et d'un ostéosarcome, et
(f) d'un rhabdomyosarcome, d'un histiocytome fibreux malin et d'un fibrosarcome ; ou

dans lequel la mesure de l'abondance relative des SEQ ID NO : 155, 179, 43, 208, 278, 17, 385, 174, 5, 52, 257, 366, 48, 49, 12, 25, 169, 34, 35, 23, 384, 189, 377, 265, 294, 293, 292 ou une séquence présentant une identité de séquence d'au moins 90 % avec celles-ci dans ledit échantillon par rapport à l'abondance de référence, permet de distinguer un cancer provenant du sarcome d'Ewing et un cancer provenant d'un ostéosarcome ; ou
dans lequel la mesure de l'abondance relative des SEQ ID NO : 33, 268, 267, 333, 276, 319, 306, 320, 334, 323, 300, 281, 59, 339, 316, 176, 348, 352, 349, 67, 357, 315, 343, 342, 355, 340, 344, 10, 341, 331, 20, 277, 318, 158, 265, 284, 36, 183, 40, 63, 147, 43, 289, 52, 190, 4, 5, 39, 169, 208 ou une séquence présentant une identité de séquence d'au moins 90 % avec celles-ci dans ledit échantillon par rapport à l'abondance de référence, permet de distinguer un cancer provenant d'un rhabdomyosarcome et un cancer provenant d'un histiocytome fibreux malin et d'un fibrosarcome.

**2.** Procédé selon la revendication 1, dans lequel ledit échantillon cancéreux est choisi parmi un fluide corporel, un échantillon de tissu, un échantillon de biopsie, un échantillon de biopsie à l'aiguille, un échantillon de biopsie à l'aiguille fine (BAF), un échantillon prélevé par chirurgie et un échantillon obtenu par des procédures de prélèvement tissulaire telles que l'endoscopie, la bronchoscopie ou des méthodes laparoscopiques ; de préférence, dans lequel ledit tissu est un tissu frais, congelé, fixé, inclus dans la cire ou un tissu fixé au formol et inclus dans la paraffine (FFIP).

**3.** Procédé selon l'une quelconque des revendications 1 ou 2, dans lequel l'abondance de séquence d'acide nucléique est mesurée par un procédé choisi parmi l'hybridation de l'acide nucléique et l'amplification de l'acide nucléique.

**4.** Procédé selon la revendication 3, dans lequel l'hybridation de l'acide nucléique est effectuée en utilisant un réseau de biopuces à acide nucléique en phase solide ou une hybridation in situ.

**5.** Procédé selon la revendication 3, dans lequel ledit procédé d'amplification de l'acide nucléique est une PCR en temps réel ; de préférence dans lequel ladite PCR en temps réel comprend des amorces avant et des amorces inverses.

**6.** Procédé selon la revendication 5, dans lequel ledit procédé de PCR en temps réel comprend en outre une sonde, ladite sonde comprenant une séquence complémentaire des SEQ ID NO : 1 ; 2 ou 156 ; 3-7 ; 9-12 ; 14-21 ; 23-27 ; 29-40 ; 42 ; 43 ; 44 ou 191 ; 45-47 ; 49-51 ; 53-56 ; 57 ou 202 ; 58 ; 59 ; 60 ou 208 ; 61 ; 62 ou 211 ; 64-69 ; et 146-148 , leurs fragments et des séquences ayant au moins environ 90 % d'identité avec celles-ci.

**7.** Procédé selon la revendication 1, dans lequel ledit algorithme classificateur est un classificateur par arbre de décision, un classificateur des K-plus proches voisins (KNN), un classificateur par régression logistique, un classificateur du voisin le plus proche, un classificateur de réseau neuronal, un modèle de mélange gaussien (GMM), un classificateur par machine à vecteurs de support (SVM), un classificateur de centroïde le plus proche, un classificateur de régression linéaire ou un classificateur de forêt aléatoire.

Figure 1A

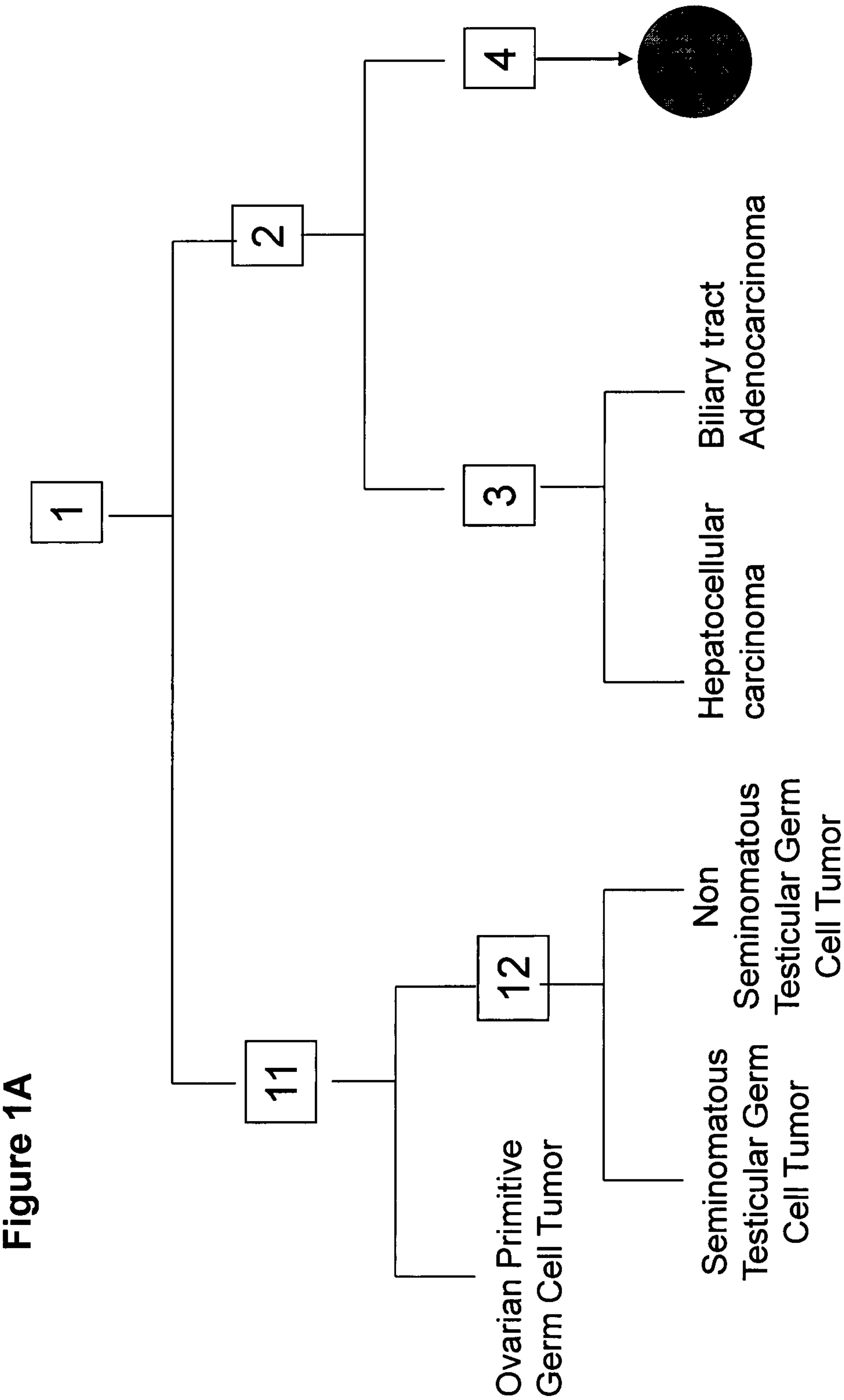
1
2
3
4
11
12
Hepatocellular carcinoma
Biliary tract Adenocarcinoma
Ovarian Primitive Germ Cell Tumor
Seminomatous Testicular Germ Cell Tumor
Non Seminomatous Testicular Germ Cell Tumor

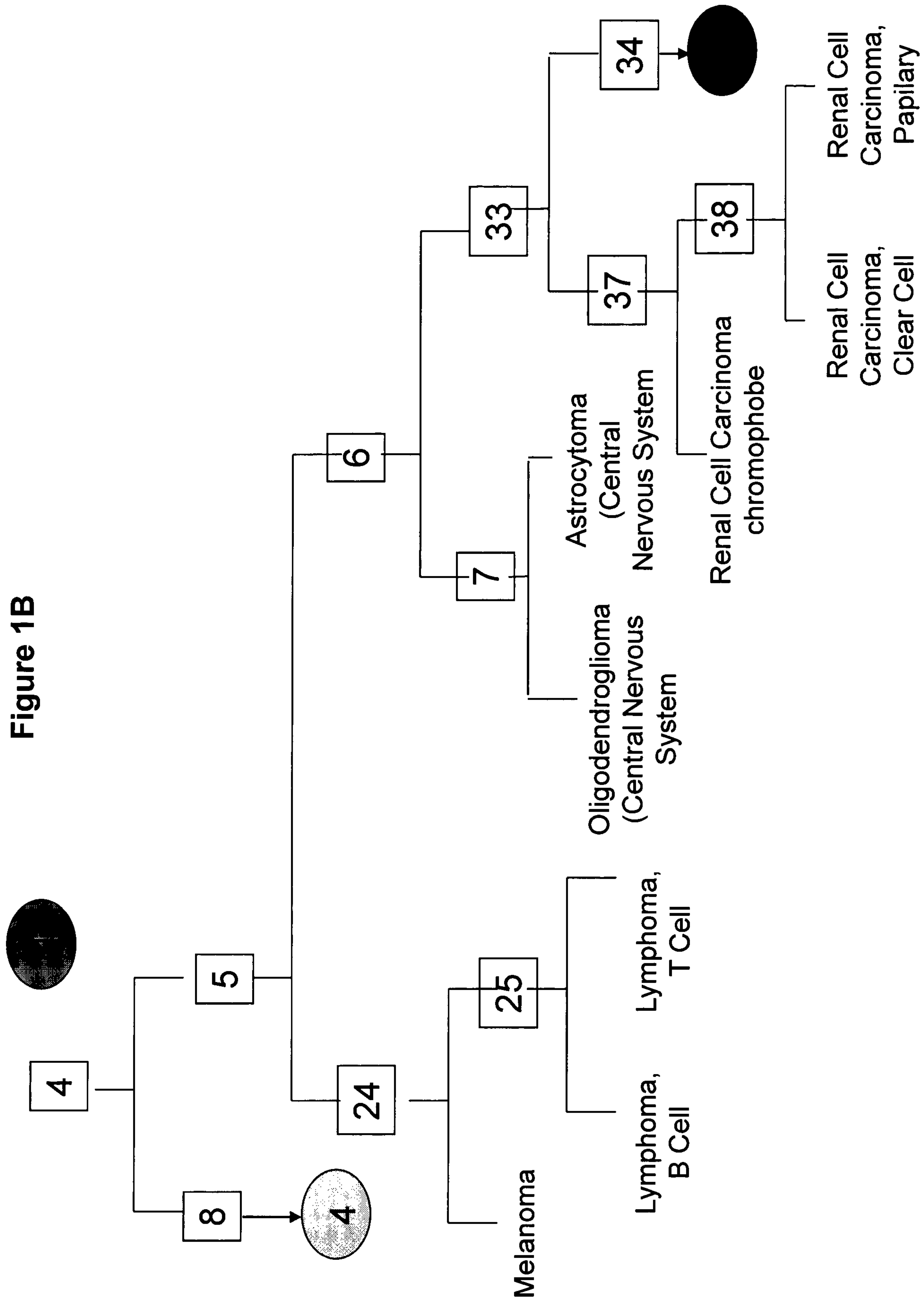
4
8
4
5
24
25
6
7
33
34
37
38
Melanoma
Lymphoma, B Cell
Lymphoma, T Cell
Oligodendroglioma (Central Nervous System
Astrocytoma (Central Nervous System
Renal Cell Carcinoma chromophobe
Renal Cell Carcinoma, Clear Cell
Renal Cell Carcinoma, Papilary

Figure 1B

Figure 1C

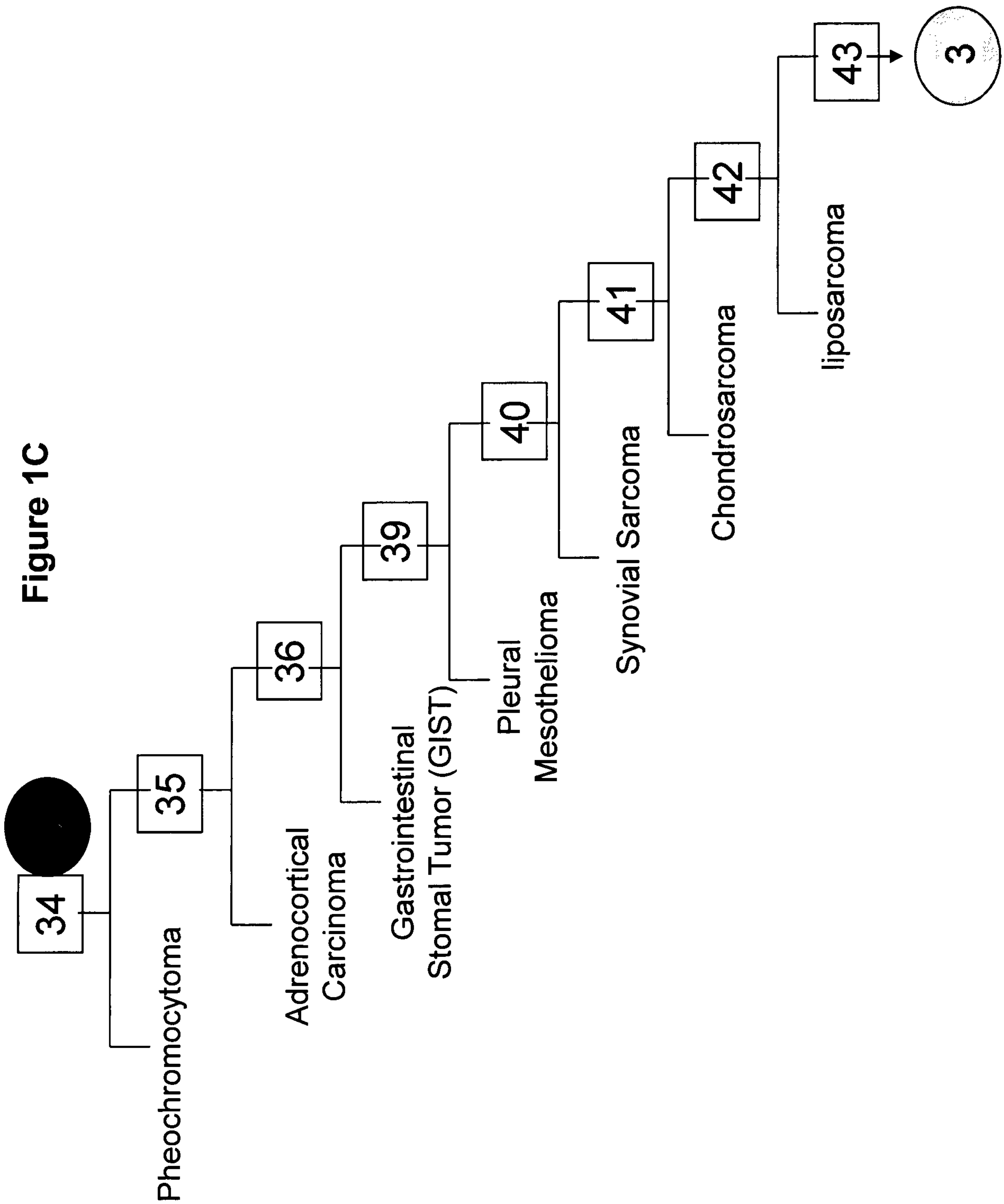
34
35
36
39
40
41
42
43
3
Pheochromocytoma
Adrenocortical Carcinoma
Gastrointestinal Stomal Tumor (GIST)
Pleural Mesothelioma
Synovial Sarcoma
Chondrosarcoma
liposarcoma

Figure 1D

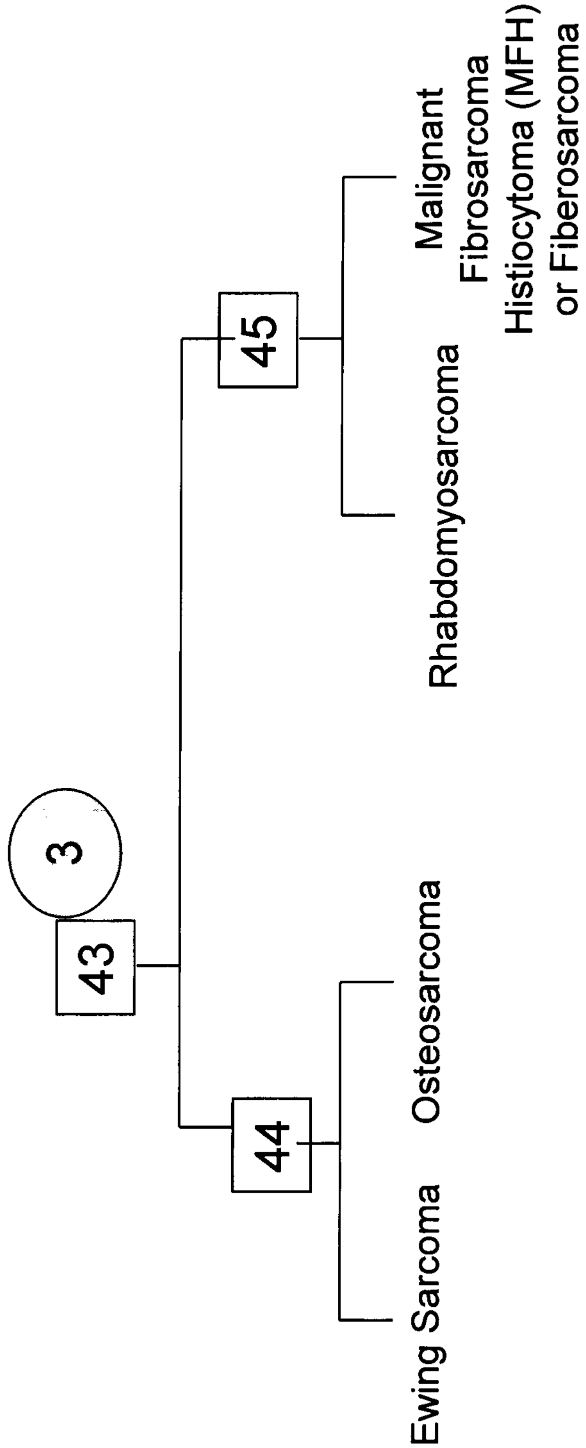
3
43
44
45
Ewing Sarcoma
Osteosarcoma
Rhabdomyosarcoma
Malignant Fibrosarcoma Histiocytoma (MFH) or Fiberosarcoma

Figure 1E

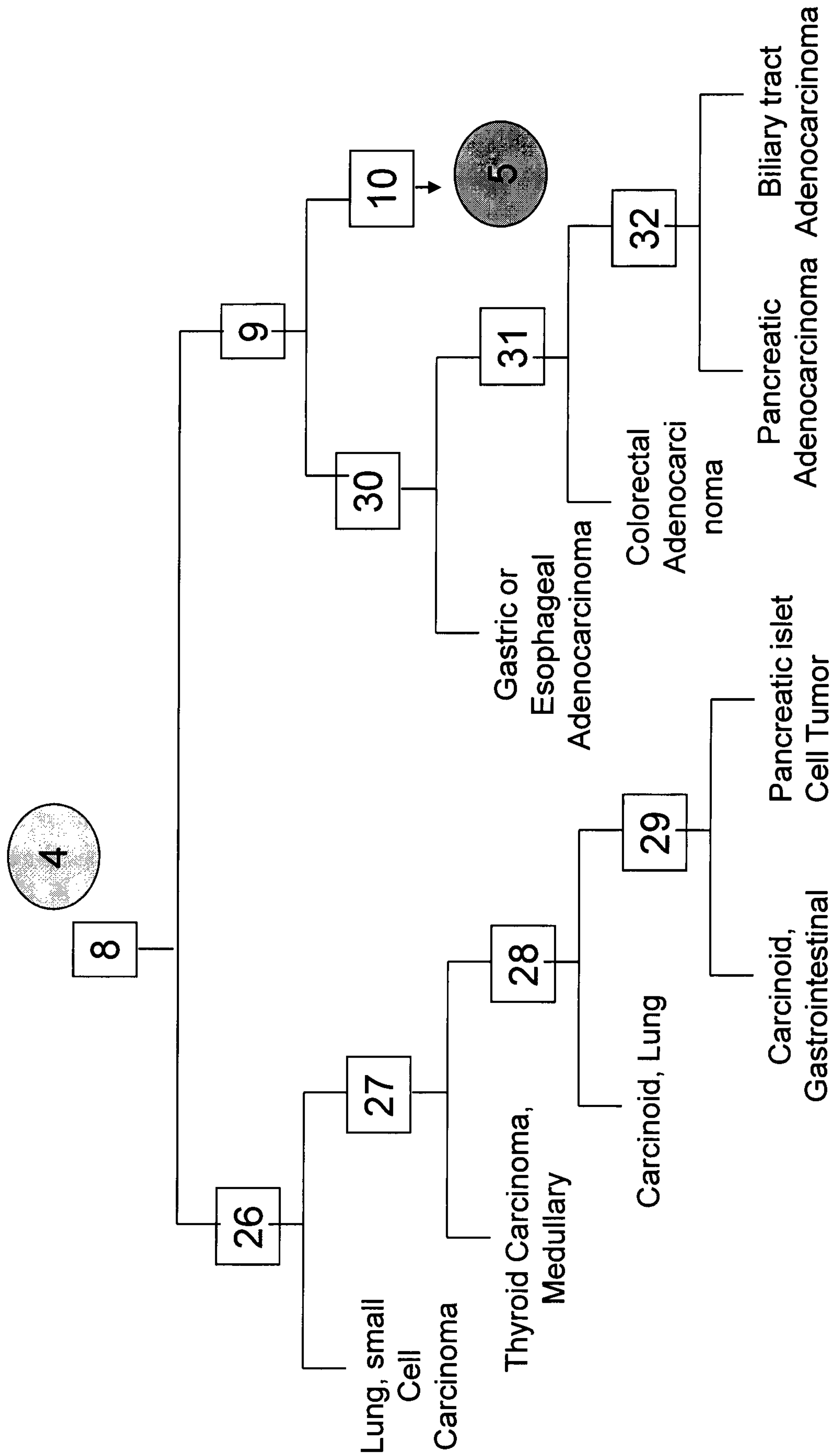
4
8
26
Lung, small Cell Carcinoma
27
Thyroid Carcinoma, Medullary
28
Carcinoid, Lung
29
Carcinoid, Gastrointestinal
Pancreatic islet Cell Tumor
9
30
Gastric or Esophageal Adenocarcinoma
31
Colorectal Adenocarci noma
32
Pancreatic Adenocarcinoma
Biliary tract Adenocarcinoma
10
5

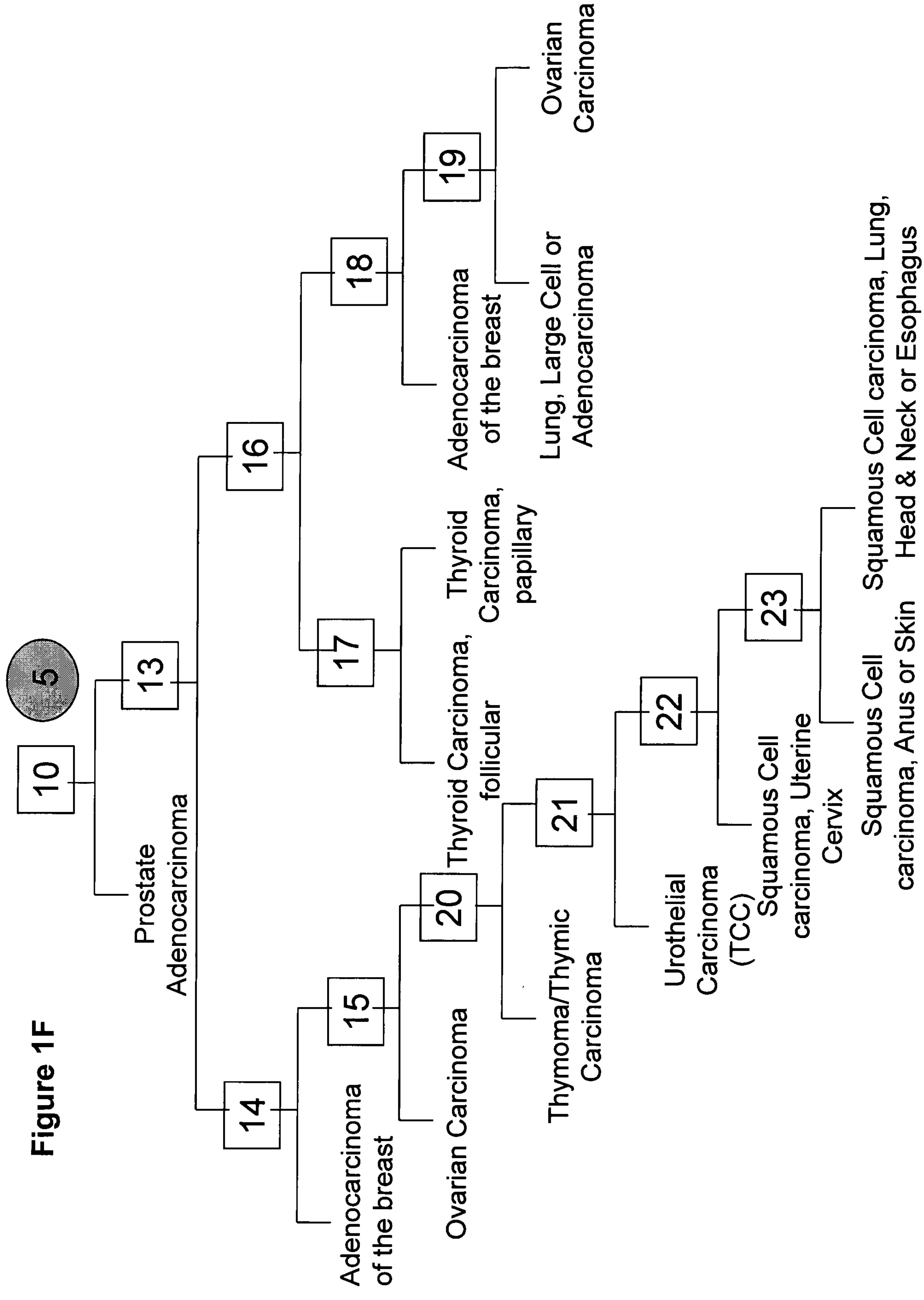
10
5
13
Prostate Adenocarcinoma
14
16
15
17
18
19
20
21
22
23
Adenocarcinoma of the breast
Ovarian Carcinoma
Thyroid Carcinoma, follicular
Thyroid Carcinoma, papillary
Adenocarcinoma of the breast
Lung, Large Cell or Adenocarcinoma
Ovarian Carcinoma
Thymoma/Thymic Carcinoma
Urothelial Carcinoma (TCC)
Squamous Cell carcinoma, Uterine Cervix
Squamous Cell carcinoma, Anus or Skin
Squamous Cell carcinoma, Lung, Head & Neck or Esophagus

Figure 1F

Figure 2A

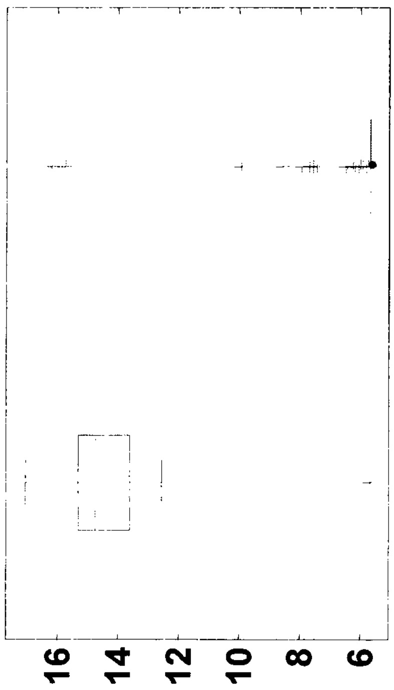


Figure 2B

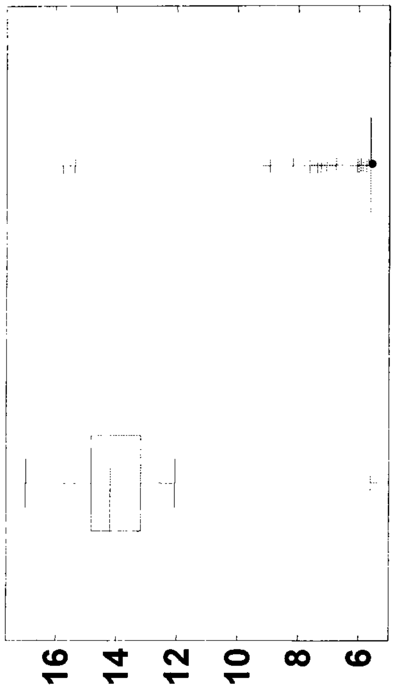


Figure 2C

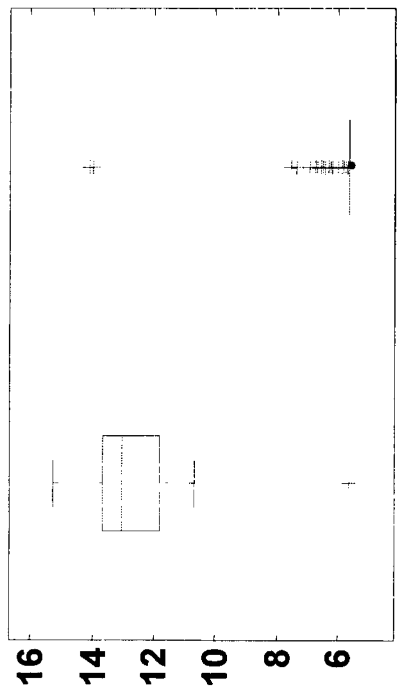


Figure 2D

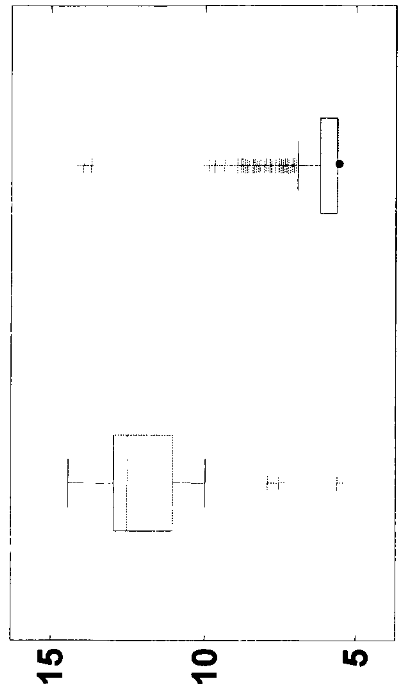

Figure 3

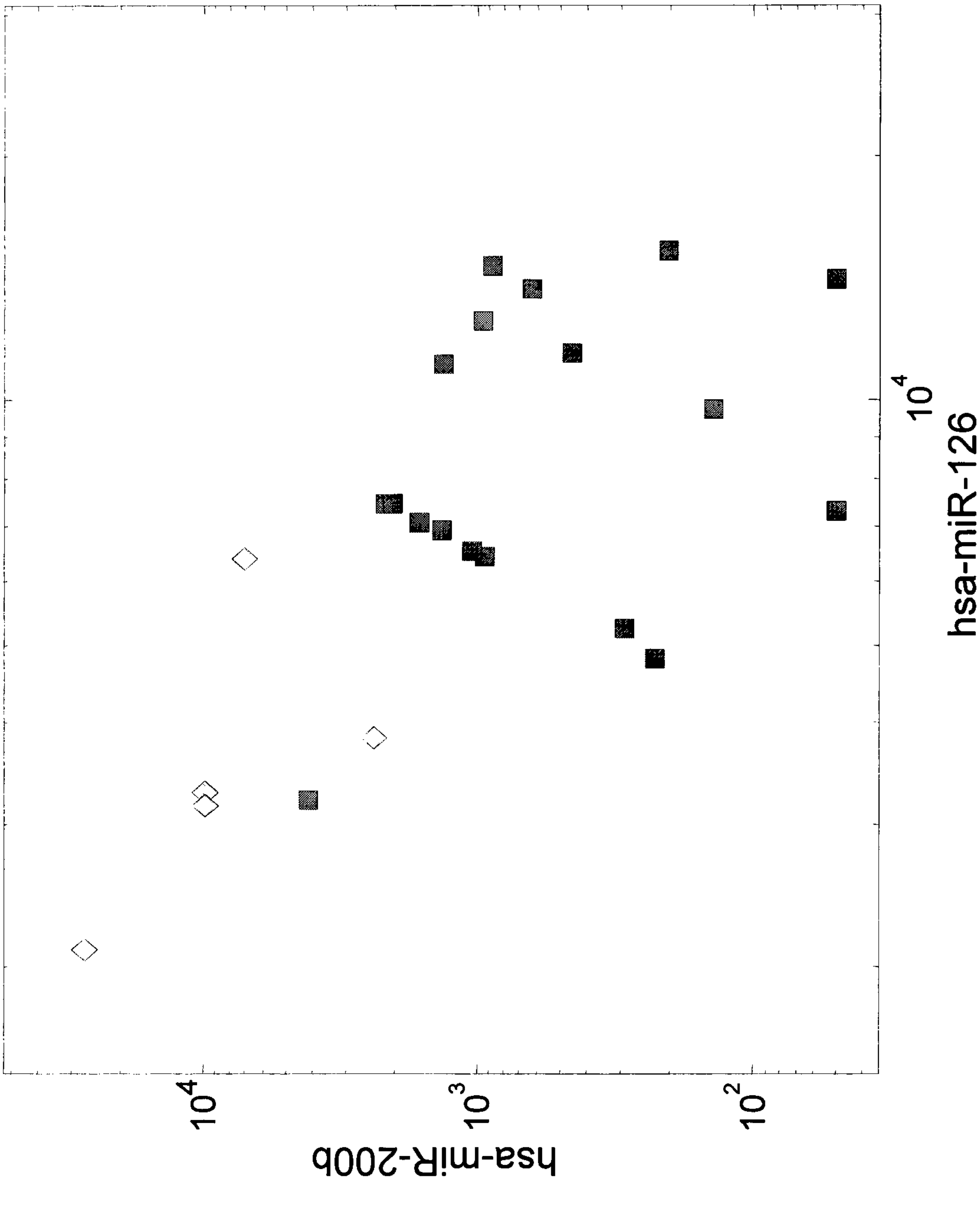

Figure 4

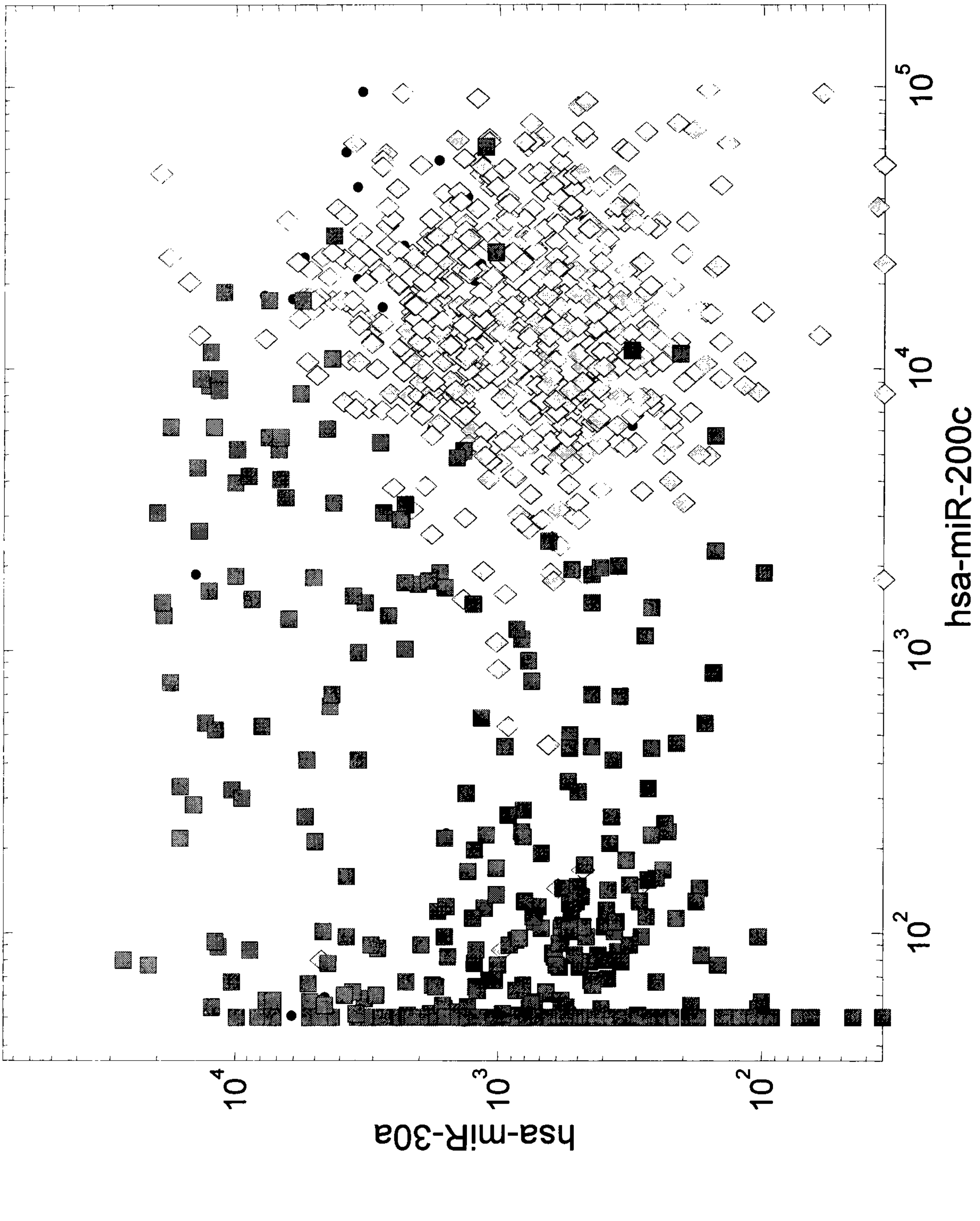

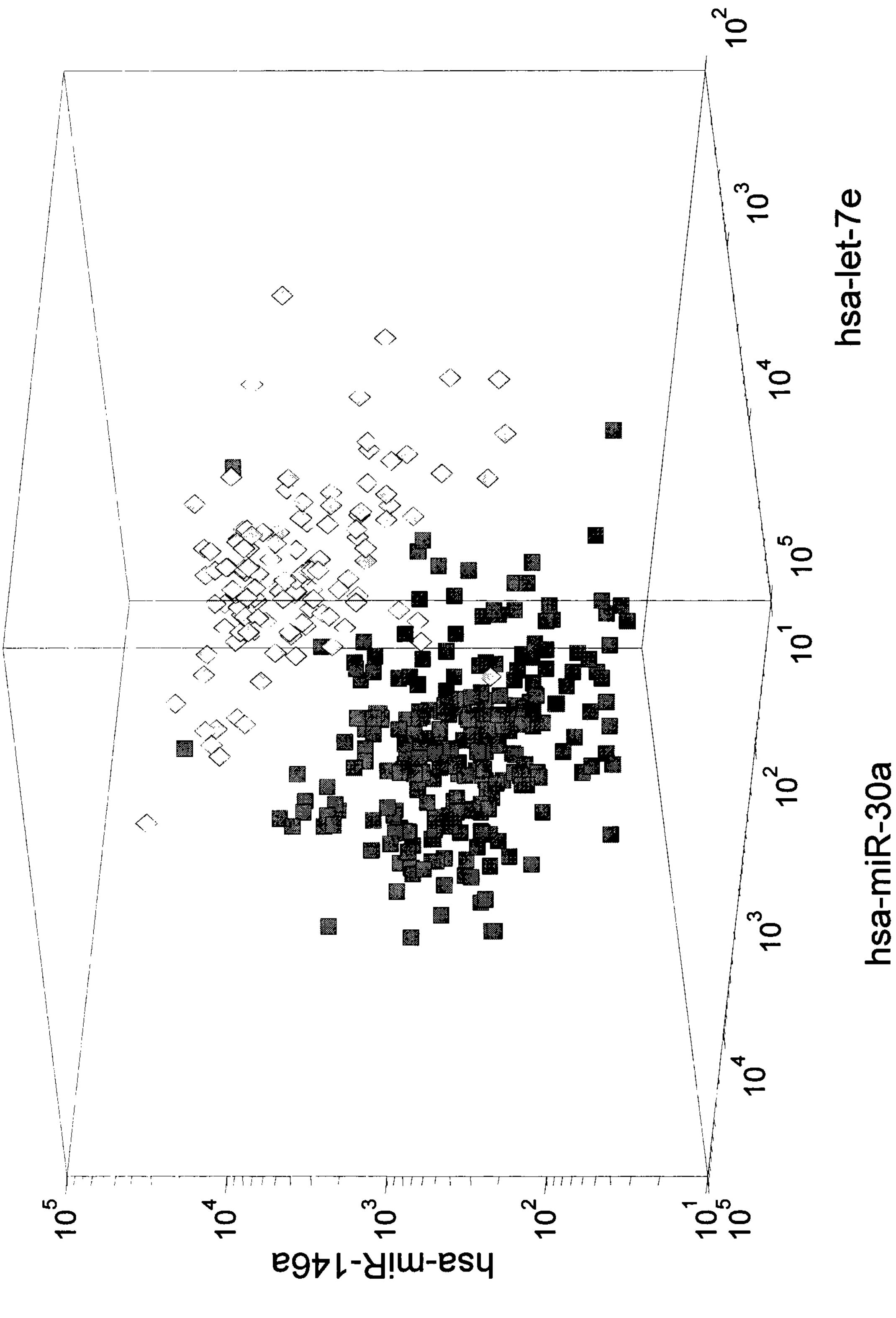
hsa-miR-146a
hsa-miR-30a
hsa-let-7e
$10^5$
$10^4$
$10^3$
$10^2$
$10^1$
$10^5$
$10^4$
$10^3$
$10^2$
$10^1$
$10^5$
$10^4$
$10^3$
$10^2$

Figure 5

Figure 6

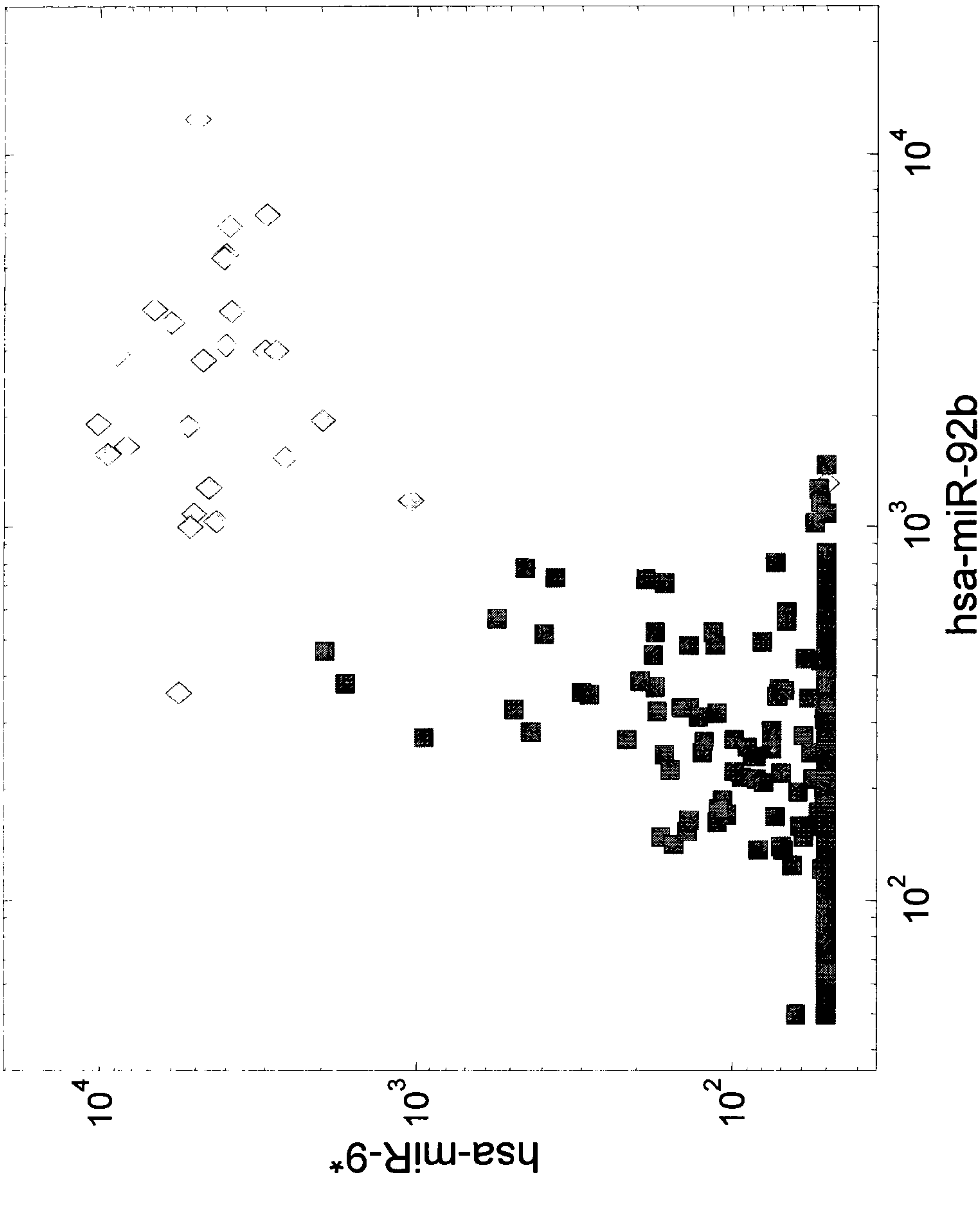

Figure 7

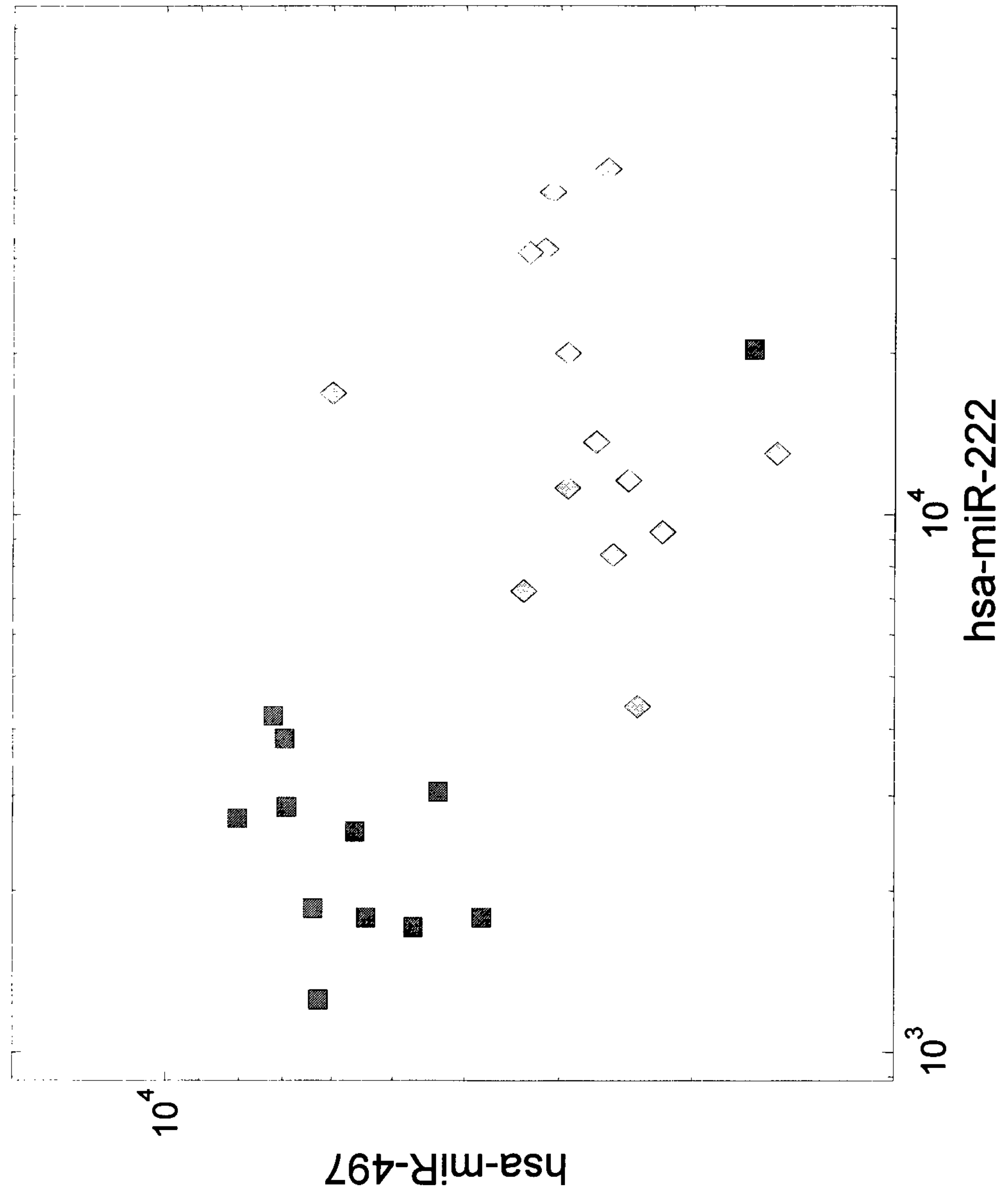

Figure 8

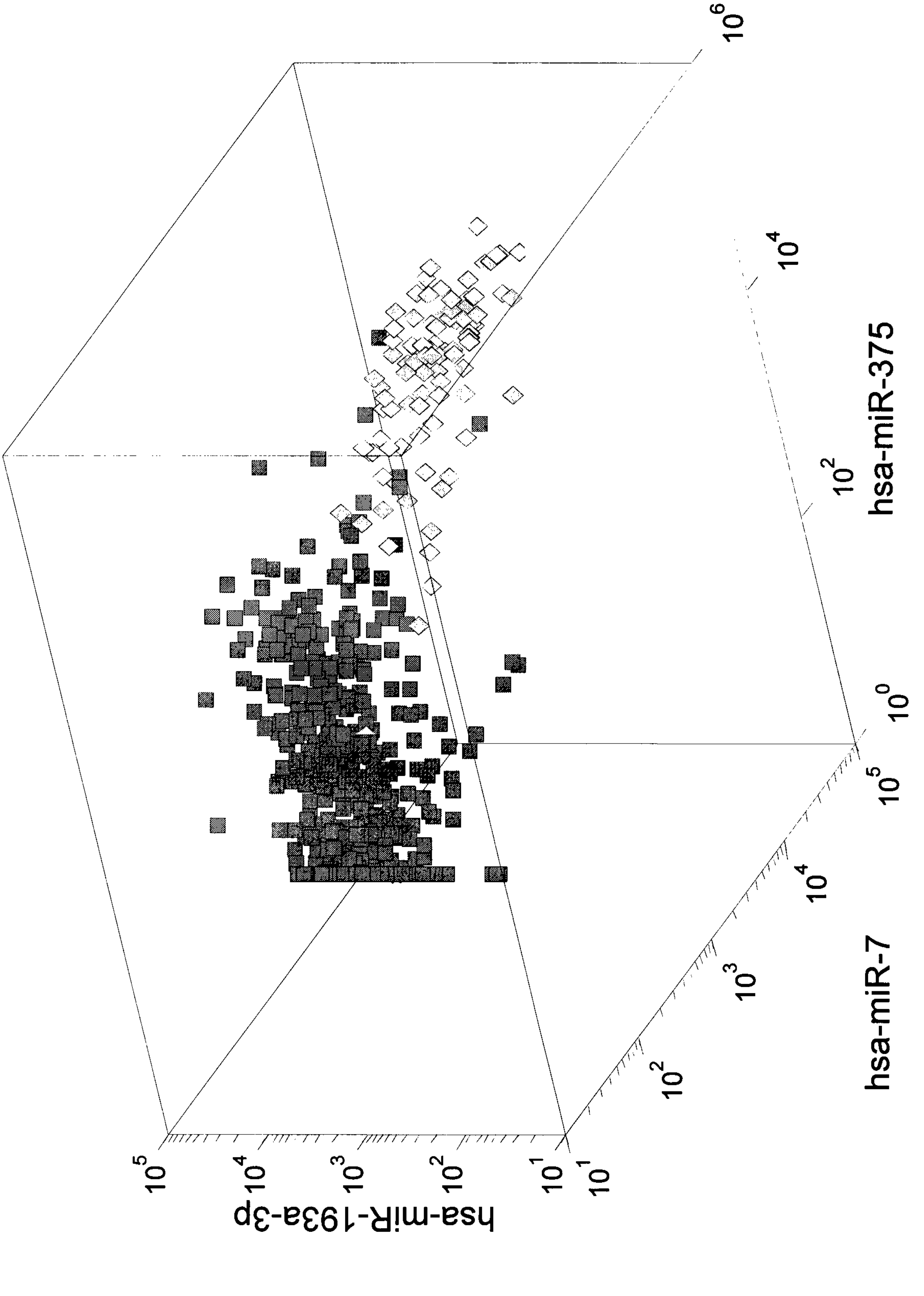

Figure 9

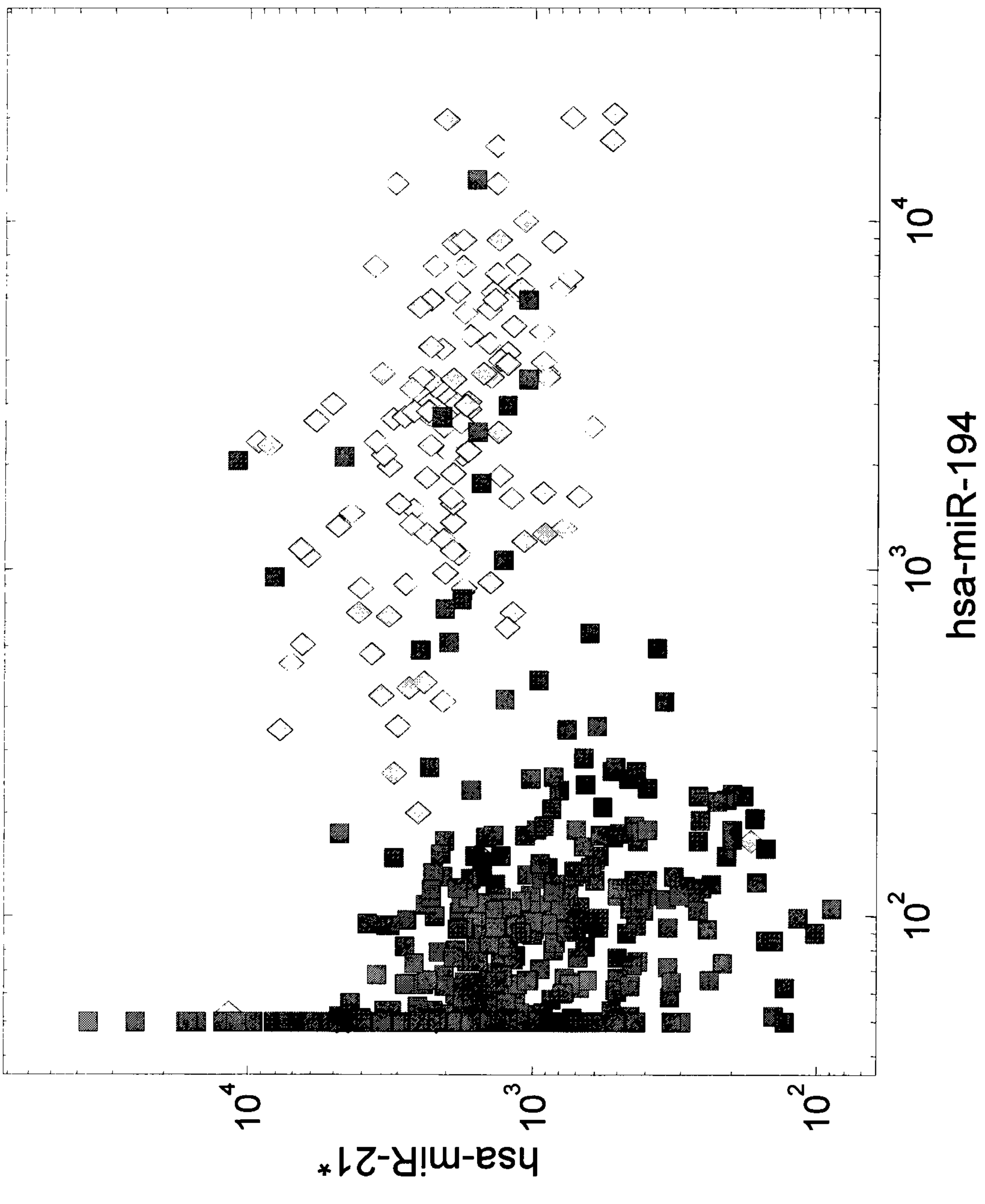

**Figure 10**

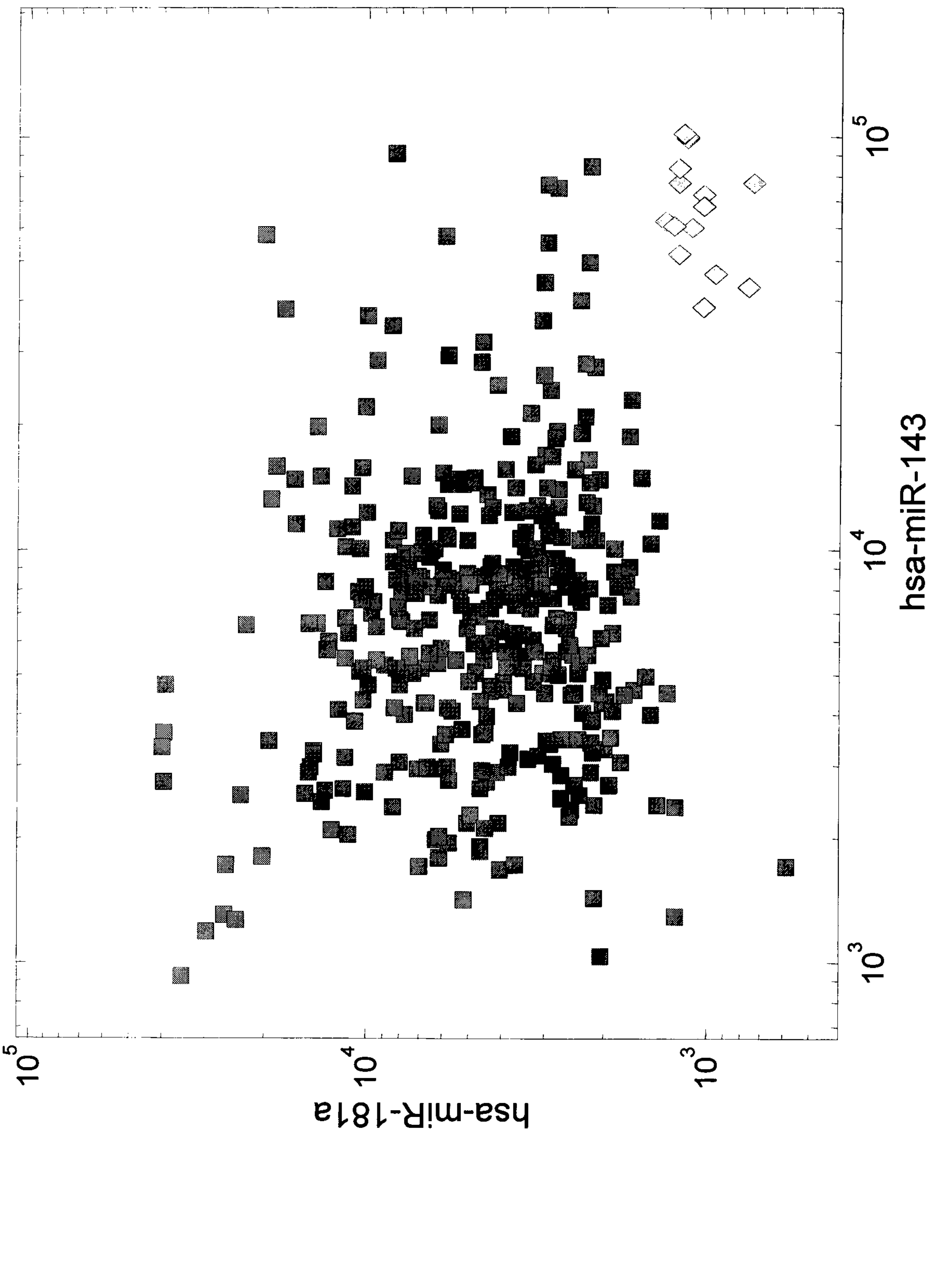

Figure 11

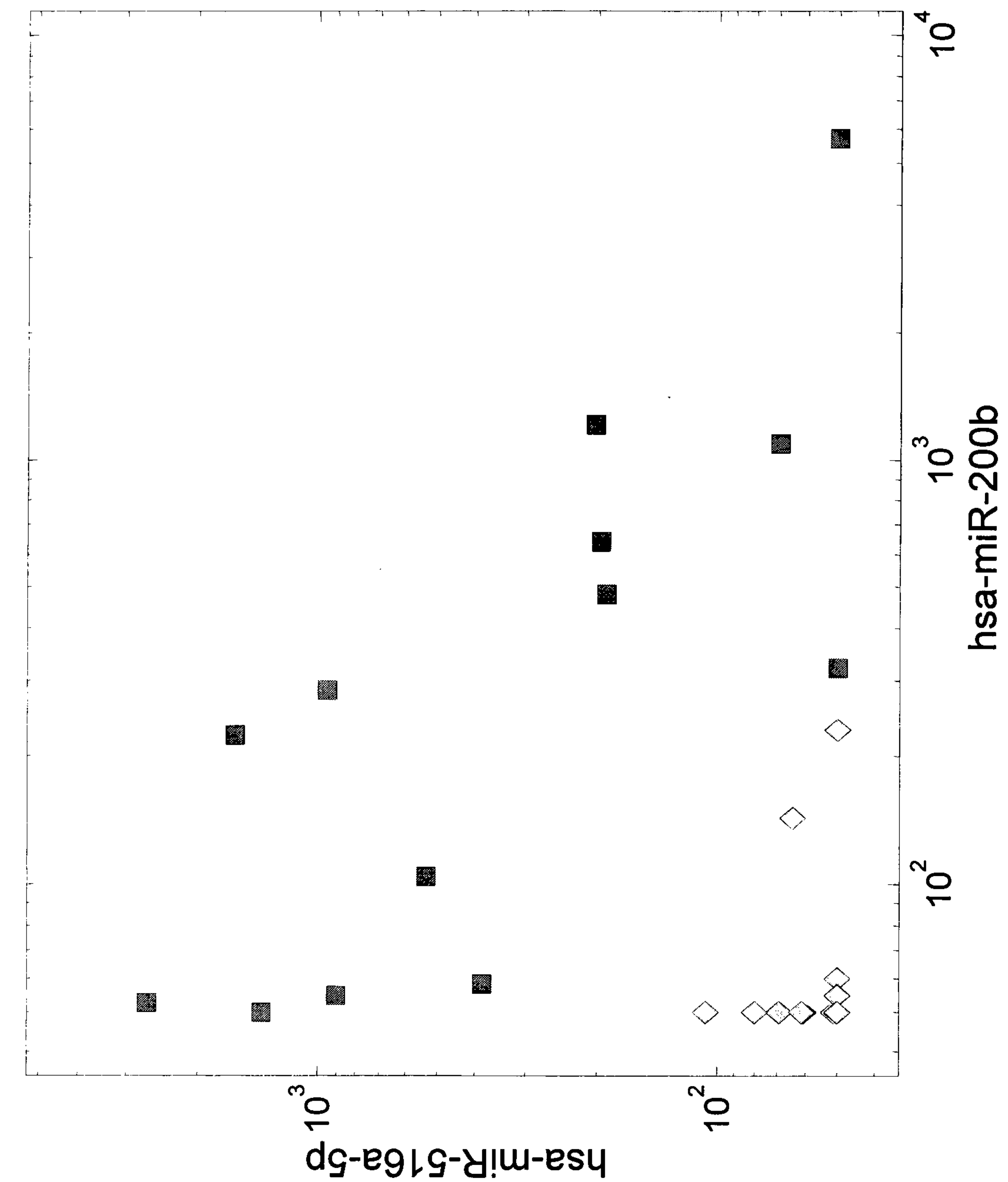

Figure 12

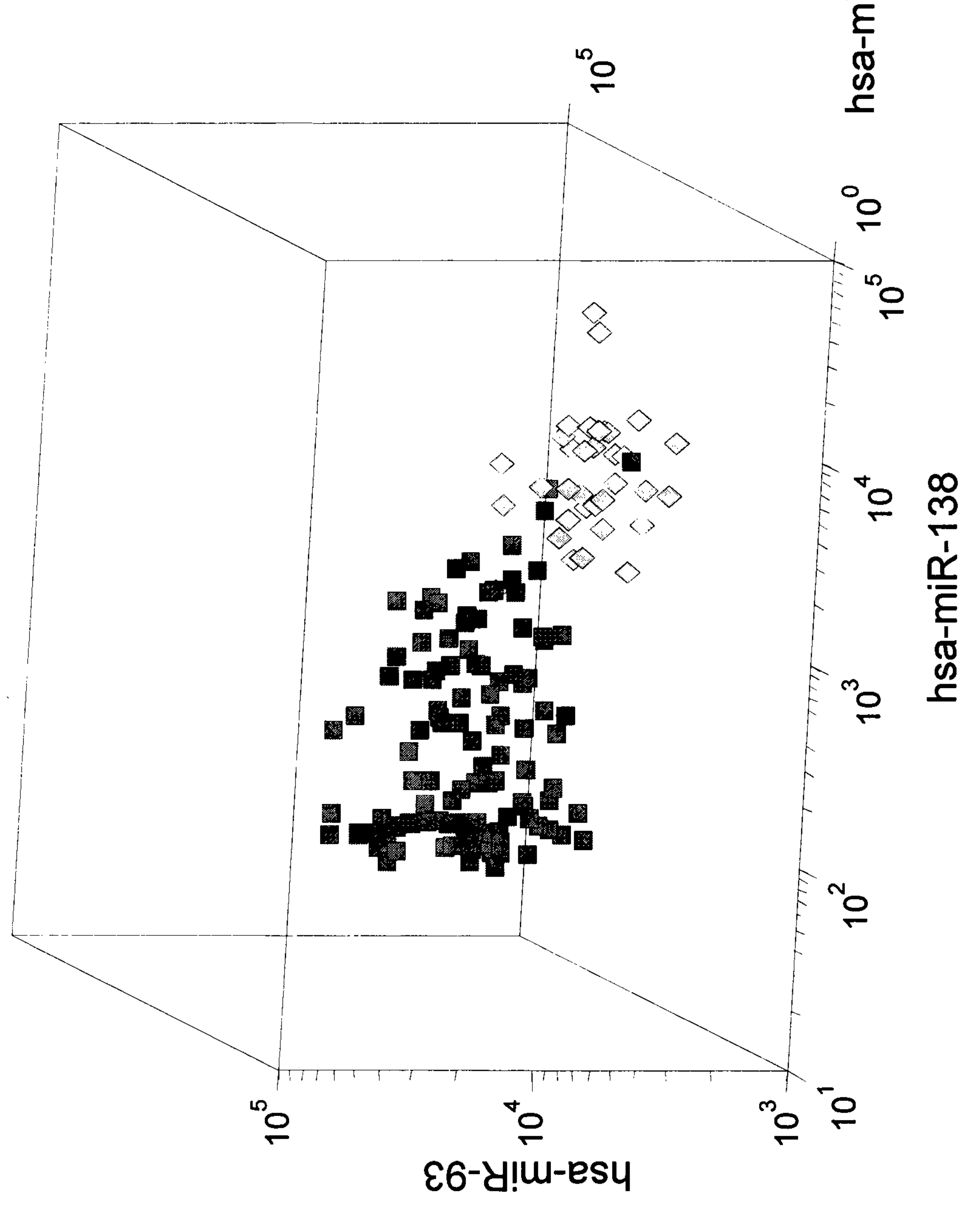
hsa-miR-93
hsa-miR-138
hsa-miR-10a
10^5
10^4
10^3
10^1
10^2
10^3
10^4
10^5
10^0
10^5

Figure 13

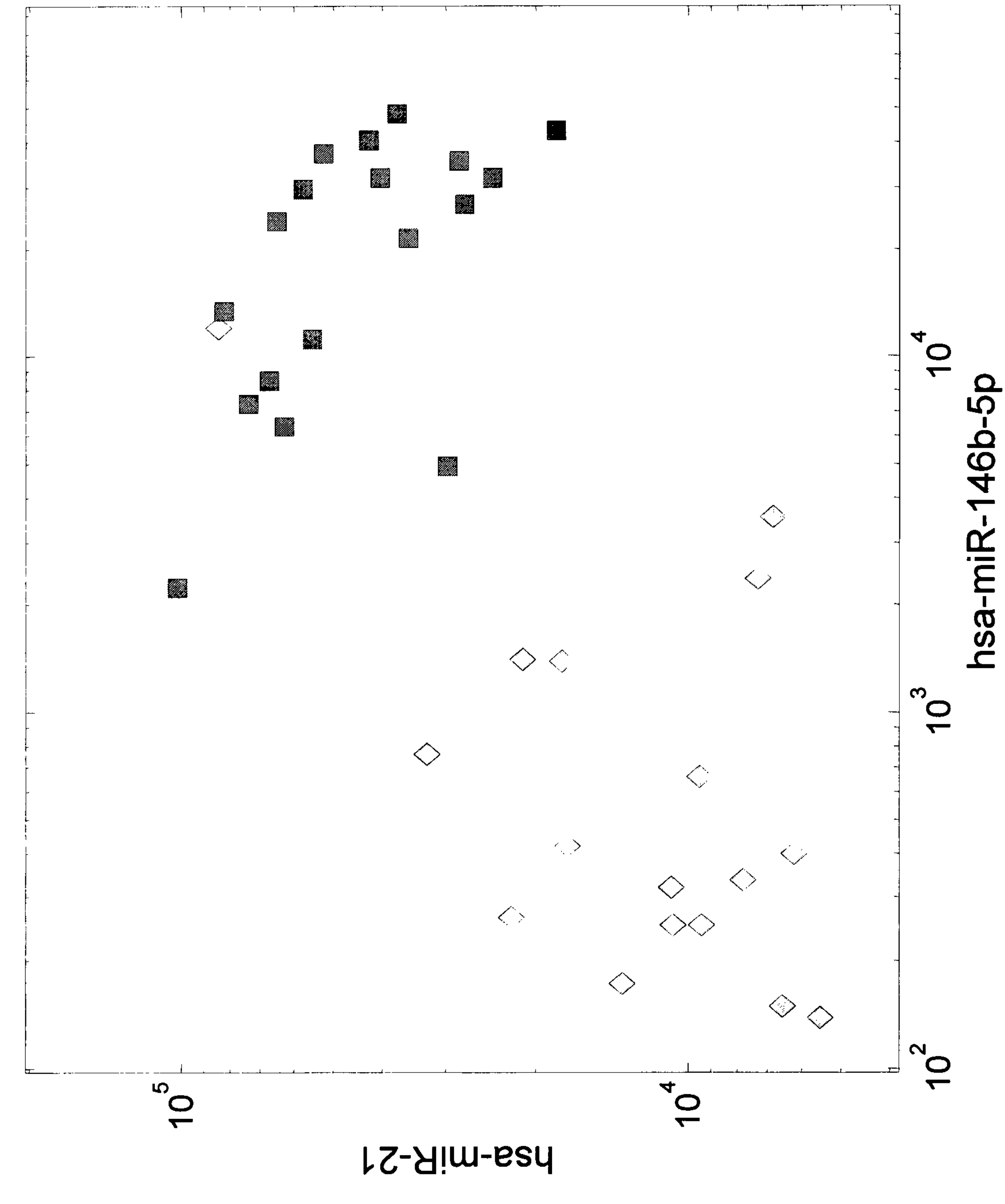

Figure 14

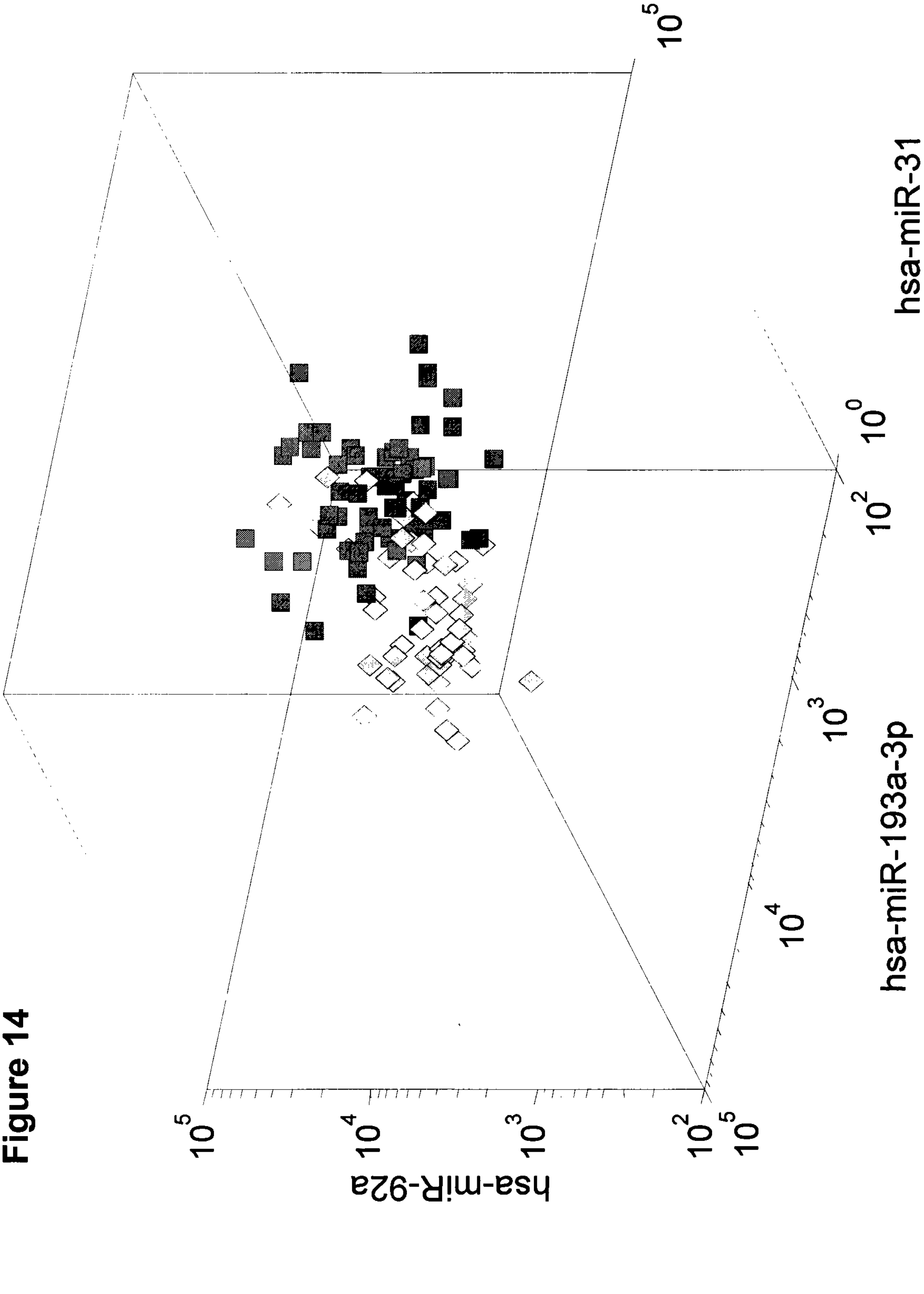
hsa-miR-92a
$10^5$
$10^4$
$10^3$
$10^2$
$10^5$
hsa-miR-193a-3p
$10^4$
$10^3$
$10^2$
$10^0$
hsa-miR-31
$10^5$

Figure 15

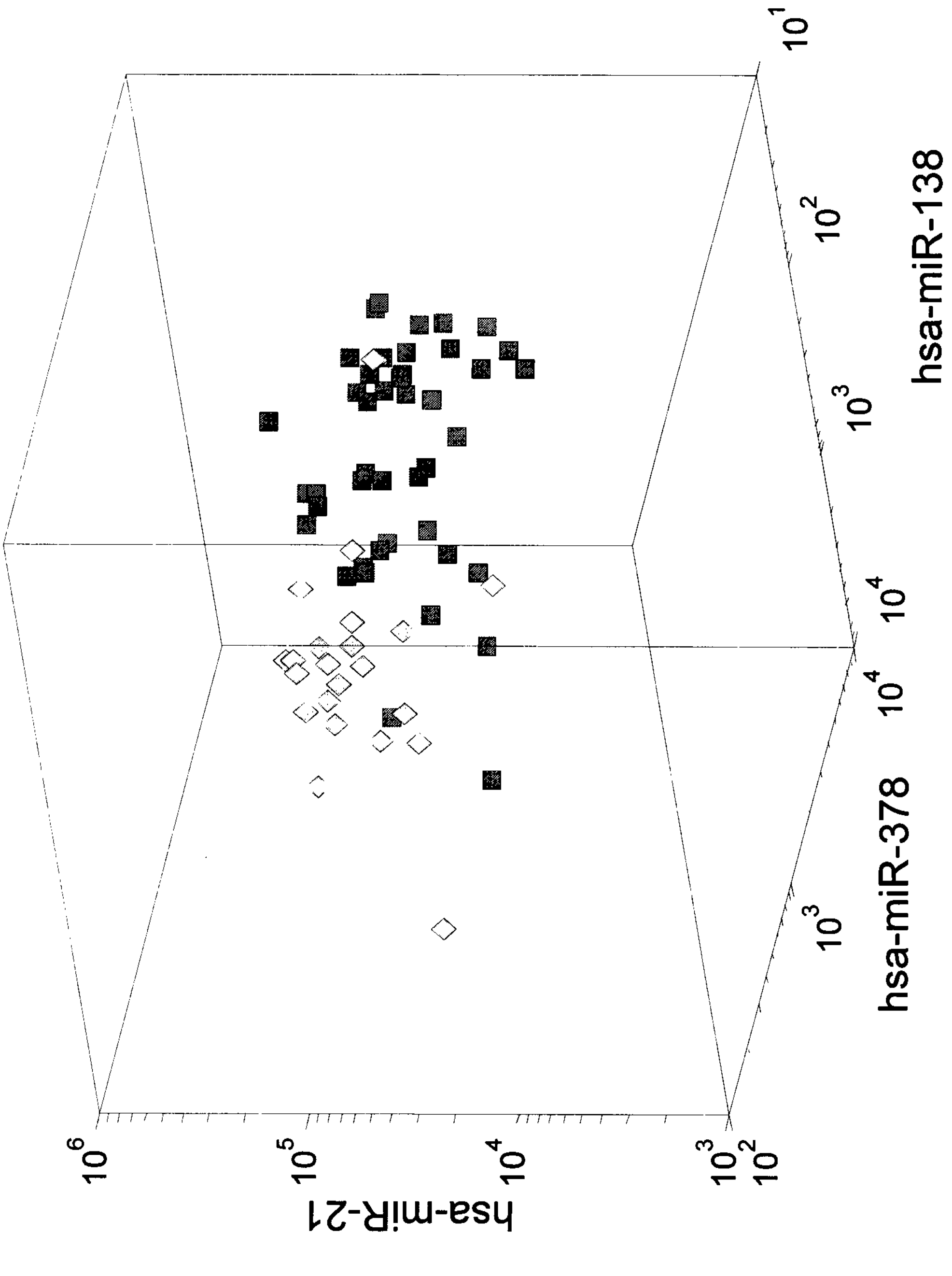
hsa-miR-21
hsa-miR-378
hsa-miR-138
$10^6$
$10^5$
$10^4$
$10^3$
$10^2$
$10^3$
$10^4$
$10^4$
$10^3$
$10^2$
$10^1$

Figure 16

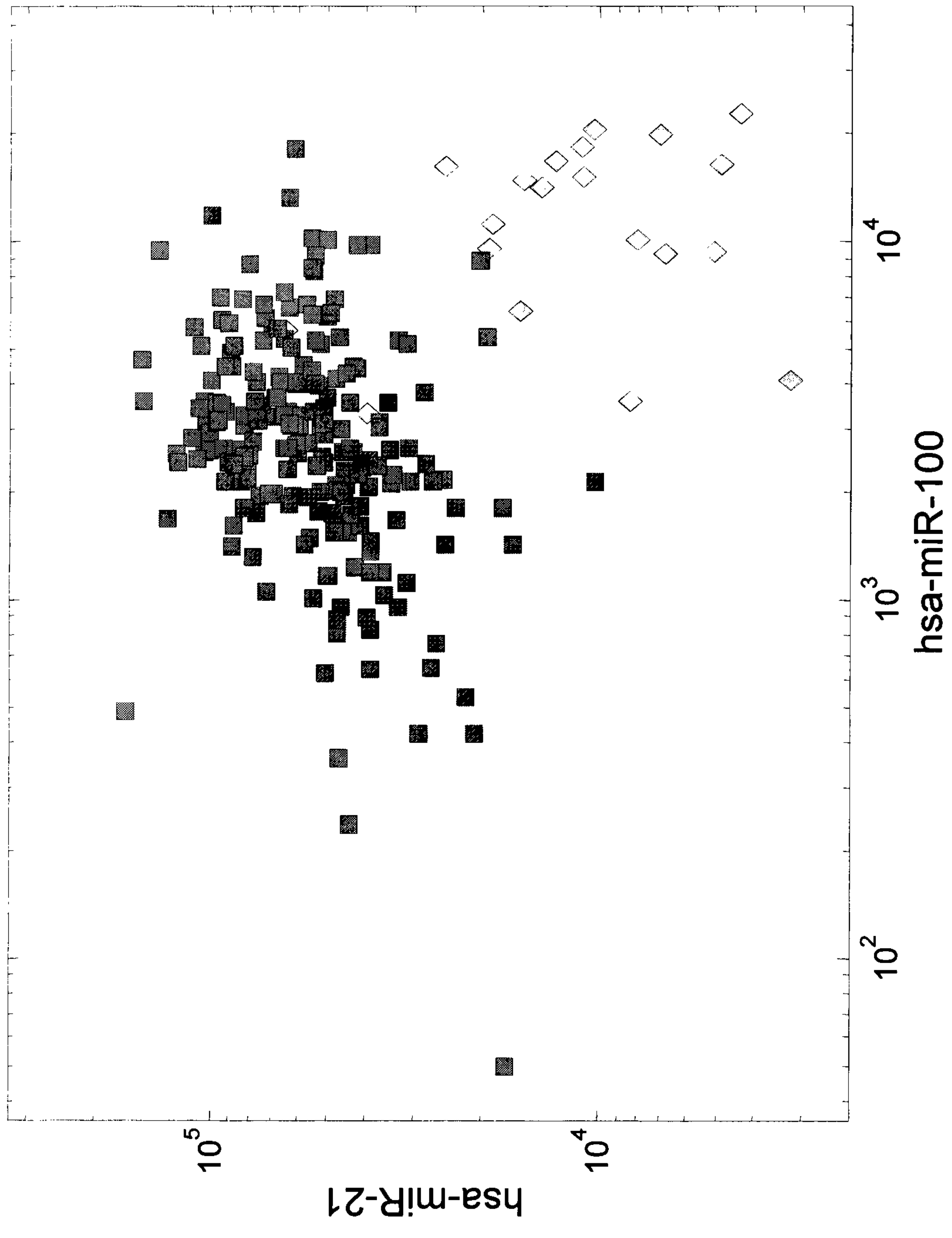

Figure 17

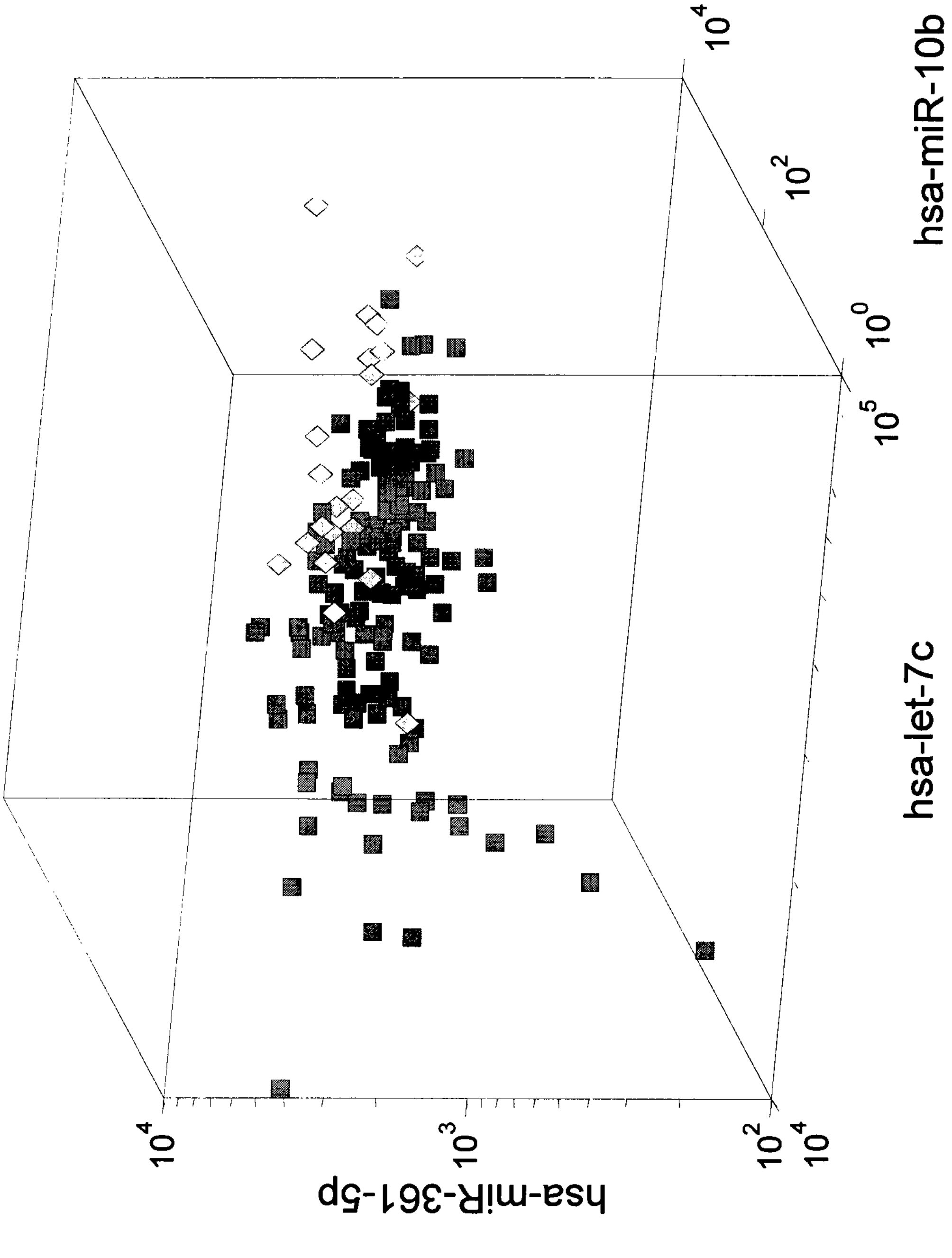
hsa-miR-361-5p
$10^4$
$10^3$
$10^2$
$10^4$
$10^5$
hsa-let-7c
$10^0$
$10^2$
$10^4$
hsa-miR-10b

Figure 18

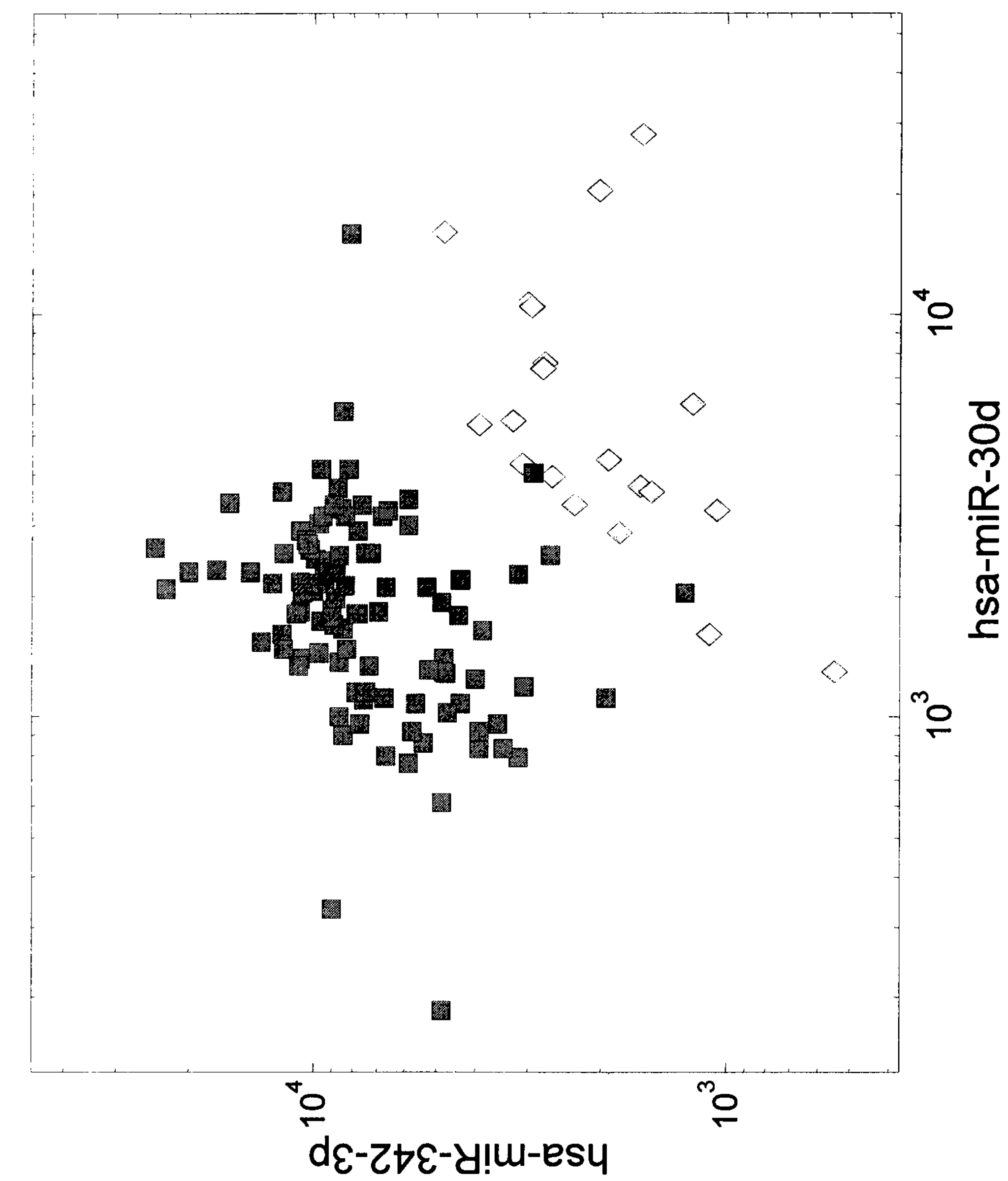

Figure 19

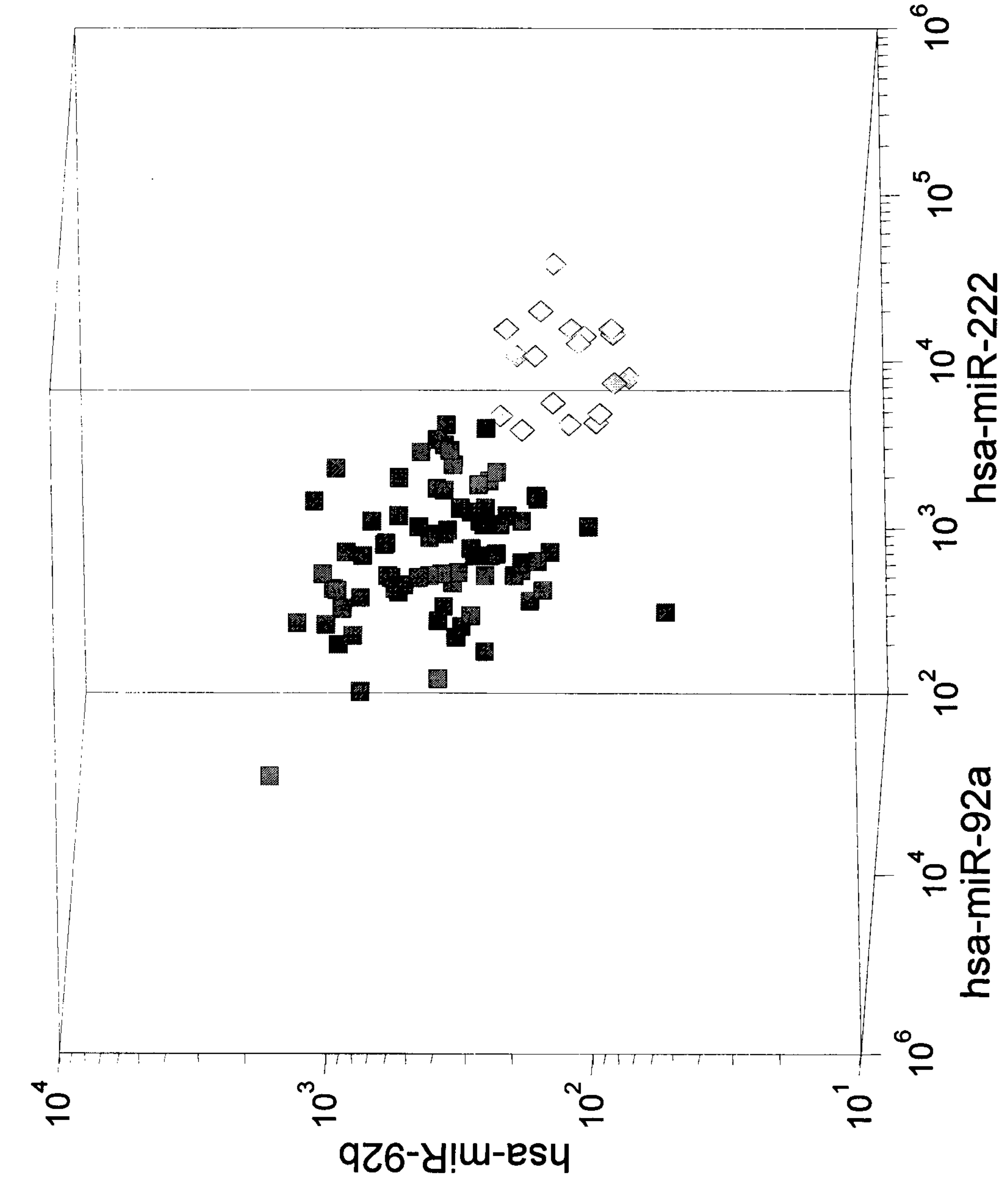
hsa-miR-92b
$10^4$
$10^3$
$10^2$
$10^1$
hsa-miR-92a
$10^6$
$10^4$
$10^2$
hsa-miR-222
$10^3$
$10^4$
$10^5$
$10^6$

**Figure 20**

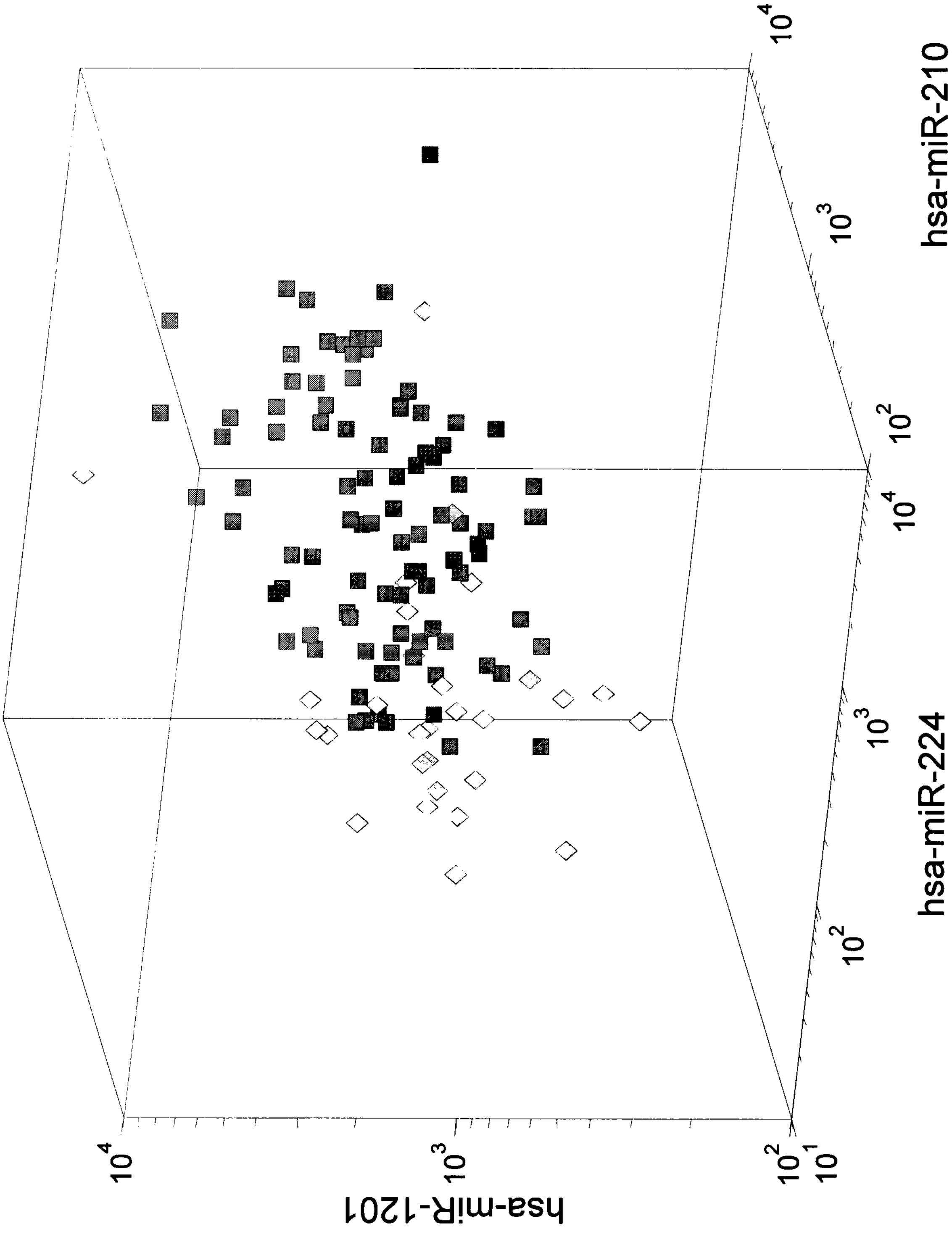
hsa-miR-1201
10^4
10^3
10^2
10^1
hsa-miR-224
10^2
10^3
10^4
hsa-miR-210
10^2
10^3
10^4

Figure 21

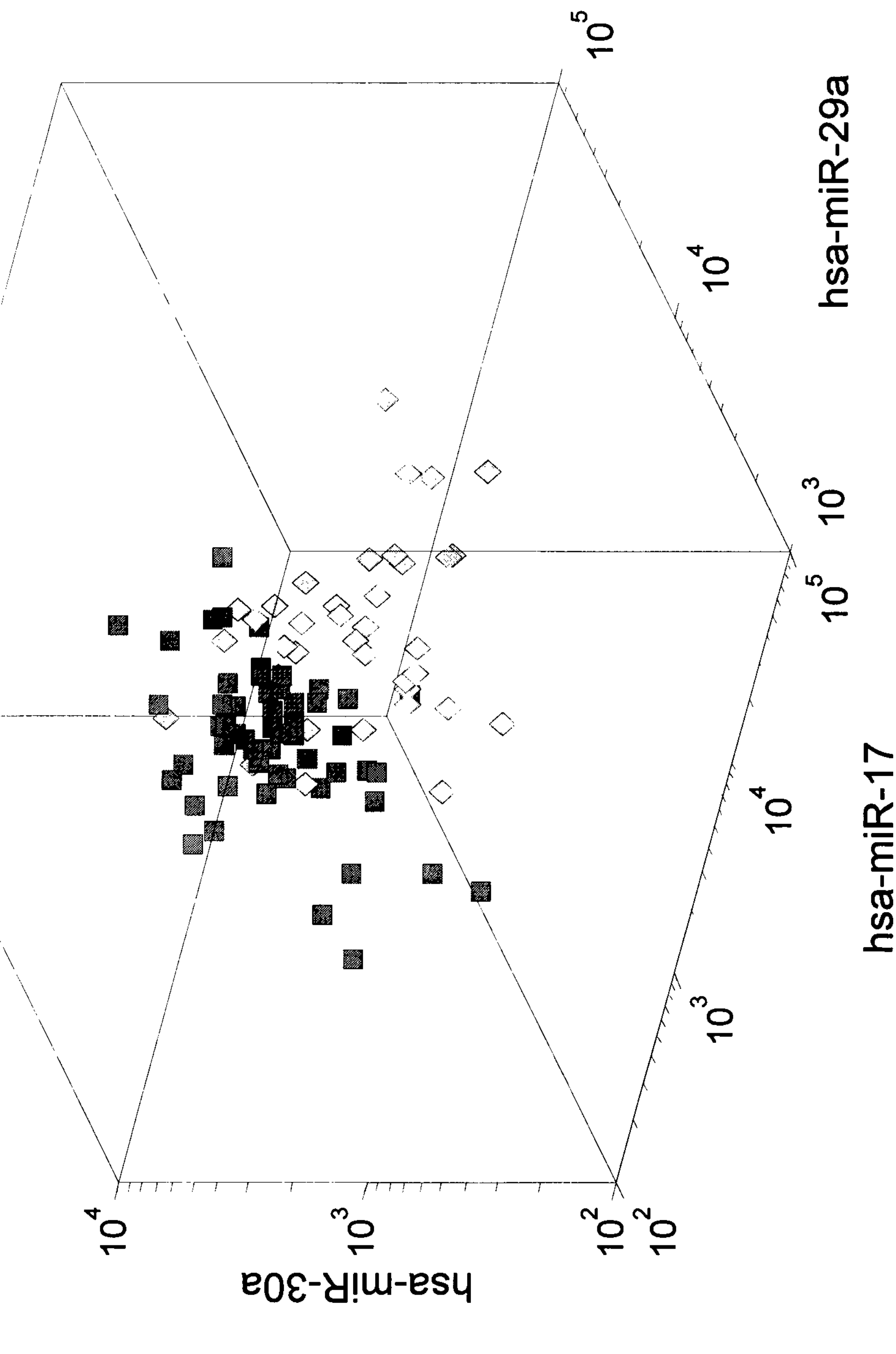
hsa-miR-30a
$10^4$
$10^3$
$10^2$
$10^2$
hsa-miR-17
$10^3$
$10^4$
$10^5$
hsa-miR-29a
$10^3$
$10^4$
$10^5$

Figure 22

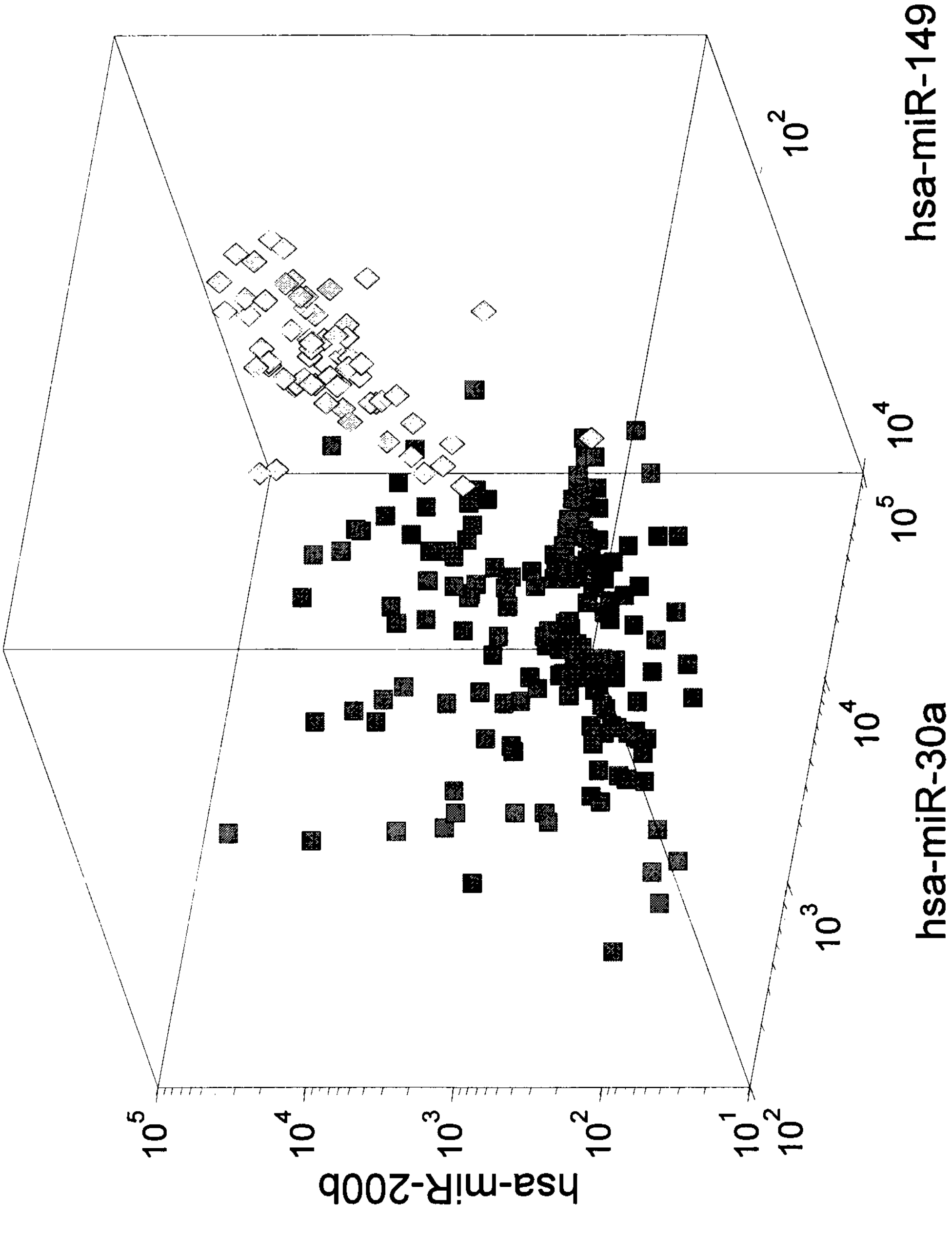
hsa-miR-200b
$10^5$
$10^4$
$10^3$
$10^2$
$10^1$
$10^2$
$10^3$
hsa-miR-30a
$10^4$
$10^5$
$10^4$
$10^2$
hsa-miR-149

Figure 23

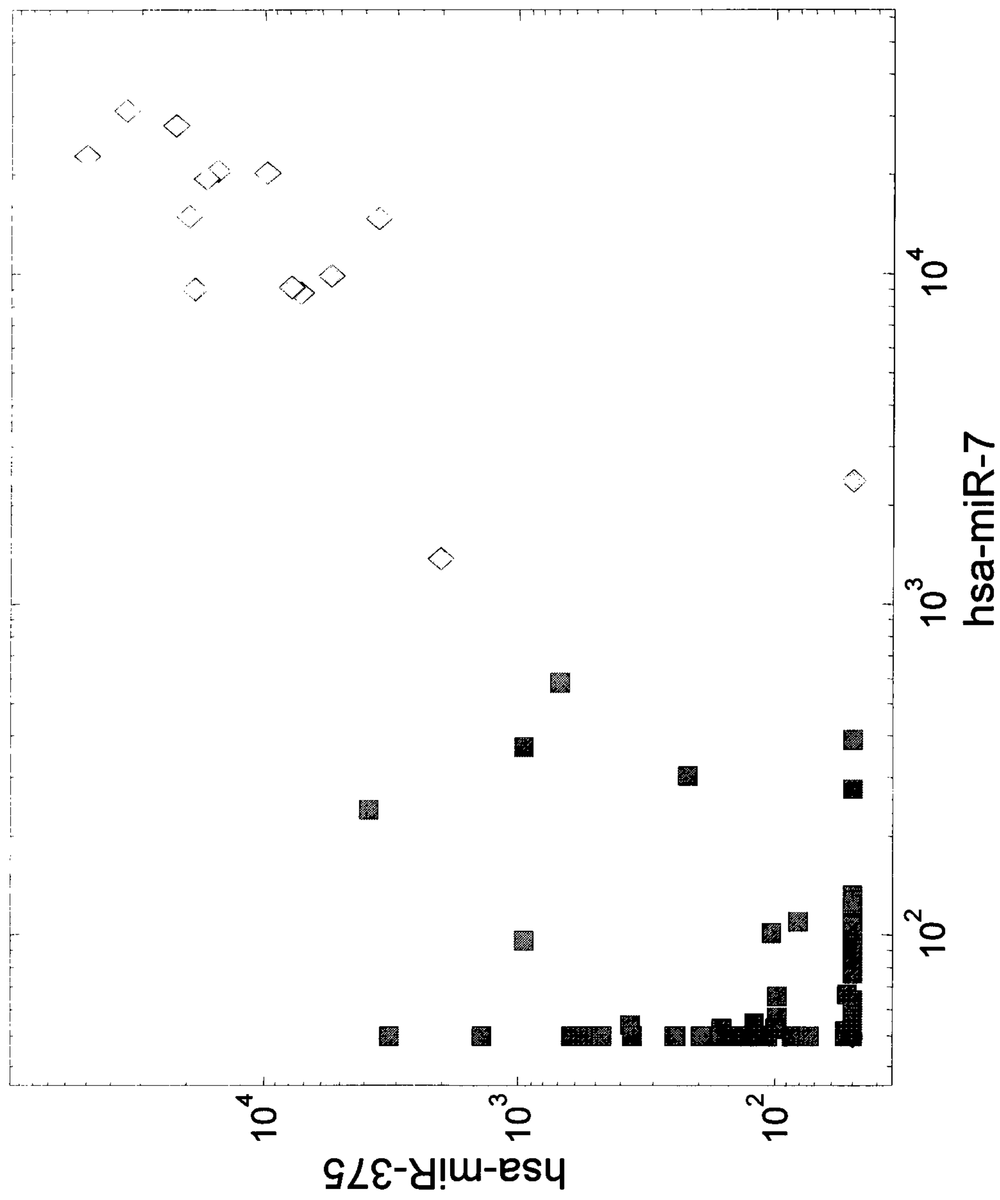

Figure 24

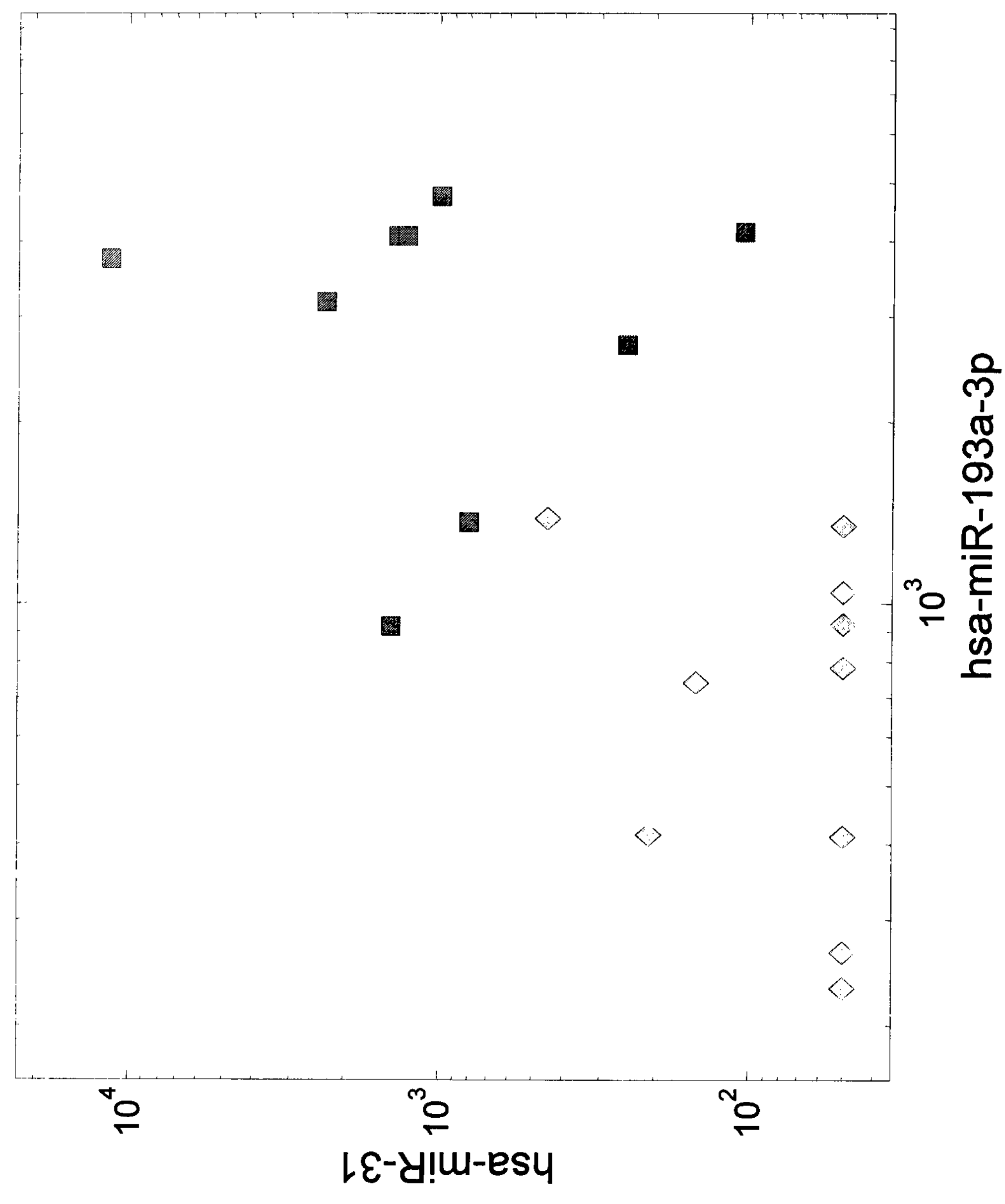

Figure 25

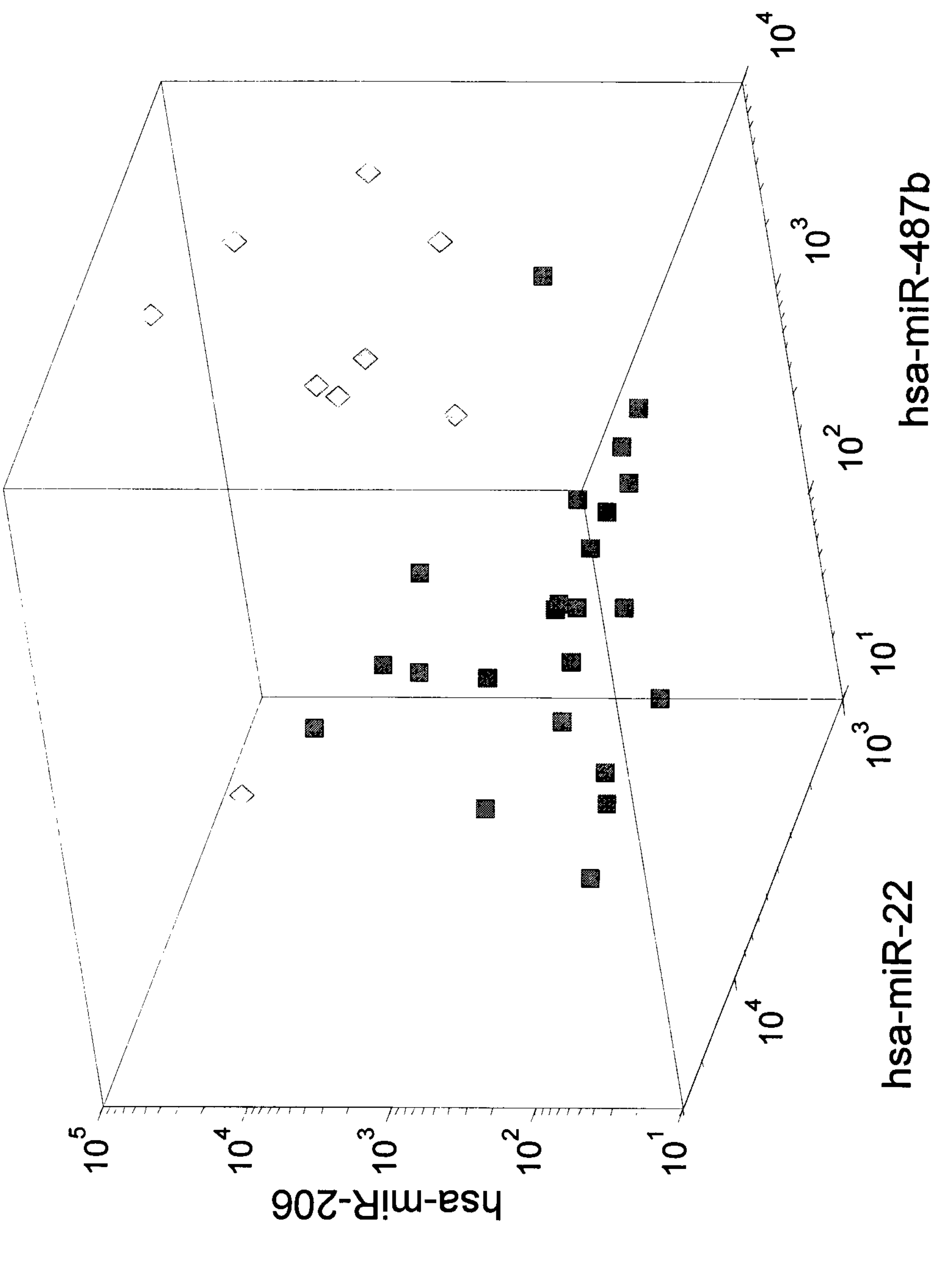

hsa-miR-206
$10^5$
$10^4$
$10^3$
$10^2$
$10^1$
hsa-miR-22
$10^4$
$10^3$
hsa-miR-487b
$10^1$
$10^2$
$10^3$
$10^4$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010073248 A
- WO 03078450 A
- US 5235033 A
- US 5034506 A
- US 20050107325 A
- US 20050182005 A
- US 20080182237 A
- US 20070259352 A
- US 6506559 B


### Non-patent literature cited in the description


- **LU et al.** *Nature,* 2005, vol. 435 (7043), 834-838
- Nucleic Acids in Chemistry and Biology. Oxford University Press, 1996
- **KRUTZFELDT et al.** *Nature,* 2005, vol. 438, 685-689
- **SOUTSCHEK et al.** *Nature,* 2004, vol. 432, 173-178
- **YEKTA et al.** *Science,* 2004, vol. 304, 594-596
- **DOENCH ; SHARP.** *GenesDev,* 2004, vol. 18, 504-511
- **BRENNECKE et al.** *PloS Biol,* 2005, vol. 3, e85
- **LEWIS et al.** *Cell,* 2005, vol. 120, 15-20
- **KREK et al.** *Nat Genet,* 2005, vol. 37, 495-500
- **HOFACKER et al.** *Monatshefte f. Chemie,* 1994, vol. 125, 167-188
- **THOMSON et al.** *Nature Methods,* 2004, vol. 1, 47-53
- **MA et al.** *Arch Pathol Lab Med,* 2006, vol. 130, 465-73
- **BENTWICH, I. et al.** Identification of hundreds of conserved and nonconserved human microRNAs. *Nat Genet,* 2005
- **FARH, K.K. et al.** The Widespread Impact of Mammalian MicroRNAs on mRNA Repression and Evolution. *Science,* 2005
- **GRIFFITHS-JONES, S. ; GROCOCK, R.J. ; VAN DONGEN, S. ; BATEMAN, A. ; ENRIGHT, A.J.** miRBase: microRNA sequences, targets and gene nomenclature. *Nucleic Acids Res,* 2006, vol. 34, D140-4
- **HE, L. et al.** A microRNA polycistron as a potential human oncogene. *Nature,* 2005, vol. 435, 828-33
- **BASKERVILLE, S. ; BARTEL, D.P.** Microarray profiling of microRNAs reveals frequent coexpression with neighboring miRNAs and host genes. *Rna,* 2005, vol. 11, 241-7
- **LANDGRAF, P. et al.** A Mammalian microRNA Expression Atlas Based on Small RNA Library Sequencing. *Cell,* 2007, vol. 129, 1401-14
- **VOLINIA, S. et al.** A microRNA expression signature of human solid tumors defines cancer gene targets. *Proc Natl Acad Sci USA,* 2006
- **LU, J. et al.** MicroRNA expression profiles classify human cancers. *Nature,* 2005, vol. 435, 834-8
- **VARADHACHARY, G.R. ; ABBRUZZESE, J.L. ; LENZI, R.** Diagnostic strategies for unknown primary cancer. *Cancer,* 2004, vol. 100, 1776-85
- **PIMIENTO, J.M. ; TESO, D. ; MALKAN, A. ; DUDRICK, S.J. ; PALESTY, J.A.** Cancer of unknown primary origin: a decade of experience in a community-based hospital. *Am J Surg,* 2007, vol. 194, 833-7
- **SHAW, P.H. ; ADAMS, R. ; JORDAN, C. ; CROSBY, T.D.** A clinical review of the investigation and management of carcinoma of unknown primary in a single cancer network. *Clin Oncol (R Coll Radiol),* 2007, vol. 19, 87-95
- **HAINSWORTH, J.D. ; GRECO, F.A.** Treatment of patients with cancer of an unknown primary site. *N Engl JMed,* 1993, vol. 329, 257-63
- **BLASZYK, H. ; HARTMANN, A. ; BJORNSSON, J.** Cancer of unknown primary: clinicopathologic correlations. *Apmis,* 2003, vol. 111, 1089-94
- **BLOOM, G. et al.** Multi-platform, multi-site, microarray-based human tumor classification. *Am J Pathol,* 2004, vol. 164, 9-16
- **MA, X.J. et al.** Molecular classification of human cancers using a 92-gene real-time quantitative polymerase chain reaction assay. *Arch Pathol Lab Med,* 2006, vol. 130, 465-73
- **TALANTOV, D. et al.** A quantitative reverse transcriptase-polymerase chain reaction assay to identify metastatic carcinoma tissue of origin. *J Mol Diagn,* 2006, vol. 8, 320-9

- **TOTHILL, R.W. et al.** An expression-based site of origin diagnostic method designed for clinical application to cancer of unknown origin. *Cancer Res,* 2005, vol. 65, 4031-40
- **SHEDDEN, K.A. et al.** Accurate molecular classification of human cancers based on gene expression using a simple classifier with a pathological tree-based framework. *Am J Pathol,* 2003, vol. 163, 1985-95
- **RAVER-SHAPIRA, N. et al.** Transcriptional Activation of miR-34a Contributes to p53-Mediated Apoptosis. *Mol Cell,* 2007
- **XIAO, C. et al.** MiR-150 Controls B Cell Differentiation by Targeting the Transcription Factor c-Myb. *Cell,* 2007, vol. 131, 146-59